# EUROPEAN PATENT SPECIFICATION

(11) **EP 3 155 008 B1**
(45) Date of publication and mention of the grant of the patent: **26.08.2020**
(21) Application number: 15805829.7
(22) Date of filing: 15.06.2015
(51) Int. Cl.: A61K 38/17, A61K 31/704, A61K 9/00, A61K 47/26, A61P 35/00, A61P 21/00

(54) **FORMULATED RECEPTOR POLYPEPTIDES AND RELATED METHODS**
FORMULIERTE REZEPTORPOLYPEPTIDE UND ZUGEHÖRIGES VERFAHREN
POLYPEPTIDES D'UN RÉCEPTEUR FORMULÉS DANS UNE COMPOSITION ET PROCÉDÉS ASSOCIÉS

(30) Priority: 13.06.2014 US 201462012104 P; 09.09.2014 US 201462047995 P; 02.10.2014 US 201462058789 P; 03.04.2015 US 201562142812 P
(43) Date of publication of application: 19.04.2017
(73) Proprietor: Atara Biotherapeutics, Inc., South San Francisco, CA 94080 (US); Amgen Inc., Thousand Oaks, California 91320-1799 (US)
(72) Inventor: HAQQ, Christopher, Michael, South San Francisco, CA 94080 (US); LATYPOV, Ramil, Wellesley, MA 02481 (US)
(74) Representative: J A Kemp LLP
(86) International application number: PCT/US2015/035818
(87) International publication number: WO 2015/192127

(56) References cited:
- WO-A1-2016/205370
- WO-A2-2008/109167
- US-A1- 2009 258 017
- US-A1- 2013 225 484
- BANKS DOUGLAS D ET AL: "Native-state solubility and transfer free energy as predictive tools for selecting excipients to include in protein formulation development studies", JOURNAL OF PHARMACEUTICAL SCIENCES, vol. 101, no. 8, August 2012 (2012-08), pages 2720-2732, XP009502028, ISSN: 0022-3549
- Anonymous: "- Atara Biotherapeutics", , 1 October 2013 (2013-10-01), XP055430566, Retrieved from the Internet: URL:http://www.atarabio.com/2014/01/atara- biotherapeutics-announces-second-closing-o f-52-million-series-b-financing/ [retrieved on 2017-11-30]

### SEQUENCE LISTING

<110> AMGEN INC. SANTA MARIA BIOTHERAPEUTICS, INC.
<120> FORMULATED RECEPTOR POLYPEPTIDES AND RELATED METHODS
<130> 29957-29502 PCT
<140> PCT/US2015/035818
   <141> 2015-06-15
<150> 62/142,812
   <151> 2015-04-03
<150> 62/058,789
   <151> 2014-10-02
<150> 62/047,995
   <151> 2014-09-09
<150> 61/012,104
   <151> 2014-06-13
<160> 53
<170> PatentIn version 3.5
<210> 1
   <211> 402
   <212> DNA
   <213> Homo sapiens
<220>
   <221> CDS
   <222> (1)..(402)
<400> 1
   atg acg gcg ccc tgg gtg gcc ctc gcc ctc ctc tgg gga tcg ctg tgc      48
   Met Thr Ala Pro Trp Val Ala Leu Ala Leu Leu Trp Gly Ser Leu Cys
   1               5                   10                  15
   gcc ggc tct ggg cgt ggg gag gct gag aca cgg gag tgc atc tac tac      96
   Ala Gly Ser Gly Arg Gly Glu Ala Glu Thr Arg Glu Cys Ile Tyr Tyr
               20                  25                  30
   aac gcc aac tgg gag ctg gag cgc acc aac cag agc ggc ctg gag cgc     144
   Asn Ala Asn Trp Glu Leu Glu Arg Thr Asn Gln Ser Gly Leu Glu Arg
           35                  40                  45
   tgc gaa ggc gag cag gac aag cgg ctg cac tgc tac gcc tcc tgg cgc     192
   Cys Glu Gly Glu Gln Asp Lys Arg Leu His Cys Tyr Ala Ser Trp Arg
       50                  55                  60
   aac agc tct ggc acc atc gag ctc gtg aag aag ggc tgc tgg cta gat     240
   Asn Ser Ser Gly Thr Ile Glu Leu Val Lys Lys Gly Cys Trp Leu Asp
   65                  70                  75                  80
   gac ttc aac tgc tac gat agg cag gag tgt gtg gcc act gag gag aac     288
   Asp Phe Asn Cys Tyr Asp Arg Gln Glu Cys Val Ala Thr Glu Glu Asn
                   85                  90                  95
   ccc cag gtg tac ttc tgc tgc tgt gaa ggc aac ttc tgc aac gag cgc        336
   Pro Gln Val Tyr Phe Cys Cys Cys Glu Gly Asn Phe Cys Asn Glu Arg
               100                 105                 110
   ttc act cat ttg cca gag gct ggg ggc ccg gaa gtc acg tac gag cca        384
   Phe Thr His Leu Pro Glu Ala Gly Gly Pro Glu Val Thr Tyr Glu Pro
           115                 120                 125
   ccc ccg aca gcc ccc acc                                                402
   Pro Pro Thr Ala Pro Thr
       130
<210> 2
   <211> 134
   <212> PRT
   <213> Homo sapiens
<400> 2
   Met Thr Ala Pro Trp Val Ala Leu Ala Leu Leu Trp Gly Ser Leu Cys
   1               5                   10                  15
   Ala Gly Ser Gly Arg Gly Glu Ala Glu Thr Arg Glu Cys Ile Tyr Tyr
               20                  25                  30
   Asn Ala Asn Trp Glu Leu Glu Arg Thr Asn Gln Ser Gly Leu Glu Arg
           35                  40                  45
   Cys Glu Gly Glu Gln Asp Lys Arg Leu His Cys Tyr Ala Ser Trp Arg
       50                  55                  60
   Asn Ser Ser Gly Thr Ile Glu Leu Val Lys Lys Gly Cys Trp Leu Asp
   65                  70                  75                  80
   Asp Phe Asn Cys Tyr Asp Arg Gln Glu Cys Val Ala Thr Glu Glu Asn
                   85                  90                  95
   Pro Gln Val Tyr Phe Cys Cys Cys Glu Gly Asn Phe Cys Asn Glu Arg
               100                 105                 110
   Phe Thr His Leu Pro Glu Ala Gly Gly Pro Glu Val Thr Tyr Glu Pro
           115                 120                 125
   Pro Pro Thr Ala Pro Thr
       130
<210> 3
   <211> 387
   <212> DNA
   <213> Homo sapiens
<220>
   <221> CDS
   <222> (1)..(387)
<400> 3
   atg gag ttt ggg ctg agc tgg gtt ttc ctc gtt gct ctt tta aga ggt       48
   Met Glu Phe Gly Leu Ser Trp Val Phe Leu Val Ala Leu Leu Arg Gly
   1               5                   10                  15
   gtc cag tgt gag aca cgg tgg tgc atc tac tac aac gcc aac tgg gag       96
   Val Gln Cys Glu Thr Arg Trp Cys Ile Tyr Tyr Asn Ala Asn Trp Glu
               20                  25                  30
   ctg gag cgc acc aac cag acc ggc ctg gag cgc tgc gaa ggc gag cag      144
   Leu Glu Arg Thr Asn Gln Thr Gly Leu Glu Arg Cys Glu Gly Glu Gln
           35                  40                  45
   gac aag cgg ctg cac tgc tac gcc tcc tgg cgc aac agc tct ggc acc      192
   Asp Lys Arg Leu His Cys Tyr Ala Ser Trp Arg Asn Ser Ser Gly Thr
       50                  55                  60
   atc gag ctc gtg aag aag ggc tgc tgg cta gat gac ttc aac tgc tac      240
   Ile Glu Leu Val Lys Lys Gly Cys Trp Leu Asp Asp Phe Asn Cys Tyr
   65                  70                  75                  80
   gat agg cag gag tgt gtg gcc act gag gag aac ccc cag gtg tac ttc      288
   Asp Arg Gln Glu Cys Val Ala Thr Glu Glu Asn Pro Gln Val Tyr Phe
                   85                  90                  95
   tgc tgc tgt gag ggc aac ttc tgc aac gag cgc ttc act cat ttg cca      336
   Cys Cys Cys Glu Gly Asn Phe Cys Asn Glu Arg Phe Thr His Leu Pro
               100                 105                 110
   gag gct ggg ggc ccg gaa gtc acg tac gag cca ccc ccg aca gcc ccc      384
   Glu Ala Gly Gly Pro Glu Val Thr Tyr Glu Pro Pro Pro Thr Ala Pro
           115                 120                 125
   acc                                                                  387
   Thr
<210> 4
   <211> 129
   <212> **PRT**
   <213> Homo sapiens
<400> 4
   Met Glu Phe Gly Leu Ser Trp Val Phe Leu Val Ala Leu Leu Arg Gly
   1               5                   10                  15
   Val Gln Cys Glu Thr Arg Trp Cys Ile Tyr Tyr Asn Ala Asn Trp Glu
               20                  25                  30
   Leu Glu Arg Thr Asn Gln Thr Gly Leu Glu Arg Cys Glu Gly Glu Gln
           35                  40                  45
   Asp Lys Arg Leu His Cys Tyr Ala Ser Trp Arg Asn Ser Ser Gly Thr
       50                  55                  60
   Ile Glu Leu Val Lys Lys Gly Cys Trp Leu Asp Asp Phe Asn Cys Tyr
   65                  70                  75                  80
   Asp Arg Gln Glu Cys Val Ala Thr Glu Glu Asn Pro Gln Val Tyr Phe
                   85                  90                  95
   Cys Cys Cys Glu Gly Asn Phe Cys Asn Glu Arg Phe Thr His Leu Pro
               100                 105                 110
   Glu Ala Gly Gly Pro Glu Val Thr Tyr Glu Pro Pro Pro Thr Ala Pro
           115                 120                 125
   Thr
<210> 5
   <211> 330
   <212> DNA
   <213> Homo sapiens
<220>
   <221> CDS
   <222> (1)..(330)
<400> 5
   gag aca cgg tgg tgc atc tac tac aac gcc aac tgg gag ctg gag cgc      48
   Glu Thr Arg Trp Cys Ile Tyr Tyr Asn Ala Asn Trp Glu Leu Glu Arg
   1               5                   10                  15
   acc aac cag acc ggc ctg gag cgc tgc gaa ggc gag cag gac aag cgg      96
   Thr Asn Gln Thr Gly Leu Glu Arg Cys Glu Gly Glu Gln Asp Lys Arg
               20                  25                  30
   ctg cac tgc tac gcc tcc tgg cgc aac agc tct ggc acc atc gag ctc     144
   Leu His Cys Tyr Ala Ser Trp Arg Asn Ser Ser Gly Thr Ile Glu Leu
           35                  40                  45
   gtg aag aag ggc tgc tgg cta gat gac ttc aac tgc tac gat agg cag     192
   Val Lys Lys Gly Cys Trp Leu Asp Asp Phe Asn Cys Tyr Asp Arg Gln
       50                  55                  60
   gag tgt gtg gcc act gag gag aac ccc cag gtg tac ttc tgc tgc tgt     240
   Glu Cys Val Ala Thr Glu Glu Asn Pro Gln Val Tyr Phe Cys Cys Cys
   65                  70                  75                  80
   gag ggc aac ttc tgc aac gag cgc ttc act cat ttg cca gag gct ggg     288
   Glu Gly Asn Phe Cys Asn Glu Arg Phe Thr His Leu Pro Glu Ala Gly
                   85                  90                  95
   ggc ccg gaa gtc acg tac gag cca ccc ccg aca gcc ccc acc             330
   Gly Pro Glu Val Thr Tyr Glu Pro Pro Pro Thr Ala Pro Thr
               100                 105                 110
<210> 6
   <211> 110
   <212> PRT
   <213> Homo sapiens
<400> 6
   Glu Thr Arg Trp Cys Ile Tyr Tyr Asn Ala Asn Trp Glu Leu Glu Arg
   1               5                   10                  15
   Thr Asn Gln Thr Gly Leu Glu Arg Cys Glu Gly Glu Gln Asp Lys Arg
               20                  25                  30
   Leu His Cys Tyr Ala Ser Trp Arg Asn Ser Ser Gly Thr Ile Glu Leu
           35                  40                  45
   Val Lys Lys Gly Cys Trp Leu Asp Asp Phe Asn Cys Tyr Asp Arg Gln
       50                  55                  60
   Glu Cys Val Ala Thr Glu Glu Asn Pro Gln Val Tyr Phe Cys Cys Cys
   65                  70                  75                  80
   Glu Gly Asn Phe Cys Asn Glu Arg Phe Thr His Leu Pro Glu Ala Gly
                   85                  90                  95
   Gly Pro Glu Val Thr Tyr Glu Pro Pro Pro Thr Ala Pro Thr
               100                 105                 110
<210> 7
   <211> 1071
   <212> DNA
   <213> Homo sapiens
<220>
   <221> CDS
   <222> (1)..(1071)
<400> 7
   atg gag ttt ggg ctg agc tgg gtt ttc ctc gtt gct ctt tta aga ggt        48
   Met Glu Phe Gly Leu Ser Trp Val Phe Leu Val Ala Leu Leu Arg Gly
   1               5                   10                  15
   gtc cag tgt gag aca cgg tgg tgc atc tac tac aac gcc aac tgg gag        96
   Val Gln Cys Glu Thr Arg Trp Cys Ile Tyr Tyr Asn Ala Asn Trp Glu
               20                  25                  30
   ctg gag cgc acc aac cag acc ggc ctg gag cgc tgc gaa ggc gag cag       144
   Leu Glu Arg Thr Asn Gln Thr Gly Leu Glu Arg Cys Glu Gly Glu Gln
           35                  40                  45
   gac aag cgg ctg cac tgc tac gcc tcc tgg cgc aac agc tct ggc acc       192
   Asp Lys Arg Leu His Cys Tyr Ala Ser Trp Arg Asn Ser Ser Gly Thr
       50                  55                  60
   atc gag ctc gtg aag aag ggc tgc tgg cta gat gac ttc aac tgc tac       240
   Ile Glu Leu Val Lys Lys Gly Cys Trp Leu Asp Asp Phe Asn Cys Tyr
   65                  70                  75                  80
   gat agg cag gag tgt gtg gcc act gag gag aac ccc cag gtg tac ttc       288
   Asp Arg Gln Glu Cys Val Ala Thr Glu Glu Asn Pro Gln Val Tyr Phe
                   85                  90                  95
   tgc tgc tgt gag ggc aac ttc tgc aac gag cgc ttc act cat ttg cca          336
   Cys Cys Cys Glu Gly Asn Phe Cys Asn Glu Arg Phe Thr His Leu Pro
               100                 105                 110
   gag gct ggg ggc ccg gaa gtc acg tac gag cca ccc ccg aca gcc ccc          384
   Glu Ala Gly Gly Pro Glu Val Thr Tyr Glu Pro Pro Pro Thr Ala Pro
           115                 120                 125
   acc gga ggg gga gga tct gtc gag tgc cca ccg tgc cca gca cca cct          432
   Thr Gly Gly Gly Gly Ser Val Glu Cys Pro Pro Cys Pro Ala Pro Pro
       130                 135                 140
   gtg gca gga ccg tca gtc ttc ctc ttc ccc cca aaa ccc aag gac acc          480
   Val Ala Gly Pro Ser Val Phe Leu Phe Pro Pro Lys Pro Lys Asp Thr
   145                 150                 155                 160
   ctc atg atc tcc cgg acc cct gag gtc acg tgc gtg gtg gtg gac gtg          528
   Leu Met Ile Ser Arg Thr Pro Glu Val Thr Cys Val Val Val Asp Val
                   165                 170                 175
   agc cac gaa gac ccc gag gtc cag ttc aac tgg tac gtg gac ggc gtg          576
   Ser His Glu Asp Pro Glu Val Gln Phe Asn Trp Tyr Val Asp Gly Val
               180                 185                 190
   gag gtg cat aat gcc aag aca aag cca cgg gag gag cag ttc aac agc          624
   Glu Val His Asn Ala Lys Thr Lys Pro Arg Glu Glu Gln Phe Asn Ser
           195                 200                 205
   acg ttc cgt gtg gtc agc gtc ctc acc gtt gtg cac cag gac tgg ctg          672
   Thr Phe Arg Val Val Ser Val Leu Thr Val Val His Gln Asp Trp Leu
       210                 215                 220
   aac ggc aag gag tac aag tgc aag gtc tcc aac aaa ggc ctc cca gcc          720
   Asn Gly Lys Glu Tyr Lys Cys Lys Val Ser Asn Lys Gly Leu Pro Ala
   225                 230                 235                 240
   ccc atc gag aaa acc atc tcc aaa acc aaa ggg cag ccc cga gaa cca          768
   Pro Ile Glu Lys Thr Ile Ser Lys Thr Lys Gly Gln Pro Arg Glu Pro
                   245                 250                 255
   cag gtg tac acc ctg ccc cca tcc cgg gag gag atg acc aag aac cag          816
   Gln Val Tyr Thr Leu Pro Pro Ser Arg Glu Glu Met Thr Lys Asn Gln
               260                 265                 270
   gtc agc ctg acc tgc ctg gtc aaa ggc ttc tat ccc agc gac atc gcc          864
   Val Ser Leu Thr Cys Leu Val Lys Gly Phe Tyr Pro Ser Asp Ile Ala
           275                 280                 285
   gtg gag tgg gag agc aat ggg cag ccg gag aac aac tac aag acc aca          912
   Val Glu Trp Glu Ser Asn Gly Gln Pro Glu Asn Asn Tyr Lys Thr Thr
       290                 295                 300
   cct ccc atg ctg gac tcc gac ggc tcc ttc ttc ctc tac agc aag ctc          960
   Pro Pro Met Leu Asp Ser Asp Gly Ser Phe Phe Leu Tyr Ser Lys Leu
   305                 310                 315                 320
   acc gtg gac aag agc agg tgg cag cag ggg aac gtc ttc tca tgc tcc         1008
   Thr Val Asp Lys Ser Arg Trp Gln Gln Gly Asn Val Phe Ser Cys Ser
                   325                 330                 335
   gtg atg cat gag gct ctg cac aac cac tac acg cag aag agc ctc tcc         1056
   Val Met His Glu Ala Leu His Asn His Tyr Thr Gln Lys Ser Leu Ser
               340                 345                 350
   ctg tct ccg ggt aaa                                                1071
   Leu Ser Pro Gly Lys
           355
<210> 8
   <211> 357
   <212> PRT
   <213> Homo sapiens
<400> 8
   Met Glu Phe Gly Leu Ser Trp Val Phe Leu Val Ala Leu Leu Arg Gly
   1               5                   10                  15
   Val Gln Cys Glu Thr Arg Trp Cys Ile Tyr Tyr Asn Ala Asn Trp Glu
               20                  25                  30
   Leu Glu Arg Thr Asn Gln Thr Gly Leu Glu Arg Cys Glu Gly Glu Gln
           35                  40                  45
   Asp Lys Arg Leu His Cys Tyr Ala Ser Trp Arg Asn Ser Ser Gly Thr
       50                  55                  60
   Ile Glu Leu Val Lys Lys Gly Cys Trp Leu Asp Asp Phe Asn Cys Tyr
   65                  70                  75                  80
   Asp Arg Gln Glu Cys Val Ala Thr Glu Glu Asn Pro Gln Val Tyr Phe
                   85                  90                  95
   Cys Cys Cys Glu Gly Asn Phe Cys Asn Glu Arg Phe Thr His Leu Pro
               100                 105                 110
   Glu Ala Gly Gly Pro Glu Val Thr Tyr Glu Pro Pro Pro Thr Ala Pro
           115                 120                 125
   Thr Gly Gly Gly Gly Ser Val Glu Cys Pro Pro Cys Pro Ala Pro Pro
       130                 135                 140
   Val Ala Gly Pro Ser Val Phe Leu Phe Pro Pro Lys Pro Lys Asp Thr
   145                 150                 155                 160
   Leu Met Ile Ser Arg Thr Pro Glu Val Thr Cys Val Val Val Asp Val
                   165                 170                 175
   Ser His Glu Asp Pro Glu Val Gln Phe Asn Trp Tyr Val Asp Gly Val
               180                 185                 190
   Glu Val His Asn Ala Lys Thr Lys Pro Arg Glu Glu Gln Phe Asn Ser
                   195                 200                 205
   Thr Phe Arg Val Val Ser Val Leu Thr Val Val His Gln Asp Trp Leu
       210                 215                 220
   Asn Gly Lys Glu Tyr Lys Cys Lys Val Ser Asn Lys Gly Leu Pro Ala
   225                 230                 235                 240
   Pro Ile Glu Lys Thr Ile Ser Lys Thr Lys Gly Gln Pro Arg Glu Pro
                   245                 250                 255
   Gln Val Tyr Thr Leu Pro Pro Ser Arg Glu Glu Met Thr Lys Asn Gln
               260                 265                 270
   Val Ser Leu Thr Cys Leu Val Lys Gly Phe Tyr Pro Ser Asp Ile Ala
           275                 280                 285
   Val Glu Trp Glu Ser Asn Gly Gln Pro Glu Asn Asn Tyr Lys Thr Thr
       290                 295                 300
   Pro Pro Met Leu Asp Ser Asp Gly Ser Phe Phe Leu Tyr Ser Lys Leu
   305                 310                 315                 320
   Thr Val Asp Lys Ser Arg Trp Gln Gln Gly Asn Val Phe Ser Cys Ser
                   325                 330                 335
   Val Met His Glu Ala Leu His Asn His Tyr Thr Gln Lys Ser Leu Ser
               340                 345                 350
   Leu Ser Pro Gly Lys
           355
<210> 9
   <211> 1014
   <212> DNA
   <213> Homo sapiens
<220>
   <221> CDS
   <222> (1)..(1014)
<400> 9
   gag aca cgg tgg tgc atc tac tac aac gcc aac tgg gag ctg gag cgc        48
   Glu Thr Arg Trp Cys Ile Tyr Tyr Asn Ala Asn Trp Glu Leu Glu Arg
   1               5                   10                  15
   acc aac cag acc ggc ctg gag cgc tgc gaa ggc gag cag gac aag cgg        96
   Thr Asn Gln Thr Gly Leu Glu Arg Cys Glu Gly Glu Gln Asp Lys Arg
               20                  25                  30
   ctg cac tgc tac gcc tcc tgg cgc aac agc tct ggc acc atc gag ctc       144
   Leu His Cys Tyr Ala Ser Trp Arg Asn Ser Ser Gly Thr Ile Glu Leu
           35                  40                  45
   gtg aag aag ggc tgc tgg cta gat gac ttc aac tgc tac gat agg cag        192
   Val Lys Lys Gly Cys Trp Leu Asp Asp Phe Asn Cys Tyr Asp Arg Gln
       50                  55                  60
   gag tgt gtg gcc act gag gag aac ccc cag gtg tac ttc tgc tgc tgt        240
   Glu Cys Val Ala Thr Glu Glu Asn Pro Gln Val Tyr Phe Cys Cys Cys
   65                  70                  75                  80
   gag ggc aac ttc tgc aac gag cgc ttc act cat ttg cca gag gct ggg        288
   Glu Gly Asn Phe Cys Asn Glu Arg Phe Thr His Leu Pro Glu Ala Gly
                   85                  90                  95
   ggc ccg gaa gtc acg tac gag cca ccc ccg aca gcc ccc acc gga ggg        336
   Gly Pro Glu Val Thr Tyr Glu Pro Pro Pro Thr Ala Pro Thr Gly Gly
               100                 105                 110
   gga gga tct gtc gag tgc cca ccg tgc cca gca cca cct gtg gca gga        384
   Gly Gly Ser Val Glu Cys Pro Pro Cys Pro Ala Pro Pro Val Ala Gly
           115                 120                 125
   ccg tca gtc ttc ctc ttc ccc cca aaa ccc aag gac acc ctc atg atc        432
   Pro Ser Val Phe Leu Phe Pro Pro Lys Pro Lys Asp Thr Leu Met Ile
       130                 135                 140
   tcc cgg acc cct gag gtc acg tgc gtg gtg gtg gac gtg agc cac gaa        480
   Ser Arg Thr Pro Glu Val Thr Cys Val Val Val Asp Val Ser His Glu
   145                 150                 155                 160
   gac ccc gag gtc cag ttc aac tgg tac gtg gac ggc gtg gag gtg cat        528
   Asp Pro Glu Val Gln Phe Asn Trp Tyr Val Asp Gly Val Glu Val His
                   165                 170                 175
   aat gcc aag aca aag cca cgg gag gag cag ttc aac agc acg ttc cgt        576
   Asn Ala Lys Thr Lys Pro Arg Glu Glu Gln Phe Asn Ser Thr Phe Arg
               180                 185                 190
   gtg gtc agc gtc ctc acc gtt gtg cac cag gac tgg ctg aac ggc aag        624
   Val Val Ser Val Leu Thr Val Val His Gln Asp Trp Leu Asn Gly Lys
           195                 200                 205
   gag tac aag tgc aag gtc tcc aac aaa ggc ctc cca gcc ccc atc gag        672
   Glu Tyr Lys Cys Lys Val Ser Asn Lys Gly Leu Pro Ala Pro Ile Glu
       210                 215                 220
   aaa acc atc tcc aaa acc aaa ggg cag ccc cga gaa cca cag gtg tac        720
   Lys Thr Ile Ser Lys Thr Lys Gly Gln Pro Arg Glu Pro Gln Val Tyr
   225                 230                 235                 240
   acc ctg ccc cca tcc cgg gag gag atg acc aag aac cag gtc agc ctg        768
   Thr Leu Pro Pro Ser Arg Glu Glu Met Thr Lys Asn Gln Val Ser Leu
                   245                 250                 255
   acc tgc ctg gtc aaa ggc ttc tat ccc agc gac atc gcc gtg gag tgg        816
   Thr Cys Leu Val Lys Gly Phe Tyr Pro Ser Asp Ile Ala Val Glu Trp
               260                 265                 270
   gag agc aat ggg cag ccg gag aac aac tac aag acc aca cct ccc atg        864
   Glu Ser Asn Gly Gln Pro Glu Asn Asn Tyr Lys Thr Thr Pro Pro Met
           275                 280                 285
   ctg gac tcc gac ggc tcc ttc ttc ctc tac agc aag ctc acc gtg gac        912
   Leu Asp Ser Asp Gly Ser Phe Phe Leu Tyr Ser Lys Leu Thr Val Asp
       290                 295                 300
   aag agc agg tgg cag cag ggg aac gtc ttc tca tgc tcc gtg atg cat        960
   Lys Ser Arg Trp Gln Gln Gly Asn Val Phe Ser Cys Ser Val Met His
   305                 310                 315                 320
   gag gct ctg cac aac cac tac acg cag aag agc ctc tcc ctg tct ccg       1008
   Glu Ala Leu His Asn His Tyr Thr Gln Lys Ser Leu Ser Leu Ser Pro
                   325                 330                 335
   ggt aaa                                                               1014
   Gly Lys
<210> 10
   <211> 338
   <212> PRT
   <213> Homo sapiens
<400> 10 Gly Lys
<210> 11
   <211> 387
   <212> DNA
   <213> Homo sapiens
<220>
   <221> CDS
   <222> (1)..(387)
<400> 11
   atg gag ttt ggg ctg agc tgg gtt ttc ctc gtt gct ctt tta aga ggt        48
   Met Glu Phe Gly Leu Ser Trp Val Phe Leu Val Ala Leu Leu Arg Gly
   1               5                   10                  15
   gtc cag tgt gag aca cgg tac tgc atc tac tac aac gcc aac tgg gag          96
   Val Gln Cys Glu Thr Arg Tyr Cys Ile Tyr Tyr Asn Ala Asn Trp Glu
               20                  25                  30
   ctg gag cgc acc aac cag acc ggc ctg gag cgc tgc gaa ggc gag cag         144
   Leu Glu Arg Thr Asn Gln Thr Gly Leu Glu Arg Cys Glu Gly Glu Gln
           35                  40                  45
   gac aag cgg ctg cac tgc tac gcc tcc tgg cgc aac agc tct ggc acc         192
   Asp Lys Arg Leu His Cys Tyr Ala Ser Trp Arg Asn Ser Ser Gly Thr
       50                  55                  60
   atc gag ctc gtg aag aag ggc tgc tgg cta gat gac ttc aac tgc tac         240
   Ile Glu Leu Val Lys Lys Gly Cys Trp Leu Asp Asp Phe Asn Cys Tyr
   65                  70                  75                  80
   gat agg cag gag tgt gtg gcc act gag gag aac ccc cag gtg tac ttc         288
   Asp Arg Gln Glu Cys Val Ala Thr Glu Glu Asn Pro Gln Val Tyr Phe
                   85                  90                  95
   tgc tgc tgt gag ggc aac ttc tgc aac gag cgc ttc act cat ttg cca         336
   Cys Cys Cys Glu Gly Asn Phe Cys Asn Glu Arg Phe Thr His Leu Pro
               100                 105                 110
   gag gct ggg ggc ccg gaa gtc acg tac gag cca ccc ccg aca gcc ccc         384
   Glu Ala Gly Gly Pro Glu Val Thr Tyr Glu Pro Pro Pro Thr Ala Pro
           115                 120                 125
   acc                                                                     387
   Thr
<210> 12
   <211> 129
   <212> PRT
   <213> Homo sapiens
<400> 12
   Met Glu Phe Gly Leu Ser Trp Val Phe Leu Val Ala Leu Leu Arg Gly
   1               5                   10                  15
   Val Gln Cys Glu Thr Arg Tyr Cys Ile Tyr Tyr Asn Ala Asn Trp Glu
               20                  25                  30
   Leu Glu Arg Thr Asn Gln Thr Gly Leu Glu Arg Cys Glu Gly Glu Gln
           35                  40                  45
   Asp Lys Arg Leu His Cys Tyr Ala Ser Trp Arg Asn Ser Ser Gly Thr
       50                  55                  60
   Ile Glu Leu Val Lys Lys Gly Cys Trp Leu Asp Asp Phe Asn Cys Tyr
   65                  70                  75                  80
   Asp Arg Gln Glu Cys Val Ala Thr Glu Glu Asn Pro Gln Val Tyr Phe
                   85                  90                  95
   Cys Cys Cys Glu Gly Asn Phe Cys Asn Glu Arg Phe Thr His Leu Pro
               100                 105                 110
   Glu Ala Gly Gly Pro Glu Val Thr Tyr Glu Pro Pro Pro Thr Ala Pro
           115                 120                 125
   Thr
<210> 13
   <211> 330
   <212> DNA
   <213> Homo sapiens
<220>
   <221> CDS
   <222> (1)..(330)
<400> 13
   gag aca cgg tac tgc atc tac tac aac gcc aac tgg gag ctg gag cgc        48
   Glu Thr Arg Tyr Cys Ile Tyr Tyr Asn Ala Asn Trp Glu Leu Glu Arg
   1               5                   10                  15
   acc aac cag acc ggc ctg gag cgc tgc gaa ggc gag cag gac aag cgg        96
   Thr Asn Gln Thr Gly Leu Glu Arg Cys Glu Gly Glu Gln Asp Lys Arg
               20                  25                  30
   ctg cac tgc tac gcc tcc tgg cgc aac agc tct ggc acc atc gag ctc       144
   Leu His Cys Tyr Ala Ser Trp Arg Asn Ser Ser Gly Thr Ile Glu Leu
           35                  40                  45
   gtg aag aag ggc tgc tgg cta gat gac ttc aac tgc tac gat agg cag       192
   Val Lys Lys Gly Cys Trp Leu Asp Asp Phe Asn Cys Tyr Asp Arg Gln
       50                  55                  60
   gag tgt gtg gcc act gag gag aac ccc cag gtg tac ttc tgc tgc tgt       240
   Glu Cys Val Ala Thr Glu Glu Asn Pro Gln Val Tyr Phe Cys Cys Cys
   65                  70                  75                  80
   gag ggc aac ttc tgc aac gag cgc ttc act cat ttg cca gag gct ggg       288
   Glu Gly Asn Phe Cys Asn Glu Arg Phe Thr His Leu Pro Glu Ala Gly
                   85                  90                  95
   ggc ccg gaa gtc acg tac gag cca ccc ccg aca gcc ccc acc               330
   Gly Pro Glu Val Thr Tyr Glu Pro Pro Pro Thr Ala Pro Thr
               100                 105                 110
<210> 14
   <211> 110
   <212> PRT
   <213> Homo sapiens
<400> 14
   Glu Thr Arg Tyr Cys Ile Tyr Tyr Asn Ala Asn Trp Glu Leu Glu Arg
   1               5                   10                  15
   Thr Asn Gln Thr Gly Leu Glu Arg Cys Glu Gly Glu Gln Asp Lys Arg
               20                  25                  30
   Leu His Cys Tyr Ala Ser Trp Arg Asn Ser Ser Gly Thr Ile Glu Leu
           35                  40                  45
   Val Lys Lys Gly Cys Trp Leu Asp Asp Phe Asn Cys Tyr Asp Arg Gln
       50                  55                  60
   Glu Cys Val Ala Thr Glu Glu Asn Pro Gln Val Tyr Phe Cys Cys Cys
   65                  70                  75                  80
   Glu Gly Asn Phe Cys Asn Glu Arg Phe Thr His Leu Pro Glu Ala Gly
                   85                  90                  95
   Gly Pro Glu Val Thr Tyr Glu Pro Pro Pro Thr Ala Pro Thr
               100                 105                 110
<210> 15
   <211> 1071
   <212> DNA
   <213> Homo sapiens
<220>
   <221> CDS
   <222> (1)..(1071)
<400> 15
   atg gag ttt ggg ctg agc tgg gtt ttc ctc gtt gct ctt tta aga ggt        48
   Met Glu Phe Gly Leu Ser Trp Val Phe Leu Val Ala Leu Leu Arg Gly
   1               5                   10                  15
   gtc cag tgt gag aca cgg tac tgc atc tac tac aac gcc aac tgg gag        96
   Val Gln Cys Glu Thr Arg Tyr Cys Ile Tyr Tyr Asn Ala Asn Trp Glu
               20                  25                  30
   ctg gag cgc acc aac cag acc ggc ctg gag cgc tgc gaa ggc gag cag       144
   Leu Glu Arg Thr Asn Gln Thr Gly Leu Glu Arg Cys Glu Gly Glu Gln
           35                  40                  45
   gac aag cgg ctg cac tgc tac gcc tcc tgg cgc aac agc tct ggc acc       192
   Asp Lys Arg Leu His Cys Tyr Ala Ser Trp Arg Asn Ser Ser Gly Thr
       50                  55                  60
   atc gag ctc gtg aag aag ggc tgc tgg cta gat gac ttc aac tgc tac       240
   Ile Glu Leu Val Lys Lys Gly Cys Trp Leu Asp Asp Phe Asn Cys Tyr
   65                  70                  75                  80
   gat agg cag gag tgt gtg gcc act gag gag aac ccc cag gtg tac ttc       288
   Asp Arg Gln Glu Cys Val Ala Thr Glu Glu Asn Pro Gln Val Tyr Phe
                   85                  90                  95
   tgc tgc tgt gag ggc aac ttc tgc aac gag cgc ttc act cat ttg cca       336
   Cys Cys Cys Glu Gly Asn Phe Cys Asn Glu Arg Phe Thr His Leu Pro
               100                 105                 110
   gag gct ggg ggc ccg gaa gtc acg tac gag cca ccc ccg aca gcc ccc       384
   Glu Ala Gly Gly Pro Glu Val Thr Tyr Glu Pro Pro Pro Thr Ala Pro
           115                 120                 125
   acc gga ggg gga gga tct gtc gag tgc cca ccg tgc cca gca cca cct      432
   Thr Gly Gly Gly Gly Ser Val Glu Cys Pro Pro Cys Pro Ala Pro Pro
       130                 135                 140
   gtg gca gga ccg tca gtc ttc ctc ttc ccc cca aaa ccc aag gac acc      480
   Val Ala Gly Pro Ser Val Phe Leu Phe Pro Pro Lys Pro Lys Asp Thr
   145                 150                 155                 160
   ctc atg atc tcc cgg acc cct gag gtc acg tgc gtg gtg gtg gac gtg      528
   Leu Met Ile Ser Arg Thr Pro Glu Val Thr Cys Val Val Val Asp Val
                   165                 170                 175
   agc cac gaa gac ccc gag gtc cag ttc aac tgg tac gtg gac ggc gtg      576
   Ser His Glu Asp Pro Glu Val Gln Phe Asn Trp Tyr Val Asp Gly Val
               180                 185                 190
   gag gtg cat aat gcc aag aca aag cca cgg gag gag cag ttc aac agc      624
   Glu Val His Asn Ala Lys Thr Lys Pro Arg Glu Glu Gln Phe Asn Ser
           195                 200                 205
   acg ttc cgt gtg gtc agc gtc ctc acc gtt gtg cac cag gac tgg ctg      672
   Thr Phe Arg Val Val Ser Val Leu Thr Val Val His Gln Asp Trp Leu
       210                 215                 220
   aac ggc aag gag tac aag tgc aag gtc tcc aac aaa ggc ctc cca gcc      720
   Asn Gly Lys Glu Tyr Lys Cys Lys Val Ser Asn Lys Gly Leu Pro Ala
   225                 230                 235                 240
   ccc atc gag aaa acc atc tcc aaa acc aaa ggg cag ccc cga gaa cca      768
   Pro Ile Glu Lys Thr Ile Ser Lys Thr Lys Gly Gln Pro Arg Glu Pro
                   245                 250                 255
   cag gtg tac acc ctg ccc cca tcc cgg gag gag atg acc aag aac cag      816
   Gln Val Tyr Thr Leu Pro Pro Ser Arg Glu Glu Met Thr Lys Asn Gln
               260                 265                 270
   gtc agc ctg acc tgc ctg gtc aaa ggc ttc tat ccc agc gac atc gcc      864
   Val Ser Leu Thr Cys Leu Val Lys Gly Phe Tyr Pro Ser Asp Ile Ala
           275                 280                 285
   gtg gag tgg gag agc aat ggg cag ccg gag aac aac tac aag acc aca      912
   Val Glu Trp Glu Ser Asn Gly Gln Pro Glu Asn Asn Tyr Lys Thr Thr
       290                 295                 300
   cct ccc atg ctg gac tcc gac ggc tcc ttc ttc ctc tac agc aag ctc      960
   Pro Pro Met Leu Asp Ser Asp Gly Ser Phe Phe Leu Tyr Ser Lys Leu
   305                 310                 315                 320
   acc gtg gac aag agc agg tgg cag cag ggg aac gtc ttc tca tgc tcc     1008
   Thr Val Asp Lys Ser Arg Trp Gln Gln Gly Asn Val Phe Ser Cys Ser
                   325                 330                 335
   gtg atg cat gag gct ctg cac aac cac tac acg cag aag agc ctc tcc     1056
   Val Met His Glu Ala Leu His Asn His Tyr Thr Gln Lys Ser Leu Ser
               340                 345                 350
   ctg tct ccg ggt aaa                                                 1071
   Leu Ser Pro Gly Lys
           355
<210> 16
   <211> 357
   <212> PRT
   <213> Homo sapiens
<400> 16
   Met Glu Phe Gly Leu Ser Trp Val Phe Leu Val Ala Leu Leu Arg Gly
   1               5                   10                  15
   Val Gln Cys Glu Thr Arg Tyr Cys Ile Tyr Tyr Asn Ala Asn Trp Glu
               20                  25                  30
   Leu Glu Arg Thr Asn Gln Thr Gly Leu Glu Arg Cys Glu Gly Glu Gln
           35                  40                  45
   Asp Lys Arg Leu His Cys Tyr Ala Ser Trp Arg Asn Ser Ser Gly Thr
       50                  55                  60
   Ile Glu Leu Val Lys Lys Gly Cys Trp Leu Asp Asp Phe Asn Cys Tyr
   65                  70                  75                  80
   Asp Arg Gln Glu Cys Val Ala Thr Glu Glu Asn Pro Gln Val Tyr Phe
                   85                  90                  95
   Cys Cys Cys Glu Gly Asn Phe Cys Asn Glu Arg Phe Thr His Leu Pro
               100                 105                 110
   Glu Ala Gly Gly Pro Glu Val Thr Tyr Glu Pro Pro Pro Thr Ala Pro
           115                 120                 125
   Thr Gly Gly Gly Gly Ser Val Glu Cys Pro Pro Cys Pro Ala Pro Pro
       130                 135                 140
   Val Ala Gly Pro Ser Val Phe Leu Phe Pro Pro Lys Pro Lys Asp Thr
   145                 150                 155                 160
   Leu Met Ile Ser Arg Thr Pro Glu Val Thr Cys Val Val Val Asp Val
                   165                 170                 175
   Ser His Glu Asp Pro Glu Val Gln Phe Asn Trp Tyr Val Asp Gly Val
               180                 185                 190
   Glu Val His Asn Ala Lys Thr Lys Pro Arg Glu Glu Gln Phe Asn Ser
           195                 200                 205
   Thr Phe Arg Val Val Ser Val Leu Thr Val Val His Gln Asp Trp Leu
       210                 215                 220
   Asn Gly Lys Glu Tyr Lys Cys Lys Val Ser Asn Lys Gly Leu Pro Ala
   225                 230                 235                 240
   Pro Ile Glu Lys Thr Ile Ser Lys Thr Lys Gly Gln Pro Arg Glu Pro
                   245                 250                 255
   Gln Val Tyr Thr Leu Pro Pro Ser Arg Glu Glu Met Thr Lys Asn Gln
               260                 265                 270
   Val Ser Leu Thr Cys Leu Val Lys Gly Phe Tyr Pro Ser Asp Ile Ala
           275                 280                 285
   Val Glu Trp Glu Ser Asn Gly Gln Pro Glu Asn Asn Tyr Lys Thr Thr
       290                 295                 300
   Pro Pro Met Leu Asp Ser Asp Gly Ser Phe Phe Leu Tyr Ser Lys Leu
   305                 310                 315                 320
   Thr Val Asp Lys Ser Arg Trp Gln Gln Gly Asn Val Phe Ser Cys Ser
                   325                 330                 335
   Val Met His Glu Ala Leu His Asn His Tyr Thr Gln Lys Ser Leu Ser
               340                 345                 350
   Leu Ser Pro Gly Lys
           355
<210> 17
   <211> 1014
   <212> DNA
   <213> Homo sapiens
<220>
   <221> CDS
   <222> (1)..(1014)
<400> 17
   gag aca cgg tac tgc atc tac tac aac gcc aac tgg gag ctg gag cgc        48
   Glu Thr Arg Tyr Cys Ile Tyr Tyr Asn Ala Asn Trp Glu Leu Glu Arg
   1               5                   10                  15
   acc aac cag acc ggc ctg gag cgc tgc gaa ggc gag cag gac aag cgg        96
   Thr Asn Gln Thr Gly Leu Glu Arg Cys Glu Gly Glu Gln Asp Lys Arg
               20                  25                  30
   ctg cac tgc tac gcc tcc tgg cgc aac agc tct ggc acc atc gag ctc       144
   Leu His Cys Tyr Ala Ser Trp Arg Asn Ser Ser Gly Thr Ile Glu Leu
           35                  40                  45
   gtg aag aag ggc tgc tgg cta gat gac ttc aac tgc tac gat agg cag       192
   Val Lys Lys Gly Cys Trp Leu Asp Asp Phe Asn Cys Tyr Asp Arg Gln
       50                  55                  60
   gag tgt gtg gcc act gag gag aac ccc cag gtg tac ttc tgc tgc tgt        240
   Glu Cys Val Ala Thr Glu Glu Asn Pro Gln Val Tyr Phe Cys Cys Cys
   65                  70                  75                  80
   gag ggc aac ttc tgc aac gag cgc ttc act cat ttg cca gag gct ggg        288
   Glu Gly Asn Phe Cys Asn Glu Arg Phe Thr His Leu Pro Glu Ala Gly
                   85                  90                  95
   ggc ccg gaa gtc acg tac gag cca ccc ccg aca gcc ccc acc gga ggg        336
   Gly Pro Glu Val Thr Tyr Glu Pro Pro Pro Thr Ala Pro Thr Gly Gly
               100                 105                 110
   gga gga tct gtc gag tgc cca ccg tgc cca gca cca cct gtg gca gga        384
   Gly Gly Ser Val Glu Cys Pro Pro Cys Pro Ala Pro Pro Val Ala Gly
           115                 120                 125
   ccg tca gtc ttc ctc ttc ccc cca aaa ccc aag gac acc ctc atg atc        432
   Pro Ser Val Phe Leu Phe Pro Pro Lys Pro Lys Asp Thr Leu Met Ile
       130                 135                 140
   tcc cgg acc cct gag gtc acg tgc gtg gtg gtg gac gtg agc cac gaa        480
   Ser Arg Thr Pro Glu Val Thr Cys Val Val Val Asp Val Ser His Glu
   145                 150                 155                 160
   gac ccc gag gtc cag ttc aac tgg tac gtg gac ggc gtg gag gtg cat        528
   Asp Pro Glu Val Gln Phe Asn Trp Tyr Val Asp Gly Val Glu Val His
                   165                 170                 175
   aat gcc aag aca aag cca cgg gag gag cag ttc aac agc acg ttc cgt        576
   Asn Ala Lys Thr Lys Pro Arg Glu Glu Gln Phe Asn Ser Thr Phe Arg
               180                 185                 190
   gtg gtc agc gtc ctc acc gtt gtg cac cag gac tgg ctg aac ggc aag        624
   Val Val Ser Val Leu Thr Val Val His Gln Asp Trp Leu Asn Gly Lys
           195                 200                 205
   gag tac aag tgc aag gtc tcc aac aaa ggc ctc cca gcc ccc atc gag        672
   Glu Tyr Lys Cys Lys Val Ser Asn Lys Gly Leu Pro Ala Pro Ile Glu
       210                 215                 220
   aaa acc atc tcc aaa acc aaa ggg cag ccc cga gaa cca cag gtg tac        720
   Lys Thr Ile Ser Lys Thr Lys Gly Gln Pro Arg Glu Pro Gln Val Tyr
   225                 230                 235                 240
   acc ctg ccc cca tcc cgg gag gag atg acc aag aac cag gtc agc ctg        768
   Thr Leu Pro Pro Ser Arg Glu Glu Met Thr Lys Asn Gln Val Ser Leu
                   245                 250                 255
   acc tgc ctg gtc aaa ggc ttc tat ccc agc gac atc gcc gtg gag tgg        816
   Thr Cys Leu Val Lys Gly Phe Tyr Pro Ser Asp Ile Ala Val Glu Trp
               260                 265                 270
   gag agc aat ggg cag ccg gag aac aac tac aag acc aca cct ccc atg        864
   Glu Ser Asn Gly Gln Pro Glu Asn Asn Tyr Lys Thr Thr Pro Pro Met
           275                 280                 285
   ctg gac tcc gac ggc tcc ttc ttc ctc tac agc aag ctc acc gtg gac        912
   Leu Asp Ser Asp Gly Ser Phe Phe Leu Tyr Ser Lys Leu Thr Val Asp
       290                 295                 300
   aag agc agg tgg cag cag ggg aac gtc ttc tca tgc tcc gtg atg cat        960
   Lys Ser Arg Trp Gln Gln Gly Asn Val Phe Ser Cys Ser Val Met His
   305                 310                 315                 320
   gag gct ctg cac aac cac tac acg cag aag agc ctc tcc ctg tct ccg       1008
   Glu Ala Leu His Asn His Tyr Thr Gln Lys Ser Leu Ser Leu Ser Pro
                   325                 330                 335
   ggt aaa                                                               1014
   Gly Lys
<210> 18
   <211> 338
   <212> PRT
   <213> Homo sapiens
<400> 18
   Glu Thr Arg Tyr Cys Ile Tyr Tyr Asn Ala Asn Trp Glu Leu Glu Arg
   1               5                   10                  15
   Thr Asn Gln Thr Gly Leu Glu Arg Cys Glu Gly Glu Gln Asp Lys Arg
               20                  25                  30
   Leu His Cys Tyr Ala Ser Trp Arg Asn Ser Ser Gly Thr Ile Glu Leu
           35                  40                  45
   Val Lys Lys Gly Cys Trp Leu Asp Asp Phe Asn Cys Tyr Asp Arg Gln
       50                  55                  60
   Glu Cys Val Ala Thr Glu Glu Asn Pro Gln Val Tyr Phe Cys Cys Cys
   65                  70                  75                  80
   Glu Gly Asn Phe Cys Asn Glu Arg Phe Thr His Leu Pro Glu Ala Gly
                   85                  90                  95
   Gly Pro Glu Val Thr Tyr Glu Pro Pro Pro Thr Ala Pro Thr Gly Gly
               100                 105                 110
   Gly Gly Ser Val Glu Cys Pro Pro Cys Pro Ala Pro Pro Val Ala Gly
           115                 120                 125
   Pro Ser Val Phe Leu Phe Pro Pro Lys Pro Lys Asp Thr Leu Met Ile
       130                 135                 140
   Ser Arg Thr Pro Glu Val Thr Cys Val Val Val Asp Val Ser His Glu
   145                 150                 155                 160
   Asp Pro Glu Val Gln Phe Asn Trp Tyr Val Asp Gly Val Glu Val His
                   165                 170                 175
   Asn Ala Lys Thr Lys Pro Arg Glu Glu Gln Phe Asn Ser Thr Phe Arg
               180                 185                 190
   Val Val Ser Val Leu Thr Val Val His Gln Asp Trp Leu Asn Gly Lys
           195                 200                 205
   Glu Tyr Lys Cys Lys Val Ser Asn Lys Gly Leu Pro Ala Pro Ile Glu
       210                 215                 220
   Lys Thr Ile Ser Lys Thr Lys Gly Gln Pro Arg Glu Pro Gln Val Tyr
   225                 230                 235                 240
   Thr Leu Pro Pro Ser Arg Glu Glu Met Thr Lys Asn Gln Val Ser Leu
                   245                 250                 255
   Thr Cys Leu Val Lys Gly Phe Tyr Pro Ser Asp Ile Ala Val Glu Trp
               260                 265                 270
   Glu Ser Asn Gly Gln Pro Glu Asn Asn Tyr Lys Thr Thr Pro Pro Met
           275                 280                 285
   Leu Asp Ser Asp Gly Ser Phe Phe Leu Tyr Ser Lys Leu Thr Val Asp
       290                 295                 300
   Lys Ser Arg Trp Gln Gln Gly Asn Val Phe Ser Cys Ser Val Met His
   305                 310                 315                 320
   Glu Ala Leu His Asn His Tyr Thr Gln Lys Ser Leu Ser Leu Ser Pro
                   325                 330                 335
   Gly Lys
<210> 19
   <211> 110
   <212> PRT
   <213> Homo sapiens
<400> 19
   Glu Thr Arg Trp Cys Ile Tyr Tyr Asn Ala Asn Trp Glu Leu Glu Arg
   1               5                   10                  15
   Thr Asn Gln Ser Gly Leu Glu Arg Cys Glu Gly Glu Gln Asp Lys Arg
               20                  25                  30
   Leu His Cys Tyr Ala Ser Trp Arg Asn Ser Ser Gly Thr Ile Glu Leu
           35                  40                  45
   Val Lys Lys Gly Cys Trp Leu Asp Asp Phe Asn Cys Tyr Asp Arg Gln
       50                  55                  60
   Glu Cys Val Ala Thr Glu Glu Asn Pro Gln Val Tyr Phe Cys Cys Cys
   65 70 75 80
   Glu Gly Asn Phe Cys Asn Glu Arg Phe Thr His Leu Pro Glu Ala Gly
   85 90 95
   Gly Pro Glu Val Thr Tyr Glu Pro Pro Pro Thr Ala Pro Thr
   100 105 110
<210> 20
   <211> 1014
   <212> DNA
   <213> Homo sapiens
<220>
   <221> CDS
   <222> (1)..(1014)
<400> 20
   gag aca cgg tgg tgc atc tac tac aac gcc aac tgg gag ctg gag cgc       48
   Glu Thr Arg Trp Cys Ile Tyr Tyr Asn Ala Asn Trp Glu Leu Glu Arg
   1               5                   10                  15
   acc aac cag agc ggc ctg gag cgc tgc gaa ggc gag cag gac aag cgg       96
   Thr Asn Gln Ser Gly Leu Glu Arg Cys Glu Gly Glu Gln Asp Lys Arg
               20                  25                  30
   ctg cac tgc tac gcc tcc tgg cgc aac agc tct ggc acc atc gag ctc      144
   Leu His Cys Tyr Ala Ser Trp Arg Asn Ser Ser Gly Thr Ile Glu Leu
           35                  40                  45
   gtg aag aag ggc tgc tgg cta gat gac ttc aac tgc tac gat agg cag      192
   Val Lys Lys Gly Cys Trp Leu Asp Asp Phe Asn Cys Tyr Asp Arg Gln
       50                  55                  60
   gag tgt gtg gcc act gag gag aac ccc cag gtg tac ttc tgc tgc tgt      240
   Glu Cys Val Ala Thr Glu Glu Asn Pro Gln Val Tyr Phe Cys Cys Cys
   65                  70                  75                  80
   gag ggc aac ttc tgc aac gag cgc ttc act cat ttg cca gag gct ggg      288
   Glu Gly Asn Phe Cys Asn Glu Arg Phe Thr His Leu Pro Glu Ala Gly
                   85                  90                  95
   ggc ccg gaa gtc acg tac gag cca ccc ccg aca gcc ccc acc gga gga      336
   Gly Pro Glu Val Thr Tyr Glu Pro Pro Pro Thr Ala Pro Thr Gly Gly
               100                 105                 110
   gga gga tct gtc gag tgc cca ccg tgc cca gca cca cct gtg gca gga      384
   Gly Gly Ser Val Glu Cys Pro Pro Cys Pro Ala Pro Pro Val Ala Gly
           115                 120                 125
   ccg tca gtc ttc ctc ttc ccc cca aaa ccc aag gac acc ctc atg atc      432
   Pro Ser Val Phe Leu Phe Pro Pro Lys Pro Lys Asp Thr Leu Met Ile
       130                 135                 140
   tcc cgg acc cct gag gtc acg tgc gtg gtg gtg gac gtg agc cac gaa      480
   Ser Arg Thr Pro Glu Val Thr Cys Val Val Val Asp Val Ser His Glu
   145                 150                 155                 160
   gac ccc gag gtc cag ttc aac tgg tac gtg gac ggc gtg gag gtg cat        528
   Asp Pro Glu Val Gln Phe Asn Trp Tyr Val Asp Gly Val Glu Val His
                       165                 170                 175
   aat gcc aag aca aag cca cgg gag gag cag ttc aac agc acg ttc cgt        576
   Asn Ala Lys Thr Lys Pro Arg Glu Glu Gln Phe Asn Ser Thr Phe Arg
                   180                 185                 190
   gtg gtc agc gtc ctc acc gtt gtg cac cag gac tgg ctg aac ggc aag        624
   Val Val Ser Val Leu Thr Val Val His Gln Asp Trp Leu Asn Gly Lys
               195                 200                 205
   gag tac aag tgc aag gtc tcc aac aaa ggc ctc cca gcc ccc atc gag        672
   Glu Tyr Lys Cys Lys Val Ser Asn Lys Gly Leu Pro Ala Pro Ile Glu
       210                 215                 220
   aaa acc atc tcc aaa acc aaa ggg cag ccc cga gaa cca cag gtg tac        720
   Lys Thr Ile Ser Lys Thr Lys Gly Gln Pro Arg Glu Pro Gln Val Tyr
   225                 230                 235                 240
   acc ctg ccc cca tcc cgg gag gag atg acc aag aac cag gtc agc ctg        768
   Thr Leu Pro Pro Ser Arg Glu Glu Met Thr Lys Asn Gln Val Ser Leu
                       245                 250                 255
   acc tgc ctg gtc aaa ggc ttc tat ccc agc gac atc gcc gtg gag tgg        816
   Thr Cys Leu Val Lys Gly Phe Tyr Pro Ser Asp Ile Ala Val Glu Trp
                   260                 265                 270
   gag agc aat ggg cag ccg gag aac aac tac aag acc aca cct ccc atg        864
   Glu Ser Asn Gly Gln Pro Glu Asn Asn Tyr Lys Thr Thr Pro Pro Met
               275                 280                 285
   ctg gac tcc gac ggc tcc ttc ttc ctc tac agc aag ctc acc gtg gac        912
   Leu Asp Ser Asp Gly Ser Phe Phe Leu Tyr Ser Lys Leu Thr Val Asp
       290                 295                 300
   aag agc agg tgg cag cag ggg aac gtc ttc tca tgc tcc gtg atg cat        960
   Lys Ser Arg Trp Gln Gln Gly Asn Val Phe Ser Cys Ser Val Met His
   305                 310                 315                 320
   gag gct ctg cac aac cac tac acg cag aag agc ctc tcc ctg tct ccg       1008
   Glu Ala Leu His Asn His Tyr Thr Gln Lys Ser Leu Ser Leu Ser Pro
                       325                 330                 335
   ggt aaa                                                               1014
   Gly Lys
<210> 21
   <211> 338
   <212> PRT
   <213> Homo sapiens
<400> 21
   Glu Thr Arg Trp Cys Ile Tyr Tyr Asn Ala Asn Trp Glu Leu Glu Arg
   1               5                   10                  15
   Thr Asn Gln Ser Gly Leu Glu Arg Cys Glu Gly Glu Gln Asp Lys Arg
               20                  25                  30
   Leu His Cys Tyr Ala Ser Trp Arg Asn Ser Ser Gly Thr Ile Glu Leu
   35 40 45
   Val Lys Lys Gly Cys Trp Leu Asp Asp Phe Asn Cys Tyr Asp Arg Gln
   50 55 60
   Glu Cys Val Ala Thr Glu Glu Asn Pro Gln Val Tyr Phe Cys Cys Cys
   65 70 75 80
   Glu Gly Asn Phe Cys Asn Glu Arg Phe Thr His Leu Pro Glu Ala Gly
   85 90 95
   Gly Pro Glu Val Thr Tyr Glu Pro Pro Pro Thr Ala Pro Thr Gly Gly
   100 105 110
   Gly Gly Ser Val Glu Cys Pro Pro Cys Pro Ala Pro Pro Val Ala Gly
   115 120 125
   Pro Ser Val Phe Leu Phe Pro Pro Lys Pro Lys Asp Thr Leu Met Ile
   130 135 140
   Ser Arg Thr Pro Glu Val Thr Cys Val Val Val Asp Val Ser His Glu
   145 150 155 160
   Asp Pro Glu Val Gln Phe Asn Trp Tyr Val Asp Gly Val Glu Val His
   165 170 175
   Asn Ala Lys Thr Lys Pro Arg Glu Glu Gln Phe Asn Ser Thr Phe Arg
   180 185 190
   Val Val Ser Val Leu Thr Val Val His Gln Asp Trp Leu Asn Gly Lys
   195 200 205
   Glu Tyr Lys Cys Lys Val Ser Asn Lys Gly Leu Pro Ala Pro Ile Glu
   210 215 220
   Lys Thr Ile Ser Lys Thr Lys Gly Gln Pro Arg Glu Pro Gln Val Tyr
   225 230 235 240
   Thr Leu Pro Pro Ser Arg Glu Glu Met Thr Lys Asn Gln Val Ser Leu
   245 250 255
   Thr Cys Leu Val Lys Gly Phe Tyr Pro Ser Asp Ile Ala Val Glu Trp
   260 265 270
   Glu Ser Asn Gly Gln Pro Glu Asn Asn Tyr Lys Thr Thr Pro Pro Met
   275 280 285
   Leu Asp Ser Asp Gly Ser Phe Phe Leu Tyr Ser Lys Leu Thr Val Asp
       290                 295                 300
   Lys Ser Arg Trp Gln Gln Gly Asn Val Phe Ser Cys Ser Val Met His
   305                 310                 315                 320
   Glu Ala Leu His Asn His Tyr Thr Gln Lys Ser Leu Ser Leu Ser Pro
                   325                 330                 335
   Gly Lys
<210> 22
   <211> 216
   <212> PRT
   <213> Homo sapiens
<400> 22
   Ala Pro Pro Val Ala Gly Pro Ser Val Phe Leu Phe Pro Pro Lys Pro
   1               5                   10                  15
   Lys Asp Thr Leu Met Ile Ser Arg Thr Pro Glu Val Thr Cys Val Val
               20                  25                  30
   Val Asp Val Ser His Glu Asp Pro Glu Val Gln Phe Asn Trp Tyr Val
           35                  40                  45
   Asp Gly Val Glu Val His Asn Ala Lys Thr Lys Pro Arg Glu Glu Gln
       50                  55                  60
   Phe Asn Ser Thr Phe Arg Val Val Ser Val Leu Thr Val Val His Gln
   65                  70                  75                  80
   Asp Trp Leu Asn Gly Lys Glu Tyr Lys Cys Lys Val Ser Asn Lys Gly
                   85                  90                  95
   Leu Pro Ala Pro Ile Glu Lys Thr Ile Ser Lys Thr Lys Gly Gln Pro
               100                 105                 110
   Arg Glu Pro Gln Val Tyr Thr Leu Pro Pro Ser Arg Glu Glu Met Thr
           115                 120                 125
   Lys Asn Gln Val Ser Leu Thr Cys Leu Val Lys Gly Phe Tyr Pro Ser
       130                 135                 140
   Asp Ile Ala Val Glu Trp Glu Ser Asn Gly Gln Pro Glu Asn Asn Tyr
   145                 150                 155                 160
   Lys Thr Thr Pro Pro Met Leu Asp Ser Asp Gly Ser Phe Phe Leu Tyr
                   165                 170                 175
   Ser Lys Leu Thr Val Asp Lys Ser Arg Trp Gln Gln Gly Asn Val Phe
               180                 185                 190
   Ser Cys Ser Val Met His Glu Ala Leu His Asn His Tyr Thr Gln Lys
           195                 200                 205
   Ser Leu Ser Leu Ser Pro Gly Lys
       210                 215
<210> 23
   <211> 217
   <212> PRT
   <213> Homo sapiens
<400> 23
   Ala Pro Glu Leu Leu Gly Gly Pro Ser Val Phe Leu Phe Pro Pro Lys
   1               5                   10                  15
   Pro Lys Asp Ile Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Gly Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys Gly Gln
               100                 105                 110
   Pro Arg Glu Pro Gln Val Tyr Thr Leu Pro Pro Ser Arg Asp Glu Leu
           115                 120                 125
   Thr Lys Asn Gln Val Ser Leu Thr Cys Leu Val Lys Gly Phe Tyr Pro
       130                 135                 140
   Ser Asp Ile Ala Val Glu Trp Glu Ser Asn Gly Gln Pro Glu Asn Asn
   145                 150                 155                 160
   Tyr Lys Thr Thr Pro Pro Val Leu Asp Ser Asp Gly Ser Phe Phe Leu
                   165                 170                 175
   Tyr Ser Lys Leu Thr Val Asp Lys Ser Arg Trp Gln Gln Gly Asn Val
               180                 185                 190
   Phe Ser Cys Ser Val Met His Glu Ala Leu His Asn His Tyr Thr Gln
           195                 200                 205
   Lys Ser Leu Ser Leu Ser Pro Gly Lys
       210                 215
<210> 24
   <211> 217
   <212> PRT
   <213> Homo sapiens
<400> 24
   Ala Pro Glu Phe Leu Gly Gly Pro Ser Val Phe Leu Phe Pro Pro Lys
   1               5                   10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys Gly Gln
               100                 105                 110
   Pro Arg Glu Pro Gln Val Tyr Thr Leu Pro Pro Ser Gln Glu Glu Met
           115                 120                 125
   Thr Lys Asn Gln Val Ser Leu Thr Cys Leu Val Lys Gly Phe Tyr Pro
       130                 135                 140
   Ser Asp Ile Ala Val Glu Trp Glu Ser Asn Gly Gln Pro Glu Asn Asn
   145                 150                 155                 160
   Tyr Lys Thr Thr Pro Pro Val Leu Asp Ser Asp Gly Ser Phe Phe Leu
                   165                 170                 175
   Tyr Ser Arg Leu Thr Val Asp Lys Ser Arg Trp Gln Glu Gly Asn Val
               180                 185                 190
   Phe Ser Cys Ser Val Met His Glu Ala Leu His Asn His Tyr Thr Gln
           195                 200                 205
   Lys Ser Leu Ser Leu Ser Leu Gly Lys
       210                 215
<210> 25
   <211> 5
   <212> PRT
   <213> Artificial Sequence
<220>
   <223> Description of Artificial Sequence: Synthetic Linker peptide
<400> 25
   Gly Gly Gly Gly Ser
   1 5
<210> 26
   <211> 36
   <212> DNA
   <213> Artificial Sequence
<220>
   <223> Description of Artificial Sequence: Synthetic Hinge linker oligonucleotide
<220>
   <221> CDS
   <222> (1)..(36)
<400> 26
   gga ggg gga gga tct gtc gag tgc cca ccg tgc cca 36
   Gly Gly Gly Gly Ser Val Glu Cys Pro Pro Cys Pro
   1 5 10
<210> 27
   <211> 12
   <212> PRT
   <213> Artificial Sequence
<220>
   <223> Description of Artificial Sequence: Synthetic Hinge linker peptide
<400> 27
   Gly Gly Gly Gly Ser Val Glu Cys Pro Pro Cys Pro
   1 5 10
<210> 28
   <211> 12
   <212> PRT
   <213> Homo sapiens
<400> 28
   Glu Arg Lys Cys Cys Val Glu Cys Pro Pro Cys Pro
   1 5 10
<210> 29
   <211> 15
   <212> PRT
   <213> Homo sapiens
<400> 29
   Glu Pro Lys Ser Cys Asp Lys Thr His Thr Cys Pro Pro Cys Pro
   1 5 10 15
<210> 30
   <211> 12
   <212> PRT
   <213> Homo sapiens
<400> 30
   Glu Ser Lys Thr Gly Pro Pro Cys Pro Ser Cys Pro
   1               5                   10
<210> 31
   <211> 18
   <212> PRT
   <213> Homo sapiens
<400> 31
   Met Thr Ala Pro Trp Val Ala Leu Ala Leu Leu Trp Gly Ser Leu Trp
   1               5                   10                  15
   Pro Gly
<210> 32
   <211> 18
   <212> PRT
   <213> Homo sapiens
<400> 32
   Met Thr Ala Pro Trp Val Ala Leu Ala Leu Leu Trp Gly Ser Leu Cys
   1               5                   10                  15
   Ala Gly
<210> 33
   <211> 512
   <212> PRT
   <213> Homo sapiens
<400> 33
<210> 34
   <211> 426
   <212> PRT
   <213> Homo sapiens
<400> 34
   Met Pro Leu Leu Trp Leu Arg Gly Phe Leu Leu Ala Ser Cys Trp Ile
   1               5                   10                  15
   Ile Val Arg Ser Ser Pro Thr Pro Gly Ser Glu Gly His Ser Ala Ala
               20                  25                  30
   Pro Asp Cys Pro Ser Cys Ala Leu Ala Ala Leu Pro Lys Asp Val Pro
           35                  40                  45
   Asn Ser Gln Pro Glu Met Val Glu Ala Val Lys Lys His Ile Leu Asn
       50                  55                  60
   Met Leu His Leu Lys Lys Arg Pro Asp Val Thr Gln Pro Val Pro Lys
   65                  70                  75                  80
   Ala Ala Leu Leu Asn Ala Ile Arg Lys Leu His Val Gly Lys Val Gly
                   85                  90                  95
   Glu Asn Gly Tyr Val Glu Ile Glu Asp Asp Ile Gly Arg Arg Ala Glu
               100                 105                 110
   Met Asn Glu Leu Met Glu Gln Thr Ser Glu Ile Ile Thr Phe Ala Glu
           115                 120                 125
   Ser Gly Thr Ala Arg Lys Thr Leu His Phe Glu Ile Ser Lys Glu Gly
       130                 135                 140
   Ser Asp Leu Ser Val Val Glu Arg Ala Glu Val Trp Leu Phe Leu Lys
   145                 150                 155                 160
   Val Pro Lys Ala Asn Arg Thr Arg Thr Lys Val Thr Ile Arg Leu Phe
                   165                 170                 175
   Gln Gln Gln Lys His Pro Gln Gly Ser Leu Asp Thr Gly Glu Glu Ala
               180                 185                 190
   Glu Glu Val Gly Leu Lys Gly Glu Arg Ser Glu Leu Leu Leu Ser Glu
           195                 200                 205
   Lys Val Val Asp Ala Arg Lys Ser Thr Trp His Val Phe Pro Val Ser
       210                 215                 220
   Ser Ser Ile Gln Arg Leu Leu Asp Gln Gly Lys Ser Ser Leu Asp Val
   225 230 235 240
   Arg Ile Ala Cys Glu Gln Cys Gln Glu Ser Gly Ala Ser Leu Val Leu
   245 250 255
   Leu Gly Lys Lys Lys Lys Lys Glu Glu Glu Gly Glu Gly Lys Lys Lys
   260 265 270
   Gly Gly Gly Glu Gly Gly Ala Gly Ala Asp Glu Glu Lys Glu Gln Ser
   275 280 285
   His Arg Pro Phe Leu Met Leu Gln Ala Arg Gln Ser Glu Asp His Pro
   290 295 300
   His Arg Arg Arg Arg Arg Gly Leu Glu Cys Asp Gly Lys Val Asn Ile
   305 310 315 320
   Cys Cys Lys Lys Gln Phe Phe Val Ser Phe Lys Asp Ile Gly Trp Asn
   325 330 335
   Asp Trp Ile Ile Ala Pro Ser Gly Tyr His Ala Asn Tyr Cys Glu Gly
   340 345 350
   Glu Cys Pro Ser His Ile Ala Gly Thr Ser Gly Ser Ser Leu Ser Phe
   355 360 365
   His Ser Thr Val Ile Asn His Tyr Arg Met Arg Gly His Ser Pro Phe
   370 375 380
   Ala Asn Leu Lys Ser Cys Cys Val Pro Thr Lys Leu Arg Pro Met Ser
   385 390 395 400
   Met Leu Tyr Tyr Asp Asp Gly Gln Asn Ile Ile Lys Lys Asp Ile Gln
   405 410 415
   Asn Met Ile Val Glu Glu Cys Gly Cys Ser
   420 425
<210> 35
   <211> 375
   <212> PRT
   <213> Homo sapiens
<400> 35
   Met Gln Lys Leu Gln Leu Cys Val Tyr Ile Tyr Leu Phe Met Leu Ile
   1 5 10 15
   Val Ala Gly Pro Val Asp Leu Asn Glu Asn Ser Glu Gln Lys Glu Asn
               20                  25                  30
   Val Glu Lys Glu Gly Leu Cys Asn Ala Cys Thr Trp Arg Gln Asn Thr
           35                  40                  45
   Lys Ser Ser Arg Ile Glu Ala Ile Lys Ile Gln Ile Leu Ser Lys Leu
       50                  55                  60
   Arg Leu Glu Thr Ala Pro Asn Ile Ser Lys Asp Val Ile Arg Gln Leu
   65                  70                  75                  80
   Leu Pro Lys Ala Pro Pro Leu Arg Glu Leu Ile Asp Gln Tyr Asp Val
                   85                  90                  95
   Gln Arg Asp Asp Ser Ser Asp Gly Ser Leu Glu Asp Asp Asp Tyr His
               100                 105                 110
   Ala Thr Thr Glu Thr Ile Ile Thr Met Pro Thr Glu Ser Asp Phe Leu
           115                 120                 125
   Met Gln Val Asp Gly Lys Pro Lys Cys Cys Phe Phe Lys Phe Ser Ser
       130                 135                 140
   Lys Ile Gln Tyr Asn Lys Val Val Lys Ala Gln Leu Trp Ile Tyr Leu
   145                 150                 155                 160
   Arg Pro Val Glu Thr Pro Thr Thr Val Phe Val Gln Ile Leu Arg Leu
                   165                 170                 175
   Ile Lys Pro Met Lys Asp Gly Thr Arg Tyr Thr Gly Ile Arg Ser Leu
               180                 185                 190
   Lys Leu Asp Met Asn Pro Gly Thr Gly Ile Trp Gln Ser Ile Asp Val
           195                 200                 205
   Lys Thr Val Leu Gln Asn Trp Leu Lys Gln Pro Glu Ser Asn Leu Gly
       210                 215                 220
   Ile Glu Ile Lys Ala Leu Asp Glu Asn Gly His Asp Leu Ala Val Thr
   225                 230                 235                 240
   Phe Pro Gly Pro Gly Glu Asp Gly Leu Asn Pro Phe Leu Glu Val Lys
                   245                 250                 255
   Val Thr Asp Thr Pro Lys Arg Ser Arg Arg Asp Phe Gly Leu Asp Cys
               260                 265                 270
   Asp Glu His Ser Thr Glu Ser Arg Cys Cys Arg Tyr Pro Leu Thr Val
           275                 280                 285
   Asp Phe Glu Ala Phe Gly Trp Asp Trp Ile Ile Ala Pro Lys Arg Tyr
       290                 295                 300
   Lys Ala Asn Tyr Cys Ser Gly Glu Cys Glu Phe Val Phe Leu Gln Lys
   305                 310                 315                 320
   Tyr Pro His Thr His Leu Val His Gln Ala Asn Pro Arg Gly Ser Ala
                   325                 330                 335
   Gly Pro Cys Cys Thr Pro Thr Lys Met Ser Pro Ile Asn Met Leu Tyr
               340                 345                 350
   Phe Asn Gly Lys Glu Gln Ile Ile Tyr Gly Lys Ile Pro Ala Met Val
           355                 360                 365
   Val Asp Arg Cys Gly Cys Ser
       370                 375
<210> 36
   <211> 217
   <212> PRT
   <213> Homo sapiens
<400> 36
   Ala Pro Glu Leu Leu Gly Gly Pro Ser Val Phe Leu Phe Pro Pro Lys
   1               5                   10                  15
   Pro Lys Asp Ile Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Gly Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys Gly Gln
               100                 105                 110
   Pro Arg Glu Pro Gln Val Tyr Thr Leu Pro Pro Ser Arg Asp Glu Leu
           115                 120                 125
   Thr Lys Asn Gln Val Ser Leu Thr Cys Leu Val Lys Gly Phe Tyr Pro
       130                 135                 140
   Ser Asp Ile Ala Val Glu Trp Glu Ser Asn Gly Gln Pro Glu Asn Asn
   145                 150                 155                 160
   Tyr Lys Thr Thr Pro Pro Val Leu Asp Ser Asp Gly Ser Phe Phe Leu
                   165                 170                 175
   Tyr Ser Lys Leu Thr Val Asp Lys Ser Arg Trp Gln Gln Gly Asn Val
               180                 185                 190
   Phe Ser Cys Ser Val Met His Glu Ala Leu His Asn His Tyr Thr Gln
           195                 200                 205
   Lys Ser Leu Ser Leu Ser Pro Gly Lys
       210                 215
<210> 37
   <211> 48
   <212> DNA
   <213> Artificial Sequence
<220>
   <223> Description of Artificial Sequence: Synthetic Hinge linker oligonucleotide
<220>
   <221> CDS
   <222> (1)..(48)
<400> 37
   gga ggg gga gga tct gag cgc aaa tgt tgt gtc gag tgc cca ccg tgc        48
   Gly Gly Gly Gly Ser Glu Arg Lys Cys Cys Val Glu Cys Pro Pro Cys
   1               5                   10                  15
<210> 38
   <211> 16
   <212> PRT
   <213> Artificial Sequence
<220>
   <223> Description of Artificial Sequence: Synthetic Hinge linker peptide
<400> 38
   Gly Gly Gly Gly Ser Glu Arg Lys Cys Cys Val Glu Cys Pro Pro Cys
   1               5                   10                  15
<210> 39
   <211> 42
   <212> DNA
   <213> Artificial Sequence
<220>
   <223> Description of Artificial Sequence: Synthetic Hinge linker oligonucleotide
<220>
   <221> CDS
   <222> (1)..(42)
<400> 39
   gga ggg gga gga tct ggt gga ggt ggt tca ggt cca ccg tgc                42
   Gly Gly Gly Gly Ser Gly Gly Gly Gly Ser Gly Pro Pro Cys
   1               5                   10
<210> 40
   <211> 14
   <212> PRT
   <213> Artificial Sequence
<220>
   <223> Description of Artificial Sequence: Synthetic Hinge linker peptide
<400> 40
   Gly Gly Gly Gly Ser Gly Gly Gly Gly Ser Gly Pro Pro Cys
   1               5                   10
<210> 41
   <211> 42
   <212> DNA
   <213> Artificial Sequence
<220>
   <223> Description of Artificial Sequence: Synthetic Hinge linker oligonucleotide
<220>
   <221> CDS
   <222> (1)..(42)
<400> 41
   gga ggg gga gga tct ggt gga ggt ggt tca ggt cca ccg gga                42
   Gly Gly Gly Gly Ser Gly Gly Gly Gly Ser Gly Pro Pro Gly
   1               5                   10
<210> 42
   <211> 14
   <212> PRT
   <213> Artificial Sequence
<220>
   <223> Description of Artificial Sequence: Synthetic Hinge linker peptide
<400> 42
   Gly Gly Gly Gly Ser Gly Gly Gly Gly Ser Gly Pro Pro Gly
   1 5 10
<210> 43
   <211> 54
   <212> DNA
   <213> Artificial Sequence
<220>
   <223> Description of Artificial Sequence: Synthetic Hinge linker oligonucleotide
<220>
   <221> CDS
   <222> (1)..(54)
<400> 43
   gga ggg gga gga tct gag cgc aaa tgt cca cct tgt gtc gag tgc cca 48
   Gly Gly Gly Gly Ser Glu Arg Lys Cys Pro Pro Cys Val Glu Cys Pro
   1 5 10 15
   ccg tgc 54
   Pro Cys
<210> 44
   <211> 18
   <212> PRT
   <213> Artificial Sequence
<220>
   <223> Description of Artificial Sequence: Synthetic Hinge linker peptide
<400> 44
   Gly Gly Gly Gly Ser Glu Arg Lys Cys Pro Pro Cys Val Glu Cys Pro
   1 5 10 15
   Pro Cys
<210> 45
   <211> 14
   <212> PRT
   <213> Artificial Sequence
<220>
   <223> Description of Artificial Sequence: Synthetic Hinge linker peptide
<400> 45
   Gly Pro Ala Ser Gly Gly Pro Ala Ser Gly Pro Pro Cys Pro
   1 5 10
<210> 46
   <211> 21
   <212> PRT
   <213> Artificial Sequence
<220>
   <223> Description of Artificial Sequence: Synthetic Hinge linker peptide
<400> 46
   Gly Pro Ala Ser Gly Gly Pro Ala Ser Gly Cys Pro Pro Cys Val Glu
   1               5                   10                  15
   Cys Pro Pro Cys Pro
               20
<210> 47
   <211> 217
   <212> PRT
   <213> Homo sapiens
<400> 47
   Ala Pro Glu Leu Leu Gly Gly Pro Ser Val Phe Leu Phe Pro Pro Lys
   1               5                   10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys Gly Gln
               100                 105                 110
   Pro Arg Glu Pro Gln Val Tyr Thr Leu Pro Pro Ser Arg Glu Glu Met
           115                 120                 125
   Thr Lys Asn Gln Val Ser Leu Thr Cys Leu Val Lys Gly Phe Tyr Pro
       130                 135                 140
   Ser Asp Ile Ala Val Glu Trp Glu Ser Asn Gly Gln Pro Glu Asn Asn
   145                 150                 155                 160
   Tyr Lys Thr Thr Pro Pro Val Leu Asp Ser Asp Gly Ser Phe Phe Leu
                   165                 170                 175
   Tyr Ser Lys Leu Thr Val Asp Lys Ser Arg Trp Gln Gln Gly Asn Val
               180                 185                 190
   Phe Ser Cys Ser Val Met His Glu Ala Leu His Asn His Tyr Thr Gln
           195                 200                 205
   Lys Ser Leu Ser Leu Ser Pro Gly Lys
       210                 215
<210> 48
   <211> 16
   <212> PRT
   <213> Artificial Sequence
<220>
   <223> Description of Artificial Sequence: Synthetic Hinge linker peptide
<400> 48
   Gly Gly Gly Gly Ser Val Asp Lys Thr His Thr Cys Pro Pro Cys Pro
   1 5 10 15
<210> 49
   <211> 16
   <212> PRT
   <213> Artificial Sequence
<220>
   <223> Description of Artificial Sequence: Synthetic Hinge linker peptide
<400> 49
   Gly Gly Gly Gly Ser Val Asp Lys Thr His Thr Gly Pro Pro Cys Pro
   1 5 10 15
<210> 50
   <211> 21
   <212> PRT
   <213> Artificial Sequence
<220>
   <223> Description of Artificial Sequence: Synthetic Hinge linker peptide
<400> 50
   Gly Gly Gly Gly Ser Gly Gly Gly Gly Ser Val Asp Lys Thr His Thr
   1 5 10 15
   Gly Pro Pro Cys Pro
   20
<210> 51
   <211> 5
   <212> PRT
   <213> Artificial Sequence
<220>
   <223> Description of Artificial Sequence: Synthetic peptide
<400> 51
   Gly Gly Gly Gly Gly
   1 5
<210> 52
   <211> 8
   <212> PRT
   <213> Artificial Sequence
<220>
   <223> Description of Artificial Sequence: Synthetic peptide
<400> 52
   Gly Gly Gly Gly Gly Gly Gly Gly
   1               5
<210> 53
   <211> 300
   <212> DNA
   <213> Homo sapiens
<400> 53
   gagacacggt ggtgcatcta ctacaacgcc aactgggagc tggagcgcac caaccagacc      60
   ggcctggagc gctgcgaagg cgagcaggac aagcggctgc actgctacgc ctcctggcgc     120
   aacagctctg gcaccatcga gctcgtgaag aagggctgct ggctagatga cttcaactgc     180
   tacgataggc aggagtgtgt ggccactgag gagaaccccc aggtgtactt ctgctgctgt     240
   gagggcaact tctgcaacga gcgcttcact catttgccag aggctggggg cccggaagtc     300

## Description

### BACKGROUND

The transforming growth factor β (TGF-β) family of proteins includes the transforming growth factors-β (TGF-β), activins, bone morphogenic proteins (BMP), nerve growth factors (NGFs), brain-derived neurotrophic factor (BDNF), and growth/ differentiation factors (GDFs). These family members are involved in the regulation of a wide range of biological processes including cell proliferation, differentiation, and other functions.

Growth/differentiation factor 8 (GDF-8), also referred to as myostatin, is a TGF-β family member expressed for the most part in the cells of developing and adult skeletal muscle tissue. Myostatin appears to play an essential role in negatively controlling skeletal muscle growth (McPherron et al., Nature (London) 387, 83-90 (1997), Zimmers et al., Science 296:1486-1488 (2002)). Antagonizing myostatin has been shown to increase lean muscle mass in animals.

Another member of the TGF-β family of proteins is a related growth/differentiation factor, growth/differentiation factor 11 (GDF-11). GDF-11 has approximately 90 % sequence identity to the amino acid sequence of myostatin. GDF-11 has a role in the axial patterning in developing animals (Oh et al., Genes Dev 11:1812-26 (1997)), and also appears to play a role in skeletal muscle development and growth.

Activins A, B and AB are the homodimers and heterodimer respectively of two polypeptide chains, βA and βB (Vale et al., Nature 321, 776-779 (1986), Ling et al., Nature 321, 779-782 (1986)). Activins were originally discovered as gonadal peptides involved in the regulation of follicle stimulating hormone synthesis, and are now believed to be involved in the regulation of a number of biological activities. Activin A is a predominant form of activin.

Activin, myostatin, GDF-11 and other members of the TGF-β superfamily bind and signal through a combination of activin type II and activin type IIB receptors, both of which are transmembrane serine/threonine kinases (Harrison et al., J. Biol. Chem. 279, 28036-28044 (2004)). Cross-linking studies have determined that myostatin is capable of binding the activin type II receptors ActRIIA and ActRIIB *in vitro* (Lee et al., PNAS USA 98:9306-11 (2001)). There is also evidence that GDF-11 binds to both ActRIIA and ActRIIB (Oh et al., Genes Dev 16:2749-54 (2002)).

TGF-β protein expression is known to be associated with a variety of diseases and disorders. Therefore, therapeutic molecules capable of antagonizing several TGF-β proteins simultaneously may be particularly effective for treating these diseases and disorders.

Related applications include: U.S. Serial No. 12/626,375, filed November 25, 2009, U.S. Serial No. 12/074,877, filed March 5, 2008, U.S. Serial No. 13/329,897, filed December 19, 2011, U.S. Serial No. 11/590,962, filed October 31, 2006, PCT/US2014/014490, filed February 3, 2014, and PCT/US2015/011396, filed January 14, 2015.

### SUMMARY

The invention provides a composition comprising a solution of a protein, a sucrose excipient, a potassium phosphate buffer, and a polysorbate 20 surfactant, wherein the composition comprises a pH of 4-12, wherein the protein comprises a polypeptide capable of binding at least one of myostatin, activin, or GDF-11, optionally wherein the polypeptide is selected from the group consisting of: (a) a polypeptide consisting of the amino acid sequence set forth in the group consisting of SEQ ID NO: 4, 6, 12, and 14; (b) a polypeptide having at least 90% sequence identity to (a), and the polypeptide has a W or a Y at the position corresponding to position 28 of the sequence set forth in SEQ ID NO:2 and a T at the position corresponding to position 44 of the sequence set forth in SEQ ID NO:2, (c) a polypeptide having at least 95% sequence identity to (a), wherein the polypeptide has a W or a Y at the position corresponding to position 28 of the sequence set forth in SEQ ID NO:2 and a T at the position corresponding to position 44 of the sequence set forth in SEQ ID NO:2; and wherein the protein in the composition retains at least 80% stability for a time period of greater than 1 month in solution when kept at 2-8°C or 5°C relative to the protein in the composition at the beginning of the time period (0 months) or relative to an identical protein kept under otherwise identical conditions for greater than 1 month at -20°C or -70°C; and, optionally wherein the composition further comprises a chemotherapeutic agent or a second therapeutic agent.

Also provided is a composition of the invention for use in a method of inhibiting a solid tumor growth in a subject or treating a solid tumor in a subject or inhibiting, reducing, or treating cachexia in a subject, comprising administering a dose of the composition of the invention to the subject.

Also provided is a composition of the invention for use in a method of inhibiting, reducing, or treating cachexia in a subject, comprising administering a fixed dose ranging from at least 0.1 mg/kg to 20 mg/kg of the composition of the invention to the subject.

Also disclosed herein is a method of treating obesity or a disease associated with obesity, increasing or maintaining muscle mass, or decreasing fat mass in a subject, comprising: administering to the subject an effective dose of a protein that inhibits at least one of activin, myostatin, and GDF-11, optionally wherein the disease associated with obesity is at least one of a genetic obesity syndrome, Prader willi syndrome, a hypothalamic disorder, familial hypercholesterolemia, Bardet-Biedl syndrome, Prader-Willi syndrome, a syndrome resulting from a loss of imprinted genes on 15q11-13, Alstrom syndrome, Cohen syndrome, Albright's hereditary osteodystrophy (pseudohypoparathyroidism), Carpenter syndrome, MOMO syndrome, Rubinstein-Taybi syndrome, a syndrome resulting from deletions of at least one of 6q16, 1p36, 2q37, and 9q34, maternal uniparental disomy of chromosome 14, fragile X syndrome, atherosclerosis, non-alcoholic steatohepatitis, a disease where visceral fat deposition results in one or more deleterious outcomes, cerebrovascular disease, fatty liver, and Börjeson-Forssman-Lehman syndrome, and optionally wherein the polypeptide is selected from the group consisting of: (a) a polypeptide consisting of the amino acid sequence set forth in the group consisting of SEQ ID NO: 4, 6, 12, and 14; (b) a polypeptide having at least 90% sequence identity to (a), and the polypeptide has a W or a Y at the position corresponding to position 28 of the sequence set forth in SEQ ID NO:2 and a T at the position corresponding to position 44 of the sequence set forth in SEQ ID NO:2, (c) a polypeptide having at least 95% sequence identity to (a), wherein the polypeptide has a W or a Y at the position corresponding to position 28 of the sequence set forth in SEQ ID NO:2 and a T at the position corresponding to position 44 of the sequence set forth in SEQ ID NO:2, and (d) a polypeptide having a sequence with at least 80 %, 85 %, 90 %, 95 %, 96 %, 97 %, 98 %, or 99 % identity to the sequence set forth in amino acids 19-25, 19, 20, 21, 22, 23, 24, or 25 through 130-134, 125, 126, 127, 128, 129, 130, 131, 132, 133, or 134 of SEQ ID NO: 2.

Also provided herein is a method of producing the composition of the invention, wherein the protein comprises a polypeptide capable of binding at least one of myostatin, activin, or GDF-11, wherein the polypeptide is selected from the group consisting of: (a) a polypeptide consisting of the amino acid sequence set forth in the group consisting of SEQ ID NO: 4, 6, 12, and 14; (b) a polypeptide having at least 90% sequence identity to (a), and the polypeptide has a W or a Y at the position corresponding to position 28 of the sequence set forth in SEQ ID NO:2 and a T at the position corresponding to position 44 of the sequence set forth in SEQ ID NO:2, (c) a polypeptide having at least 95% sequence identity to (a), wherein the polypeptide has a W or a Y at the position corresponding to position 28 of the sequence set forth in SEQ ID NO:2 and a T at the position corresponding to position 44 of the sequence set forth in SEQ ID NO:2, wherein the method comprises: culturing a CS9 Chinese hamster ovary cell line engineered to express the protein in a cell culture; harvesting the protein from the culture; and purifying the protein.

### BRIEF DESCRIPTION OF THE SEVERAL VIEWS OF THE DRAWINGS

These and other features, aspects, and advantages will become better understood with regard to the following description, and accompanying drawings, where:
Figure 1 shows an overview of the cell culture process for making STM 434.
Figure 2 shows an overview of the purification process for STM 434.
Figure 3 shows a flow diagram for preparing and packaging STM 434.
Figure 4 shows that FSH levels decreased in 2 of 3 subjects following administration of STM 434 dosed at 0.25 mg/kg.
Figure 5 shows an interim PK analysis for STM 434 in humans.
Figure 6 shows the baseline scan with the tumor indicated in the circle.
Figure 7 shows the follow-on scan taken at the beginning of cycle 4, with the tumor again indicated in the circle.

### DETAILED DESCRIPTION

### Compositions

Described herein is an isolated protein comprising a stabilized human activin IIB receptor (svActRIIB) polypeptide and related formulations. Proteins and polypeptides can be characterized by their ability to bind to at least one of three TGF-β proteins, myostatin (GDF-8), activin A, or GDF-11, to inhibit the activities of at least one of these proteins, and, optionally, to have improved manufacturability properties compared with other ActRIIB soluble receptors. The stabilized human activin IIB receptor polypeptide can be characterized by amino acid substitutions at both positions E28 and S44 with reference to the extracellular domain of ActRIIB, as set forth in SEQ ID NO: 2. In one embodiment, a stabilized human activin IIB receptor polypeptide can have a further substitution of alanine at position 64 with respect to SEQ ID NO: 2. In some aspects, a protein is an antibody, an ActRIIB-based protein, and/or an Fc-Fusion protein. In some aspects, an Fc-Fusion protein is an ActRIIB Fc-Fusion protein. In some aspects, an ActRIIB protein comprises a sequence with at least 80 %, 85 %, 90 %, 95 %, 96 %, 97 %, 98 %, or 99 % identity to the sequence set forth in amino acids 19-25, 19, 20, 21, 22, 23, 24, or 25 through 130-134, 125, 126, 127, 128, 129, 130, 131, 132, 133, or 134 of SEQ ID NO: 2, optionally wherein the Fc is IgG, optionally wherein IgG is human IgG. In some aspects, an ActRIIB protein comprises or consists of the sequence set forth in SEQ ID NO:6 or SEQ ID NO:10. In some aspects, an ActRIIB protein comprises a sequence with at least 80 %, 85 %, 90 %, 95 %, 96 %, 97 %, 98 %, or 99 % identity to the sequence set forth in SEQ ID NO: 6. In some aspects, an ActRIIB protein comprises a sequence the differs by less than 1-2, 1, 2, 3, 4, or 5 amino acids from the sequence set forth in SEQ ID NO:6. In some aspects, a protein inhibits at least two of activin, myostatin, and GDF-11. In some aspects, a protein inhibits activin, myostatin, and GDF-11. In some aspects, a protein inhibits activin.

As used herein the term "TGF-β family members" or "TGF-β proteins" refers to the structurally related growth factors of the transforming growth factor family including activins, and growth and differentiation factor (GDF) proteins (Kingsley et al. Genes Dev. 8: 133-146 (1994), McPherron et al., Growth factors and cytokines in health and disease, Vol. 1B, D. LeRoith and C. Bondy. ed., JAI Press Inc., Greenwich, Conn, USA: pp 357-393).

GDF-8, also referred to as myostatin, is a negative regulator of skeletal muscle tissue (McPherron et al. PNAS USA 94:12457-12461 (1997)). Myostatin is synthesized as an inactive protein approximately 375 amino acids in length, having GenBank Accession No: AAB86694 for human. The precursor protein is activated by proteolytic cleavage at a tetrabasic processing site to produce an N-terminal inactive prodomain and an approximately 109 amino acid C-terminal protein which dimerizes to form a homodimer of about 25 kDa. This homodimer is the mature, biologically active protein (Zimmers et al., Science 296, 1486 (2002)).

As used herein, the term "prodomain" or "propeptide" refers to the inactive N-terminal protein which is cleaved off to release the active C-terminal protein. As used herein the term "myostatin" or "mature myostatin" refers to the mature, biologically active C-terminal polypeptide, in monomer, dimer or other form, as well as biologically active fragments or related polypeptides including allelic variants, splice variants, and fusion peptides and polypeptides. The mature myostatin has been reported to have 100% sequence identity among many species including human, mouse, chicken, porcine, turkey, and rat (Lee et al., PNAS 98, 9306 (2001)).

As used herein GDF-11 refers to the BMP (bone morphogenic protein) having Swissprot accession number O95390, as well as variants and species homologs of that protein. GDF-11 is involved in the regulation of anterior/posterior patterning of the axial skeleton (McPherron et al, Nature Genet. 22 (93): 260-264 (1999); Gamer et al, Dev. Biol. 208 (1), 222-232 (1999)) but postnatal functions are unknown.

Activin A is the homodimer of the polypeptide chains βA. As used herein the term "activin A" refers to the activin protein having GenBank Accession No: NM_002192. Activins A, B, and AB are the homodimers and heterodimer respectively of two polypeptide chains, βA and βB. As used herein, "activin" refers to activin A, B, and AB, as well as variants and species homologs of that protein.

### Receptor Polypeptides

As used herein, the term activin type II B receptors (ActRIIB) refers to human activin receptors having accession number NP_001097 or variants thereof, such as those having arginine at position 64 substituted with alanine. The term soluble ActRIIB (wild type) refers to the extracellular domain of ActRIIB, e.g., amino acids 1 to 134 (with signal sequence), or amino acids 19 through 134 of SEQ ID NO: 2 (without signal sequence), or amino acids 20, 21, 22, 23, 24, or 25 through 134 of SEQ ID NO: 2 (without signal sequence).

### Stabilized receptor polypeptides

Also disclosed herein is an isolated protein comprising a stabilized ActIIB receptor polypeptide (referred to herein as "svActRIIB polypeptide"). As used herein the term "svActRIIB protein" refers to a protein comprising a stabilized ActRIIB polypeptide. These polypeptides and proteins are characterized as having the ability to bind and inhibit the activity of any one of activin A, myostatin, or GDF-11, in addition to having improved manufacturability characteristics.

The stabilized ActRIIB polypeptide can be characterized by having an amino acid substitution at both position 28 and 44 with respect to SEQ ID NO: 2. For consistency, the amino acid positions on the stabilized ActRIIB polypeptides and proteins are referred to with respect to the positions in SEQ ID NO: 2, regardless of whether the polypeptide is mature or truncated. As used herein, the term "mature" refers to a polypeptide or peptide without its signal sequence. As used herein, the term "truncated" refers to polypeptides having N terminal amino acids or C terminal amino acids removed.

In one aspect, the isolated stabilized activin IIB receptor polypeptide (svActRIIB) has the polypeptide sequence set forth in SEQ ID NO: 2. In another aspect, the polypeptide has the sequence set forth in amino acids 19 through 134 of SEQ ID NO: 2. In another aspect, the polypeptide has the sequence set forth in amino acids 23 through 134 of SEQ ID NO: 2. In another aspect, the polypeptide has the sequence set forth in amino acids 25 through 134 of SEQ ID NO: 2. In another aspect, the polypeptide has an amino acid sequence with at least 80 %, 85 %, 90 %, 95 %, 96 %, 97 %, 98 % or 99 % identity to any one of the polypeptides above. In one aspect, the polypeptide is capable of binding myostatin, activin A, or GDF-11. In one aspect, the isolated stabilized activin IIB receptor polypeptide (svActRIIB) comprises a sequence with at least 80 %, 85 %, 90 %, 95 %, 96 %, 97 %, 98 % or 99 % identity to the sequence set forth in amino acids 19-24, 19, 20, 21, 22, 23, 24, or 25 through 130-134, 125, 126, 127, 128, 129, 130, 131, 132, 133, or 134 of SEQ ID NO: 2.

In one aspect, the isolated stabilized activin IIB receptor polypeptide (svActRIIB) has the polypeptide sequence set forth in SEQ ID NO: 2, except for an amino acid substitution at position 28, and an amino acid substitution at position 44, wherein the substitution at position 28 is selected from W or Y, and the substitution at position 44 is T. In another aspect, the polypeptide has the sequence set forth in amino acids 19 through 134 of SEQ ID NO: 2, except for an amino acid substitution at position 28, and an amino acid substitution at position 44, wherein the substitution at position 28 is selected from W or Y, and the substitution at position 44 is T. In another aspect, the polypeptide has the sequence set forth in amino acids 23 through 134 of SEQ ID NO: 2, except for an amino acid substitution at position 28, and an amino acid substitution at position 44, wherein the substitution at position 28 is selected from W or Y, and the substitution at position 44 is T. In another aspect, the polypeptide has the sequence set forth in amino acids 25 through 134 of SEQ ID NO: 2, except for an amino acid substitution at position 28, and an amino acid substitution at position 44, wherein the substitution at position 28 is selected from W or Y, and the substitution at position 44 is T. In another aspect, the polypeptide has an amino acid sequence with at least 80 %, 85 %, 90 %, 95 %, 96 %, 97 %, 98 % or 99 % identity to any one of the polypeptides above, wherein the polypeptide has an amino acid substitution at position 28, and an amino acid substitution at position 44, optionally wherein the substitution at position 28 is selected from W or Y, and the substitution at position 44 is T, and wherein the polypeptide is capable of binding myostatin, activin A, or GDF-11. In one aspect, the substitution of the above polypeptides at position 28 is W, and the substitution at position 44 is T, wherein the polypeptide is capable of binding myostatin, activin A, or GDF-11.

In one embodiment, svActRIIB polypeptide includes a signal sequence, for example, the sequences shown in SEQ ID NO: 4, 8, 12, and 16. However, various signal peptides can be used in the preparation of the polypeptides of the instant application. The signal peptides can have the sequence set forth in amino acids 1 to 19 of SEQ ID NO: 4, for example. Any other signal peptides useful for expressing svActRIIB polypeptides can be used. In other embodiments, the signal sequence is removed, leaving the mature peptide. Examples of svActRIIB polypeptides lacking a signal sequence includes, for example, the sequences shown in SEQ ID NO: 6, 10, 14 and 18.

In one embodiment, the protein comprises a stabilized activin IIB receptor polypeptide, wherein the polypeptide is selected from the group consisting of polypeptides having the sequence set forth in the group consisting of SEQ ID NO: 4, 6, 12 and 14. These polypeptides represent amino acids 25 to 134 of SEQ ID NO: 2, wherein the polypeptide has an amino acid substitution at position 28, and an amino acid substitution at position 44, wherein the substitution at position 28 is selected from W or Y, and the substitution at position 44 is T, and wherein the polypeptide is capable of binding myostatin, activin A, or GDF-11, with and without a signal sequence different from that shown in SEQ ID NO: 2. In another aspect the protein comprises a polypeptide having at least 80-100%, 90-100%, 85-95%, 90-95%, 80 %, 85 %, 90 %, 95 %, 96 %, 97 %, 98 %, or 99 % sequence identity to SEQ ID NO: 4, 6, 12 or 14, optionally wherein the polypeptide has a W or Y at position 28 and a T at position 44, and wherein the polypeptide is capable of binding myostatin, activin A, or GDF-11. In one embodiment, the substitution at position 28 is W and the substitution at position 44 is T, wherein the polypeptide is capable of binding myostatin, activin A or GDF-11.

In a further embodiment svActRIIB protein further comprises a heterologous protein. In one embodiment, the heterologous protein is an Fc domain. In a further embodiment, the Fc domain is a human IgG Fc domain. In one embodiment, the protein comprises a polypeptide having the sequence set forth in the group consisting of SEQ ID NO: 8, 10, 16 and 18. In another aspect, the protein comprises a polypeptide having at least 80-100%, 90-100%, 85-95%, 90-95%, 80 %, 85 %, 90 %, 95 %, 96 %, 97 %, 98 %, or 99 % sequence identity to the sequence shown in SEQ ID NO: 8, 10, 16 or 18, optionally wherein the polypeptide has a W or Y at position 28 and a T at position 44, and wherein the polypeptide is capable of binding myostatin, activin A, or GDF-11. In one embodiment, the substitution at position 28 is W and the substitution at position 44 is T, wherein the polypeptide is capable of binding myostatin, activin A or GDF-11. In certain aspects, the protein comprises or consists of the sequence shown in SEQ ID NO:10. In certain aspects, the sequence is glycosylated.

In a further embodiment svActRIIB protein is STM 434, described in the Examples.

In a further aspect, the protein comprises any one of the polypeptides described above, wherein the amino acid residue at position 64 is alanine.

In another embodiment, the term svActRIIB polypeptide and protein encompasses proteins comprising fragments of SEQ ID NO: 2, 4, 6,12 and 14, including N and C terminal truncations, wherein position 28 is W or Y, and position 44 is T, and wherein the polypeptide is capable of binding myostatin, activin A or GDF-11.

As used herein the term "derivative" of a svActRIIB polypeptide refers to the attachment of at least one additional chemical moiety, or at least one additional polypeptide to form covalent or aggregate conjugates such as glycosyl groups, lipids, acetyl groups, or C-terminal or N-terminal fusion polypeptides, conjugation to PEG molecules, and other modifications which are described more fully below. Stabilized ActRIIB receptor polypeptides can also include additional modifications and derivatives, including modifications to the C and N termini which arise from processing due to expression in various cell types such as mammalian cells, E. coli, yeasts and other recombinant host cells.

svActRIIB proteins can further comprise heterologous polypeptides attached to svActRIIB polypeptide either directly or through a linker to form a fusion protein. As used herein the term "fusion protein" refers to a protein having a heterologous polypeptide attached to another polypeptide such as an svActRIIB. Heterologous polypeptides include but are not limited to Fc polypeptides, his tags, and leucine zipper domains to promote oligomerization and further stabilization of the stabilized ActRIIB polypeptides as described in, for example, WO 00/29581. In one embodiment, the heterologous polypeptide is an Fc polypeptide or domain. In one embodiment, the Fc domain is selected from a human IgG1 Fc (SEQ ID NO: 23), a modified IgG1 Fc, IgG2 Fc (SEQ ID NO: 22), and IgG4 Fc (SEQ ID NO: 24) domain. SvActRIIB protein can further comprise all or a portion of the hinge sequence of the IgG1, IgG2, or IgG4. Exemplary svActRIIB polypeptides are selected from polypeptides consisting of the sequences as set forth in SEQ ID NO: 8, 10, 16 and 18, as well as those polypeptides having substantial similarity to these sequences, wherein the substitutions at positions 28 and 44 are retained. As used herein, "substantial similarity" refers to sequences that are at least 80-100%, 90-100%, 85-95%, 90-95%, 80 % identical, 85 % identical, 90 % identical, 95 % identical, 96 % identical, 97 % identical, 98 % identical, 99 % identical to any of SEQ ID NO: 8, 10, 16, and 18, wherein the polypeptides retain W or Y at position 28 and T at position 44, and wherein the polypeptide is capable of binding myostatin, activin A or GDF-11. In one embodiment, the substitution at position 28 is W and the substitution at position 44 is T, wherein the polypeptide is capable of binding myostatin, activin A or GDF-11.

svActRIIB polypeptides can optionally further comprise a "linker". Linkers serve primarily as a spacer between a polypeptide and a second heterologous polypeptide or other type of fusion or between two or more stabilized ActRIIB polypeptides. In one embodiment, a linker is made up of amino acids linked together by peptide bonds, preferably from 1 to 20 amino acids linked by peptide bonds, wherein the amino acids are selected from the 20 naturally occurring amino acids. One or more of these amino acids can be glycosylated, as is understood by those of skill in the art. In one embodiment, the 1 to 20 amino acids can be selected from glycine, alanine, proline, asparagine, glutamine, and lysine. In one embodiment, a linker is made up of a majority of amino acids that are sterically unhindered, such as glycine and alanine. Exemplary linkers are polyglycines, particularly (Gly)s (SEQ ID NO: 51), (Gly)₈ (SEQ ID NO: 52), poly(Gly-Ala), and polyalanines. One exemplary suitable linker is (Gly)₄Ser (SEQ ID NO: 25). In a further embodiment, svActRIIB can comprise a "hinge linker", that is a linker sequence provided adjacent to a hinge region or a partial hinge region of an IgG, as exemplified in SEQ ID NO: 27. Hinge sequences include IgG2Fc, IgG1Fc, and IgG4Fc.

Hinge linker sequences can also be designed to improve manufacturability and stability of svActRIIB-Fc proteins. In one embodiment, the hinge linkers of SEQ ID NO: 27, 38, 40, 42, 44, 45, and 46 are designed to improve manufacturability with the IgG2 Fc (SEQ ID NO: 22) when attached to svActRIIB polypeptides. In one embodiment, the hinge linker sequences is designed to improve manufacturability when attaching svActRIIB polypeptides to a human IgG1 Fc (SEQ ID NO: 23) or a modified human IgG1 Fc.

Linkers can also be non-peptide linkers. For example, alkyl linkers such as -NH-(CH₂)s-C(O)-, wherein s = 2-20 can be used. These alkyl linkers may further be substituted by any non-sterically hindering group such as lower alkyl (e.g., C₁-C₆) lower acyl, halogen (e.g., Cl, Br), CN, NH2, phenyl, etc.

svActRIIB polypeptides disclosed herein can also be attached to a non-polypeptide molecule for the purpose of conferring desired properties such as reducing degradation and/or increasing half-life, reducing toxicity, reducing immunogenicity, and/or increasing the biological activity of svActRIIB polypeptides. Exemplary molecules include but are not limited to linear polymers such as polyethylene glycol (PEG), polylysine, a dextran; a lipid; a cholesterol group (such as a steroid); a carbohydrate, or an oligosaccharide molecule.

svActRIIB proteins and polypeptides can have improved manufacturability properties when compared to other ActRIIB soluble polypeptides. As used herein, the term "manufacturability" refers to the stability of a particular protein during recombinant expression and purification of that protein. Manufacturability is believed to be due to the intrinsic properties of the molecule under conditions of expression and purification. Examples of improved manufacturability characteristics include uniform glycosylation of a protein, increased cell titer, growth and protein expression during recombinant production of the protein, improved purification properties, and improved stability at low pH. svActRIIB proteins and polypeptides demonstrate the improved manufacturability, along with retention of *in vitro* and *in vivo* activity, compared with other soluble ActRIIB polypeptides. Further, additional hinge linker sequences can confer additional manufacturability benefits.

As used herein, the term a "svActRIIB polypeptide activity" or "a biological activity of a soluble ActRIIB polypeptide" refers to one or more *in vitro* or *in vivo* activities of svActRIIB polypeptides. Activities of svActRIIB polypeptides include, but are not limited to, the ability to bind to myostatin or activin A or GDF-11, and the ability to inhibit or neutralize an activity of myostatin or activin A or GDF-11. As used herein, the term "capable of binding" to myostatin, activin A, or GDF-11 refers to binding measured by methods known in the art, such as the KinExA™ method. *In vitro* inhibition of myostatin, activin A, or GDF-11 can be measured using, for example, the pMARE C2C12 cell-based assay. *In vivo* activity, is demonstrated by increased lean muscle mass in mouse models. *In vivo* activities of svActRIIB polypeptides and proteins include but are not limited to increasing body weight, increasing lean muscle mass, and increasing the ratio of lean muscle to fat mass. Therapeutic activities further include reducing or preventing cachexia caused by certain types of tumors, preventing the growth of certain types of tumors, and increasing survival of certain animal models. Further discussion of svActRIIB protein and polypeptide activities is provided below.

In another aspect, an isolated nucleic acid molecule comprising a polynucleotide encoding an svActRIIB polypeptide is disclosed.

In one aspect, the polynucleotide encodes a polypeptide having the sequence set forth in SEQ ID NO: 2, except for an amino acid substitution at position 28, and an amino acid substitution at position 44, wherein the substitution at position 28 is selected from W or Y, and the substitution at position 44 is T. In another aspect, the polynucleotide encodes a polypeptide having the sequence set forth in amino acids 19 through 134 of SEQ ID NO: 2, except for an amino acid substitution at position 28, and an amino acid substitution at position 44, wherein the substitution at position 28 is selected from W or Y, and the substitution at position 44 is T. In another aspect, the polynucleotide encodes a polypeptide having the sequence set forth in amino acids 23 through 134 of SEQ ID NO: 2, except for an amino acid substitution at position 28, and an amino acid substitution at position 44, wherein the substitution at position 28 is selected from W or Y, and the substitution at position 44 is T. In another aspect, the polynucleotide encodes a polypeptide having the sequence set forth in amino acids 25 through 134 of SEQ ID NO: 2, except for an amino acid substitution at position 28, and an amino acid substitution at position 44, wherein the substitution at position 28 is selected from W or Y, and the substitution at position 44 is T. In another aspect, the polynucleotide encodes the a polypeptide having an amino acid sequence at least 80-100%, 90-100%, 85-95%, 90-95%, 80 %, 85 %, 90 %, 95 %, 98 % or 99 % identity to any one of the polypeptides above, wherein the polypeptide has single amino acid substitution at position 28, and an amino acid substitution at position 44, optionally wherein the substitution at position 28 is selected from W or Y, and the substitution at position 44 is T, and wherein the polypeptide is capable of binding myostatin, activin A, or GDF-11. In one aspect, the polynucleotide of the above aspects encodes a polypeptide wherein the substitution at position 28 is W and the substitution at position 44 is T.

In one aspect, the isolated nucleic acid molecule comprises a polynucleotide encoding a polypeptide having the sequence set forth in the group consisting of SEQ ID NO: 4, 6, 12, and 14. In another aspect, the nucleic acid comprises a polynucleotide encoding a polypeptide having at least 80-100%, 90-100%, 85-95%, 90-95%, 80 %, 90 %, 95 %, 96 %, 97 %, 98 %, or 99 % sequence identity to SEQ ID NO: 4, 6, 12 or 14, optionally wherein the polypeptide has a W or Y at position 28 and a T at position 44, and wherein the polypeptide is capable of binding activin A, GDF-11, or myostatin. In one aspect, the polynucleotide of the above aspects encodes a polypeptide wherein the substitution at position 28 is W and the substitution at position 44 is T, and wherein the polypeptide is capable of binding activin A, GDF-11 or myostatin.

In another aspect, an isolated nucleic acid molecule further comprises a polynucleotide encoding at least one heterologous protein. In one aspect, the heterologous protein is an Fc domain, in a further aspect, the Fc domain is a human IgG Fc domain. In another aspect, the nucleic acid molecule further comprises polynucleotides encoding linkers and hinge linkers set forth in, e.g., SEQ ID NO: 25, 27.

In one aspect, the nucleic acid molecule comprises a polynucleotide encoding a polypeptide consisting of the sequence set forth in the group consisting of SEQ ID NO: 8, 10, 16 and 18. In another aspect, the nucleic acid comprises a polynucleotide encoding a polypeptide having at least 80-100%, 90-100%, 85-95%, 90-95%, 80 %, 90 %, 95 %, 96 %, 97 %, 98 %, 99 % sequence identity to the group consisting of SEQ ID NO: 8, 10, 16 and 18, optionally wherein the polypeptide has a W or Y at position 28 and a T at position 44, and wherein the polypeptide is capable of binding activin A, GDF-11, or myostatin. In one aspect, the polynucleotide of the above aspects encodes a polypeptide wherein the substitution at position 28 is W and the substitution at position 44 is T, and wherein the polypeptide is capable of binding myostatin, activin A or GDF-11.

In one aspect, the isolated nucleic acid molecule comprises a polynucleotide having the sequence selected from the group consisting of SEQ ID NO: 3, 5, 11 or 13, or its complement. In another aspect, the isolated nucleic acid molecule comprises a polynucleotide having the sequence selected from the group consisting of the sequence SEQ ID NO: 7, 9, 15 and 17, or its complement. In a further aspect the isolated nucleic acid molecule hybridizes under stringent or moderate conditions with SEQ ID NO: 3, 5, 7, 9, 11, 13, 15 or 17 wherein the encoded polypeptide is substantially similar to SEQ ID NO: 4, 6, 8, 10, 12, 14, 16, or 18, optionally wherein the polypeptide comprises an amino acid sequence having W or Y at position 28, and T at position 44, and wherein the encoded polypeptide is capable of binding or inhibiting activin A, myostatin or GDF-11.

Nucleic acid molecules include DNA in both single-stranded and double-stranded form, as well as the RNA complement thereof. DNA includes, for example, cDNA, genomic DNA, synthetic DNA, DNA amplified by PCR, and combinations thereof. Genomic DNA may be isolated by conventional techniques, such as by using the DNA of SEQ ID NO: 3, 5, 11 or 13, or a suitable fragment thereof, as a probe. Genomic DNA encoding ActRIIB polypeptides is obtained from genomic libraries which are available for a number of species. Synthetic DNA is available from chemical synthesis of overlapping oligonucleotide fragments followed by assembly of the fragments to reconstitute part or all of the coding regions and flanking sequences. RNA may be obtained from procaryotic expression vectors which direct high-level synthesis of mRNA, such as vectors using T7 promoters and RNA polymerase. cDNA is obtained from libraries prepared from mRNA isolated from various tissues that express ActRIIB. The DNA molecules include full length genes as well as polynucleotides and fragments thereof. The full length gene may also include sequences encoding the N-terminal signal sequence.

Also disclosed are the nucleic acid molecule describe above, wherein the polynucleotide is operably linked to a transcriptional or translational regulatory sequence.

In another aspect expression vectors containing the nucleic acid molecules and polynucleotides are also provided, and host cells transformed with such vectors, and methods of producing svActRIIB polypeptides are also disclosed. The term "expression vector" refers to a plasmid, phage, virus or vector for expressing a polypeptide from a polynucleotide sequence. Vectors for the expression of svActRIIB polypeptides contain at a minimum sequences required for vector propagation and for expression of the cloned insert. An expression vector comprises a transcriptional unit comprising an assembly of (1) a genetic element or elements having a regulatory role in gene expression, for example, promoters or enhancers, (2) a sequence that encodes svActRIIB polypeptides and proteins to be transcribed into mRNA and translated into protein, and (3) appropriate transcription initiation and termination sequences. These sequences may further include a selection marker. Vectors suitable for expression in host cells are readily available and the nucleic acid molecules are inserted into the vectors using standard recombinant DNA techniques. Such vectors can include promoters which function in specific tissues, and viral vectors for the expression of svActRIIB polypeptides in targeted human or animal cells. An exemplary expression vector suitable for expression of svActRIIB is the pDSRa, (described in WO 90/14363) and its derivatives, containing svActRIIB polynucleotides, as well as any additional suitable vectors known in the art or described below.

### Polypeptides

In some embodiments, compositions disclosed herein include a polypeptide that is less than 100% identical to an amino acid sequence disclosed herein. In some aspects, the polypeptide is 80%, 85%, 90%, 91%, 92%, 93%, 94%, 95%, 96%, 97%, 98%, 99%, or between 99 and 100% identical to a sequence disclosed herein.

The term "percent identical" in the context of two or more amino acid or nucleic acid sequences, refer to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection. Depending on the application, the percent identity can exist over a region of the sequence being compared, e.g., over a functional domain, or, alternatively, exist over the full length of the two sequences to be compared.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Ausubel et al., infra).

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information (www.ncbi.nlm.nih.gov/).

### Variants

The compositions described herein also encompass variants of the polypeptides described herein. As used herein, the term "variants" refers to polypeptides having one or more amino acid residues inserted, deleted or substituted into the original amino acid sequence and which retain at least a portion of the function of the polypeptide described herein. As used herein, fragments of the polypeptides are included within the definition of "variants". It is understood that any given peptide or peptibody may contain one or two or all three types of variants. Insertional and substitutional variants may contain natural amino acids, as well as non-naturally occurring amino acids or both. Variants can include, e.g., polypeptides that include a leader or signal sequence; polypeptides with additional amino terminal residues, e.g., Met1 or Lys 2; polypeptides with expression tags, e.g., histidine tags; and polypeptides expressed as fusion proteins.

Variants of the polypeptides described herein can include amino acid substitutions. Stereoisomers (e.g., D-amino acids) of the twenty conventional (naturally occurring) amino acids, non-naturally occurring amino acids such as α-, α-disubstituted amino acids, N-alkyl amino acids, lactic acid, and other unconventional amino acids may also be suitable components for polypeptides of the present invention. Examples of non-naturally occurring amino acids include, for example: aminoadipic acid, beta-alanine, beta-aminopropionic acid, aminobutyric acid, piperidinic acid, aminocaprioic acid, aminoheptanoic acid, aminoisobutyric acid, aminopimelic acid, diaminobutyric acid, desmosine, diaminopimelic acid, diaminopropionic acid, N-ethylglycine, N-ethylaspargine, hyroxylysine, all0-hydroxylysine, hydroxyproline, isodesmosine, allo-isoleucine, N-methylglycine, sarcosine, N-methylisoleucine, N-methylvaline, norvaline, norleucine, orithine, 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and amino acids (e.g., 4-hydroxyproline).

Naturally occurring residues may be divided into (overlapping) classes based on common side chain properties:
1) neutral hydrophobic: Met, Ala, Val, Leu, Ile, Pro, Trp, Met, Phe;
2) neutral polar: Cys, Ser, Thr, Asn, Gln, Tyr, Gly;
3) acidic: Asp, Glu;
4) basic: His, Lys, Arg;
5) residues that influence chain orientation: Gly, Pro; and
6) aromatic: Trp, Tyr, Phe.

Substitution with naturally occurring amino acids can be conservative or non-conservative. Conservative amino acid substitutions involve exchanging a member of one of the above classes for another member of the same class. Conservative changes may encompass unconventional amino acid residues, which are typically incorporated by chemical peptide synthesis rather than by synthesis in biological systems. These include peptidomimetics and other reversed or inverted forms of amino acid moieties.

### Methods of Making

Also disclosed are methods of making svActRIIB polypeptides. A variety of other expression/host systems may be utilized. These systems include but are not limited to microorganisms such as bacteria transformed with recombinant bacteriophage, plasmid or cosmid DNA expression vectors; yeast transformed with yeast expression vectors; insect cell systems infected with virus expression vectors (e.g., baculovirus); plant cell systems transfected with virus expression vectors (e.g., cauliflower mosaic virus, CaMV; tobacco mosaic virus, TMV) or transformed with bacterial expression vectors (e.g., Ti or pBR322 plasmid); or animal cell systems. Mammalian cells useful in recombinant protein production include but are not limited to VERO cells, HeLa cells, Chinese hamster ovary (CHO) cell lines, or their derivatives such as Veggie CHO and related cell lines which grow in serum-free media (see Rasmussen et al., 1998, Cytotechnology 28:31) or CHO strain DX-B11, which is deficient in DHFR (see Urlaub et al., 1980, Proc. Natl. Acad. Sci. USA 77:4216-20) COS cells such as the COS-7 line of monkey kidney cells (ATCC CRL 1651) (see Gluzman et al., 1981, Cell 23:175), W138, BHK, HepG2, 3T3 (ATCC CCL 163), RIN, MDCK, A549, PC12, K562, L cells, C127 cells, BHK (ATCC CRL 10) cell lines, the CV1/EBNA cell line derived from the African green monkey kidney cell line CV1 (ATCC CCL 70) (see McMahan et al., 1991, EMBO J. 10:2821), human embryonic kidney cells such as 293, 293 EBNA or MSR 293, human epidermal A431 cells, human Colo205 cells, other transformed primate cell lines, normal diploid cells, cell strains derived from in vitro culture of primary tissue, primary explants, HL-60, U937, HaK or Jurkat cells. Mammalian expression allows for the production of secreted or soluble polypeptides which may be recovered from the growth medium.

Using an appropriate host-vector system, svActRIIB polypeptides are produced recombinantly by culturing a host cell transformed with an expression vector containing the nucleic acid molecules under conditions allowing for production. Transformed cells can be used for long-term, high-yield polypeptide production. Once such cells are transformed with vectors that contain selectable markers as well as the desired expression cassette, the cells can be allowed to grow in an enriched media before they are switched to selective media, for example. The selectable marker is designed to allow growth and recovery of cells that successfully express the introduced sequences. Resistant clumps of stably transformed cells can be proliferated using tissue culture techniques appropriate to the cell line employed. An overview of expression of recombinant proteins is found in Methods of Enzymology, v. 185, Goeddell, D.V., ed., Academic Press (1990).

In some cases, such as in expression using procaryotic systems, the expressed polypeptides may need to be "refolded" and oxidized into a proper tertiary structure and disulfide linkages generated in order to be biologically active. Refolding can be accomplished using a number of procedures well known in the art. Such methods include, for example, exposing the solubilized polypeptide to a pH usually above 7 in the presence of a chaotropic agent. The selection of chaotrope is similar to the choices used for inclusion body solubilization, however a chaotrope is typically used at a lower concentration. Exemplary chaotropic agents are guanidine and urea. In most cases, the refolding/oxidation solution will also contain a reducing agent plus its oxidized form in a specific ratio to generate a particular redox potential which allows for disulfide shuffling to occur for the formation of cysteine bridges. Some commonly used redox couples include cysteine/cystamine, glutathione/dithiobisGSH, cupric chloride, dithiothreitol DTT/dithiane DTT, and 2-mercaptoethanol (bME)/dithio-bME. In many instances, a co-solvent may be used to increase the efficiency of the refolding. Commonly used cosolvents include glycerol, polyethylene glycol of various molecular weights, and arginine.

In addition, the polypeptides can be synthesized in solution or on a solid support in accordance with conventional techniques. Various automatic synthesizers are commercially available and can be used in accordance with known protocols. See, for example, Stewart and Young, Solid Phase Peptide Synthesis, 2d.Ed., Pierce Chemical Co. (1984); Tam et al., J Am Chem Soc, 105:6442, (1983); Merrifield, Science 232:341-347 (1986); Barany and Merrifield, The Peptides, Gross and Meienhofer, eds, Academic Press, New York, 1-284; Barany et al., Int J Pep Protein Res, 30:705-739 (1987).

The polypeptides and proteins can be purified according to protein purification techniques are well known to those of skill in the art. These techniques involve, at one level, the crude fractionation of the proteinaceous and non-proteinaceous fractions. Having separated the peptide polypeptides from other proteins, the peptide or polypeptide of interest can be further purified using chromatographic and electrophoretic techniques to achieve partial or complete purification (or purification to homogeneity). The term "isolated polypeptide" or "purified polypeptide" as used herein, is intended to refer to a composition, isolatable from other components, wherein the polypeptide is purified to any degree relative to its naturally-obtainable state. A purified polypeptide therefore also refers to a polypeptide that is free from the environment in which it may naturally occur. Generally, "purified" will refer to a polypeptide composition that has been subjected to fractionation to remove various other components, and which composition substantially retains its expressed biological activity. Where the term "substantially purified" is used, this designation will refer to a peptide or polypeptide composition in which the polypeptide or peptide forms the major component of the composition, such as constituting about 50 %, about 60 %, about 70 %, about 80 %, about 85 %, or about 90 % or more of the proteins in the composition. The term isolated can include a synthesized component such as a polypeptide.

Various techniques suitable for use in purification will be well known to those of skill in the art. These include, for example, precipitation with ammonium sulphate, PEG, antibodies (immunoprecipitation) and the like or by heat denaturation, followed by centrifugation; chromatography such as affinity chromatography (Protein-A columns), ion exchange, gel filtration, reverse phase, hydroxylapatite, hydrophobic interaction chromatography, isoelectric focusing, gel electrophoresis, and combinations of these techniques. As is generally known in the art, it is believed that the order of conducting the various purification steps may be changed, or that certain steps may be omitted, and still result in a suitable method for the preparation of a substantially purified polypeptide. Exemplary purification steps are provided in the Examples below.

Various methods for quantifying the degree of purification of polypeptide will be known to those of skill in the art in light of the present disclosure. These include, for example, determining the specific binding activity of an active fraction, or assessing the amount of peptide or polypeptide within a fraction by SDS/PAGE analysis. A preferred method for assessing the purity of a polypeptide fraction is to calculate the binding activity of the fraction, to compare it to the binding activity of the initial extract, and to thus calculate the degree of purification, herein assessed by a "-fold purification number." The actual units used to represent the amount of binding activity will, of course, be dependent upon the particular assay technique chosen to follow the purification and whether or not the polypeptide or peptide exhibits a detectable binding activity.

### Methods of Treatment

Also disclosed are methods, proteins, and compositions for reducing or neutralizing the amount or activity of at least one of myostatin, activin A, or GDF-11 *in vivo* and *in vitro.* svActRIIB polypeptides have a high binding affinity for myostatin, activin A, and GDF-11, and are capable of reducing and inhibiting the biological activities of at least one of myostatin, activin A and GDF-11. In some aspects, a protein is an antibody or an Fc-Fusion protein. In some aspects, an Fc-Fusion protein is an ActRIIB Fc-Fusion protein. In some aspects, an ActRIIB Fc-Fusion protein comprises a sequence with at least 80 %, 85 %, 90 %, 95 %, 96 %, 97 %, 98 %, or 99 % identity to the sequence set forth in amino acids 19-25, 19, 20, 21, 22, 23, 24, or 25 through 130-134, 125, 126, 127, 128, 129, 130, 131, 132, 133, or 134 of SEQ ID NO: 2, optionally wherein the Fc is IgG, optionally wherein IgG is human IgG. In some aspects, an ActRIIB Fc-Fusion protein comprises or consists of the sequence set forth in SEQ ID NO:6 or SEQ ID NO:10. In some aspects, a protein inhibits at least two of activin, myostatin, and GDF-11. In some aspects, a protein inhibits activin, myostatin, and GDF-11. In some aspects, a protein inhibits activin.

In one aspect, methods and reagents are disclosed for treating myostatin-related and/or activin A related disorders in a subject in need of such a treatment by administering an effective dosage of an svActRIIB composition to the subject. As used herein the term "subject" refers to any animal, such as mammals including humans.

The compositions are useful for increasing lean muscle mass in a subject. The compositions may also be useful to increase lean muscle mass in proportion to fat mass, and thus decrease fat mass as percentage of body weight in a subject.

The disorders that can be treated by an svActRIIB include but are not limited to various forms of muscle wasting, as well as metabolic disorders such as diabetes and related disorders, and bone degenerative diseases such as osteoporosis.

Muscle wasting disorders also include dystrophies such as Duchenne's muscular dystrophy, progressive muscular dystrophy, Becker's type muscular dystrophy, Dejerine-Landouzy muscular dystrophy, Erb's muscular dystrophy, and infantile neuroaxonal muscular dystrophy. Additional muscle wasting disorders arise from chronic diseases or disorders such as amyotrophic lateral sclerosis, congestive obstructive pulmonary disease, cancer, AIDS, renal failure, organ atrophy, androgen deprivation, and rheumatoid arthritis.

Over-expression of myostatin and/or activin may contribute to cachexia, a severe muscle wasting syndrome. Cachexia results from cancers, and also arises due to rheumatoid arthritis, diabetic nephropathy, renal failure, chemotherapy, injury due to burns, as well as other causes. In another example, serum and intramuscular concentrations of myostatin-immunoreactive protein was found to be increased in men exhibiting AIDS-related muscle wasting and was inversely related to fat-free mass (Gonzalez-Cadavid et al., PNAS USA 95: 14938-14943 (1998)). Myostatin levels have also been shown to increase in response to burns injuries, resulting in a catabolic muscle effect (Lang et al, FASEB J 15, 1807-1809 (2001)). Additional conditions resulting in muscle wasting may arise from inactivity due to disability such as confinement in a wheelchair, prolonged bed rest due to stroke, illness, spinal chord injury, bone fracture or trauma, and muscular atrophy in a microgravity environment (space flight). For example, plasma myostatin immunoreactive protein was found to increase after prolonged bed rest (Zachwieja et al. J Gravit Physiol. 6(2):11(1999). It was also found that the muscles of rats exposed to a microgravity environment during a space shuttle flight expressed an increased amount of myostatin compared with the muscles of rats which were not exposed (Lalani et al., J.Endocrin 167 (3):417-28 (2000)).

In addition, age-related increases in fat to muscle ratios, and age-related muscular atrophy appear to be related to myostatin. For example, the average serum myostatin-immunoreactive protein increased with age in groups of young (19-35 yr. old), middle-aged (36-75 yr. old), and elderly (76-92 yr old) men and women, while the average muscle mass and fat-free mass declined with age in these groups (Yarasheski et al. J Nutr Aging 6(5):343-8 (2002)). In addition, myostatin has now been found to be expressed at low levels in heart muscle and expression is upregulated in cardiomyocytes after infarct (Sharma et al., J Cell Physiol. 180 (1):1-9 (1999)). Therefore, reducing myostatin levels in the heart muscle may improve recovery of heart muscle after infarct.

Myostatin also appears to influence metabolic disorders including type 2 diabetes, noninsulin-dependent diabetes mellitus, hyperglycemia, and obesity. For example, lack of myostatin has been shown to improve the obese and diabetic phenotypes of two mouse models (Yen et al. FASEB J. 8:479 (1994). svActRIIB polypeptides of the present disclosure are suitable for treating such metabolic disorders. Therefore, administering the compositions will improve diabetes, obesity, and hyperglycemic conditions in suitable subjects. In addition, compositions containing svActRIIB polypeptides can decrease food intake in obese individuals.

Administering stabilized ActRIIB polypeptides can improve bone strength and reduce osteoporosis and other degenerative bone diseases. It has been found, for example, that myostatin-deficient mice showed increased mineral content and density of the mouse humerus and increased mineral content of both trabecular and cortical bone at the regions where the muscles attach, as well as increased muscle mass (Hamrick et al. Calcif Tissue Int 71(1):63-8 (2002)). In addition, svActRIIBs can be used to treat the effects of androgen deprivation in cases such as androgen deprivation therapy used for the treatment of prostate cancer, for example.

Also disclosed are methods and compositions for increasing muscle mass in food animals by administering an effective dosage of svActRIIB proteins to the animal. Since the mature C-terminal myostatin polypeptide is similar or identical in all species tested, svActRIIB polypeptides would be expected to be effective for increasing lean muscle mass and reducing fat in any agriculturally important species including cattle, chicken, turkeys, and pigs.

In some aspects, disclosed herein are methods of treating obesity or a disease associated with obesity, increasing muscle mass, or decreasing fat mass in a subject, comprising: administering to the subject an effective dose of a protein that inhibits at least one of activin, myostatin, and GDF-11.

In some aspects, a disease associated with obesity is at least one of a genetic obesity syndrome, Prader willi syndrome, a hypothalamic disorder, familial hypercholesterolemia, Bardet-Biedl syndrome, Prader-Willi syndrome, a syndrome resulting from a loss of imprinted genes on 15q11-13, Alstrom syndrome, Cohen syndrome, Albright's hereditary osteodystrophy (pseudohypoparathyroidism), Carpenter syndrome, MOMO syndrome, Rubinstein-Taybi syndrome, a syndrome resulting from deletions of at least one of6q16, 1p36, 2q37, and 9q34, maternal uniparental disomy of chromosome 14, fragile X syndrome, atherosclerosis, non-alcoholic steatohepatitis, a disease where visceral fat deposition results in one or more deleterious outcomes, cerebrovascular disease, fatty liver, and Börjeson-Forssman-Lehman syndrome.

In some aspects, a subject is a human subject in need treatment or administration. In some aspects, a subject has at least one of a genetic obesity syndrome, Prader willi syndrome, a hypothalamic disorder, familial hypercholesterolemia, Bardet-Biedl syndrome, Prader-Willi syndrome, a syndrome resulting from a loss of imprinted genes on 15q11-13, Alstrom syndrome, Cohen syndrome, Albright's hereditary osteodystrophy (pseudohypoparathyroidism), Carpenter syndrome, MOMO syndrome, Rubinstein-Taybi syndrome, a syndrome resulting from deletions of at least one of 6q16, 1p36, 2q37, and 9q34, maternal uniparental disomy of chromosome 14, fragile X syndrome, atherosclerosis, non-alcoholic steatohepatitis, a disease where visceral fat deposition results in one or more deleterious outcomes, cerebrovascular disease, fatty liver, and Börjeson-Forssman-Lehman syndrome. In some aspects, a subject has at least one of insulin resistance, chronic kidney disease, cancer, and a catabolic condition.

In some aspects, a protein is an antibody or an Fc-Fusion protein. In some aspects, an Fc-Fusion protein is an ActRIIB Fc-Fusion protein. In some aspects, an ActRIIB Fc-Fusion protein comprises a sequence with at least 80 %, 85 %, 90 %, 95 %, 96 %, 97 %, 98 %, or 99 % identity to the sequence set forth in amino acids 19-25, 19, 20, 21, 22, 23, 24, or 25 through 130-134, 125, 126, 127, 128, 129, 130, 131, 132, 133, or 134 of SEQ ID NO: 2, optionally wherein the Fc is IgG, optionally wherein IgG is human IgG. In some aspects, an ActRIIB Fc-Fusion protein comprises or consists of the sequence set forth in SEQ ID NO:6 or SEQ ID NO:10. In some aspects, a protein inhibits at least two of activin, myostatin, and GDF-11. In some aspects, a protein inhibits activin, myostatin, and GDF-11. In some aspects, a protein inhibits activin.

In some aspects, a method disclosed herein decreases at least one of total fat mass, subcutaneous fat mass, and visceral fat mass. In some aspects, a method disclosed herein results in a greater percent decrease in visceral fat mass relative to the percent decrease in total fat mass. In some aspects, a method disclosed herein increases at least one of lean body mass and appendicular lean mass. In some aspects, a method disclosed herein results in an increase in at least one of lean body mass and appendicular lean mass by at least 1-50%, 1%, 2%, 3%, 4%, 5%, 6%, 7%, 8%, 9%, 10%, 15%, 20%, 25%, 30%, 35%, 40%, 45%, 50%, or more. In some aspects, a method disclosed herein results in a decrease in at least one of total fat mass, subcutaneous fat mass, and visceral fat mass by at least 1-99%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 99%, or more.

svActRIIB polypeptides and compositions also antagonize the activity of activin A, as shown in the *in vitro* assays below. Activin A is known to be expressed in certain types of cancers, particularly gonadal tumors such as ovarian carcinomas, and to cause severe cachexia. (Ciprano et al. Endocrinol 141 (7):2319-27 (2000), Shou et al., Endocrinol 138 (11):5000-5 (1997); Coerver et al, Mol Endocrinol 10(5):534-43 (1996); Ito et al. British J Cancer 82(8):1415-20 (2000), Lambert-Messerlian, et al, Gynecologic Oncology 74:93-7 (1999). Therefore, the compositions of the present disclosure may be used to treat conditions related to activin A overexpression, as well as myostatin expression, such as cachexia from certain cancers and the treatment of certain gonadal type tumors.

In addition, svActRIIB polypeptides are useful for detecting and quantitating myostatin, activin A, or GDF-11 in any number of assays. In general, stabilized ActRIIB polypeptides are useful as capture agents to bind and immobilize myostatin, activin A, or GDF-11 in a variety of assays, similar to those described, for example, in Asai, ed., Methods in Cell Biology, 37, Antibodies in Cell Biology, Academic Press, Inc., New York (1993). The polypeptides may be labeled in some manner or may react with a third molecule such as an antibody which is labeled to enable myostatin to be detected and quantitated. For example, a polypeptide or a third molecule can be modified with a detectable moiety, such as biotin, which can then be bound by a fourth molecule, such as enzyme-labeled streptavidin, or other proteins. (Akerstrom, J Immunol 135:2589 (1985); Chaubert, Mod Pathol 10:585 (1997)).

In some aspects, a composition of the present disclosure can be used to treat cancers such as solid tumors. The terms "cancer" and "cancerous" refer to or describe the physiological condition in mammals that is typically characterized by unregulated cell growth. Examples of cancer include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. More particular examples of such cancers include squamous cell cancer (e.g. epithelial squamous cell cancer), lung cancer including small-cell lung cancer (SCLC), non-small cell lung cancer (NSCLC), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer (including metastatic breast cancer), colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophagael cancer, tumors of the biliary tract, as well as head and neck cancer. In some aspects, a composition of the present disclosure can be used to treat cancers such as these or ovarian cancer (granulosa cell, clear cell, serous, endometrioid, germ cell tumors), head and neck cancer, non-small cell lung cancer, small cell lung cancer, pancreatic cancer, prostate cancer, small intestinal tumors, colon cancer, renal cell carcinoma, adenoid cystic carcinoma, gastric cancer, esophageal cancer, squamous cell carcinoma (skin), melanoma, breast cancer, bladder carcinoma, hepatocellular carcinoma, and/or uterine cancer (endometrial, cervical, leiomyoma, vulva, vaginal).

Dosages for use with the proteins and polypeptides described herein can be 0.1-10, 0.25-5, 1-3, 0.1, 0.2, 0.25, 0.3, 0.4, 0.5, 0.6, 0.7, 0.75, 0.8, 0.9, 1, 2, 3, 4, 5, or greater than 5 mg/kg, inclusive. In some aspects each dosage can be administered every less than 1-10, 2-9, 3-8, 4-7, 5-6, 1, 1, 2, 3, 4, 5, 6, 7, 8, or greater than 8 weeks, inclusive. In some aspects each dosage can be administered every less than 1-10, 2-9, 3-8, 4-7, 5-6, 1, 1, 2, 3, 4, 5, 6, 7 days, inclusive. In some aspects each dosage is administered IV. In some aspects, the subject is administered at least 1-30, 5-20, 5-10, 10-20, 15-20, 1-5,2,3,4,5,6, 7, 8,9, 10, 11, 12, 13, 14, 15, 16, 17, 18, 19, or 20 doses, inclusive. In some aspects, the amount of at least one of the plurality of doses is at least 0.3, 0.5, 1, 2, 3, 4, 5, 6, 7, 8, 9, 10, 11, 12, 13, 14, 15, 16, 17, 18, 19, or 20 mg/kg, inclusive. In some aspects, the amount of each of the plurality of doses is at least 0.1-30, 0.25-20, 0.25-10, 1-5, 1-3, 0.1-1, 0.3, 0.5, 1, 2, 3, 4, 5, 6, 7, 8,9, 10, 11, 12, 13, 14, 15, 16, 17, 18, 19, or 20 mg/kg, inclusive. In some aspects, each dose is administered at least daily, weekly, or monthly. In some aspects, each dose is administered at least every 1-30, 5-20, 5-10, 10-20, 15-20, 1-5, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days, inclusive. In some aspects, treatment continues for at least 1-30, 5-20, 5-10, 10-20, 15-20, 1-5, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days; at least 1-30, 5-20, 5-10, 10-20, 15-20, 1-5, 1, 2, 3, 4, 5, 6, 7, 8, 9, 10, 11, 12, 13, 14, 15, 16, 17, 18, 19, or 20 weeks; or at least 1-30, 5-20, 5-10, 10-20, 15-20, 1-5, 1,2,3,4,5,6, 7, 8,9, 10, 11, 12, 13, 14, 15, 16, 17, 18, 19, or 20 months, inclusive.

In some aspects, a subject can be selected for treatment of cancer using certain criteria such as inclusion criteria or exclusion criteria.

In some aspects the criteria are inclusion criteria. In some aspects, inclusion criteria can include: males and postmenopausal females ≥ 18 years of age, presence of advanced solid tumors with histologic diagnosis confirming cancer, presence of recurrent metastatic or locally advanced disease after failure of at least one line of prior standard treatment (if available), measurable disease using Response Evaluation Criteria in Solid Tumors (RECIST 1.1) criteria, an Eastern Cooperative Oncology Group (ECOG) performance status of 0 or 1, ablity to walk at least 30 meters without assistance from another person (use of assistive devices such as a cane or walking frame can be allowed), 12 months of spontaneous amenorrhea in postmenopausal women, 6 months of spontaneous amenorrhea with FSH > 40 IU/L in postmenopausal women, post-surgical bilateral oophorectomy with or without hysterectomy in postmenopausal women, prior treatment with a platinum-based chemotherapy regimen, and/or documented as unable to receive platinum-based chemotherapy.

In some aspects the criteria are exclusion criteria. In some aspects, exclusion criteria can include: concurrent serious uncontrolled or unresolved medical condition (such as infection) limiting protocol compliance or exposing the subject to extreme risk, unresolved toxicities from prior anti-cancer therapy, such as motor or sensory neuropathy, with a CTCAE (version 4.03) Grade ≥ 2 with the exception of alopecia, history of gastrointestinal bleeding within 6 months of starting treatment, presence of QTcF > 470 msec, history of hereditary prolonged QT interval, or any arrhythmia (such as bundle branch blocks) that would preclude assessment of the QT interval, myocardial infarction, unstable angina within 6 months of Cycle 1 Day 1, or congestive heart failure New York Heart Association ≥ class II, elevated liver function tests, including total bilirubin > 1.5 × the upper limit of normal (ULN; unless subject has documented Gilbert's disease), aspartate aminotransferase (AST) or alanine aminotransferase (ALT) > 3.0 × ULN (for subjects with known liver metastasis, AST or ALT > 5 × ULN), creatinine > 1.5 × ULN and an estimated creatinine clearance of < 60 mL/min (using the Cockcroft-Gault equation), hemoglobin < 9 g/dL; platelet < 100 × 10⁹/L; absolute neutrophil count (ANC) < 1.5 × 10⁹/L (without granulocyte colony-stimulating factor support within 2 weeks of starting treatment), chemotherapy, hormonal therapy, or radiation therapy within 3 weeks of starting treatment, antibody/biologic therapy within 4 weeks of starting treatment, major surgery within 8 weeks or minor surgery within 4 weeks of starting treatment, current bowel obstruction, brain metastasis, presence of ascites or pleural effusion requiring frequent (more than 1 × per week) medical intervention, presence of portal-venous shunt device or history of extensive hepatic resection (more than one segment), known human immunodeficiency virus (HIV) infection, active Hepatitis B or C infection, prior treatment with any investigational product within 4 weeks of starting treatment, female of childbearing potential, or male with a female partner of childbearing potential, unwilling to use a highly effective method of contraception (i.e., one that results in pregnancy less than 1% per year) when used consistently and correctly, such as implants, injectables, combined oral contraceptives, some intrauterine contraceptive devices, sexual abstinence, or a vasectomized partner, hypersensitivity reactions to a conventional formulation of doxorubicin HCl or the components of liposomal doxorubicin, cumulative dose of prior doxorubin HC1 > 300 mg/m², or cumulative dose of prior epirubicin > 500 mg/m², decreased cardiac ejection fraction less than the lower limit of normal by a MUGA scan or an echocardiogram (ECHO) within 28 days of starting treatment, and/or women who are breast-feeding.

### Pharmaceutical compositions and formulations

Pharmaceutical compositions containing proteins and polypeptides disclosed herein are also provided. In some aspects, a protein is an antibody or an Fc-Fusion protein. In some aspects, an Fc-Fusion protein is an ActRIIB Fc-Fusion protein. In some aspects, an ActRIIB Fc-Fusion protein comprises a sequence with at least 80 %,85 %, 90 %, 95 %, 96 %, 97 %, 98 %, or 99 % identity to the sequence set forth in amino acids 19-25, 19, 20, 21, 22, 23, 24, or 25 through 130-134, 125, 126, 127, 128, 129, 130, 131, 132, 133, or 134 of SEQ ID NO: 2, optionally wherein the Fc is IgG, optionally wherein IgG is human IgG. In some aspects, an ActRIIB Fc-Fusion protein comprises or consists of the sequence set forth in SEQ ID NO:6 or SEQ ID NO:10. In some aspects, a protein inhibits at least two of activin, myostatin, and GDF-11. In some aspects, a protein inhibits activin, myostatin, and GDF-11. In some aspects, a protein inhibits activin.

Such compositions comprise a therapeutically or prophylactically effective amount of the polypeptide or protein in admixture with pharmaceutically acceptable materials, and physiologically acceptable formulation materials. The pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. Suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates, other organic acids); bulking agents (such as mannitol or glycine), chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides and other carbohydrates (such as glucose, mannose, or dextrins); proteins (such as serum albumin, gelatin or immunoglobulins); coloring; flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate 80, triton, tromethamine, lecithin, cholesterol, tyloxapal); stability enhancing agents (sucrose or sorbitol); tonicity enhancing agents (such as alkali metal halides (preferably sodium or potassium chloride, mannitol sorbitol); delivery vehicles; diluents; excipients and/or pharmaceutical adjuvants. (Remington's Pharmaceutical Sciences, 18th Edition, A.R. Gennaro, ed., Mack Publishing Company, 1990).

As used herein, the term "buffer" is intended to mean a substance that stabilizes the pH of a liquid, either its acidity or alkalinity. The term as it is used herein is intended to refer to a solution having a buffering substance, such as an acid, in equilibrium with its conjugate base. Exemplary buffers useful in a formulation disclosed herein include a potassium phosphate buffer. Exemplary salt forms of buffers that can be included in a buffer of the invention include, for example, sodium, potassium, calcium, organic amino or magnesium salt. The term "buffer" as it is used herein also is intended to include all buffers other than potassium phosphate buffer that are well known to those skilled in the art and applicable for use with biopharmaceuticals such as therapeutic polypeptides. Given the teachings and guidance provided herein, those skilled in the art will understand that buffers other than potassium phosphate buffer can be equally substituted in the formulations of the disclosure to maintain or enhance the stability of a therapeutic polypeptide. Any of a wide variety of buffer components well known in the art can be used in a formulation of the disclosure. Such buffer components include, for example, acetic acid, glutamic acid, succinic acid, propionic acid, maleic acid, gluconate, histidine or other amino acids, citrate, phosphate, or salt forms thereof. A wide variety of other buffers including, for example, other organic acids, are well known in the art and can similarly be used as a buffer component in a formulation of the disclosure. Given the teachings and guidance provided herein, those skilled in the art will known that any of the above buffer components or others well known in the art can be selected and used in a formulation of the disclosure given the desired pH of the formulation and excipients, if any, included in the formulation. The buffer component can be supplied to the buffering system in a variety of different forms. In some aspects, a formulation can include 1-30, 5-20, 5-10, 10-20, 15-20, 1-5, 1, 2, 3, 4, 5, 6, 7, 8, 9, 10, 11, 12, 13, 14, 15, 16, 17, 19, 20, or greater than 20 mM of buffer, inclusive.

As used herein, the term "excipient" is intended to mean a therapeutically inactive substance. Excipients can be included in a formulation for a wide variety of purposes including, for example, as a diluent, vehicle, buffer, stabilizer, tonicity agent, bulking agent, surfactant, cryoprotectant, lyoprotectant, anti-oxidant, metal ion source, chelating agent and/or preservative. Excipients include, for example, polyols such as sorbitol or mannitol; sugars such as sucrose, lactose or dextrose; polymers such as polyethylene glycol; salts such as NaCl, KC1 or calcium phosphate, amino acids such as glycine, methionine or glutamic acid, surfactants, metal ions, buffer salts such as propionate, acetate or succinate, preservatives and polypeptides such as human serum albumin, as well as saline and water. Particularly useful excipients of the disclosure include sugars including sugar alcohols, reducing sugars, non-reducing sugars and sugar acids. Excipients are well known in the art and can be found described in, for example, Wang W., Int. J. Pharm. 185:129-88 (1999) and Wang W., Int. J. Pharm. 203:1-60 (2000). Non-reducing sugars contain an anomeric carbon that is an acetal and is not substantially reactive with amino acids or polypeptides to initiate a Maillard reaction. Sugars that reduce Fehling's solution or Tollen's reagent also are known as reducing sugars. Specific examples of non-reducing sugars include sucrose, trehalose, sorbose, sucralose, melezitose and raffinose. Buffer excipients maintain the pH of liquid formulations through product shelf-life and maintain the pH of lyophilized formulations during the lyophilization process and upon reconstitution, for example. In general, excipients can be chosen on the basis of the mechanisms by which they stabilize proteins against various chemical and physical stresses.

As described herein, certain excipients are beneficial to include so as to alleviate the effects of a specific stress or to regulate a particular susceptibility of a specific polypeptide. Other excipients are beneficial to include because they have more general effects on the physical and covalent stabilities of proteins. Particularly useful excipients include those chemically and functionally innocuous or compatible with aqueous buffer solutions and polypeptides so as to optimize the stability properties of a formulation. Various such excipients are described herein as exemplary excipients exhibiting chemical compatibility with the aqueous formulations of the invention and functional compatibility with the polypeptide included in such formulations. Those skilled in the art will understand that the teachings and guidance provided herein with respect to the exemplified excipients are equally applicable to the use of a wide range of other excipients well known in the art. For example, optimal excipients chosen to enhance or confer stability of a polypeptide within a formulation include those that are substantially free from reacting with functional groups on the polypeptide. In this regard, both reducing and non-reducing sugars can be used as an excipient in a formulation of the invention. However, because reducing sugars contain a hemiacetal group they can react and form adducts or other modifications with amino groups on amino acid side chains of polypeptides (i.e., glycosylation). Similarly, excipients such as citrate, succinate or histidine also can form adducts with amino acid side chains. Given the teachings and guidance provided herein, those skilled in the art will know that greater retention of stability for a given polypeptide can be achieved by choosing a non-reducing sugar over a reducing sugar or over other amino acid-reactive excipients such as those exemplified above. Optimal excipients also are chosen to enhance or provide stabilization with reference to the mode of administration for an aqueous formulation of the invention. For example, parenteral routes of intravenous (IV), subcutaneous (SC) or intramuscular (IM) administration can be more safe and efficacious when all components of the formulation maintain physical and chemical stability during manufacture, storage and administration. Those skilled in the art will know to employ one or more excipients that maintain maximal stability of the active form of a polypeptide given, for example, a particular manufacturing or storage condition or a particular mode of administration. The excipients exemplified herein for use in a formulation exhibit these and other characteristics.

The amount or concentration of excipient to use in a formulation of the invention will vary depending on, for example, the amount of polypeptide included in the formulation, the amount of other excipients included in the desired formulation, whether a diluent is desired or needed, the amount or volume of other components of the formulation, the total amount of components within a formulation, the specific activity of the polypeptide and the desired tonicity or osmolality to be achieved. Specific examples for excipient concentrations are exemplified further below. Further, different types of excipients can be combined into an formulation. Accordingly, a formulation of the invention can contain an excipient, two, three or four or more different types of excipients. Combinations of excipients can be particularly useful in conjunction with a formulation that contains two or more different polypeptides. The excipients can exhibit similar or different chemical properties. Given the teachings and guidance provided herein, those skilled in the art will know what amount or range of excipient can be included in any particular formulation to achieve a formulation of the invention that promotes retention in stability of the polypeptide. For example, the amount and type of a salt to be included in a formulation of the invention can be selected based on to the desired osmolality (i.e., isotonic, hypotonic or hypertonic) of the final solution as well as the amounts and osmolality of other components to be included in the formulation. Similarly, by exemplification with reference to the type of polyol or sugar included in a formulation, the amount of such an excipient will depend on its osmolality. Inclusion of about 5% sorbitol can achieve isotonicity while about 9% of a sucrose excipient is needed to achieve isotonicity. Selection of the amount or range of concentrations of one or more excipients that can be included within a formulation of the invention has been exemplified above by reference to salts, polyols and sugars. However, those skilled in the art will understand that the considerations described herein and further exemplified by reference to specific excipients are equally applicable to all types and combinations of excipients including, for example, salts, amino acids, other tonicity agents, surfactants, stabilizers, bulking agents, cryoprotectants, lyoprotectants, anti-oxidants, metal ions, chelating agents and/or preservatives.

Excipients can be included in a formulation of the invention at concentration ranges generally between about 1-40% (w/v), between about 5-35% (w/v), between about 8-30% (w/v), between about 8-25% (w/v) or about 8% (w/v). Concentrations as high as about 45% (w/v), 50% (w/v) or more than 50% (w/v) in certain instances also can be employed in the formulations of the invention. For example, in some instances, it can be desirable to include concentrations up to 60% (w/v) or 75% (w/v) to produce a hypertonic formulation of the invention. Such hypertonic solutions can be diluted to produce an isotonic formulation prior to use if desired. Other useful concentration ranges include between about 1-20%, particularly between about 2-18% (w/v), more particularly between about 4-16% (w/v), even more particularly between about 6-14% (w/v) or between about 8-12% (w/v) or about 10% (w/v). Excipient concentrations and/or amounts less than, greater than or in between these ranges also can be used in a formulation of the invention. For example, one or more excipients can be included in a formulation which constitute less than about 1% (w/v). Similarly, a formulation can contain a concentration of one or more excipients greater than about 40% (w/v). Accordingly, a formulation of the invention can be produced that contains essentially any desired concentration or amount of one or more excipients including, for example, 1-30, 5-20, 5-10, 10-20, 15-20, 1-5, 1, 2, 3, 4, 5, 6, 7, 8, 9, 10, 11, 12, 13, 14, 15, 16, 17, 18, 19 or 20% (w/v) or more, inclusive.

A buffer component of a formulation of the invention can include one or more excipients. As described previously, one role of an included excipient is to provide stabilization of the polypeptide against stresses that can occur during manufacturing, shipping and storage. To accomplish this role, at least one excipient can function as a buffer, stabilizer, tonicity agent, bulking agent, surfactant, cryoprotectant, lyoprotectant, anti-oxidant, metal ion source, chelating agent and/or preservative. In addition, at least one excipient also can function as a diluent and/or vehicle or be employed to reduce viscosity in high concentration formulations in order to enable their delivery and/or enhance patient convenience. Similarly, at least one excipient additionally can confer more than one of the above functions onto a formulation of the invention. Alternatively, two or more excipients can be included in a formulation of the invention to perform more than one of the above or other functions. For example, an excipient can be included as a component in a formulation of the invention to change, adjust or optimize the osmolality of the formulation, thereby acting as a tonicifier. Similarly, a tonicity agent and a surfactant can both be included in a formulation of the disclosure to both adjust the osmolality and control aggregation. Excipients, their use, formulation and characteristics are well known in the art and can be found described in, for example, Wang W., Int. J. Pharm. 185:129-88 (1999) and Wang W., Int. J. Pharm. 203:1-60 (2000).

Tonicity agents and/or stabilizers included in liquid formulations can be used, for example, to provide isotonicity, hypotonicity or hypertonicity to a formulation such that it is suitable for administration. Such excipients also can be used, for example, to facilitate maintenance of a polypeptides' structure and/or to minimize electrostatic, solution protein-protein interactions. Specific examples of tonicity agents and/or stabilizers include polyols, salts and/or amino acids. Tonicity agents and/or stabilizers included in lyophilized formulations can be used, for example, as a cryoprotectant to guard polypeptides from freezing stresses or as a lyoprotectant to stabilize polypeptides in the freeze-dried state. Specific examples of such cryo- and lyoprotectants include polyols, sugars and polymers.

As used herein, the term "surfactant" is intended to mean a substance that functions to reduce the surface tension of a liquid in which it is dissolved. Surfactants can be included in a formulation for a variety of purposes including, for example, to prevent or control aggregation, particle formation and/or surface adsorption in liquid formulations or to prevent or control these phenomena during the lyophilization and/or reconstitution process in lyophilized formulations. Surfactants include, for example, amphipathic organic compounds that exhibit partial solubility in both organic solvents and aqueous solutions. General characteristics of surfactants include their ability to reduce the surface tension of water, reduce the interfacial tension between oil and water and also form micelles. Surfactants of the invention include non-ionic and ionic surfactants. Surfactants are well known in the art and can be found described in, for example, Randolph T. W. and Jones L. S., Surfactant-protein interactions. Pharm Biotechnol. 13:159-75 (2002). Briefly, non-ionic surfactants include, for example, alkyl poly (ethylene oxide), alkyl polyglucosides such as octyl glucoside and decyl maltoside, fatty alcohols such as cetyl alcohol and oleyl alcohol, cocamide MEA, cocamide DEA, and cocamide TEA. Specific examples of non-ionic surfactants include the polysorbates including, for example, polysorbate 20, polysorbate 28, polysorbate 40, polysorbate 60, polysorbate 65, polysorbate 80, polysorbate 81, polysorbate 85 and the like; the poloxamers including, for example, poloxamer 188, also known as poloxalkol or poly(ethylene oxide)-poly(propylene oxide), poloxamer 407 or polyethylene-polypropylene glycol and the like, and polyethylene glycol (PEG). Polysorbate 20 is synonymous with TWEEN 20, sorbitan monolaurate and polyoxyethylenesorbitan monolaurate.

Optimal surfactants to include in a formulation of the invention can be chosen, for example, to enhance or promote retention in stability of the polypeptide by preventing or reducing aggregation and/or adsorption. For example, sorbitan fatty acid esters such as the polysorbates are surfactants exhibiting with a wide range of hydrophilic and emulsifying characteristics. They can be used individually or in combination with other surfactants to cover a wide range of stabilization needs. Such characteristics are particularly suitable for use with polypeptides because they can be tailored to cover the wide range of hydrophobic and hydrophilic characteristics of polypeptides. Considerations for selecting a surfactant include those described previously with reference to excipients in general as well as the hydrophobic character and critical micellar concentration of the surfactant. The surfactants exemplified herein, as well as many others well known in the art can be used in a formulation of the disclosure.

Surfactant concentration ranges for a formulation of the invention include those described previously with reference to excipients in general with particularly useful concentrations being less than about 1% (w/v). In this regard, surfactant concentrations generally can be used at ranges between about 0.0001-0.10% (w/v), particularly between about 0.002-0.05% (w/v), more particularly between about 0.003-0.01% (w/v), even more particularly between about 0.004-0.008% (w/v) or between about 0.005-0.006% (w/v). Surfactant concentrations and/or amounts less than, greater than or in between these ranges also can be used in a formulation of the invention. For example, one or more surfactants can be included in a formulation which constitute less than about 0.001% (w/v). Similarly, a formulation can contain a concentration of one or more surfactants greater than about 0.10% (w/v). Accordingly, a formulation of the invention can be produced that contains essentially any desired concentration or amount of one or more surfactants including, for example, 0.001, 0.002, 0.003, 0.004, 0.005, 0.006, 0.007, 0.008, 0.009, 0.010, 0.02, 0.03, 0.04, 0.05, 0.06, 0.07, 0.08, 0.09 or 0.10% (w/v) or more, inclusive. Various surfactants useful as an excipient in a formulation of the invention have been described previously. Other surfactants useful in either a liquid or lyophilized formulation of the disclosure include, for example, sugar esters such as esters lauric acid (C12), palmitic acid (C16), stearic acid (C18), macrogol cetostearyl ethers, macrogol lauryl ethers, macrogol oleyl ether, macrogol oleate, macrogol stearate, macrogol glycerol ricinoleate, macrogol glycerol hydroxystearate; alkyl polyglucosides such as octyl glucoside and decyl maltoside; fatty alcohols such as cetyl alcohol and oleyl alcohol, and cocamides such as cocamide MEA, DEA, TEA, other non-ionic surfactants and other ionic surfactants.

Stability of a formulation of the invention, including a liquid formulation of the invention, refers to the retention of structure and/or function of a polypeptide within a formulation. A polypeptide in a formulation of the invention will exhibit attributes such as resistance to change or deterioration that affect stability or function and therefore maintain consistent functional characteristics over time.

A buffer component of a formulation of the invention also can include one or more surfactants as an excipient. As described previously, one role of surfactants in a formulation of the invention is to prevent or minimize aggregation and/or adsorption such as surface-induced degradation. At sufficient concentrations, generally about the surfactant's critical micellar concentration, a surface layer of surfactant molecules serve to prevent protein molecules from adsorbing at the interface. Thereby, surface-induced degradation is minimized. Surfactant, their use, formulation and characteristics for formulations are well known in the art and can be found described in, for example, Randolph and Jones, supra, (2002).

In another embodiment, the stability of a polypeptide within a formulation of the invention includes, for example, the retention of physical and/or chemical stability. Polypeptide stability can be assessed by, for example, determining whether the polypeptide has been subjected to a physical degradation and/or chemical degradation pathway such as those described previously, including chemical modification of its structure. Retention in stability of a polypeptide in a formulation of the invention includes, for example, retention of physical or chemical stability between about 80-100%, 85-99%, 90-98%, 92-96% or 94-95% compared to the stability of the polypeptide at an initial time point or relative to an identical control kept at a lower temperature, e.g., -70 degrees Celsius. Accordingly, stability of a polypeptide within a formulation of the invention includes retention of stability greater than 99.5%, at least about 99%, 98%, 97%, 96%, 95%, 94%, 93%, 92%, 91%, 90%, 89%, 88%, 87%, 86%, 85%, 84%, 83%, 82%, 81% or 80% compared to the stability of the polypeptide at an initial time point relative to an identical control kept at a lower temperature, e.g., -70 degrees Celsius, inclusive.

In a further embodiment, stability of a polypeptide within a formulation of the invention includes, for example, retention of activity. Polypeptide activity can be assessed using, for example, an in vitro, in vivo and/or in situ assay indicative of the polypeptide's function. Retention of stability of a polypeptide in a formulation of the invention includes, for example, retention of activity between about 50-100% or more, depending on the variability of the assay. For example, retention in stability can include retention of activity between about 60-90% or 70-80% compared to the activity of the polypeptide at an initial time point. Accordingly, stability of a polypeptide within a formulation of the invention includes retention of activity of at least about 50%, 55%, 60%, 65%, 70%, 75%, 80%, 85%, 90%, 95% or 100% and can include activity measurements greater than 100% such as 105%, 110%, 115%, 120%, 125% or 150% or more compared to the activity of the polypeptide at an initial time point, inclusive. Generally, an initial time point is selected to be the time that a polypeptide is first prepared in a formulation of the invention or first examined for quality (i.e., meets release specifications). An initial time point also can include the time at which a polypeptide is reformulated in a formulation of the invention. The reformulation can be, for example, at a higher concentration, lower concentration or at the same concentration of an initial preparation.

A formulation of the invention can be prepared to be isotonic with a reference solution or fluid (i.e., blood serum). An isotonic solution has a substantially similar amount of dissolved solute in it compared to the things around it so that it is osmotically stable. Unless expressly compared to a specific solution or fluid, isotonic or isotonicity is exemplary used herein by reference to human blood serum (e.g., 300 mOsmol/kg). Therefore, an isotonic formulation of the invention will contain a substantially similar concentration of solutes or exhibit substantially similar osmotic pressure as human blood. In general, an isotonic solution contains about the same concentration of solutes as normal saline for humans and many other mammals, which is about 0.9 weight percent (0.009 g/ml) salt in aqueous solution (e.g., 0.009 g/ml NaCl). Formulations of the invention also can include hypotonic or hypertonic solution preparations.

The optimal pharmaceutical composition will be determined by one skilled in the art depending upon, for example, the intended route of administration, delivery format, and desired dosage. See for example, Remington's Pharmaceutical Sciences, *supra.* Such compositions may influence the physical state, stability, rate of *in vivo* release, and rate of *in vivo* clearance of the polypeptide. For example, suitable compositions may be water for injection, physiological saline solution for parenteral administration. Ionic surfactants include, for example, anionic, cationic and zwitterionic surfactants. Anionic surfactants include, for example, sulfonate-based or carboxylate-based surfactants such as soaps, fatty acid salts, sodium dodecyl sulfate (SDS), ammonium lauryl sulfate and other alkyl sulfate salts. Cationic surfactants include, for example, quaternary ammonium-based surfactants such as cetyl trimethylammonium bromide (CTAB), other alkyltrimethylammonium salts, cetyl pyridinium chloride, polyethoxylated tallow amine (POEA) and benzalkonium chloride. Zwitterionic or amphoteric surfactants include, for example, dodecyl betaine, dodecyl dimethylamine oxide, cocamidopropyl betaine and coco ampho glycinate.

The primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffers, or acetate buffers, which may further include sorbitol or a suitable substitute thereof. In one embodiment, compositions may be prepared for storage by mixing the selected composition having the desired degree of purity with optional formulation agents (Remington's Pharmaceutical Sciences, supra) in the form of a lyophilized cake or an aqueous solution. Further, the therapeutic composition may be formulated as a lyophilizate using appropriate excipients such as sucrose.

The formulations can be delivered in a variety of methods, for example, by inhalation therapy, orally, or by injection. When parenteral administration is contemplated, the therapeutic compositions may be in the form of a pyrogen-free, parenterally acceptable aqueous solution comprising the desired polypeptide in a pharmaceutically acceptable vehicle. A particularly suitable vehicle for parenteral injection is sterile distilled water in which a polypeptide is formulated as a sterile, isotonic solution, properly preserved. Yet another preparation can involve the formulation of the desired molecule with an agent, such as injectable microspheres, bio-erodible particles, polymeric compounds (polylactic acid, polyglycolic acid), beads, or liposomes that provides for the controlled or sustained release of the product which may then be delivered via a depot injection. Hyaluronic acid may also be used, and this may have the effect of promoting sustained duration in the circulation. Other suitable means for the introduction of the desired molecule include implantable drug delivery devices.

In another aspect, pharmaceutical formulations suitable for injectable administration may be formulated in aqueous solutions, preferably in physiologically compatible buffers such as Hanks' solution, Ringer's solution, or physiologically buffered saline. Aqueous injection suspensions may contain substances that increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils, such as sesame oil, or synthetic fatty acid esters, such as ethyl oleate, triglycerides, or liposomes. Non-lipid polycationic amino polymers may also be used for delivery. Optionally, the suspension may also contain suitable stabilizers or agents to increase the solubility of the compounds and allow for the preparation of highly concentrated solutions. In another embodiment, a pharmaceutical composition may be formulated for inhalation. Inhalation solutions may also be formulated with a propellant for aerosol delivery. In yet another embodiment, solutions may be nebulized. Pulmonary administration is further described in PCT Application No. PCT/US94/001875, which describes pulmonary delivery of chemically modified proteins.

In some aspects, proteins can be formulated as a sterile aqueous solution, containing 50-100, 60-80, 65-75, 60-70, 70-80, 60, 61, 62, 63, 64, 65, 66, 67, 68, 69, 70, 71, 72, 73, 74, 75, 76, 77, 78, 79, or 80 mg/mL protein, 1-30, 5-20, 5-10, 10-20, 15-20, 1-5, 1, 2, 3, 4, 5, 6, 7, 8,9, 10, 11, 12, 13, 14, 15, 16, 17, 19, 20, or greater than 20 mM potassium phosphate buffer, less than 1-10, 5-10, 5, 5, 6, 7, 8, 8.8, 9, 10, or greater than 10 % (w/v) sucrose, and/or less than 0.006, 0.006, or greater than 0.006% (w/v) polysorbate 20 at pH 4-12, 5-6, 5-7, 6-7, 5, 6, 6.7, 7, or 8, inclusive. In some aspects, a protein can be formulated as a sterile aqueous solution, containing protein, potassium phosphate buffer, sucrose, and/or polysorbate 20. In some aspects, a protein can be formulated with potassium phosphate buffer, sucrose, and/or polysorbate 20. In some aspects, a protein can be formulated for IV administration. In some aspects, a protein can be formulated at neutral pH. In some aspects, a protein can be formulated at a pH of about 4-12, 5-6, 5-7, 6-7, 5, 6, 6.7, 7, or 8, inclusive. In some aspects, a protein described herein can be formulated with a non-naturally occurring component such as, e.g., a non-naturally occurring excipient.

It is also contemplated that certain formulations may be administered orally. In one embodiment, molecules that are administered in this fashion can be formulated with or without those carriers customarily used in the compounding of solid dosage forms such as tablets and capsules. For example, a capsule may be designed to release the active portion of the formulation at the point in the gastrointestinal tract when bioavailability is maximized and pre-systemic degradation is minimized. Additional agents can be included to facilitate absorption of the therapeutic molecule. Diluents, flavorings, low melting point waxes, vegetable oils, lubricants, suspending agents, tablet disintegrating agents, and binders may also be employed. Pharmaceutical compositions for oral administration can also be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combining active compounds with solid excipient and processing the resultant mixture of granules (optionally, after grinding) to obtain tablets or dragee cores. Suitable auxiliaries can be added, if desired. Suitable excipients include carbohydrate or protein fillers, such as sugars, including lactose, sucrose, mannitol, and sorbitol; starch from corn, wheat, rice, potato, or other plants; cellulose, such as methyl cellulose, hydroxypropylmethyl-cellulose, or sodium carboxymethylcellulose; gums, including arabic and tragacanth; and proteins, such as gelatin and collagen. If desired, disintegrating or solubilizing agents may be added, such as the cross-linked polyvinyl pyrrolidone, agar, and alginic acid or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, *i.e.,* dosage.

Pharmaceutical preparations that can be used orally also include push-fit capsules made of gelatin, as well as soft, sealed capsules made of gelatin and a coating, such as glycerol or sorbitol. Push-fit capsules can contain active ingredients mixed with fillers or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Additional pharmaceutical compositions will be evident to those skilled in the art, including formulations involving polypeptides in sustained- or controlled-delivery formulations. Techniques for formulating a variety of other sustained- or controlled-delivery means, such as liposome carriers, bio-erodible microparticles or porous beads and depot injections, are also known to those skilled in the art. See for example, PCT/US93/00829 that describes controlled release of porous polymeric microparticles for the delivery of pharmaceutical compositions. Additional examples of sustained-release preparations include semipermeable polymer matrices in the form of shaped articles, e.g. films, or microcapsules. Sustained release matrices may include polyesters, hydrogels, polylactides (U.S. 3,773,919, EP 58,481), copolymers of L-glutamic acid and gamma ethyl-L-glutamate (Sidman et al., Biopolymers, 22:547-556 (1983), poly (2-hydroxyethyl-methacrylate) (Langer et al., J. Biomed. Mater. Res., 15:167-277, (1981); Langer et al., Chem. Tech.,12:98-105(1982)), ethylene vinyl acetate (Langer et al., supra) or poly-D(-)-3-hydroxybutyric acid (EP 133,988). Sustained-release compositions also include liposomes, which can be prepared by any of several methods known in the art. See e.g., Eppstein et al., PNAS (USA), 82:3688 (1985); EP 36,676; EP 88,046; EP 143,949.

The pharmaceutical composition to be used for *in vivo* administration typically must be sterile. This may be accomplished by filtration through sterile filtration membranes. Where the composition is lyophilized, sterilization using this method may be conducted either prior to or following lyophilization and reconstitution. The composition for parenteral administration may be stored in lyophilized form or in solution. In addition, parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, or a dehydrated or lyophilized powder. Such formulations may be stored either in a ready-to-use form or in a form (e.g., lyophilized) requiring reconstitution prior to administration.

In a specific aspect, kits for producing an-dose administration unit are disclosed. The kits may each contain both a first container having a dried protein and a second container having an aqueous formulation. Also included within the scope of this disclosure are kits containing single and multi-chambered pre-filled syringes (e.g., liquid syringes and lyosyringes).

An effective amount of a pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the polypeptide is being used, the route of administration, and the size (body weight, body surface or organ size) and condition (the age and general health) of the patient. Accordingly, the clinician may titer the dosage and modify the route of administration to obtain the optimal therapeutic effect. A typical dosage may range from about 0.1mg/kg to up to about 100 mg/kg or more, depending on the factors mentioned above. Polypeptide compositions may be preferably injected or administered intravenously (IV). Long-acting pharmaceutical compositions may be administered every three to four days, every week, or biweekly depending on the half-life and clearance rate of the particular formulation. The frequency of dosing will depend upon the pharmacokinetic parameters of the polypeptide in the formulation used. Typically, a composition is administered until a dosage is reached that achieves the desired effect. The composition may therefore be administered as an dose, or as multiple doses (at the same or different concentrations/dosages) over time, or as a continuous infusion. Further refinement of the appropriate dosage is routinely made. Appropriate dosages may be ascertained through use of appropriate dose-response data.

Dosages for use with the proteins and polypeptides described herein can be 0.1-10, 0.1-5, 0.25-5, 0.25-3, 1-3, 0.1, 0.2, 0.25, 0.3, 0.4, 0.5, 0.6, 0.7, 0.75, 0.8, 0.9, 1, 2, 3, 4, 5, or greater than 5 mg/kg, inclusive. In some aspects each dosage can be administered every less than 1-10, 2-8, 2-5, 1-4, 1, 1, 2, 3, 4, 5, 6, 7, 8, or greater than 8 weeks, inclusive. In some aspects each dosage can be administered every less than 1-10, 2-8, 2-5, 1-4, 1, 1, 2, 3, 4, 5, 6, 7 days, inclusive. In some aspects each dosage is administered IV.

The route of administration of the pharmaceutical composition is in accord with known methods, e.g. orally, through injection by intravenous, intraperitoneal, intracerebral (intra-parenchymal), intracerebroventricular, intramuscular, intra-ocular, intraarterial, intraportal, intralesional routes, intramedullary, intrathecal, intraventricular, transdermal, subcutaneous, or intraperitoneal; as well as intranasal, enteral, topical, sublingual, urethral, vaginal, or rectal means, by sustained release systems or by implantation devices. Where desired, the compositions may be administered by bolus injection or continuously by infusion, or by implantation device. Alternatively or additionally, the composition may be administered locally via implantation of a membrane, sponge, or another appropriate material on to which the desired molecule has been absorbed or encapsulated. Where an implantation device is used, the device may be implanted into any suitable tissue or organ, and delivery of the desired molecule may be via diffusion, timed-release bolus, or continuous administration.

In some cases, svActRIIB polypeptides can be delivered by implanting certain cells that have been genetically engineered, using methods such as those described herein, to express and secrete the polypeptide. Such cells may be animal or human cells, and may be autologous, heterologous, or xenogeneic. Optionally, the cells may be immortalized. In order to decrease the chance of an immunological response, the cells may be encapsulated to avoid infiltration of surrounding tissues. The encapsulation materials are typically biocompatible, semi-permeable polymeric enclosures or membranes that allow the release of the polypeptide product(s) but prevent the destruction of the cells by the patient's immune system or by other detrimental factors from the surrounding tissues.

svActRIIB gene therapy *in vivo* is also envisioned wherein a nucleic acid molecule encoding svActRIIB, or a derivative of svActRIIB is introduced directly into the subject. For example, a nucleic acid sequence encoding a svActRIIB is introduced into target cells via local injection of a nucleic acid construct with or without an appropriate delivery vector, such as an adeno-associated virus vector. Alternative viral vectors include, but are not limited to, retroviruses, adenovirus, herpes simplex, virus and papilloma virus vectors. Physical transfer of the virus vector may be achieved *in vivo* by local injection of the desired nucleic acid construct or other appropriate delivery vector containing the desired nucleic acid sequence, liposome-mediated transfer, direct injection (naked DNA), or microparticle bombardment (gene-gun).

The compositions of the present disclosure may be used alone or in combination with other therapeutic agents, e.g., to enhance their therapeutic effects or decrease potential side effects. In some aspects, doxorubicin can be administered in a combination. In some aspects, doxorubicin is liposomal doxorubicin. In some aspects, doxorubicin is given at 40 mg/m². In some aspects, doxorubicin is given at 5-200, 10-150, 25-100, 30-50, 35-45, 10, 20, 30, 40, 50, 60, 70, 80, 90, 100, 110, 120, 130, 140, or 150 mg/m², inclusive.

A formulation of the invention also can include combinations of polypeptides in the formulation. For example, a formulation of the invention can include an polypeptide for treatment of one or more conditions. A formulation of the invention also can include two or more different polypeptides. Use of multiple polypeptides in a formulation of the invention can be directed to, for example, the same or different indications. Similarly, multiple polypeptides can be used in a formulation of the invention to treat, for example, both a pathological condition and one or more side effects caused by the primary treatment. Multiple polypeptides also can be included in a formulation of the invention to accomplish different medical purposes including, for example, simultaneous treatment and monitoring of the progression of the pathological condition. Multiple, concurrent therapies such as those exemplified above as well as other combinations well known in the art are particularly useful for patient compliance because an formulation can be sufficient for some or all suggested treatments and/or diagnosis. Those skilled in the art will know those polypeptides that can be admixed for a wide range of combination therapies. Similarly, a formulation of the invention also can be used with small molecule pharmaceuticals and combinations of one or more polypeptides together with one or more small molecule pharmaceuticals. Therefore, the invention provides for a formulation of the invention containing 1-10, 2-5, 1, 2, 3, 4, 5 or 6 or more different polypeptides as well as for one or more polypeptides combined with one or more small molecule pharmaceuticals.

A formulation of the invention also can include one or more preservatives and/or additives well known in the art. Similarly, a formulation of the invention can further be formulated into any of various known delivery formulations. For example, a formulation of the invention can include lubricating agents, emulsifying agents, suspending agents, preserving agents such as methyl- and propylhydroxy-benzoates, sweetening agents and flavoring agents. Such optional components, their chemical and functional characteristics are well known in the art. Similarly well known in the art are formulations that facilitate rapid, sustained or delayed release of the polypeptide after administration. A formulation of the invention can be produced to include these or other formulation components well known in the art.

A formulation of the invention also can be produced, for example, in states other than an aqueous liquid. For example, as a lyophilized formulation. A lyophilized formulation will generally contain, for example, a bulking or caking agent and an amorphous stabilizer.

Once a formulation of the invention is prepared as described herein, stability of the one or more polypeptides contained within the formulation can be assessed using methods well known in the art. Several of such methods are exemplified further below in the Examples and include size exclusion chromatography and particle counting. Any of a variety of functional assays including, for example, binding activity, other biochemical activity and/or physiological activity can be assessed at two or more different time points to determine the stability of the polypeptide in the buffered formulation of the invention.

A formulation of the invention will, in general, be prepared according to pharmaceutical standards and using pharmaceutical grade reagents. Similarly, a formulation of the invention will, in general, be prepared using sterile reagents in a sterile manufacturing environment or sterilized following preparation. Sterile injectable solutions can be prepared using well known procedures in the art including, for example, by incorporating one or more polypeptides in the required amount in an acetic acid, glutamic acid or succinic acid buffer or excipient of the invention with one or a combination of formulation components described herein followed by sterilization microfiltration. In the specific embodiment of sterile powders for the preparation of sterile injectable solutions, particularly useful methods of preparation include, for example, vacuum drying and freeze-drying (lyophilization) as described previously. Such drying methods will yield a powder of the one or more polypeptides together with any additional desired components from a previously sterile-filtered solution thereof.

### Kits

Also described herein are kits comprising a vial comprising a protein disclosed herein and instructions for use. The protein can be in any suitable pharmaceutical composition as described herein, e.g., a liquid suspension suitable for IV infusion. Alternatively, the protein can be in a lyophilized state suitable for re-suspension before use. The instructions for use can include storage instructions, patient selection, dosages, administration methods, time periods for use, clinical endpoints, and the like. In some aspects, the instructions include instructions to perform a method disclosed herein.

It must be noted that, as used in the specification and the appended claims, the singular forms "a," "an" and "the" include plural referents unless the context clearly dictates otherwise.

### EXAMPLES

Below are examples of specific embodiments. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

The practice of the present invention can employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B(1992).

### Example 1: Expression and Purification of STM 434.

### Cell Culture Process

STM 434 drug substance was expressed by a serum-free CS9 Chinese hamster ovary cell line. The sequence of STM 434 is shown in SEQ ID NO:10, where STM 434 is a homodimer of the sequence set forth in SEQ ID NO:10.

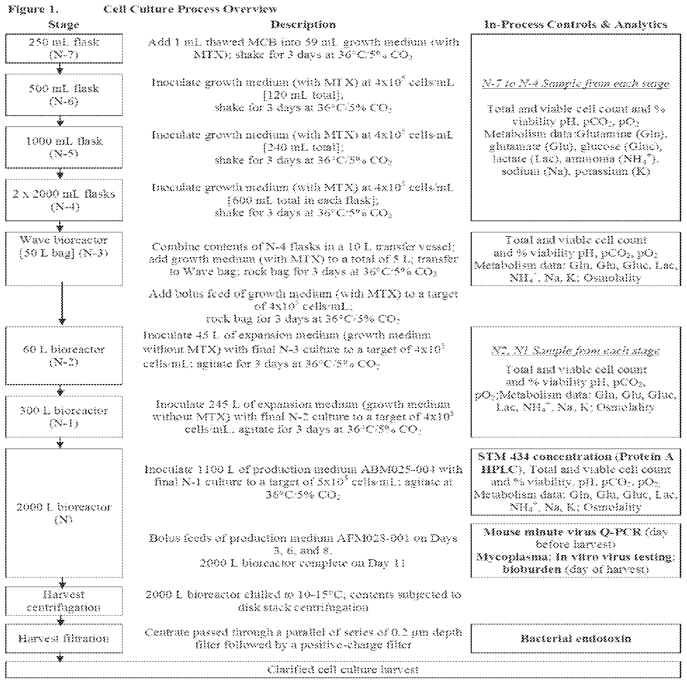

The creation of the Master Cell Bank (MCB) that expresses STM 434 was designated AMG 434 MCB. Seed expansion and cell culture production utilizes chemically defined media without animal-derived raw materials. The details of the cell culture process, including process controls, are described below, with a graphical depiction of the process in Figure 1. Certain parameters include product concentration determined by Protein A HPLC, Q-PCR for detection of contaminant MMV DNA, bacterial endotoxin, mycoplasma, adventitious virus, and total aerobic microbial count. In addition, two lots were previously manufactured (Lots 0010039909 and 0010039910), which were further processed by CMC Biologics to the formulated bulk drug substance (Lots 13-0066 and 13-0067).

### Thaw and Initial Expansion (Stages N-7 to N-4)

One vial of AMG 434 MCB was thawed, and the contents were transferred to a 250 mL shaker flask containing 59 ± 1 mL of the growth medium IMX 5.0, containing 300 nM methotrexate (MTX) and 100 µg/L insulin-like growth factor 1 (IGF-1), pre-warmed to 36.0 ± 1.0°C. The inoculated shaker flask was then incubated at 36.0 ± 1.0°C in a 5% CO₂ atmosphere using vented caps and agitation between 155 ± 5 revolutions per minute (rpm) for 72 ± 12 hours. After analyzing the cell culture, as defined in Figure 1, a portion of the cell culture was inoculated into pre-warmed growth medium in a 500 mL shaker flask to a target volume of 120 mL and target density of 4 x 10⁵ cells/mL. The shaker flask was incubated under the same conditions as above for 72 ± 12 hours. After analysis of the cell culture, a portion of the cell culture was inoculated into pre-warmed growth media in a 1 L shaker flask to a target volume of 240 mL and a target density of 4 x 10⁵ cells/mL, and the shaker flask was incubated under the same conditions as above for 72 ± 12 hours.

After analysis of the cell culture from the 1 L flask, this cell culture was used to inoculate two 2 L shaker flasks containing pre-warmed growth medium, each with a target volume of 600 mL and a target density of 4 x 10⁵ cells/mL. Both shaker flasks were incubated at 36.0 ± 1.0°C in a 5% CO₂ atmosphere using vented caps and agitation of 150-160 rpm for 72 ± 12 hours. When the incubation of the 2 L flasks was complete, samples from each flask were analyzed (Figure 1), after which the contents of the two flasks were combined. The cell density of the combined pool was measured, and the pool was then inoculated into pre-warmed growth medium in a 10 L glass transfer vessel to a target of 5 L and a target density of 4 x 10⁵ cells/mL. This culture was kept mixing at 90 ± 5 rpm prior to the next step of the process, which initiates less than 90 minutes from the time of pooling.

### Intermediate Seed Train (Stages N-3 to N-1)

The N-3 intermediate seed train stage was executed in a 50 L Wave bioreactor bag (GE Healthcare). After inflating the Wave bag, the 5 L pooled cell culture was transferred into the bag. Incubation was initiated under the conditions listed in Table 1. Samples were pulled daily and analyzed as described in Figure 1. On Day 3, after assessing viable cell density and percent viability (minimum of 1.6 x 10⁶ cells/mL and 90%, respectively), growth medium IMX 5.0 containing 300 nM MTX and 100 µg/L IGF-1 was transferred through an Opticap XL03 sterile filter (Millipore) into the Wave bag to achieve a cell density of 4 x 10⁵ cells/mL. The total cell culture volume after this bolus feed was generally 20-25 L. The incubation was continued at the same conditions (Table 1) for 3 more days, with daily sampling for analysis as described in Figure 1. Prior to harvest, the viable cell density and viability are expected to meet the criteria of no less than 1.6 x 10⁶ cells/mL and 90%, respectively.

**Table 1. 50 L Wave Bioreactor Process Parameters**

| **Parameter** | **Set points** |
|---|---|
| Wave bag capacity | 50 L |
| Volume of cell culture | 5 L |
| Temperature | 36.0°C |
| Rocker speed | 22 rpm |
| Rocker angle | 7° |
| CO₂ overlay | 5% @ 0.3 liters per minute (LPM) |

The second and third intermediate seed train stages (N-2 and N-1) were performed in 60 L and 300 L bioreactors, respectively. After transferring 45.0 ± 1.0 L of expansion media (IMX 5.0 containing 100 µg/L IGF-1 and no MTX), the bioreactor was set to the parameters described in Table 2. The bioreactor was then inoculated with the volume, within a range of ± 3%, of the final Wave bag cell culture required to achieve a starting cell density of 4 x 10⁵ cells/mL. The contents of the 60 L bioreactor were incubated for 3 days according to the conditions described in Table 2. Samples were taken daily for analysis, as described in Figure 1. During incubation, 1.0 M sodium carbonate was added automatically to control pH. Prior to harvest, the viable cell density and viability are expected to meet the criteria of no less than 2.0 x 10⁶ cells/mL and 90%, respectively.

**Table 2. 60 L Bioreactor Process Parameters**

| **Parameter** | **Set point** | **Range** |
|---|---|---|
| Temperature | 36.0°C | 35.0-37.0°C |
| Agitation | 83 rpm | 78-88 rpm |
| Pressure | 2.0 psig | 1.0-3.0 psig |
| pH | 7.00 | 6.90-7.10 |
| Dissolved oxygen (DO) | 48.0 mmHg | 28.0-220.0 mmHg |

Once the 60 L bioreactor incubation was completed, a 300 L bioreactor was prepared for inoculation by transferring 245.0 ±2.0 L of expansion media into the bioreactor, and setting it to the parameters described in Table 3. The bioreactor was then inoculated with the volume, within a range of ± 3%, of the final 60 L bioreactor culture required to achieve a starting cell density of 4 x 10⁵ cells/mL. The contents of the 300 L bioreactor were incubated for 3 days according to the conditions described in Table 3. Samples were taken daily for analysis, as described in Figure 1. During incubation, 1.0 M sodium carbonate was added automatically to control pH. Prior to harvest, the viable cell density and viability are expected to meet the criteria of no less than 2.3 x 10⁶ cells/mL and 90%, respectively.

**Table 3. 300 L Bioreactor Process Parameters**

| **Parameter** | **Set point** | **Range** |
|---|---|---|
| Temperature | 36.0°C | 35.0-37.0°C |
| Agitation | 80 rpm | 75-85 rpm |
| Pressure | 2.0 psig | 1.0-3.0 psig |
| pH | 7.00 | 6.90-7.10 |
| DO | 48.0 mmHg | 28.0-220.0 mmHg |

### Production Bioreactor

The final production bioreactor in the upstream processing of STM 434 has a nominal capacity of 2000 L. To initiate this stage of production, the bioreactor was charged with 1100.0 ±5.0 L of production medium ABM025-004, a chemically defined medium that includes custom components and the recipe for which is found in Table 4. The bioreactor was then set to the parameters defined in Table 5 to prepare for inoculation. The bioreactor was then inoculated with the volume, within a range of ± 3%, of the final 300 L bioreactor culture required to achieve a starting cell density of 5 x 10⁵ cells/mL. The contents of the 2000 L bioreactor were incubated for 11 days according to the conditions described in Table 5. During incubation, 1.0 M sodium carbonate was added automatically to control pH. On Days 3, 6, and 8 of the production bioreactor run, bolus feeds of production medium AFM028-001 (Table 4) were made at 8% of the initial culture. A 1% solution of simethicone was added (in 100 gram amounts) daily to the bioreactor as an antifoaming agent. Samples were taken daily for analysis, as described in Figure 1, including samples before and after bolus feed of AFM028-001. If the glucose concentration fell below 4.0 g/L, a 50% glucose solution was added to bring the concentration in the bioreactor to 8 g/L. The in-process testing for this process step is described in Table 13.

**Table 4. Production Bioreactor Medium Recipes**

| **Production medium** | **Component** | **Concentration** |
|---|---|---|
| ABM0025-004 (Target pH: 6.9 ± 0.1) | WFI | QS |
| | ABM025 (custom medium) | 11.04 g/L |
| | ASM003 (custom medium) | 7.68 g/L |
| | L-Tyrosine, Disodium, Dihydrate | 1.15 g/L |
| | L-Glutamine | 1.25 g/L |
| | Sodium Bicarbonate | 1.2 g/L |
| | Sodium Chloride | 1.0 N/A |
| | Potassium Chloride | 1.0 g/L |
| | Dextrose | 5.5 g/L |
| | Spermine Tetrahydrochloride (100 mM) | 100.0 µL/L |
| | 10 N Sodium Hydroxide | 550.0 µL/L |
| | 6 N Hydrochloric Acid | As needed |
| | IGF-1 (1 mg/mL) [added pre-filtration} | 100.0 µL/L |
| AFM028-001 (Target pH: 6.8 ± 0.1) | WFI | QS |
| | AFM023 (custom medium) | 11.1 g/L |
| | AFM028 (custom medium) | 74.0 g/L |
| | L-Tyrosine, Disodium, Dihydrate | 0.8 g/L |
| | Sodium Bicarbonate | 1.2 g/L |
| | L-Glutamine | 3.0 g/L |
| | 10 N Sodium Hydroxide | 4.0 mL/L |
| | 6 N Hydrochloric Acid | As needed |

**Table 5. 2000 L Production Bioreactor Process Parameters**

| **Parameter** | **Set point** | **Range** |
|---|---|---|
| Temperature | 36.5°C | 36.0-37.0°C |
| Agitation | 74 rpm | 69-79 rpm |
| Pressure | 2.0 psig | 1.0-3.0 psig |
| pH | 7.05 | 7.0-7.1 |
| DO | 48.0 mmHg | 28.0-220.0 mmHg |

### Cell Culture Harvest Clarification and Filtration

At the end of the production bioreactor run, after sampling has been performed, the bioreactor was chilled to 10-15°C, with the pressure adjusted to 8.0-12.0 psig. A transfer line was placed between the 2000 L bioreactor and a CSC-20 disk stack centrifuge (Westfalia), along with an outlet line from the centrifuge to a centrate collection tank. The packed cell volume of the bioreactor contents was measured, and this value was used to determine the shot interval value, which was included in the programming of the centrifuge, along with other parameters as shown in Figure 1. The line between the bioreactor and the centrifuge was opened, and centrifugation was initiated. Once the centrifugation was completed, the centrifuge bowl was flushed, and the product was chased to the centrate collection tank with a solution of 25 mM Tris, 100 mM sodium chloride, pH 7.4 (Tris-buffered saline; TBS). Centrifugation operational parameters are summarized in Table 6.

**Table 6. Harvest Centrifugation Operational Parameters**

| **Parameter** | **Set point** |
|---|---|
| Feed Rate | 12 LPM |
| Back Pressure | 75 psig |
| Target Shot Mass | 4.0 ±1.0 kg |
| Speed | 8400 rpm |

After centrifugation operations, the harvest pool was subjected to depth filtration through parallel series of 2 filters. The first filter in each train was a Sartopore 2 0.2 µm Maxicap cartridge (Sartorius; 1.2 m² membrane area) for sterilizing depth filtration. The second filter was a Zeta Plus Maximizer 120ZA capsule (Cuno; 1.84 m² membrane area) for the purpose of reducing nucleic acid levels. After the filters were flushed with TBS, the contents of the centrate collection tank were pumped through the filter trains into a filtrate collection tank according to the conditions shown in Table 7. Once the centrate was completely transferred, 220 L TBS was added to the centrate collection tank, after which 100 L are pushed through each filter train into the filtrate collection tank. The filtration process was completed by pushing air through each filter train, collecting the liquid in the filtrate collection tank. At the end of filtration, the filtrate pool was mixed in a manner which avoids foaming. The filtrate pool was held at 2-8°C for no more than 72 hours, with samples taken for analysis according to Table 13.

**Table 7. Depth Filtration Operational Parameters**

| **Parameter** | **Set point** |
|---|---|
| Flow Rate | ≤ 12.3 LPM |
| Inlet Pressure | ≤ 30 psig |
| Sartopore Filter Pressure Differential | ≤ 20 psig |
| Zeta Plus Filter Pressure Differential | ≤ 20 psig |
| Filtrate Tank Agitation | Moderate; initiated after 300 L collected |
| Pressure Differential During Air Push | ≤ 20 psig |
| Filtrate Pool Mixing | Moderate; ≥ 20 minutes |

### Purification Process

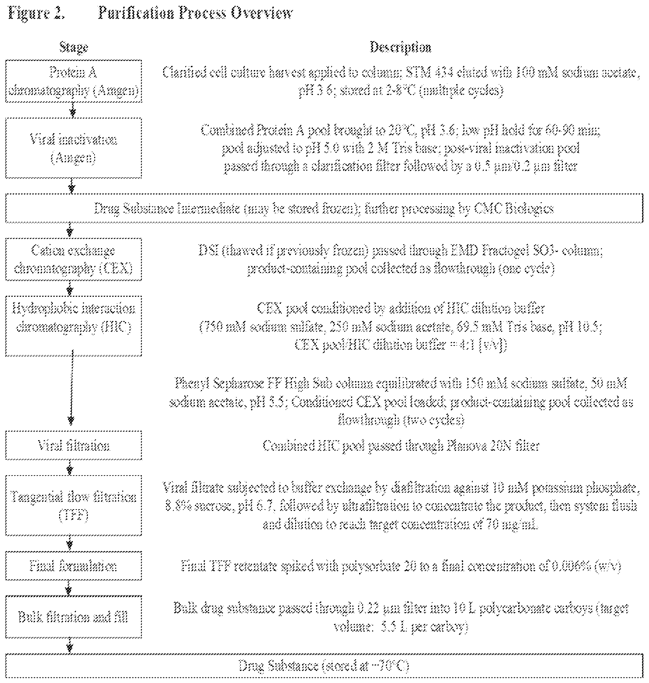

The STM 434 downstream purification process includes three column chromatography steps, a low pH viral inactivation step, a viral filtration step, and a final formulation step using tangential flow filtration. All of the steps in this process were performed at ambient temperature, except where specifically noted. Figure 2 depicts the STM 434 purification process. For each cell culture run, an run of the purification process was performed. The final formulation of STM 434 drug substance was 10 mM potassium phosphate, 8.8% (w/v) sucrose, 0.006% (w/v) polysorbate 20, pH 6.7, at a target protein concentration of 70 mg/mL. The purification process includes two robust orthogonal methods for viral clearance using two model viruses (xMuLV and MMV).

### Protein A Chromatography

Protein A chromatography was used to capture antibodies and Fc-fusion proteins, such as STM 434, through the affinity of Protein A for Fc domains. The STM 434 purification process utilizes the MabSelect SuRe Protein A resin (GE Healthcare). The Protein A affinity purification step provides extensive reduction of host cell proteins and DNA, in addition to potential viruses. The operational parameters for the Protein A chromatography step are described in Table 8. For the two cGMP purification runs, a column with a resin volume of 39.3 L was utilized. Based on the 20 g/L load density, the Protein A step was run in cycles, the number of cycles and the volume of clarified cell culture harvest loaded in each cycle being determined by the total mass of STM 434 in the final clarified harvest of each of two bioreactor runs.

The Protein A column was prepared for the processing by flushing with equilibration buffer (TBS). The first bolus of clarified cell culture harvest was loaded onto the column, followed by washing the column with a minimum of 80 L of equilibration buffer. The column was then washed with a minimum of 160 L of 25 mM Tris, 500 mM calcium chloride, pH 7.5. This step was introduced to provide additional clearance of host cell DNA. The column was then washed with a minimum of 120 L of equilibration buffer to remove any residual calcium chloride. STM 434 was then eluted from the column with 100 mM sodium acetate, pH 3.6. The collection criteria, based on absorbance of the column effluent, are provided in Table 8. The elution pool was kept in a tank maintained at 2-8°C. After the elution step, the column was stripped with a minimum of 120 L of 100 mM phosphoric acid. This cycle was repeated until all of the clarified cell culture harvest was consumed. After the last cycle was completed, the column was flushed with a minimum of 40 L of equilibration buffer, followed by a minimum of 120 L of 300 mM sodium hydroxide to regenerate the resin. The column was stored in between runs in 2% benzyl alcohol, 50 mM citrate, pH 5.0. Multiple samples were collected from this unit operation as described in Table 13.

**Table 8. Protein A Chromatography Operational Parameters**

| **Parameter** | **Set Point** |
|---|---|
| Bed Height | 25 ± 2 cm |
| Maximum Load Density | 20.0 g STM 434 per L resin |
| Flow rate | ≤ 6.6 LPM (target: 250 cm/hr) |

### Low pH Viral Inactivation

The combined Protein A pool was subjected to viral inactivation through the use of low pH. The pool was brought to a temperature of 20 ± 2°C, after which it was adjusted to pH 3.6 ± 0.1 by the slow addition of 10% (v/v) acetic acid. The titrated pool was held at low pH for a 60-90 minutes. After the inactivation period, the pool was adjusted to pH 5.0 ± 0.1 by the slow addition of 2.0 M Tris base.

The viral inactivation pool was then passed through a filter train including three different types of filters. The first filter was a Millistak + HC Pod (Millipore; 1.1m² membrane area), which removes particles that may have formed during acid treatment. The second filter in the train was the Sartobind Q (Sartorius; 1.98 m² membrane area), with three 30-inch capsules arranged in parallel. This ion exchanger serves to further reduce nucleic acids and other negatively charged impurities. The final filter was an Express SHC Opticap XL 10 0.5 µm/0.2 µm capsule (Millipore; 049 m² membrane area). The filtration was executed at a flow rate no greater than 6.6 L/minute. The filtered viral inactivation pool (FVIP) was collected into a tank. When the pre-filtration viral inactivation pool was near the bottom of its holding vessel, the remainder was chased with a minimum of 40 L of 50 mM sodium acetate, 70 mM sodium chloride, pH 5.0 into the FVIP collection tank. Multiple samples of the FVIP were collected from this unit operation as described in Table 13.

For the first two cGMP runs the FVIP, referred to as the drug substance intermediate (DSI), was stored in sterile 20 L Flexboy bags (Sartorius), and kept them at -30°C. Portions of first lot of DSI (Lot #0010039909) were stored in polycarbonate bottles and stored at -30°C, to serve as stability samples to assess the potential impact of long-term storage on product quality. The results of 24 and 35 month stability studies indicate that the quality of the DSI stored at -30°C for 35 months was retained throughout the storage period, and that the DSI could be used to evaluate suitability for use in cGMP drug substance manufacturing through the Demo DS lot.

### Cation Exchange Chromatography

STM 434 DSI was further processed by the use of cation exchange chromatography (CEX); this step was performed in flow-through mode using EMD Fractogel SO3- resin (Merck). This step was developed for the reduction of host-cell DNA and proteins, in addition to other potential impurities, and was also shown to be capable of increasing the level of more highly sialylated forms of STM 434. The operational parameters for the CEX step are described in Table 9. For the two cGMP purification runs, a column with a packed resin height of 21.5 cm, corresponding to a column volume of 33.6 L, was utilized. Based on the load density range of 75-125 g/L, the CEX step was run in an cycle.

Prior to initiating chromatography operations, frozen STM 434 DSI was statically thawed at 2-8°C for 4-6 days, followed by overnight storage at ambient temperature. The CEX column was prepared for use by first flowing a minimum of three column-volumes (CVs) of 50 mM sodium acetate, 500 mM sodium chloride, pH 5.0, followed by a minimum of three CVs of 50 mM sodium acetate, 70 mM sodium chloride, pH 5.0. The thawed STM 434 DSI was then loaded onto the column. For the two cGMP purification runs, different total volumes of STM 434 DSI were loaded onto the CEX column, each lot coming from a different upstream process run. The total volume of STM 434 DSI lot 0010039909 in the first run was 260.6 L (22 Flexboys), for a total STM 434 mass of 2907 grams. The total volume of STM 434 DSI lot 0010039910 in the second run was 294 L (25 Flexboys), for a total STM 434 mass of 3376 grams.

The collection of product-containing eluate was initiated by switching the eluate flow toward a sterile collection bag through a sterile filter when the UV absorbance reaches 0.50 ± 0.1 OD at 280 nm. After loading all the DSI onto the column, the CEX column was then washed with 50 mM sodium acetate, pH 5.0. The collection of product-containing eluate (CEX pool) continues until the UV absorbance reaches 0.50 ± 0.1 OD at 280 nm. A minimum of three CVs of the wash buffer were flowed through the column prior to switching back to 50 mM sodium acetate, 500 mM sodium chloride, pH 5.0 to strip the column, followed by a minimum of three CVs of 0.5 N sodium hydroxide for column sanitization, followed by a minimum of three CVs of 0.1 N sodium hydroxide for column storage. The CEX pool was aseptically sampled for in-process testing as described in Table 13, and was kept overnight at ambient temperature.

**Table 9. Cation Exchange Chromatography Operational Parameters**

| **Parameter** | **Target Ranges** |
|---|---|
| Bed Height | 20 ± 2 cm |
| Load Density | 75-125 g STM 434 per L resin |
| Flow rate | ≤ 4.7 LPM (target: 250 cm/hr) |
| Pre-load pH | pH 4.8-5.2 at 17-25°C |
| Pre-load Conductivity | 9-11 mS/cm |

### Hydrophobic Interaction Chromatography

The STM 434 CEX pool was subjected to hydrophobic interaction chromatography (HIC); this step was performed in flow-through mode using Phenyl Sepharose FF High Sub resin (GE Healthcare). This step was developed for the reduction of product aggregates and other potential process impurities. The operational parameters for the HIC step are described in Table 10. For the two cGMP purification runs, a column with a packed resin height of 27.0 cm, corresponding to a column volume of 42.2 L, was utilized. Based on the maximum load density of 50 g/L, the HIC step was performed in two cycles for each lot.

The CEX pool was prepared for the HIC step by conditioning the pool with a dilution buffer consisting of 750 mM sodium sulfate, 250 mM sodium acetate, 69.5 mM Tris base, pH 10.5. The conditioning ratio was one part dilution buffer per four parts CEX pool. While the conditioned CEX pool was being mixed, the HIC column was equilibrated by flowing a minimum of three CVs of 150 mM sodium sulfate, 50 mM sodium acetate, pH 5.5. Once the starting pH and conductivity of the column effluent were met, as noted in Table 10, half of the conditioned CEX pool (HIC load) was loaded onto the column. The collection of product-containing eluate (HIC pool) was initiated by switching the eluate flow toward a collection bag when the UV absorbance reaches 0.2 ± 0.1 OD at 280 nm. After loading the entire HIC load onto the column, the HIC column was then washed with 150 mM sodium sulfate, 50 mM sodium acetate, pH 5.5. The collection of the HIC pool continued until the UV absorbance reaches 1.50 ± 0.1 OD at 280 nm, when the effluent was switched back to waste collection. A minimum of three CVs of the wash buffer were pumped through the column, followed by a minimum of three CVs of 0.5 N sodium hydroxide to strip the column. The column was then washed with a minimum of three CVs of WFI to complete the first cycle. During the regeneration steps, the first cycle HIC pool was mixed to ensure homogeneity and then transferred through a sterile filter into a sterile bioprocess bag.

Upon completion of the WFI column wash, the HIC equilibration process was repeated, followed by HIC processing of the remaining half of the HIC load as described above. The second cycle HIC pool was mixed and then transferred through a sterile filter to the bioprocess bag holding the first cycle HIC pool. The combined HIC pool was kept at ambient temperature for overnight storage. The HIC column was stored in 0.1 N sodium hydroxide by pumping a minimum of three CVs through the column after the 0.5 N sodium hydroxide column strip. Multiple samples for in-process testing were collected as described in Table 13.

**Table 10. Hydrophobic Interaction Chromatography Operational Parameters**

| **Parameter** | **Target Ranges** |
|---|---|
| Bed Height | 25 ± 2 cm |
| Maximum Load Density | 50 g STM 434 per L resin |
| Flow rate | ≤ 4.7 LPM |
| Pre-load pH | pH 5.4-5.6 at 17-25°C |
| Pre-load Conductivity | 23-27 mS/cm |

### Viral Filtration

The combined HIC pool was filtered through a Planova 20 N filter (Asahi Kasei Bioprocess; 4.0 m² membrane area) as an orthogonal viral reduction step. Prior to processing the product-containing pool, the filter was leak-tested with an inlet pressure of 13.5-14.0 psig. Upon passing the leak test, a 0.2 µm Sartopore 2 capsule (Sartorius; 0.2 m² membrane area) was installed upstream of the viral filter, and this system was equilibrated by flushing with 40 kg ± 2 kg of 150 mM sodium sulfate, 50 mM sodium acetate, pH 5.5, according to the parameters listed in Table 11. After mixing and sampling the combined HIC pool was pumped through the viral filter at a target pressure of 12 ± 2 psig. The viral filtrate was collected through direct connection of the filter train to a sterile bioprocess bag. Once filtration of the combined HIC pool was completed, the residual product was chased from the filter and transfer lines with no less than 16 kg of 150 mM sodium sulfate, 50 mM sodium acetate, pH 5.5. After the run was complete, the 0.2 µm filter undergoes integrity testing (using a bubble point test), and the viral filter was flushed with a minimum of 16 L of 0.25 N sodium hydroxide, 0.5% (v/v) Triton X-100, followed by a minimum of 32 L of WFI. Following these flushes, the viral filter was subjected to gold particle testing to ensure pore size distribution, followed by post-use leak testing. The viral filtrate was transferred to a post-viral suite, which has the highest positive pressure in the facility and also a dedicated air handling unit with no recirculated air, and was kept at ambient temperature. Samples for in-process testing were collected as described in Table 13.

**Table 11. Viral Filtration Operational Parameters**

| **Parameter** | **Set Point** |
|---|---|
| Pre-use Leak Test (Viral Filter) | 13.5-14.0 psig |
| Operating Pressure | 12 ± 2 psig |
| Filter Loading Limit | ≤ 325 L/m² |
| Equilibration pH | pH 5.4-5.6 at 17-25°C |
| Equilibration Conductivity | 23-27 mS/cm |
| Post-use Integrity Test (0.2 µm filter) | WFI bubble point ≥ 46 psig |
| Post-use Leak Test (Viral Filter) | 13.5-14.0 psig |

### Ultrafiltration/Diafiltration by Tangential Flow Filtration

The STM 434 viral filtrate was subjected to ultrafiltration (UF) and diafiltration (DF) to achieve buffer-exchange and concentration of the drug substance to its final formulation through the use of tangential flow filtration (TFF). Two critical solutions were used to obtain the formulated bulk drug substance. These solutions were: (1) 10 mM potassium phosphate, 8.8% (w/v) sucrose, pH 6.7, and (2) 1% (w/v) Tween-20 (polysorbate 20). These solutions were prepared on an as-needed basis.

For the TFF step, four Pellicon 3 Ultracel TFF membranes (Millipore; 10 kDa nominal molecular weight limit; 1.14 m² membrane area each) were used to process the entirety of the viral filtrate in an cycle, based on a target load of 400 ± 50 g/m². New membranes were flushed with WFI until the retentate conductivity went below its set point, as described in Table 12, followed by flushing with 0.5 N sodium hydroxide until the permeate flush volume reaches a minimum of 40 L. After initial membrane flushing, and at the beginning of TFF processing with used membranes, the base was flushed from the system with WFI until the retentate and permeate conductivities go below their set point (Table 12). After filter integrity testing was performed (using a normalized water permeability test), sanitization was performed by pumping a minimum of 40 L of 0.5 N sodium hydroxide through the system, with the sanitization solution being held in the system for 1-24 hours. After sanitization, the TFF system was again flushed with WFI until the retentate and permeate conductivities go below their set point (Table 12), along with the retentate flush volume reaching a minimum of 40 L.

To initiate STM 434 viral filtrate processing, the system was equilibrated with 10 mM potassium phosphate, 8.8% (w/v) sucrose, pH 6.7. Equilibration was complete once the retentate and permeate pH reach the target range of pH 6.5-6.9, with a permeate flush volume of no less than 20 L. The viral filtrate was mixed and sampled from the upper and lower portions of the retentate container to measure protein concentration, to ensure complete mixing and that the protein/surface area ratio meets its target (Table 12). Protein concentration by UF was then initiated, with the feed flow rate adjusted to achieve a transmembrane pressure of 20 ± 5 psig. When the retentate volume was reached that corresponds to a protein concentration of 60 g/L, the retentate sampled from the upper and lower portions of the retentate container to confirm the protein concentration. The retentate was then subjected to DF against 10 diavolumes of 10 mM potassium phosphate, 8.8% (w/v) sucrose, pH 6.7. After DF was complete, UF was resumed to bring the retentate concentration to a target of 105 g/L, based on the retentate volume that achieves this concentration. After draining the retentate side of the TFF system into the retentate container, the system was flushed with 5 L of 10 mM potassium phosphate, 8.8% (w/v) sucrose, pH 6.7, and the flush was added to the retentate container. After 10-15 minutes of mixing, samples were taken from upper and lower portions of the retentate for protein concentration measurement, to ensure sufficient mixing and concentration of the product during the DF step. The retentate was further diluted with 10 mM potassium phosphate, 8.8% (w/v) sucrose, pH 6.7 as necessary to bring the final concentration to 70.0 ± 3.5 g/L. The final TFF pool was sterile filtered into a bioprocess bag for storage prior to final formulation and bulk fill. Retentate samples for in-process testing were collected as described in Table 13.

**Table 12. Tangential Flow Filtration Operational Parameters**

| **Parameter** | **Set Point** |
|---|---|
| Protein/Surface Area Ratio | ≤ 600 g/ m² (target: 400 ± 50 g/m²) |
| Transmembrane Pressure | 15-25 psig |
| Feed Flow | 270 L per m² per hour (LMH) |
| Retentate Conductivity (initial WFI flush) | ≤ 1000 µS/cm |
| Retentate and Permeate Conductivity (post-NaOH flushes) | ≤ 1000 µS/cm |
| Permeate pH After Equilibration | pH 6.5-6.9 |
| Concentration Target at First Stage of Ultrafiltration | 60 g/L |
| Diafiltration Volume | ≥ 10 diavolumes |
| Concentration Target at Second Stage of Ultrafiltration | 105 g/L |
| Concentration Target (final) | 70.0 ± 3.5 g/L |

### Final Formulation and Fill of Drug Substance

The final formulated STM 434 drug substance was prepared and filled following the TFF step. A sufficient volume of a 1% (w/v) solution of polysorbate 20 is added to the TFF pool to yield a final polysorbate 20 concentration of 0.006% ± 0.003% (w/v). After addition of the surfactant, the bulk drug substance was mixed for 10-15 minutes. The laminar air flow hood in the fill suite was cleaned, and the fill equipment was set up, including the Opticap XL5 0.22 µm filter capsule (Millipore; 0.35 m² membrane area). Prior to filtering the final formulated drug substance, the filter was flushed with 30 ± 1 L of STM 434 formulation buffer (10 mM potassium phosphate, 8.8% [w/v] sucrose, 0.006% [w/v] polysorbate 20, pH 6.7) to saturate the membrane with polysorbate 20 and avoid its removal from the formulated drug substance. Following the flush with formulation buffer, the filter was flushed with 340-360 g of the formulated drug substance, the flush being discarded. The remaining formulated drug substance was then filtered into 10 L polycarbonate carboys, to a target volume of 5500 ± 500 mL per carboy. The filter was integrity tested (using a bubble point test) following filtration. The final carboy can be filled with more than the target volume of formulated drug substance, but not generally more than 8 L. Samples were taken for in-process testing (for information only) and lot release testing, after which the carboy closures were secured at a torque setting of 80 in-lb. The carboys were labeled, and then stored at -70°C.

### In-Process Monitoring During the STM 434 Purification Process

Samples were collected and evaluated during the purification processes as described in Table 13.

**Table 13. In-Process Monitoring During the STM 434 Purification Process**

| **Test Method** | **Parame ter Reported** | **Step/Samples Tested** | | | | | | |
|---|---|---|---|---|---|---|---|---|
| | | **Harvest** | **Protein A chromatogr aphy** | **Viral Inactivati on** | **CEX** | **HIC** | **Viral filtrati on** | **TFF** |
| Protein A HPLC | STM 434 concentration | Depth filtrate | - | - | | - | | |
| Protein Concentration by UV Absorbance | STM 434 concentration | - | All cycles Combined pool | FVIP | Load, Pool | Both cycles, Pool | Filtrate | Retenta te |
| CHO HCP ELISA | HCP content | Depth filtrate | Combined pool | FVIP | Load, Pool | Pool | | |
| Q-PCR for CHO DNA | CHO DNA content | Depth filtrate | Combined pool | FVIP | Load, Pool | Pool | | |
| Residual Protein A ELISA | Residual Protein A content | - | First & last cycles Combined pool | FVIP | Load, Pool | Pool | | |
| TAMC by Membrane Filtration | Bioburden | Depth filtrate | Combined pool | FVIP | Load, Pool | - | | Retenta te |
| TYMC by Membrane Filtration | Bioburden | - | - | - | Load, Pool | - | | Retenta te |
| Bacterial Endotoxin Content (Kinetic LAL) | Endotoxin | Depth filtrate | Combined pool | FVIP | Load, Pool | - | | Retenta te |
| Size Exclusion HPLC | Purity | - | Combined pool | FVIP | Load, Pool | Both cycles, Pool | Filtrate | Retenta te |
| CE-SDS, Reduced | Purity | - | Combined pool | FVIP | Load, Pool | - | | Retenta te |
| CE-SDS, Nonreduced | Purity | - | - | FVIP | Load, Pool | - | | |
| icIEF | Purity | - | - | - | Load, Pool | Both cycles, Pool | | Retenta te |
| Sialic Acid Content | Purity | - | - | - | Load, Pool | Both cycles, Pool | | |

| | | | | | | | | |
|---|---|---|---|---|---|---|---|---|
| CE-SDS = sodium dodecyl sulfate capillary electrophoresis; CEX = cation exchange chromatography; CFU = colony forming unit; CHO = Chinese hamster ovary; ELISA = enzyme-linked immunosorbent assay; FVIP = filtered viral inactivation pool; HCP = host cell protein; HIC = hydrophobic interaction chromatography; icIEF = imaged capillary isoelectric focusing; LAL = Limulus amebocyte lysate; Q-PCR = quantitative polymerase chain reaction; TAMC = total aerobic microbial count; TFF = tangential flow filtration; TYMC = total yeast and mold count | | | | | | | | |

### Process Step and Overall Yields

The individual step yields for the STM 434 cGMP drug substance manufacturing runs are provided in Table 14. Overall, the process was reproducible and provided sufficient material for Phase 1 clinical trials.

**Table 14. STM 434 Step and Overall Yield Calculations**

| **Unit operations performed by Amgen** | **DSI Lot 0010039909** | | | | | **DSI Lot 0010039910** | | | |
|---|---|---|---|---|---|---|---|---|---|
| | **Volume (L)** | **Concentration (g/L)** | **Total Protein (g)** | **Step Yield (%)*** | | **Volume (L)** | **Concentration (g/L)** | **Total Protein (g)** | **Step Yield (%)*** |
| Harvest Media | 1653 | 2.35 | 3884.6 | | | 1790 | 2.64 | 4725.6 | |
| Harvest Depth Filtrate | 1730 | 2.06 | 3562.8 | 92% | | 1820 | 2.36 | 4295.2 | 91% |
| Protein A Chromatography Load* | 216.2 | 15.2 | 3286.2 | 92% | | 1615 | 2.36 | 3811.4 | 89%* |
| Protein A Chromatography Pool | 216.2 | 15.2 | 3286.2 | 100% | | 225.9 | 15.7 | 3546.6 | 83% |
| Filtered Viral Inactivation Pool (DSI) | 289.8 | 11.2 | 3245.8 | 99% | | 296.9 | 11.6 | 3444.0 | 97% |
| **Overall Yield*** | | | | 84% | | **Overall Yield*** | | | 73% |
| | | | | | | | | | |

| **Unit operations performed by CMC Biologics** | **DS Lot 13-0066 (from DSI Lot 0010039909)** | | | | | **DS Lot 13-0067 (from DSI Lot 0010039910)** | | | |
|---|---|---|---|---|---|---|---|---|---|
| | **Volume (L)** | **Concentration (g/L)** | **Total Protein (g)** | **Step Yield (%)**** | | **Volume (L)** | **Concentration (g/L)** | **Total Protein (g)** | **Step Yield (%)**** |
| CEX Load (equivalent to DSI) | 259.9 | 11.256 | 2925.4 | | | 294.2 | 11.472 | 3375.1 | |
| CEX Pool | 269.3 | 8.844 | 2381. 7 | 81% | | 302.8 | 9.405 | 2847.8 | 84% |
| HIC Load (conditioned CEX Pool) | 338.8 | 7.038 | 2384.5 | 100% | | 377.5 | 7.107 | 2682.9 | 94% |
| HIC Pool | 442.7 | 4.882 | 2161.3 | 91% | | 491.7 | 5.067 | 2491.4 | 93% |
| Viral Filtrate/TFF Load | 457.3 | 4.535 | 2073.9 | 96% | | 504.3 | 4.906 | 2474.1 | 99% |
| TFF Pool | 28.0 | 67.416 | 1887.6 | 91% | | 36.7 | 67.788 | 2487.8 | 101% |
| Bulk Drug Substance | 28.060 | 68.800 | 1930.5 | 102% | | 36.640 | 70.300 | 2575.8 | 104% |
| **Overall Yield by CMC Biologies**** | | | | 66% | | **Overall Yield by CMC Biologies**** | | | 76% |

| | | | | | | | | | |
|---|---|---|---|---|---|---|---|---|---|
| *Yield percentages reported for Amgen are based on the initial input of cell culture harvest for each manufacturing run. The differences in the two runs are due to different bioreactor yields, and Amgen's decision to limit the Protein A chromatography step to five cycles while maintaining the target protein loading parameters. **Yield percentages reported for CMC Biologics are based on the initial input of DSI, the available volumes of which were different for each manufacturing run. The DSI concentration values reported in this section of the table are those determined by CMC Biologics. | | | | | | | | | |

### Example 2: Description of Manufacturing Process and Process Controls

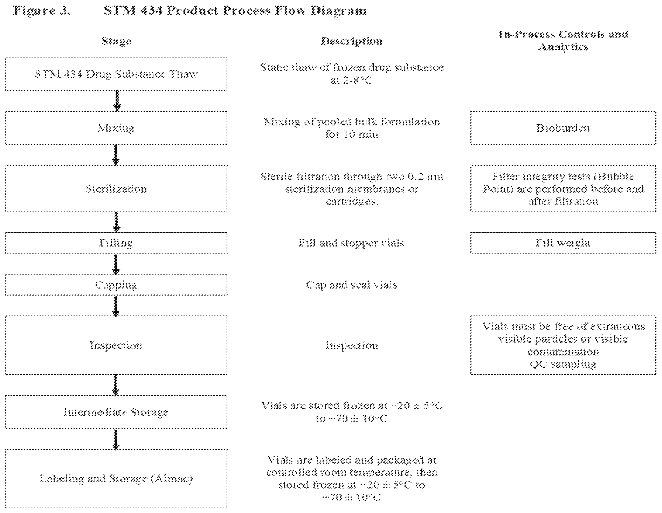

The formulated bulk drug substance was shipped frozen for aseptic processing to unlabeled drug product, which was then shipped for labeling, packaging, and distribution to clinical sites. Each STM 434 Injection lot includes a minimum of 7000 filled vials available for use in clinical trials. A process flow chart for the final drug product process is shown in Figure 3.

### Preparation of Components and Equipment

The vials were removed from the glass manufacturer's packaging and washed with hot Water for Injection (WFI). Washed vials were then placed in covered, stainless steel trays and depyrogenated in a dry heat-sterilizing oven. The sterilizing oven air was HEPA-filtered. Stoppers were washed and rinsed with hot WFI, followed by sterilization and drying in an autoclave. All sterile/depyrogenated components were stored in a controlled environment before use.

An overkill approach was used in developing and validating steam sterilization cycles. The cycles used provide a sterility assurance level (SAL) of at least 1 x 10⁻⁶. The dry-heat depyrogenation cycles used provide a minimum 3-log reduction in endotoxin.

### Manufacturing Process

In-coming material controls included identity testing and confirmation of the acceptability of the formulated bulk drug substance certificate of analysis. STM 434 drug substance was thawed statically (for no less than 7 days) at 2-8°C and held at this temperature for further processing. Material was moved to the operational area and allowed to equilibrate to room temperature (30-60 minutes). For a typical batch, 2 carboys, containing 5.5 L each, were pooled into a 13 L glass carboy and mixed for no less than 10 minutes at 300 rpm. A sample was collected for bioburden testing. Pooling and mixing operations occur in an ISO Class 7 (formerly FS209E Class 10,000) controlled environment area. The container of pooled formulated bulk solution was then transferred in preparation for product filter sterilization. This pooled container was connected to the autoclaved filter assembly. The filter assembly [tubing] was also connected to the filling surge vessel. The solution is sterilized by membrane filtration through two sterilizing grade filters, used in series, with a pore size rating of 0.22 µm. The membrane filters were sterilized and integrity tested (using a bubble point test) before and after use. Samples were tested according to written specifications.

The filling surge vessel containing the sterile pooled formulated bulk solution was then aseptically connected to the filler;. The sterile pooled formulated bulk solution was aseptically filled into the sterile, depyrogenated vials. Sterile stoppers and seals were then placed on each vial within the RABS unit. The filling operation was performed by suitably gowned and trained operators. These operations were designed, equipped, and operated to provide environmental conditions suitable for aseptic product processing.

Fill weight checks were conducted during the filling operation. After vials were filled, stoppered and sealed, they were all inspected to ensure no extraneous visible particles, precipitate, visible contamination, evidence of spillage, vial breakage, or unacceptable stoppers or seals. Additionally, a statistical sampling of the entire lot of vials was performed and inspected by the quality unit.

Sample vials were collected at this point for QC testing. All acceptable vials were placed in quarantine storage at -20 ± 5°C to -70 ± 10°C.

Controlled environment areas were maintained under positive pressure with HEPA-filtered air to reduce the possibility of airborne contamination. The performance of the filters was periodically monitored. Access to filling rooms was restricted to personnel associated with the filling operation who have completed the necessary training in aseptic operations.

During aseptic operations such as filling and stoppering, the ISO Class 5 environment was monitored for microbial content and non-viable particulate content. Appropriate action and alert levels were established for both viable contaminants and non-viable particulates to ensure appropriate quality of the filling environment air and important surfaces.

### Media Fill Validation

Media fills that simulate filling operations were conducted at routine intervals (6 months) to provide additional assurance of aseptic processing capability of staff, facility, and equipment. Media fills were conducted per approved protocol.

Data from process simulations (media fills) that serve to validate the specific filtration, filling, stoppering, and capping processes used in the manufacture of the drug product, included but are not limited to, notation of filling room, container closure type and size, volume of media, type of media, number of units filled, duration of filling and holding periods, number of units incubated, number of units positive, incubation parameters, date of media fill, process interventions, microbial monitoring, and process parameters.

Media fill sample vials were initially Swirled/Inverted to ensure that the TSB has been in contact with all of the internal surfaces, the containers were incubated upright for 7 days (not less than 168 hours) at 20° 25°C, followed by incubation at 30°-35°C inverted for an additional 7 days (not less than 168 hours). After incubation, compendial (USP) Growth Promotion Testing was performed on representative samples of the media vials. Failure of the post-incubation growth promotion test results in the invalidation of a media fill run. All media fills runs that are aborted or invalidated were followed by an investigation. Process simulations were designed to incorporate worst-case and/or challenge conditions. A bracketing concept was applied for the definition of worst case container closure combinations. The definition of the worst-case formats was based on vial volume, dimensions, aperture, and the complexity of the process (ie, liquid or freeze-dry). Freeze-drying processes can be representative for liquid processes as a result of the higher complexity of a lyophilization process. Routine interventions were performed in each media fill.

### Labeling and Packaging (Primary Container)

Following fill/finish operations, the STM 434 Injection vials were placed in paperboard cartons for intermediate storage. The cartons were labeled with product-specific and lot-specific information and then placed in frozen storage. These cartons of vials were shipped at -70 ± 10°C, and the vials in the cartons are stored at -20 ± 5°C. The labeling operation occurs in a room-temperature suite. Small batches of vials were brought to the suite and labeled within 12 hours then returned to -20 ± 5°C. Each vial was prepared for labeling by wiping residual condensation from the exterior, and then affixing the label.

Printed components were inspected to assure the content was appropriate per the master label. Upon completion of the inspection, the labels were released for use on primary containers. Approved written procedures were used for labeling and packaging processes.

### Example 3: Batch Formula for STM 434

STM 434 Injection was formulated as 70 mg/mL STM 434, 10 mM potassium phosphate, 8.8% (w/v) sucrose, 0.006% (w/v) polysorbate 20, pH 6.7. Each 5 mL vial was nominally filled with no less than 1.0 mL STM 434 drug substance. For each of the first two cGMP batches of STM 434 Injection, 11 L of STM 434 drug substance was received from CMC Biologics to execute drug product manufacturing. This volume is used in Table 155 to define the amount of each component per batch.

**Table 15. Components of STM 434 Injection and Typical Batch Size**

| **Ingredient** | **Grade** | **Amount per mL** | **Amount per Batch (11 L)** |
|---|---|---|---|
| STM 434 | S-SPEC-QA-002 | 70 mg | 770 grams |
| Potassium Phosphate Monobasic | NF, Ph. Eur. | 0.714 mg | 7.854 grams |
| Potassium Phosphate Dibasic | USP, Ph. Eur. | 0.827 mg | 9.097 grams |
| Sucrose | NF, Ph. Eur., BP, JP | 88.0 mg | 968 grams |
| Polysorbate 20 | NF, Ph. Eur., BP | 0.06 mg | 0.66 grams |
| Hydrochloric acid | NF, Ph. Eur., BP | As needed | As needed |
| Water for Injection | USP, Ph. Eur. | q.s. to target weight | q.s. to target weight |

### Example 4: STM 434 Study Protocol.

### STUDY OBJECTIVES

The primary objective of the study is to define the maximum tolerated dose (MTD) of STM 434 administered as monotherapy or in combination with liposomal doxorubicin chemotherapy in subjects with ovarian cancer or other advanced solid tumors.

The secondary objectives are as follows:
- To define the recommended Phase 2 dose (RP2D) in the event there is no MTD
- To assess the incidence of adverse events (AEs) and clinically significant changes in laboratory tests, electrocardiograms (ECGs), and vital signs during therapy with STM 434
- To assess the incidence of anti-STM 434 antibody formation
- To collect pharmacokinetic (PK) data during therapy with STM 434
- To collect ECG data during therapy with STM 434
- To collect preliminary antitumor efficacy data during therapy with STM 434
- To assess the relationship between antitumor efficacy data and each of the following: serum activin A, tumor INHBA/ACVR2B mRNA levels, and clear cell/endometrial/granulosa tumor mutation status
- To collect lean body mass, fat mass, bone mineral density (in subjects without bone metastasis), lipid profiles, fasting glucose, fasting insulin, homeostatic model assessment (HOMA), hemoglobin A1c (HbA1c), and 6-minute walk distance during therapy with STM 434
- To collect baseline and on-treatment data for biomarkers (such as cancer antigen-125 [CA-125], prostate-specific antigen [PSA], carbohydrate antigen 19-9 [CA 19-9], carcinoembryonic antigen [CEA]), activin A, follicle stimulating hormone (FSH), estradiol, and testosterone

The exploratory objectives of the study are to assess the relationship between PK parameters and PD parameters, body weight, body surface area, lean body mass, fat mass, appendicular lean mass, bone mineral density (in subjects without bone metastasis), lipid profiles, fasting glucose, fasting insulin, 6-minute walk distance, anti-drug antibody formation, biomarker, and ECG data.

### STUDY DESIGN

This is a multicenter, open-label, single-arm study in adult subjects with ovarian cancer or advanced solid tumors. The study will be conducted in three parts, as described below. An outline of the study procedures are shown in Table 16.

### Part 1 of the Study

Part 1 is an open-label dose-escalation study incorporating a 3+3 design in subjects with advanced solid tumors. A total of 15-30 subjects will be enrolled in 5 planned sequential dose cohorts to evaluate the safety, tolerability, and PK profiles of IV STM 434 at 5 dose levels (0.25 mg/kg IV, 0.5 mg/kg IV, 1 mg/kg IV, 2 mg/kg IV, and 4 mg/kg IV). In the absence of a safety signal during the dose-limiting toxicity (DLT) assessment window, STM 434 will be escalated in the planned 100% dose increments. If a safety signal (≥ 2 clinically significant STM 434-related Grade 2 AEs or one STM 434-related Grade 3 toxicity) is observed, all further dose escalations between cohorts will occur in ≤ 50% dose increments and an additional appropriate dose level or levels may be studied to avoid excessive toxicity. Each dose level will be evaluated sequentially, and each dose cohort will consist of 3-6 subjects (the number will depend on whether DLTs are observed).

Dose escalation from one cohort to the next will be determined by the sponsor in conjunction with the investigators and will be based on treatment-emergent AEs, clinical laboratory data, physical examination findings, including vital signs, ECGs, and available PK data after all 3 subjects within a cohort have completed 28 days of treatment. Dose escalation will occur if the subject incidence of DLTs or STM 434-related serious AEs (SAEs) during the first 28 days of study treatment is < 33%.

In a given cohort, if none of the 3 subjects experience a DLT during the 28 days from the initial administration of STM 434, then dose escalation will occur, and 3 subjects will be enrolled in the cohort at the next dose level. However, if ≥ 2 clinically significant Grade 2 STM 434-related AEs or one STM 434-related Grade 3 AE is observed, all further dose escalation will be reduced to ≤ 50% dose increments. If 1 of the 3 subjects in a cohort experiences a DLT, 3 additional subjects will be enrolled at the same dose level. If only 1 of 6 subjects within a cohort experiences a DLT or STM 434-related SAE during the first 28 days of treatment, the next cohort may begin enrollment. If a DLT is observed, all further dose escalation will be reduced to ≤ 50% dose increments. If 2 or more of the 6 subjects within a cohort experience a DLT or STM 434-related SAE during the first 28 days of treatment, this dose will be considered the toxic dose. Dose escalation may proceed at a lower dose or less frequent schedule based on emerging toxicity, PK, or PD data, until the MTD is determined. Doses higher than 4 mg/kg every 4 weeks may be explored if the observed PK data are below predicted levels.

### Part 2 of the Study

Part 2 is an open-label study, designed to obtain additional safety and exploratory efficacy data in subjects with advanced ovarian/endometrial clear cell, granulosa, and ovarian/fallopian tube/primary peritoneal serous tumors. A total of 24 subjects will be enrolled in 2 cohorts in this portion of the study. One cohort will include 6 subjects with clear cell adenocarcinoma and 6 subjects with granulosa cell tumors; the second cohort will include 12 subjects with serous tumors. All subjects will receive IV STM 434 at the RP2D. Enrollment in Part 2 is contingent upon establishment of the RP2D in Part 1.

### Part 3 of the Study

Part 3 is an open-label dose escalation study of STM 434 in combination with liposomal doxorubicin chemotherapy incorporating a 3+3 design in subjects with ovarian, fallopian tube, or primary peritoneal cancer who have received prior treatment with a platinum-based chemotherapy regimen or are unable to receive platinum-based chemotherapy. After Part 1 is completed and the RP2D is established, 6-12 subjects will be enrolled in Part 3. Parts 2 and 3 will enroll subjects concurrently. Subjects in Part 3 will be evaluated in 2 dose cohorts of 3-6 subjects each (the number of subjects per cohort depends on whether DLTs are observed). One cohort will receive STM 434 at one dose level below the RP2D and the second cohort will receive STM 434 at the RP2D; both cohorts will receive liposomal doxorubicin at 40 mg/m².

For both cohorts, subjects will receive an infusion of liposomal doxorubicin prior to receiving STM 434. Laboratory studies, including multigated acquisition (MUGA) scan, will be evaluated before each administration of liposomal doxorubicin. If needed, dose reduction from 40 mg/m² to 30 mg/m² can be implemented according to the toxicity management guidelines.

The safety, tolerability, and PK profiles of STM 434 when administered concomitantly with liposomal doxorubicin will be evaluated. The lower dose of STM 434 (ie, one dose level below the RP2D) will be evaluated first; dose escalation will be determined by the sponsor in conjunction with the investigators and will be based on treatment-emergent AEs, clinical laboratory data, physical examination findings including vital signs, ECGs, and available PK data after all 3 subjects within the cohort have completed 28 days of therapy using the 3+3 design outlined for Part 1.

### Dose Limiting Toxicities

A DLT will be defined as any related Grade ≥ 3 (according to the Common Terminology Criteria for Adverse Events [CTCAE], version 4.03) non-hematologic toxicity (excluding unrelated toxicities), or any Grade ≥ 4 hematologic toxicity lasting 7 days, febrile neutropenia, or Grade 3 thrombocytopenia with active bleeding.

A subject experiencing a DLT who wishes to resume therapy with STM 434 may do so at the discretion of the investigator when the toxicity has resolved to CTCAE Grade ≤ 1 or baseline values, and if the subject has not experienced disease progression.

### Maximum Tolerated Dose

The MTD is defined as the highest dose level with a DLT incidence < 33% of cohort subjects.

### Recommended Phase 2 Dose

The RP2D will be defined in consideration of the MTD, PK, pharmacodynamic biomarker and antitumor response data.

### Randomization and Blinding

This is an open-label, non-randomized, non-blinded study. Subjects will be assigned to a dose cohort in the order in which they qualify for the study.

### SUBJECT SELECTION CRITERIA

### Inclusion Criteria

A subject will be considered eligible to participate in this study if all of the following inclusion criteria are satisfied:
1. Males and postmenopausal females ≥ 18 years of age
2. Advanced solid tumors with histologic diagnosis confirming cancer
3. Subjects with recurrent metastatic or locally advanced disease considered refractory or intolerant to all standard treatment available for their tumor, or those with tumors for which no standard treatment is available
4. Subjects with serous ovarian/fallopian tube/primary peritoneal, granulosa cell tumors or clear cell tumors considered platinum refractory/resistant, defined as having at least one prior platinum-based chemotherapeutic regimen with a subsequent platinum-free interval of < 12 months, having progression during platinum-based therapy, or having persistent disease after a platinum-based therapy, are eligible. Intolerant subjects, defined as unable to receive further platinum due to toxicity, are eligible.
5. Measurable disease using Response Evaluation Criteria in Solid Tumors (RECIST 1.1) criteria
6. Eastern Cooperative Oncology Group (ECOG) performance status of 0 or 1
7. Able to walk at least 30 meters without assistance from another person (use of assistive devices such as a cane or walking frame is allowed)
8. Willing and able to provide written informed consent
9. Postmenopausal females must meet one or more of the following:
   a. 12 months of spontaneous amenorrhea
   b. 6 months of spontaneous amenorrhea with FSH > 40 IU/L
   c. Post-surgical bilateral oophorectomy with or without hysterectomy

For Part 2 of the study, the following additional inclusion criteria must be met:
10. For Cohort 6, subjects must have platinum refractory/resistant/intolerant (as defined in inclusion criterion #4) ovarian/endometrial clear cell carcinoma and ovarian granulosa cell tumors
11. For Cohort 7, subjects must have platinum refractory/resistant/intolerant (as defined in inclusion criterion #4) serous ovarian/fallopian tube/primary peritoneal cancer

For Part 3 of the study, the following additional inclusion criterion must be met:
12. For Cohorts 8 and 9, subjects must have advanced platinum refractory/resistant/intolerant (as defined in inclusion criterion #4) ovarian/fallopian tube/primary peritoneal cancer

### Exclusion Criteria

A subject will not be eligible to participate in the study if any of the following criteria are met:
1. Concurrent serious uncontrolled or unresolved medical condition, such as infection, limiting protocol compliance or exposing the subject to extreme risk
2. Unresolved toxicities from prior anti-cancer therapy, such as motor or sensory neuropathy, with a CTCAE (version 4.03) Grade ≥ 2 with the exception of alopecia
3. History of gastrointestinal bleeding within 6 months of Cycle 1 Day 1
4. Presence of QTcF > 470 msec, history of hereditary prolonged QT interval, or any arrhythmia (such as bundle branch blocks) that would preclude assessment of the QT interval
5. Myocardial infarction, unstable angina within 6 months of Cycle 1 Day 1, or congestive heart failure New York Heart Association ≥ class II
6. Elevated liver function tests, including total bilirubin > 1.5 × the upper limit of normal (ULN; unless subject has documented Gilbert's disease), aspartate aminotransferase (AST) or alanine aminotransferase (ALT) > 3.0 × ULN (for subjects with known liver metastasis, AST or ALT > 5 × ULN)
7. Creatinine > 1.5 × ULN and an estimated creatinine clearance of < 60 mL/min (using the Cockcroft-Gault equation)
8. Hemoglobin < 9 g/dL; platelet < 100 × 10⁹/L (must not receive transfusion within 4 weeks of Cycle 1 Day 1); absolute neutrophil count (ANC) < 1.5 × 10⁹/L (without granulocyte colony-stimulating factor support within 2 weeks of Cycle 1 Day 1)
9. Chemotherapy, hormonal therapy, or radiation therapy within 3 weeks of Cycle 1 Day 1 (Note: subjects taking ongoing gonadotropin releasing hormone therapy are eligible)
10. Antibody/biologic therapy within 5 half-lives or 4 weeks (whichever is longer) of Cycle 1 Day 1
11. Major surgery within 8 weeks or minor surgery within 4 weeks of Cycle 1 Day 1
12. Current bowel obstruction (those with a history of bowel obstruction are eligible)
13. Brain metastasis
14. Presence of ascites or pleural effusion requiring frequent (more than 1 × per week) medical intervention
15. Presence of portal-venous shunt device or history of extensive hepatic resection (more than one segment)
16. Known human immunodeficiency virus (HIV) infection
17. Active Hepatitis B or C infection
18. Prior treatment with any investigational product within 4 weeks of Cycle 1 Day 1
19. Female of childbearing potential, or male with a female partner of childbearing potential, unwilling to use a highly effective method of contraception (ie, one that results in pregnancy less than 1% per year) when used consistently and correctly, such as implants, injectables, combined oral contraceptives, some intrauterine contraceptive devices, sexual abstinence, or a vasectomized partner. Men unwilling to use a highly effective contraceptive measure during their participation in this study with female partners and/or unwilling to refrain from donating sperm while undergoing treatment with STM 434 and for 3 months after the last dose administration.
20. Women who are breast-feeding
21. History of epistaxis requiring medical or surgical intervention (such as nasal packing) within 6 months of Cycle 1 Day 1
22. History of central nervous system haemorrhage
23. History of bleeding diathesis or known qualitative platelet defect (including von Willebrand disease)
24. History of heredity hemorrhagic telangiectasia (HHT, Osler-Weber-Rendu syndrome)
25. Ongoing need for chronic use of aspirin or antiplatelet agents (ticlopidine or clopidogrel); if aspirin/antiplatelet agents are discontinued during screening, the washout period must be ≥ 2 weeks

For Part 3 of the study, subjects will not be eligible if they meet any of the following additional exclusion criteria:
26. Hypersensitivity reactions to a conventional formulation of doxorubicin HC1 or the components of liposomal doxorubicin
27. Cumulative dose of prior doxorubin HC1 > 300 mg/m², or cumulative dose of prior epirubicin > 500 mg/m²
28. Decreased cardiac ejection fraction less than the lower limit of normal by a MUGA scan or an echocardiogram (ECHO) within 30 days of Cycle 1 Day 1

### Subject Withdrawal Criteria

A subject may withdraw prior to completing the study for any of the following reasons:
- Withdrawal of consent by subject
- Occurrence of a DLT or other AE that precludes further participation
- Sustained side effects related to STM 434 and/or liposomal doxorubicin (for Part 3 only) that do not return to CTCAE Grade 1 or less with appropriate medical management within 30 days. A subject can be discontinued from the study if an individual clinically important toxicity (due to either STM 434 or liposomal doxorubicin) continues for more than 30 days without returning to CTCAE Grade 1 or less.
- Subject's significant non-compliance with the protocol
- Subject chooses to take a prohibited treatment, such as use of an investigational medications or device
- Worsening of clinical conditions, which, in the opinion of the investigator, requires immediate, specific medical treatment
- Early closing of the study by the sponsor

### TREATMENT WITH INVESTIGATIONAL PRODUCT

### Description and Handling of Study Drug

### Formulation

STM 434 is formulated as a sterile aqueous solution intended for IV administration, containing 70 mg/mL STM 434, 10 mM potassium phosphate buffer, 8.8% (w/v) sucrose, and 0.006% (w/v) polysorbate 20 at pH 6.7.

### Packing and Labeling

Formulated STM 434 solution is packaged into 5-mL glass vials (with a target fill volume of 1.2 mL to deliver 1.0 mL), with 13 mm fluoropolymer stoppers and 13 mm seals. Each vial is intended for single-use.

### Storage and Handling

Vials of STM 434 are stored in a non-frost-free freezer at a temperature of -20°C (± 5°C). The product should be stored according to the information provided on the label.

Prior to use, the product should be thawed overnight in a refrigerator at 2°C to 8°C; the product may be held at this temperature for up to 7 days. Exposure to higher temperatures and vigorous shaking of the vial should be avoided because these conditions may lead to a loss of STM 434 potency and structural integrity. Once thawed, the product should not be refrozen.

Refer to the Pharmacy Manual for any additional storage, handling or updated stability data of the study drug supply.

### Administration of Study Drug

### STM 434

STM 434 will be administered at a dose planned for each cohort, as specified below.

In Part 1, the planned dose cohorts are as follows:
- Cohort 1: 0.25 mg/kg STM 434 IV every 4 weeks
- Cohort 2: 0.5 mg/kg STM 434 IV every 4 weeks
- Cohort 3: 1 mg/kg STM 434 IV every 4 weeks
- Cohort 4: 2 mg/kg STM 434 IV every 4 weeks
- Cohort 5: 4 mg/kg STM 434 IV every 4 weeks

In Part 2, the planned dose cohorts are as follows:
- Cohort 6: STM 434 at the RP2D IV every 4 weeks to 6 subjects with clear cell adenocarcinoma and to 6 subjects with granulosa cell tumors
- Cohort 7: STM 434 at the RP2D IV every 4 weeks to 12 subjects with serous ovarian tumors

In Part 3, subjects will receive both STM 434 and liposomal doxorubicin. Subjects will first receive the doxorubicin infusion; after completion of this infusion, subjects will then receive IV STM 434. The planned dose cohorts for Part 3 are as follows:
- Cohort 8: STM 434 at one dose level below the RP2D IV + liposomal doxorubicin (40 mg/m² IV) every 4 weeks
- Cohort 9: STM 434 at the RP2D IV + liposomal doxorubicin (40 mg/m² IV) every 4 weeks

Subjects in Parts 1,2, and 3 will be treated with STM 434 until disease progression, unless an unacceptable toxicity is observed. Disease progression will be based on radiographic measurements according to RECIST 1.1 criteria or tumor marker measurements.

Subjects in Part 3 may continue to receive STM 434 if liposomal doxorubicin is withheld or discontinued for any reason other than a DLT observed during the first 28 days of combination treatment. Dose modification and adjustment for liposomal doxorubicin will follow the Prescribing Information for this product. The maximum number of liposomal doxorubicin cycles that will be administered is 6.

### Liposomal Doxorubicin

Subjects in Part 3 (Cohorts 8 and 9) will first receive a liposomal doxorubicin (40 mg/m²) infusion; when this infusion is completed, subjects will then receive their IV dose of STM 434.

Doses of liposomal doxorubicin up to 90 mg are to be diluted in 250 mL of 5% dextrose solution (D5W). Doses ≥ 90 mg should be diluted in 500 mL D5W. The first infusion of liposomal doxorubicin should be administered at a rate of 1 mg/minute. If no infusion reactions are observed, subsequent infusions may be administered over 1 hour.

Antiemetics for chemotherapy with moderate emetogenicity should be prescribed according to institutional guidelines.

### Duration of Treatment

Screening will begin up to 14 days prior to dosing (Cycle 1 Day 1), with the exception that radiographic scans can be collected within 30 days prior to starting the first dose of study medication. Subjects in Parts 1,2, and 3 will be treated with STM 434 until disease progression, unless an unacceptable toxicity is observed. Disease progression will be based on radiographic measurements according to RECIST 1.1 criteria or tumor marker measurements. If CA-125 is used as the tumor marker measure, the results will be evaluated with the Gynecologic Cancer Intergroup (GCIG) consensus guidelines. If PSA is used as the tumor marker measure, the results will be evaluated with the Prostate Cancer Working Group (PCWG) consensus guidelines.

Subjects in Part 3 may continue to receive STM 434 if liposomal doxorubicin is withheld or discontinued for any reason other than a DLT observed during the first 28 days of combination treatment.

### EFFICACY AND SAFETY ASSESSMENTS

Each efficacy and safety variable is described below.

### Pharmacokinetics

STM 434 levels in blood samples will be determined by a central laboratory. The following STM 434 PK parameters will be estimated from blood levels using standard noncompartmental pharmacokinetic methods:
- Terminal half-life (t_{1/2})
- Clearance (CL)
- Mean residence time (MRT)
- Volume of distribution (V_{d})
- Volume of steady state (Vₛₛ)

In addition, for subjects in Part 3 (Cohorts 8 and 9), who will receive liposomal doxorubicin in addition to STM 434, blood samples will be collected and sent to a central laboratory for determination of doxorubicin/doxorubicinol levels. PK parameters, as above, will be estimated.

### Efficacy Assessments

### Radiographic Tumor Progression

Radiographic tumor progression will be determined by the investigator's assessment using RECIST 1.1 criteria based on CT (or MRI) and bone scan (subjects with bone metastases only). The selection of CT or MRI modality will be per the investigator. Target lesions (if present, up to 5 per subject) will be measured at baseline and tracked throughout the study. Duration of radiographic response will be recorded as the time from initial observation of an objective radiographic response until the date of disease progression or death. Radiographic progression free survival will be recorded as the time from Cycle 1 Day 1 until the date of radiographic disease progression or death.

### Body Composition

Lean body mass, appendicular lean mass, and fat mass, and fat distribution (visceral and subcutaneous) will be determined by DXA as detailed in the Radiology Manual. DXA scans will be analysed by the central radiology laboratory.

### Muscle Function Test: 6-Minute Walk Test

### Description

The 6MWT measures the distance the subject covers when walking on a flat surface over a 6-minute time period. In this study, the 6MWT will be performed in accordance with guidelines from the American Thoracic Society (ATS). These guidelines were developed for patients who may have concurrent heart or lung morbidities and have an overall reduced level of physical activity, similar to advanced oncology patients. Thus, the procedure for the 6MWT in the current study has been planned in accordance with the ATS guidelines.

### Staff Training

Only site staff trained by the sponsor/sponsor's representative may conduct the 6MWT. Sites may not commence conduct of the 6MWT before the sponsor/sponsor's representative evaluates the area to be used, and trains the site staff who will conduct the test. The same area must be used for all assessments for each individual subject for the duration of the study.

### Laboratory Parameters for Efficacy

### Biomarkers

### Tumor Biomarkers

Serum tumor biomarkers (such as CA-125 for subjects with ovarian cancer, PSA for subjects with prostate cancer, CA 19-9 for subjects with pancreatic cancer, and CEA for subjects with colon cancer) will be monitored at baseline and throughout the study. Tumor marker response and tumor marker progression (TTTMP) will be assessed. If CA-125 is used as the tumor marker measure, the results will be evaluated with the Gynecologic Cancer Intergroup (GCIG) consensus guidelines. If PSA is used as the tumor marker measure, the results will be evaluated with the Prostate Cancer Working Group (PCWG) consensus guidelines.

### Serum Tumor Biomarkers (Activin A, FSH, Estradiol, Testosterone)

As STM 434 inhibits activin signaling, we will determine if serum activin levels are a pharmacodynamics biomarker reflecting the extent of target inhibition by STM 434. Since activins are part of an endocrine feedback mechanism stimulating the release of FSH and the sex hormones (estradiol, testosterone), these endocrine biomarkers will also be assessed as potential pharmacodynamic efficacy biomarkers.

Determination of activin A will be performed by the central lab to ensure comparability across subjects. FSH, estradiol, and testosterone will be assessed by local laboratories. Instructions for sample handling are provided in the Laboratory Manual.

### Tissue Tumor Biomarkers

Formalin Fixed Paraffin embedded tumor sections will be examined for autocrine/paracrine signaling by measuring mRNA for the receptor ACVR2B and ligand INHBA (all subjects) at screening and the Cycle 4 Day 1 visits using a validated RNAScope® assay. FOXL2 Gene mutation status (for subjects with granulosa cell tumors) will be determined by DNA sequencing at screening. ARID 1A gene mutation status will be determined by next-generation sequencing, comparing tumor DNA with somatic DNA from whole blood (for subjects with clear cell/endometrial tumors); this assay may also determine the sequence of other cancer genes present on the microchip. Because the clinical relevance of these data is not yet established, subjects will not be informed of the results of mutation analysis. Samples (tissue sections or tumor block) will be shipped to a central laboratory according to the instructions in the Laboratory Manual.

### Circulating Tumor Cells

Activin signaling is necessary to maintain the cancer stem cell populations in some tumor types. Therefore, reduced levels of CTCs may reflect the pharmacodynamic activity of STM 434. In this study, CTCs will be enumerated using the validated CellSearch® platform at screening and Cycle 2 Day 1. Samples will be shipped to a central laboratory according to instructions in the Laboratory Manual.

### Serum Lipids

Since the anti-activin and anti-myostatin pharmacology of STM 434 may lead to increased muscle mass, we will determine if serum lipid levels are affected in treated subjects.

### Fasting Serum Glucose and Insulin by Homeostatic Model Assessment

Insulin resistance (IR) and hyperglycemia are risk factors for development of cardiovascular complications. In preclinical studies, the STM 434 surrogate, STM 217, improved IR. While the hyperinsulinemic euglycemic glucose clamp is the gold standard for measuring IR, the complexity of this method limits its use. A more practical method, HOMA, which uses fasting levels of glucose and insulin, has been studied in dialysis subjects. Measurements obtained with HOMA correlate well with those obtained using the hyperinsulinemic euglycemic glucose clamp.

HbA1c is recommended by the American Diabetes Association for evaluation of glycemic control. Evaluation of HbA1c in this study complements the fasting glucose and fasting insulin tests and will provide an integrated measure of glucose control over time.

### STATISTICAL ANALYSIS

### Endpoints

### Primary

The primary endpoints are as follows:
- Part 1: the MTD of STM 434 monotherapy in subjects with ovarian cancer or other advanced solid tumor
- Part 2: The incidence of AEs and clinically significant changes in laboratory tests, ECGs, and vital signs in subjects with ovarian/fallopian tube/primary peritoneal/endometrial tumors
- Part 3: The MTD of STM 434 administered in combination with liposomal doxorubicin chemotherapy in subjects with ovarian/fallopian tube/primary peritoneal cancer

### Secondary

The secondary endpoints (Parts 1,2, and 3, unless otherwise noted) include the following:
- The RP2D in the event there is no MTD (Parts 1 and 3)
- The incidence of AEs and clinically significant changes in laboratory tests, ECGs and vital signs (Parts 1 and 3)
- The incidence of STM 434 antibody formation
- PK profiles of STM 434
- PK profiles of doxorubicin/doxorubicinol (Part 3)
- Subject responses on the FACT-NTX neuropathy questionnaire
- Radiographic response rate (rRR)
- Duration of radiographic response (dRR)
- Radiographic progression-free survival (rPFS)
- Tumor marker response rate (TMRR)
- Time to tumor marker progression (TTTMP)
- Correlation of baseline and on-treatment activin A serum concentrations, tumor mRNA levels for INHBA/ACVR2B, and tumor mutation status (in subjects with clear cell/endometrial/granulosa cell tumors) to antitumor efficacy measures
- Change from baseline in lean body mass, appendicular lean mass, fat mass (visceral and subcutaneous), lipid profiles, fasting glucose, fasting insulin, HOMA, HbA1c, and 6-minute walk distance

### Exploratory

The exploratory endpoints (Parts 1,2, and 3) are as follows:
- Correlation between PK parameters and antitumor efficacy, bone mineral density (in subjects without bone metastasis), lipid profiles, fasting glucose, fasting insulin, HbA1c, 6-minute walk distance, anti-drug antibody formation, biomarker, or ECG data

### Analysis Populations

Analysis populations for efficacy, safety, and PK, respectively, are described below.
- The efficacy analysis population will include all subjects who receive at least 4 weeks of STM 434 administration. A secondary analysis of the efficacy endpoints will be performed for all subjects who receive at least 12 weeks of STM 434 administration.
- The safety analysis population will include all subjects who receive at least 1 dose of STM 434.
- The PK analysis population will include all subjects who receive at least 1 dose of STM 434 and who provided at least 1 PK sample.

### Efficacy Analysis

The rRR, dRR, and rPFS will be defined according to RECIST 1.1 and analyzed using descriptive statistics, and Kaplan-Meier methods. For rPFS, subjects who do not experience disease progression or death will be censored at the date of their last imaging scan for disease assessment.

The TMRR and TTTMP will be defined by appropriate consensus guidelines, and analyzed using descriptive statistics and Kaplan-Meier methods.

### Pharmacokinetic Analysis

The PK parameters of STM 434 alone and in combination with liposomal doxorubicin will be estimated using standard noncompartmental PK methods and summarized using descriptive statistics (means, standard deviations, medians, minima and maxima).

Exploratory analyses correlating exposure to antitumor efficacy, lean body weight, fat mass, lipid profiles, fasting glucose, fasting insulin, HOMA, HbA1c, 6MWT, anti-drug antibody formation, biomarker or ECG data may be performed ad hoc or post hoc.

### Safety Analysis

Safety assessments will include AEs, concomitant medications, laboratory results and vital signs. All reported AEs will be coded using the Medical Dictionary for Regulatory Activities, summarized by number and percent of subjects with AEs, and attribution (serious versus non-serious and investigator reported relationship [unrelated, possibly related, related]). Only AEs occurring after the initiation of the first dose of treatment until 30 days following the last treatment will be included in the tables. The incidence of anti-STM 434 antibody formation will be summarized using descriptive statistics.

### Sample Size Determination

The study is planned to enroll up to a total of 66 subjects (consisting of up to 30 subjects in Part 1, 24 subjects in Part 2, and up to 12 subjects in Part 3). This sample size is not based on power calculations, but rather on clinical judgement and the expectation that the objectives of the study will be met with the planned sample size.

For Part 2 of the study, in the cohort for the rare ovarian cancer subtypes of clear cell and granulosa cell tumors, only 6 subjects of each subtype will be enrolled due to the small number of subjects expected at the clinical trial sites, whereas the cohort for the more common serous ovarian tumors will enroll 12 subjects.

### Conclusion

Formulated STM 434 administered as monotherapy or in combination with liposomal doxorubicin chemotherapy treats ovarian cancer and other advanced solid tumors with relatively limited side effects in selected subjects.

| **Table 16: Study Procedures** | | | | | | | |
|---|---|---|---|---|---|---|---|
| **Evaluation** | **Screening** | | **Treatment Phase** | | | | **End of Study²** |
| | | | **Cycle 1** | | **Cycles 2, 3, 5, 6.** | **Cycles 4, 7, 10.** | |
| | **Days -14 to 1** | **Day -1** | **Day 1** | **Day 15** | **Day 1** | **Day 1 and at Treatment Discontinuation¹** | |
| Consent form signed | X | | | | | | |
| Medical history, Demographics, Prior cancer therapies | X | | | | | | |
| Physical exam, Height and Weight³ | X | X⁴ | | X | X | X | X |
| Stool guaiac exam | X⁵ | | | | | | |
| Vital signs⁶ | X | X⁴ | | X | X | X | X |
| ECOG | X | X⁴ | | X | X | X | X |
| ECG⁷ | X | X | | | X | X | X |
| FACT /GOG-NTX⁸ | X | | | X | X | X | X |
| MUGA or ECHO | X⁹ | | | | | X⁹ | X⁹ |
| STM 434 administration | | | X | | X | X | |
| Liposomal doxorubicin dosing administration¹⁰ | | | X | | X | X | |
| Concomitant medications | X | | X | X | X | X | X |
| Adverse events | X¹¹ | | X | X | X | X | X¹² |
| Hematology | X | X⁴ | | X | X | X | X |
| Coagulation panel (PT, PTT, INR, TT¹³, D-dimer¹³) | X | X⁴ | | X | X | X | X |
| Serum chemistry | X | X⁴ | | X | X | X | X |
| Fasting glucose and fasting insulin | X | | | | | X | |
| HbA1c¹⁴ | X | | | | | X¹⁴ | X |
| Serum lipids | X | | | | | X | X |
| Serum tumor biomarkers¹⁵ | X | x⁴ | | | X | X | X |
| Tissue tumor | X | | | | | | |
| biomarkers¹⁶ | | | | | | | |
| Urinalysis (dipstick) | X | | | | | | |
| Serum pregnancy | X | | | | | | |
| Activin A (free and total) | X | X⁴ | | X | X | | X |
| CTCs | X¹⁷ | | | | X¹⁷ | | |
| FSH, estradiol (female subjects), testosterone (male subjects) | X | X⁴ | | X | X | | X |
| Central lab DXA | X¹⁸ | | | | | X¹⁹ | |
| 6-minute walk test | X | | | | X²⁰ | X | |
| CT/MRI | X¹⁸ | | | | | X¹⁹ | |
| Bone scan²¹ | X¹⁸ | | | | | X¹⁹ | |
| Disease progression assessment | | | | | | X | |
| Anti-STM 434 antibody | X | X⁴ | | | X | X | X |
| Predose PK | | | X²² | | X²² | X²² | |
| Postdose PK | | | X²² | X | X²² | | |
| ¹ The Treatment Discontinuation visit can occur at any scheduled or unscheduled visit when appropriate. At this visit, documentation to confirm progressive disease or unacceptable toxicity is determined. | | | | | | | |
| ² The EOS visit is scheduled 28 days following the last administration of STM 434. | | | | | | | |
| ³ Subject weight is recorded at each visit; height is recorded at the screening visit. | | | | | | | |
| ⁴ Procedures scheduled for Day -1, with the exception of the ECG Holter monitor, can be performed prior to dosing on Day 1 of Cycle 1. | | | | | | | |
| ⁵ Stool guaiac is performed at screening and may be repeated at the discretion of the investigator post-baseline. | | | | | | | |
| ⁶ Vital signs: aural, transdermal or oral temperature, sitting blood pressure, heart rate, and respiratory rate | | | | | | | |
| ⁷ After the screening visit, ECGs must be collected using equipment from the central laboratory. Starting on Day-1 subjects wear a Holter monitor continuously until the Day 2 visit. Subsequently, 12-lead ECGs will be obtained with the central lab machine. | | | | | | | |
| ⁸ The FACT/GOG-NTX may be collected at unscheduled visits if needed to assess an AE of neuropathy. | | | | | | | |
| ⁹ Only subjects in Part 3 use MUGA or ECHO to document the cardiac ejection fraction for liposomal doxorubicin administration. The choice of MUGA or ECHO is at the investigator's discretion, but once a modality is selected, the same method should be used for the subject throughout the study period. MUGA/ECHO is to be performed at screening and Cycle 4 Day 1. In the event that a doxorubicin-treated subject in Part 3 discontinues prior to Cycle 4 Day 1, a MUGA/ECHO is performed at the EOS visit. | | | | | | | |
| ¹⁰ Liposomal doxorubicin administration is for subjects in Part 3 of the study. | | | | | | | |
| ¹¹ Pretreatment SAEs is reported from the time the informed consent document is signed until the administration of study medication on Cycle 1 Day 1. | | | | | | | |
| ¹² AE follow-up of 30 days following the last administration of STM 434 to determine if there are any new or ongoing AEs or SAEs regardless of attribution of causality. Follow-up may be conducted via telephone and is documented in the | | | | | | | |
| source notes. | | | | | | | |
| ¹³ Screening and Cycle 2 Day 1. | | | | | | | |
| ¹⁴ HbA1c will be collected and analyzed at screening, Cycle 4 Day 1, Treatment Discontinuation and EOS only. | | | | | | | |
| ¹⁵ Serum tumor biomarkers can include CA-125, PSA, CA 19-9, CEA, or other biomarkers as appropriate based on the subject | | | | | | | |
| ¹⁶ Tissue tumor biomarkers include tumor sections/tumor block for ACVR2B and INHBA gene expression (all subjects) as well as gene mutation status (for subjects with clear cell/endometrial/granulosa cell tumors). | | | | | | | |
| ¹⁷ CTCs will be collected at screening and Cycle 2 Day 1. | | | | | | | |
| ¹⁸ Scans (DXA, CT, MRI, Bone) performed up to 30 days prior to Cycle 1 Day 1 can be used for the baseline assessments. An MRI of the brain is used to rule out any brain metastases. | | | | | | | |
| ¹⁹ On-study DXA, CT/MRI and bone scans can be done within ± 8 days of the Day 1 visit. | | | | | | | |
| ²⁰ 6-minute walk test will be completed at the Cycle 2 Day 1 visit. | | | | | | | |
| ²¹ Bone scan assessments are conducted ofor subjects with tumors metastatic to bone. | | | | | | | |
| ²² The detailed schedule for PK assessments is presented herein | | | | | | | |

### Example 5: Interim Results for STM 434 Study.

Three human subjects were selected for treatment with STM 434 using the above protocol. Each subject had an advanced form of cancer: serous ovarian, granulosa ovarian or colon. Subject 1 had a serous ovarian cancer, Subject 2 had granulosa cell ovarian cancer, and Subject 3 had a colon cancer. Subject 1 was diagnosed with Stage IV ovarian cancer in January 2014. She had progression of her serous adenocarcinoma tumor after receiving prior carboplatin and paclitaxel in 2014. Subject 2 was diagnosed with ovarian cancer in 1998 and had progression of her granulosa cell tumor after receiving prior cisplatin, etoposide, bleomycin chemotherapy in 2010, carboplatin and paclitaxel in 2012, bevacizumab, medroxyprogesterone, anastrozole in 2013, and RX-3117 (an investigational cytidine analog) in 2014. Subject 3 was diagnosed with Stage IV colon cancer in June 2012. She had progression of her colon tumor after receiving prior 5-fluorouacil, leucovorin, oxaliplatin chemotherapy in 2012, irinotecan, 5-fluorouacil, leucovorin , bevacizumab in 2013 and panitumumab in 2014.

### STM 434 Formulation, Packing, and Storage

STM 434 was formulated as a sterile aqueous solution intended for IV administration, containing 70 mg/mL STM 434, 10 mM potassium phosphate buffer, 8.8% (w/v) sucrose, and 0.006% (w/v) polysorbate 20 at pH 6.7. Formulated STM 434 solution was packaged into 5-mL glass vials, with 13 mm fluoropolymer stoppers and 13 mm seals. Vials of STM 434 were stored in a non-frost-free freezer at a temperature of -20°C (± 5°C). Prior to use, STM 434 was thawed overnight in a refrigerator at 2°C to 8°C.

### STM 434 Administration

STM 434 was administered to each subject at 0.25 mg/kg IV approximately every 4 weeks. Subject 1 began therapy on 17 October 2014, and received two additional doses on 14 November 2014 and 15 December 2014, prior to discontinuing further study treatment on 30 December 2014. Subject 2 commenced therapy on 29 October 2014, and received three additional doses on 24 November 2014, 24 December 2014, and 21 January 2015 - this subject continues to receive STM 434 as of 17 February 2015, Subject 3 began therapy on 6 November 2014, and received one additional dose on 4 December 2014, prior to discontinuing further study treatment on 05 January 2015.

### STM 434 Human Safety and Efficacy

No adverse events considered to be related to STM 434 administration were observed in any subject. No adverse events related to neuropathy, bleeding events, or endocrine symptoms were observed in any subject. No anti-STM 434 antibodies were observed in any subject. Subject 1 reported improved appetite, energy, and increasing performance status. The investigators considered STM 434 to be safe and well-tolerated. The investigators recommended escalation to the next dose level.

### STM 434 Inhibition of Activin In Vivo

Since activins are part of an endocrine feedback mechanism stimulating the release of FSH, FSH was assessed as a potential pharmacodynamic efficacy biomarker. A reduction in FSH is expected to be indicative of activin inhibition by STM 434.

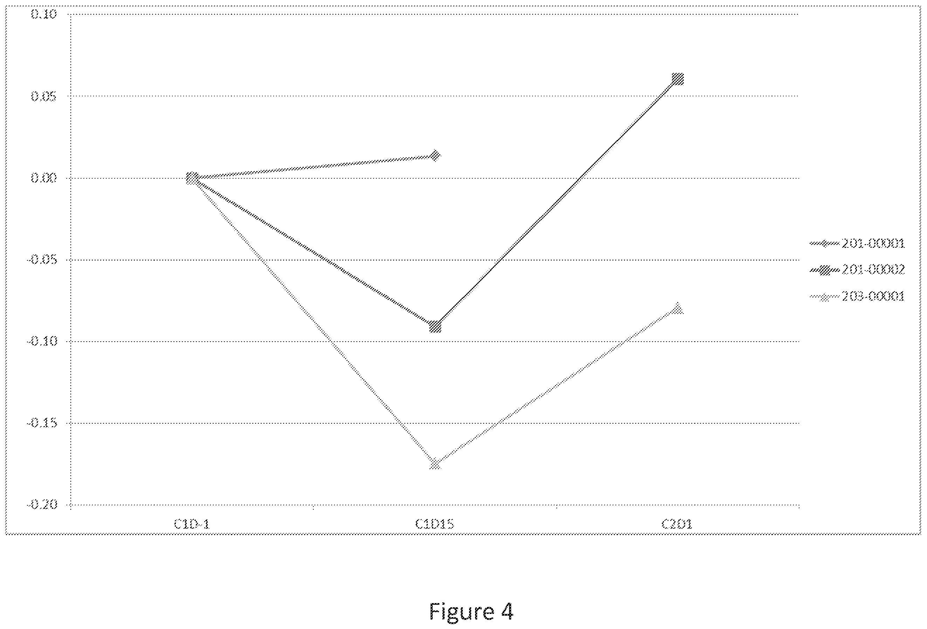

FSH was assessed using ELISA Quantitative Immunoassay. It was determined that FSH levels decreased in 2 of 3 subjects following administration of STM 434 dosed at 0.25 mg/kg. Figure 4. Percent change from baseline is plotted on the y axis, over time plotted on the x axis; C1D1 indicates Cycle 1 Day 1 (baseline prior to treatment), C1D15 indicates Cycle 1 Day 15, 14 days after initial administration of STM 434, and C2D1 indicates Cycle 2 Day 1 (predose, prior to the second dose administration of STM 434 4 weeks after first administration). A reduction in FSH observed at C1D15 indicates that STM 434 inhibits activin signaling in humans. The FSH levels return to approximate baseline, indicating that a new dose of STM 434 is required to re-suppress activin signaling in humans. These results indicate that STM 434 can inhibit activin in humans.

### STM 434 PK Results

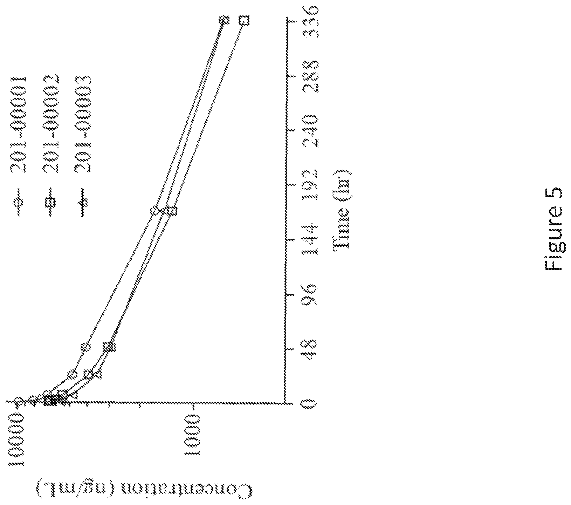

PK was determined by ELISA Quantitative Immunoassay. Interim PK analysis showed 2-fold higher clearance and T½ of 111, 114, or 147 hours (4-5 days) compared to prior data showing 163-259 hours (7-11 days) in monkeys. Figure 5. This suggests that subjects may benefit from a more frequent dosage scheme of STM 434, e.g., IV every 2 weeks or every 3 weeks.

### STM 434 Induced Tumor Infarction

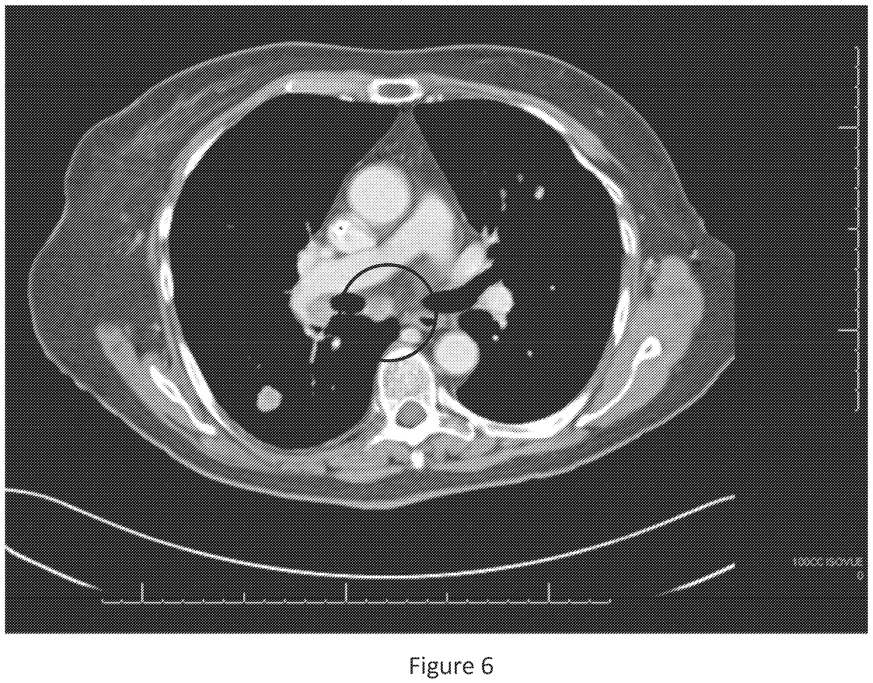

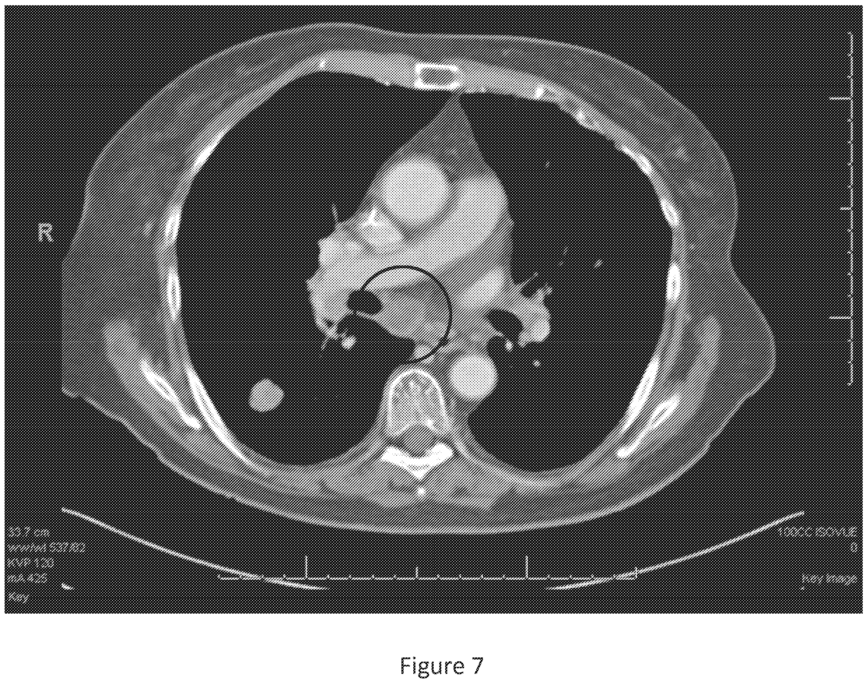

One subject (Subject 2) having granulosa tumor received a CT scan at baseline and again at the beginning of cycle 4 (day 1). There were 12 weeks, and three doses between the two scans. Figure 6 shows the baseline scan with the tumor indicated in the circle. Figure 7 shows the follow-on scan taken at the beginning of cycle 4, with the tumor again indicated in the circle. As can be seen by comparison of the scans, the tumor has undergone substantial infarction. This indicates that a low dose of STM 434 is effective in treating a subject having granulosa tumor.

### Example 6: Modified STM 434 Protocol.

Based, at least in part, on the results of Example 5 the STM 434 protocol of Example 4 was modified. The modified protocol is set forth below in Table 17, its subtables, and the associated appendices.

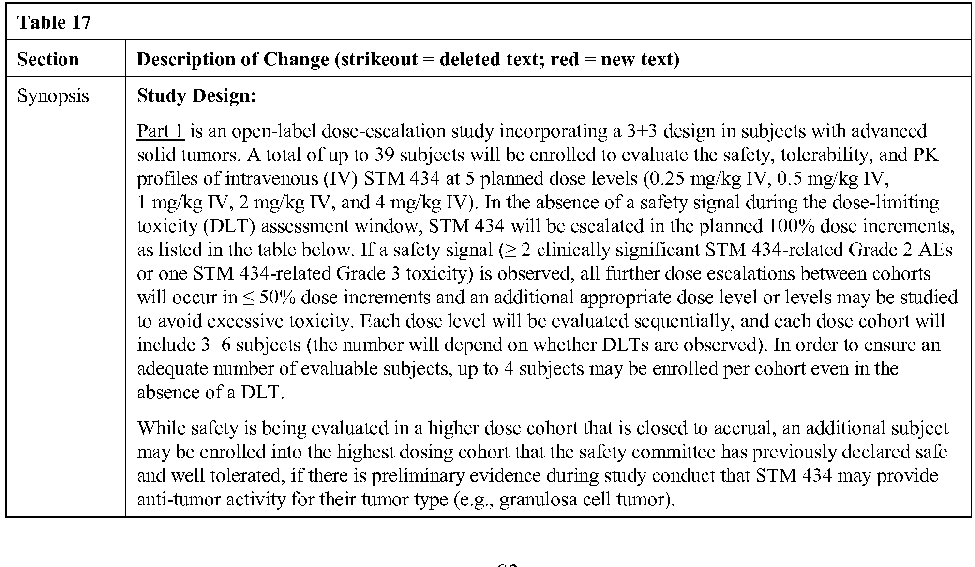

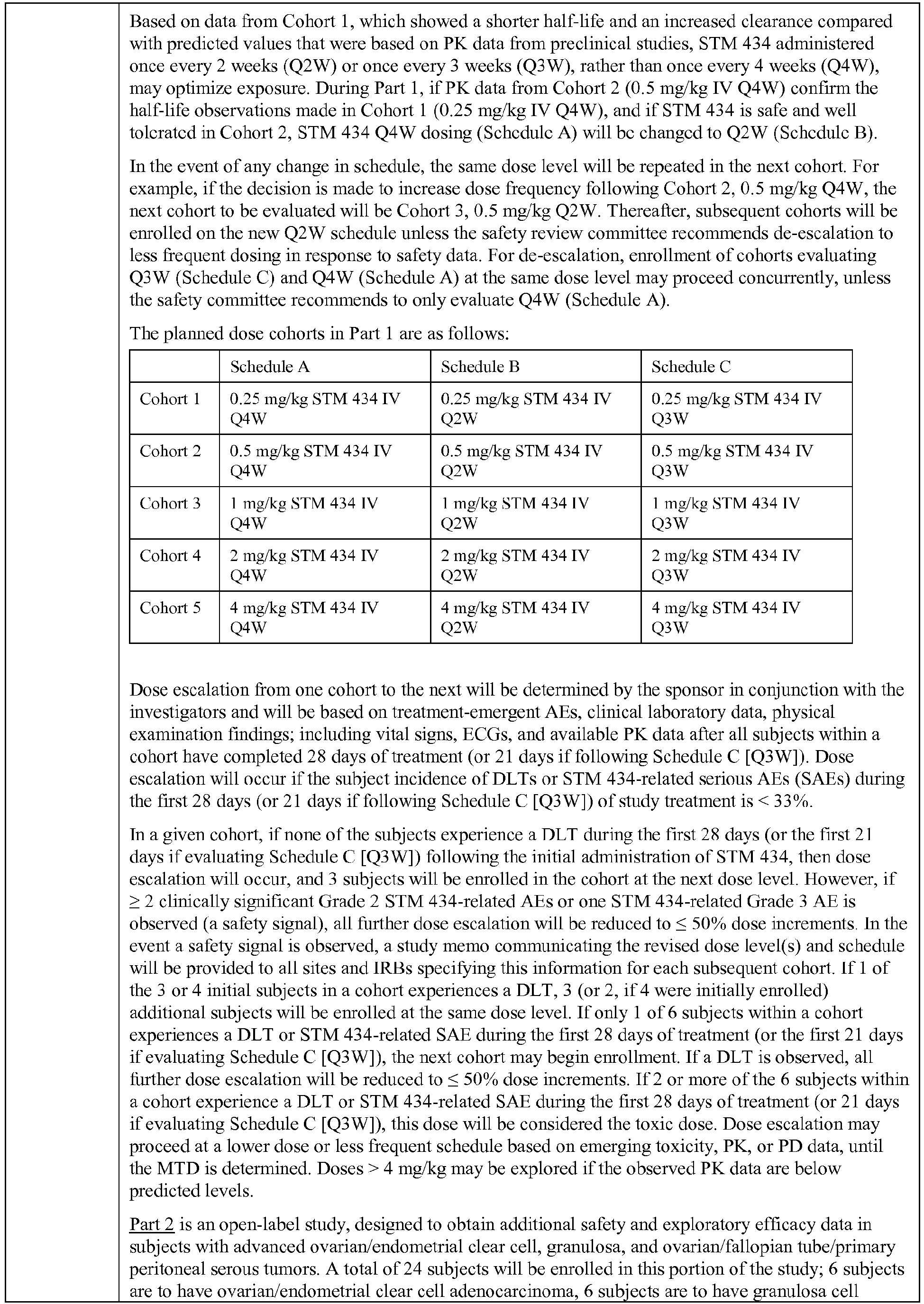

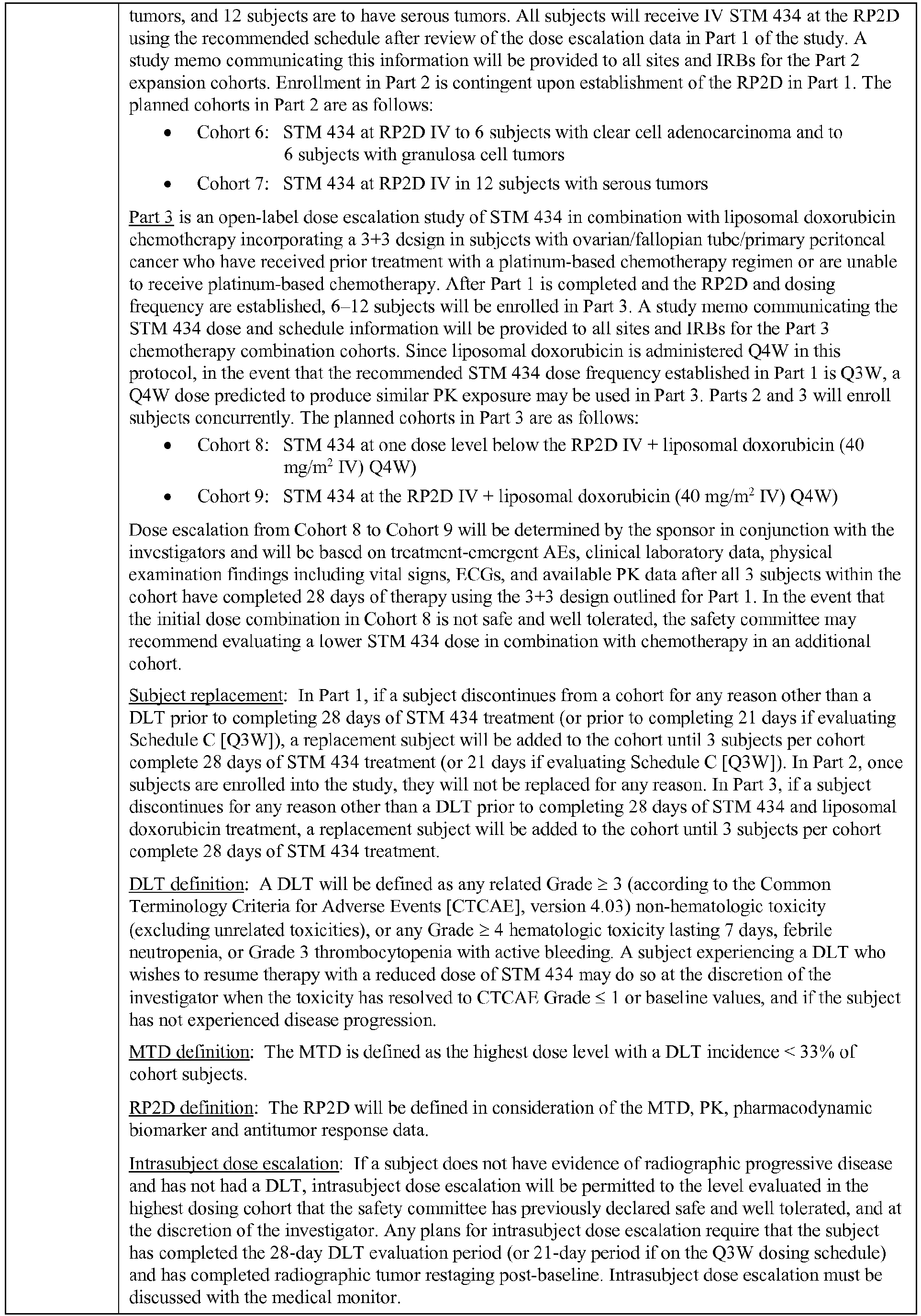

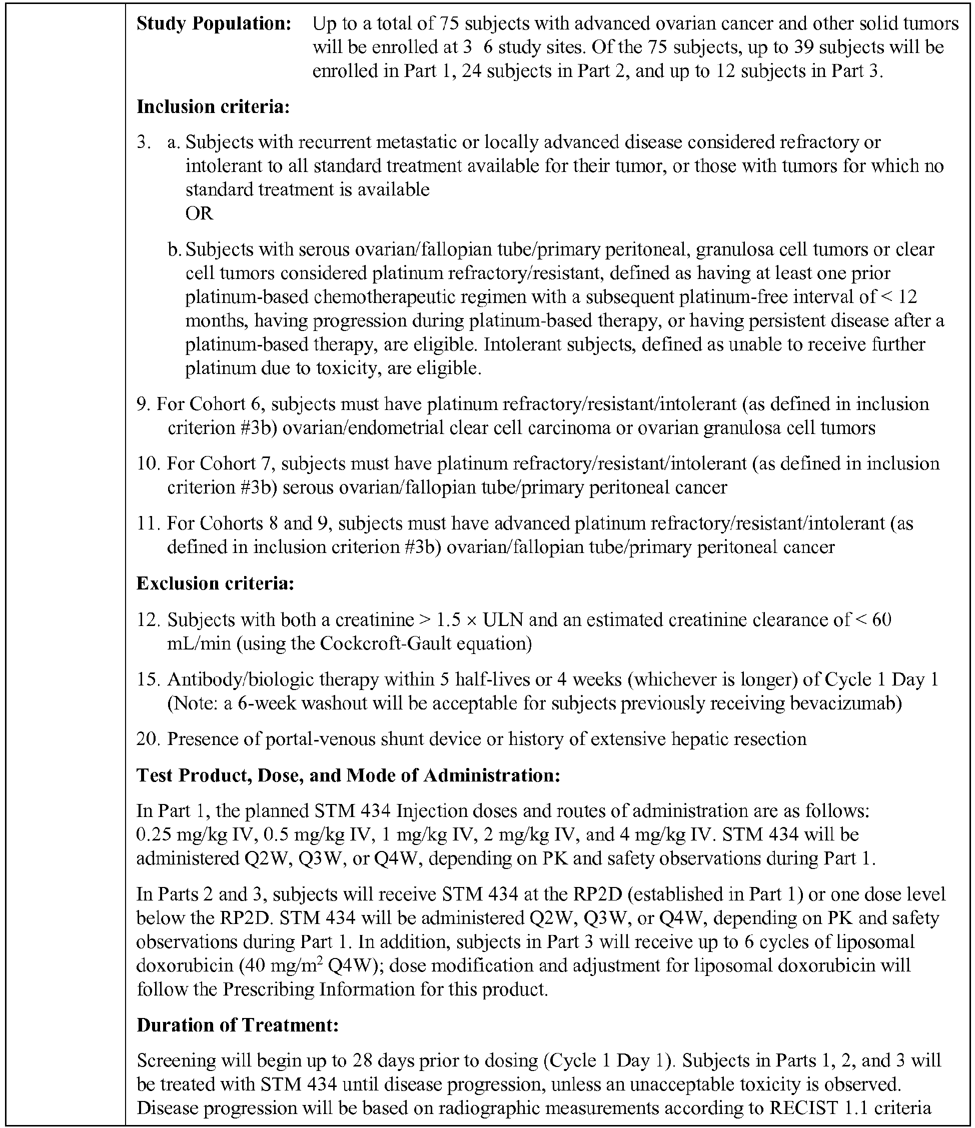

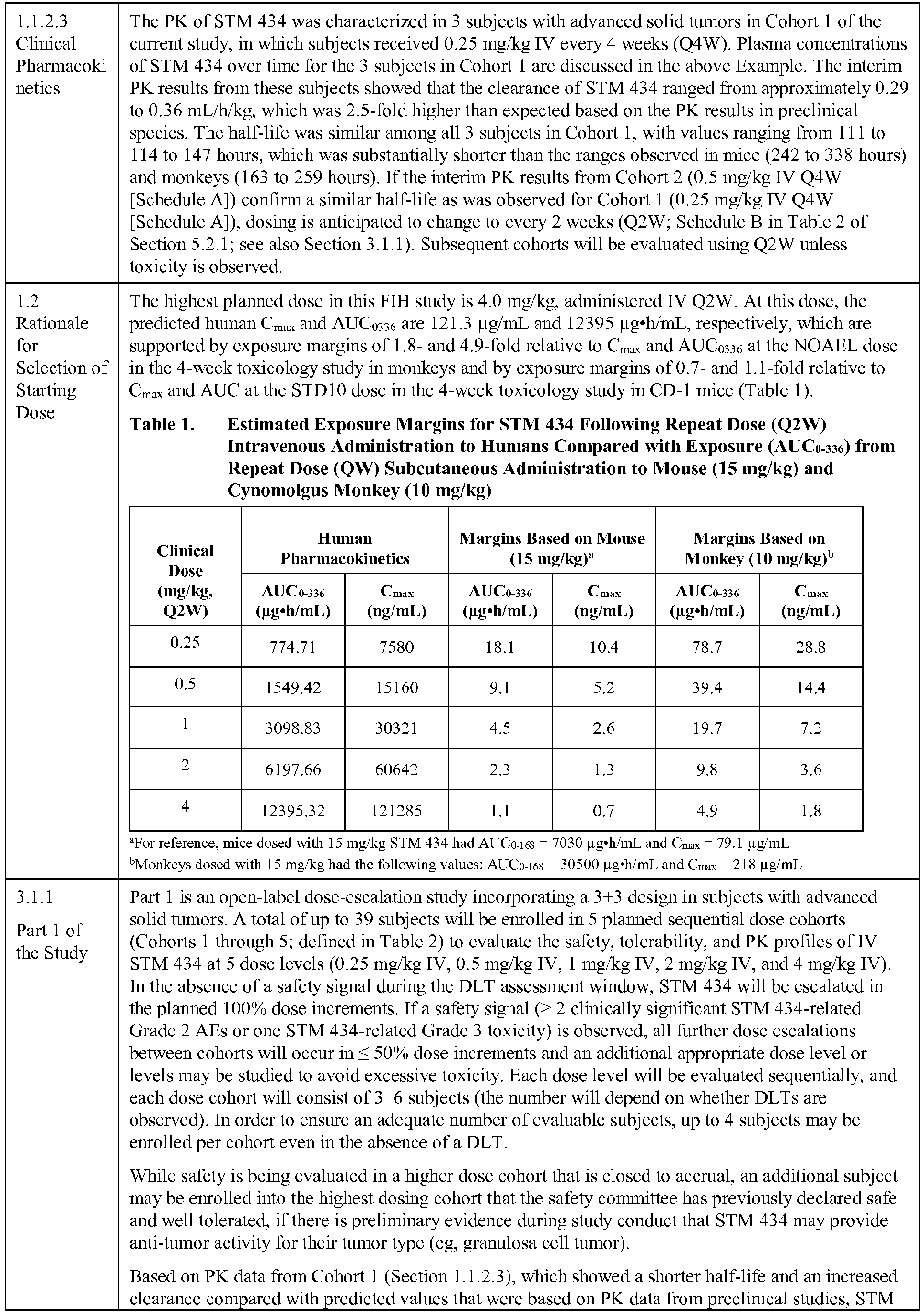

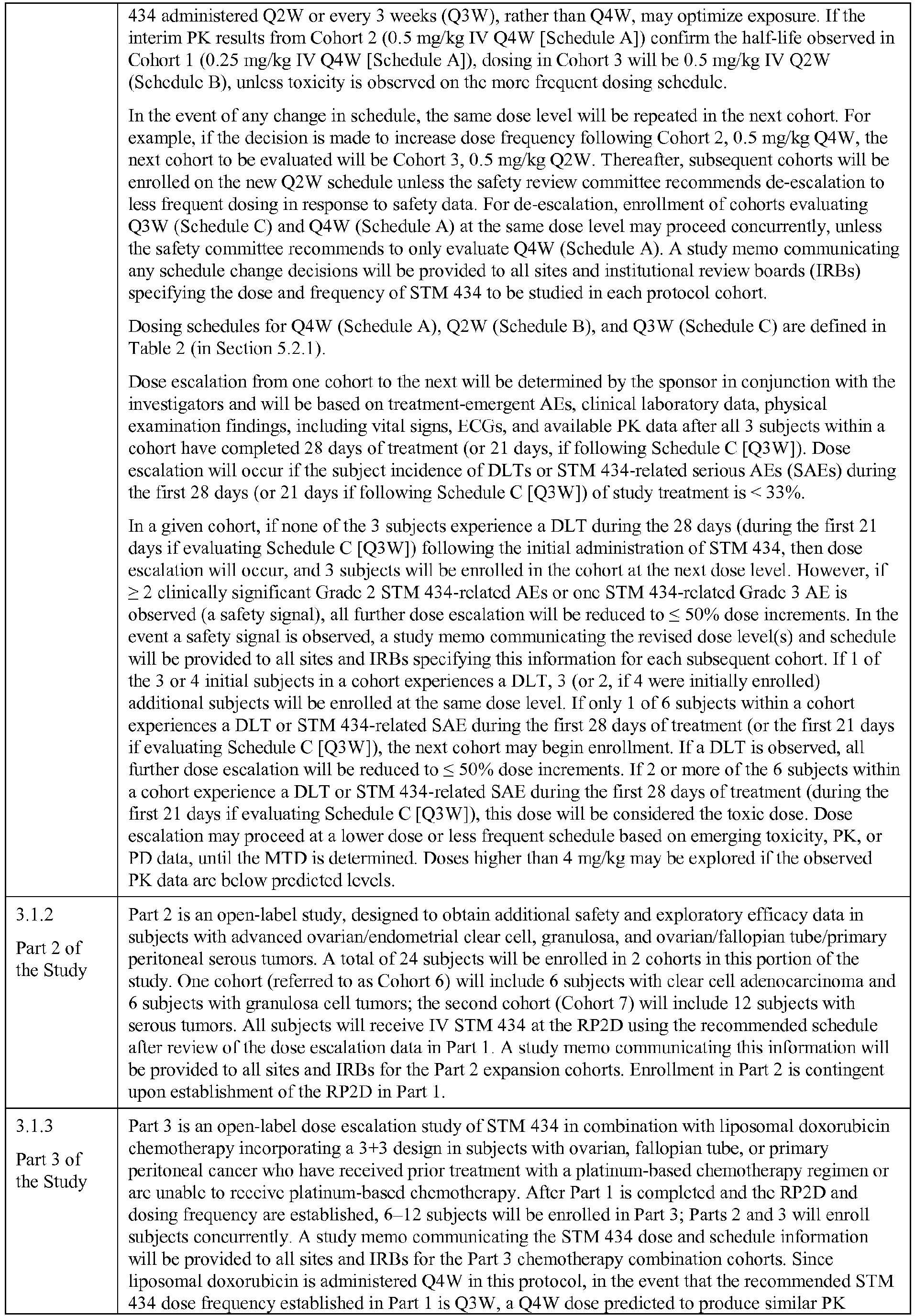

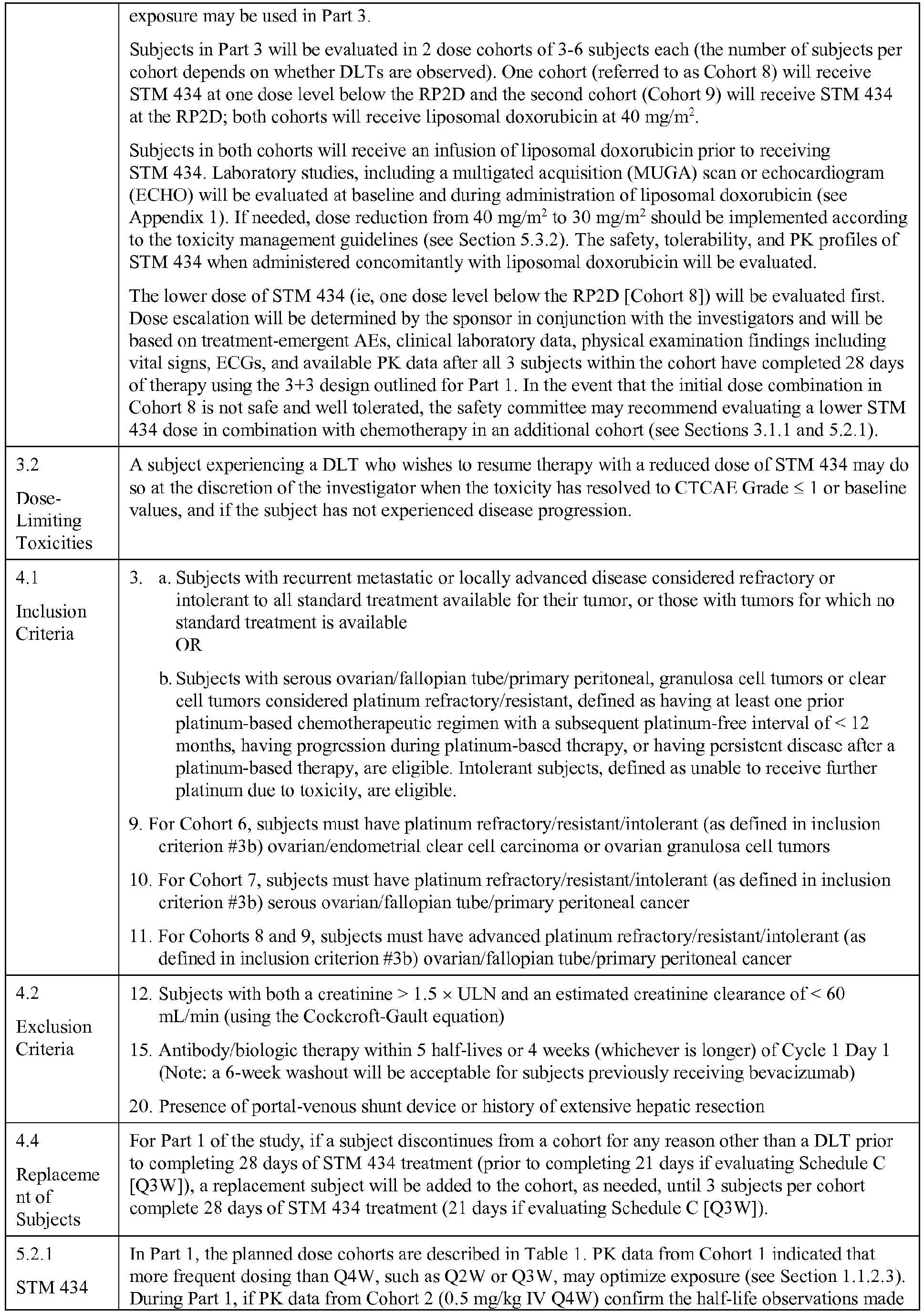

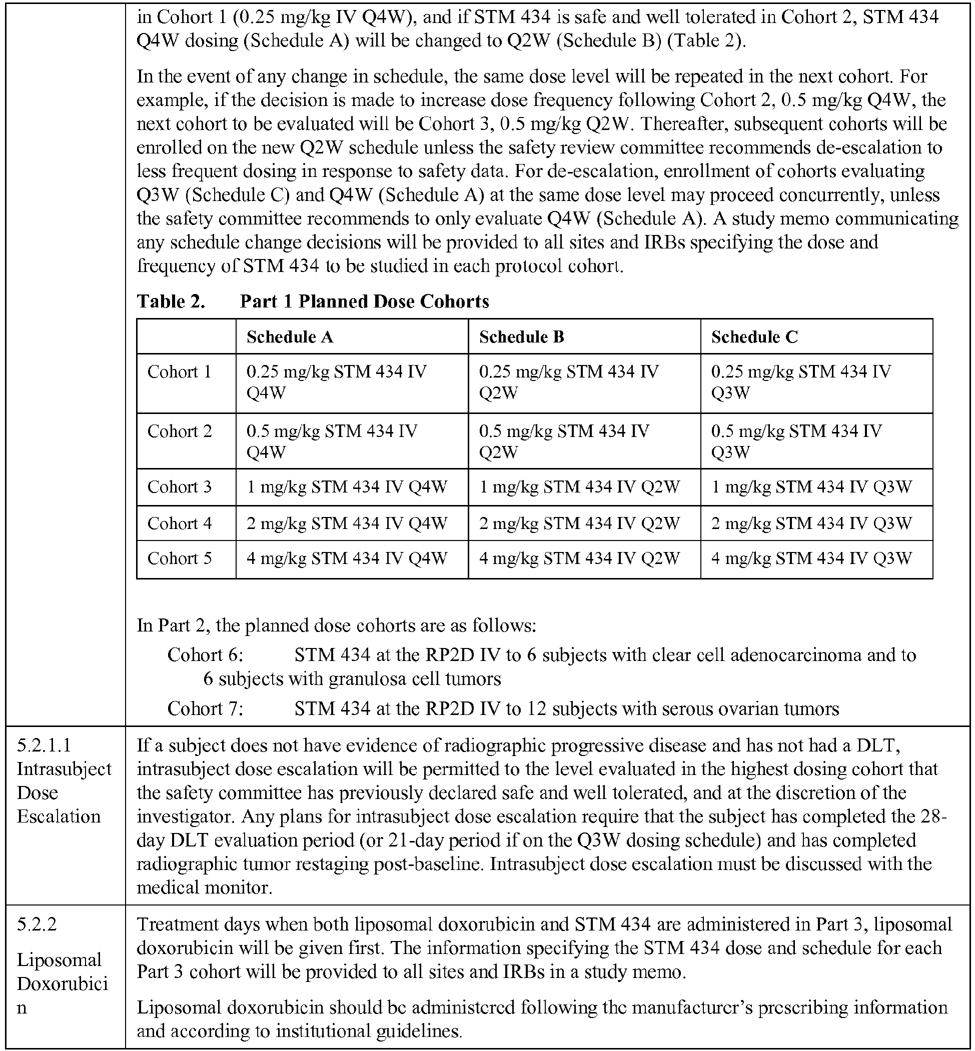

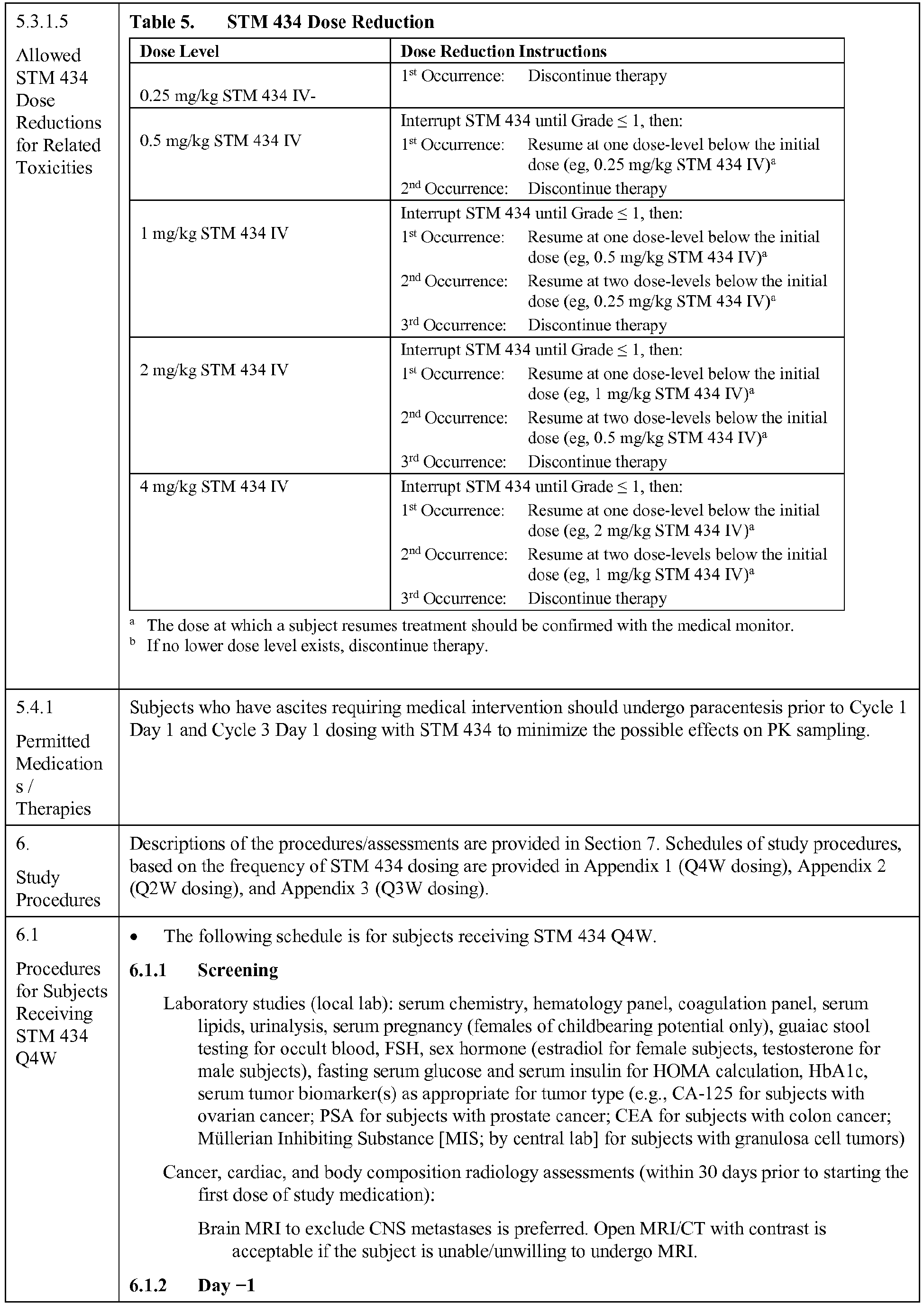

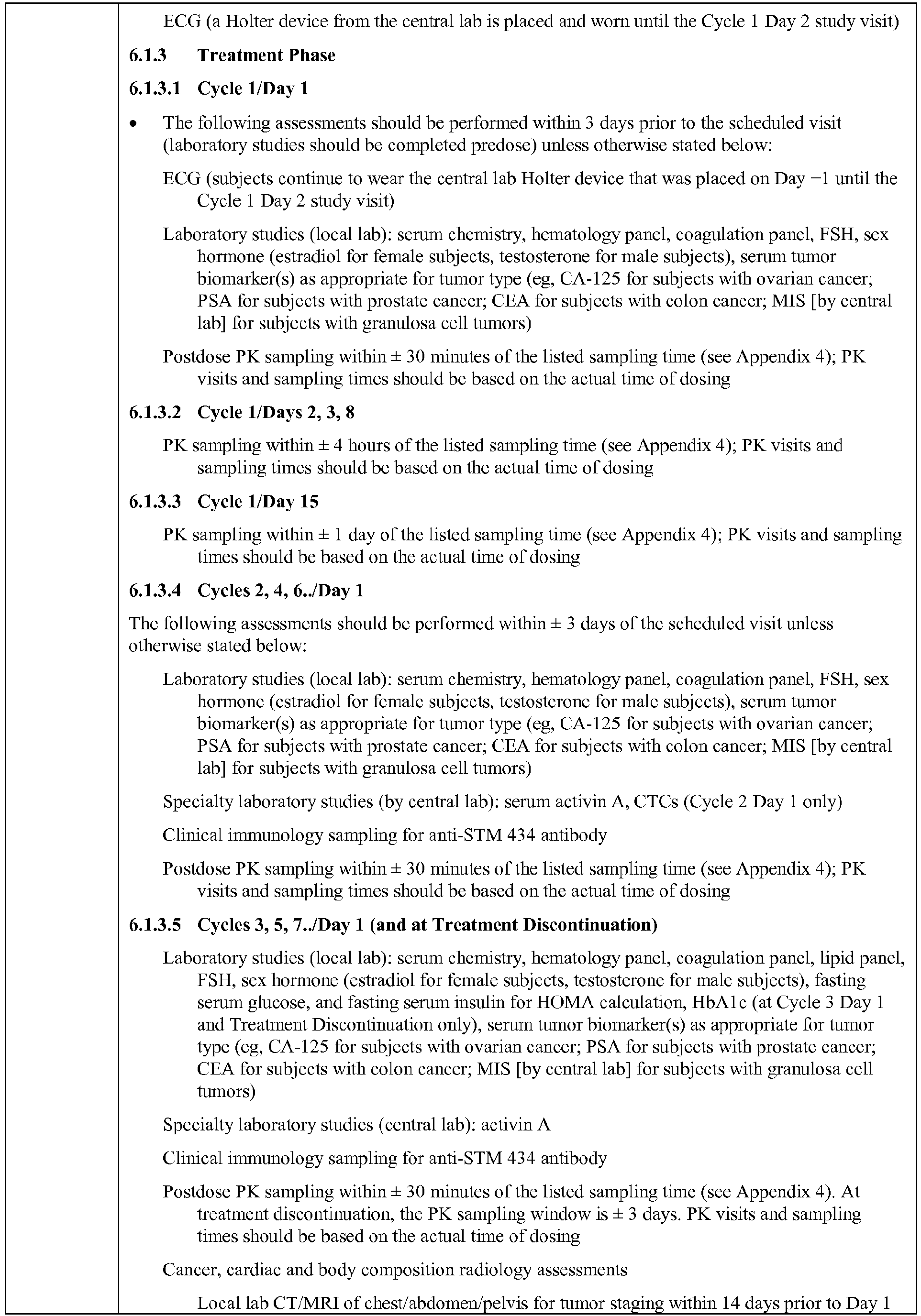

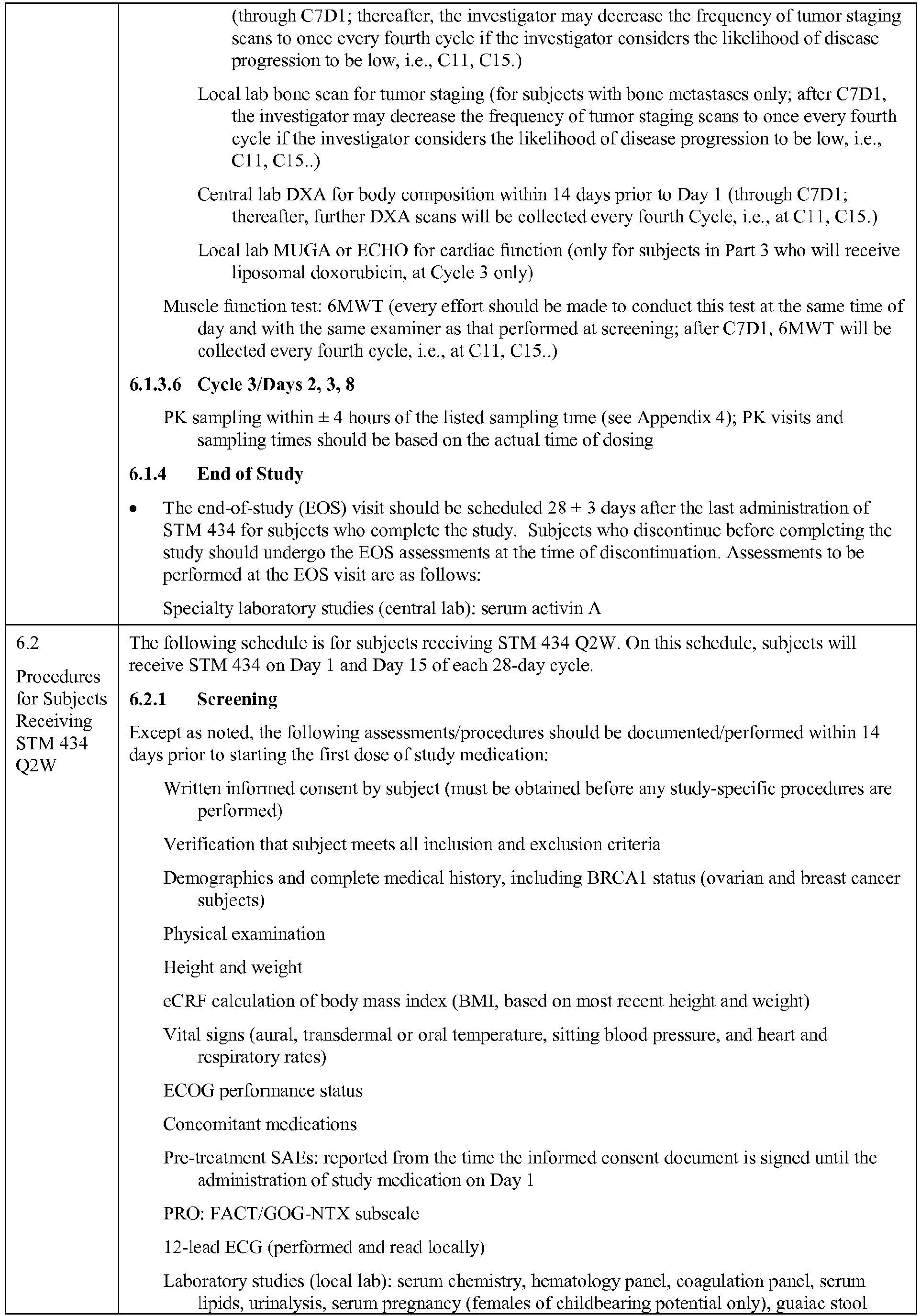

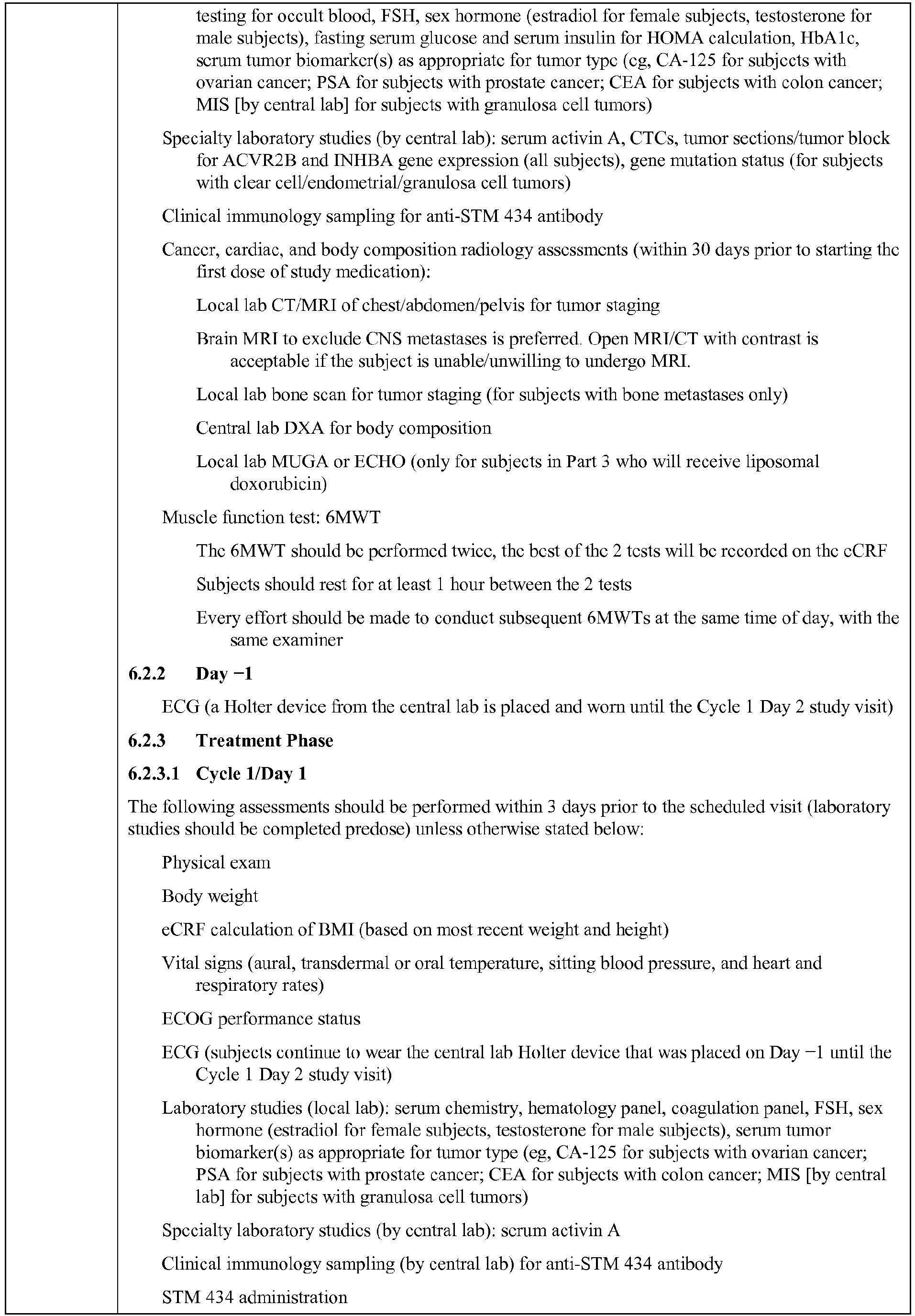

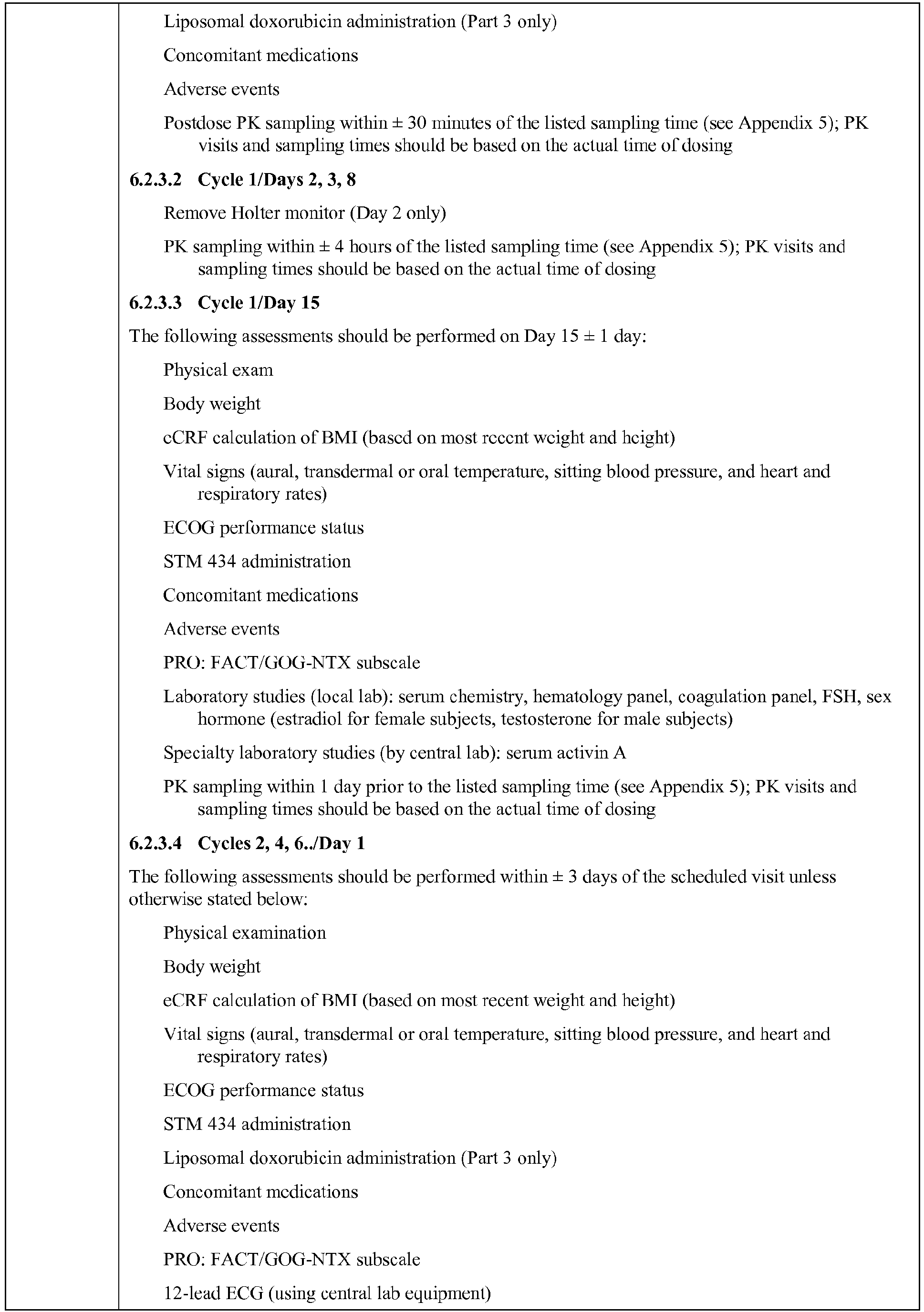

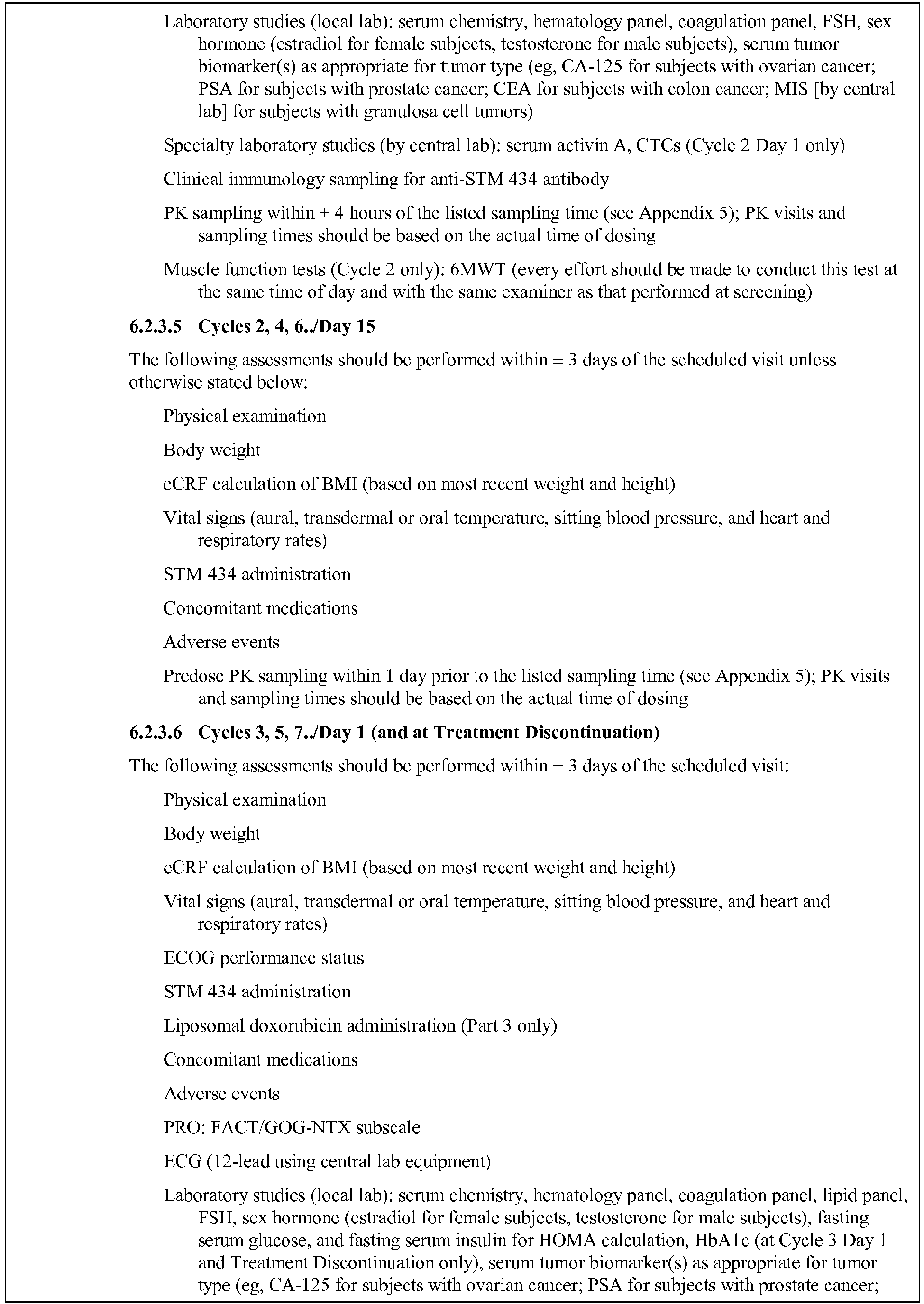

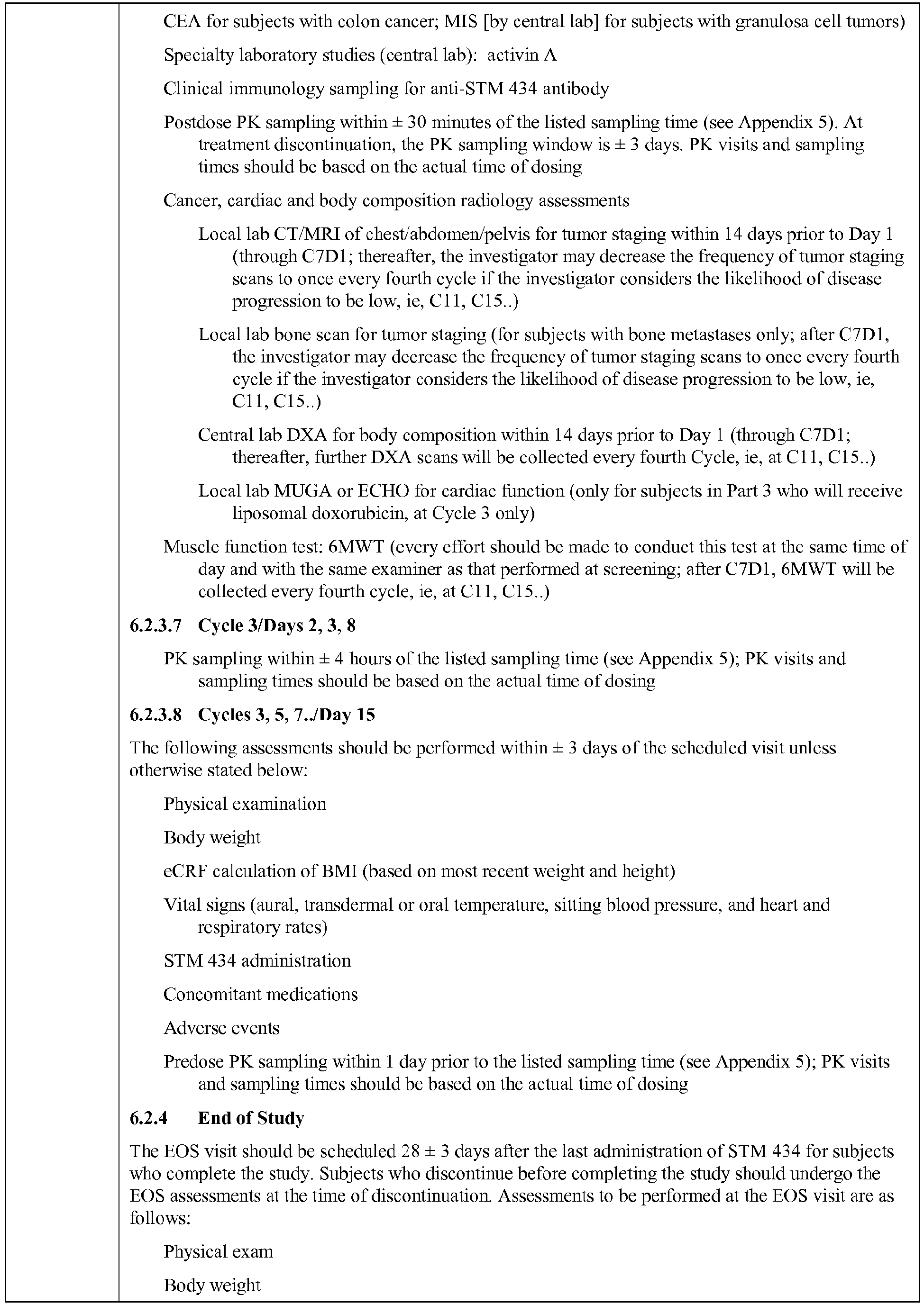

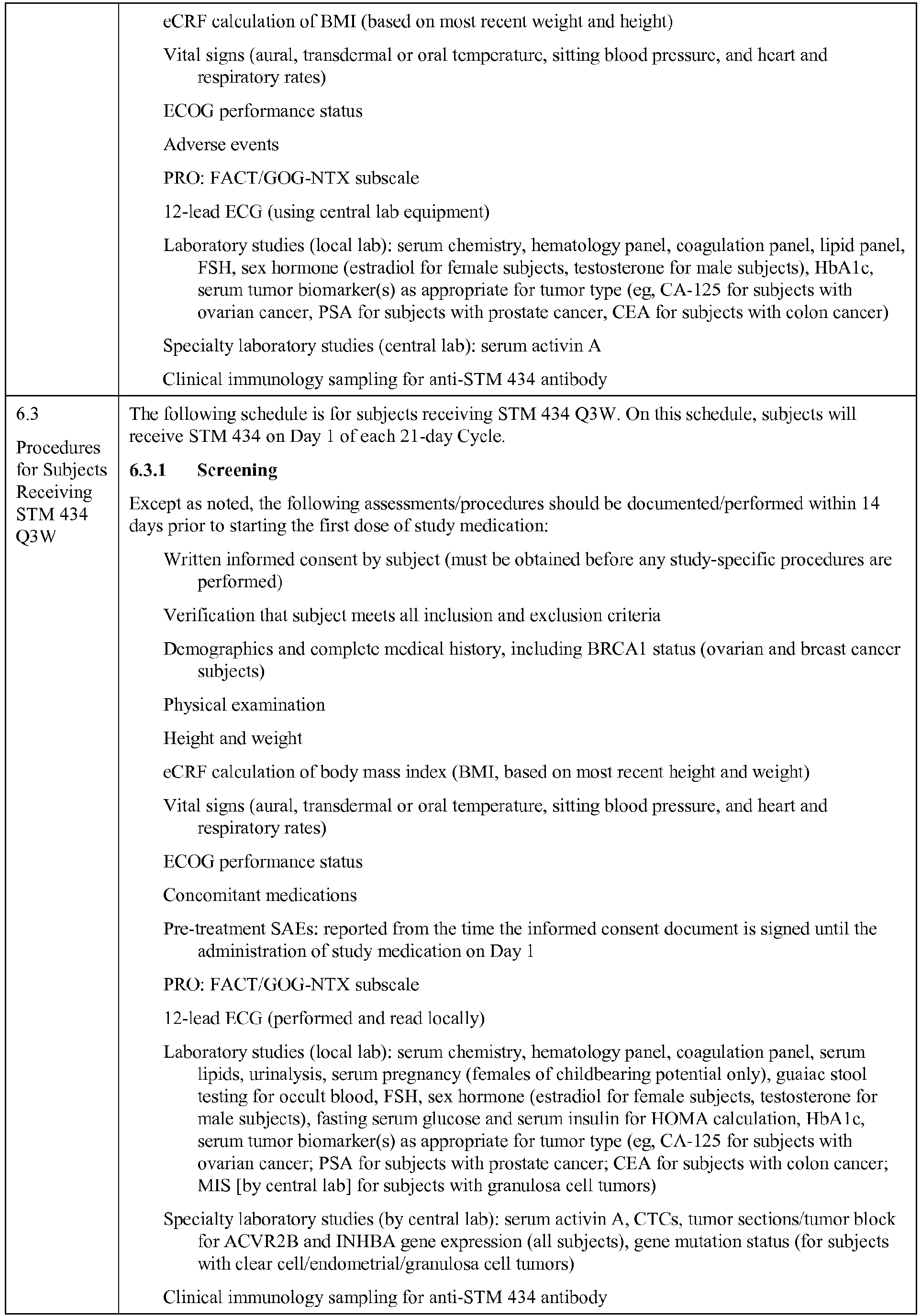

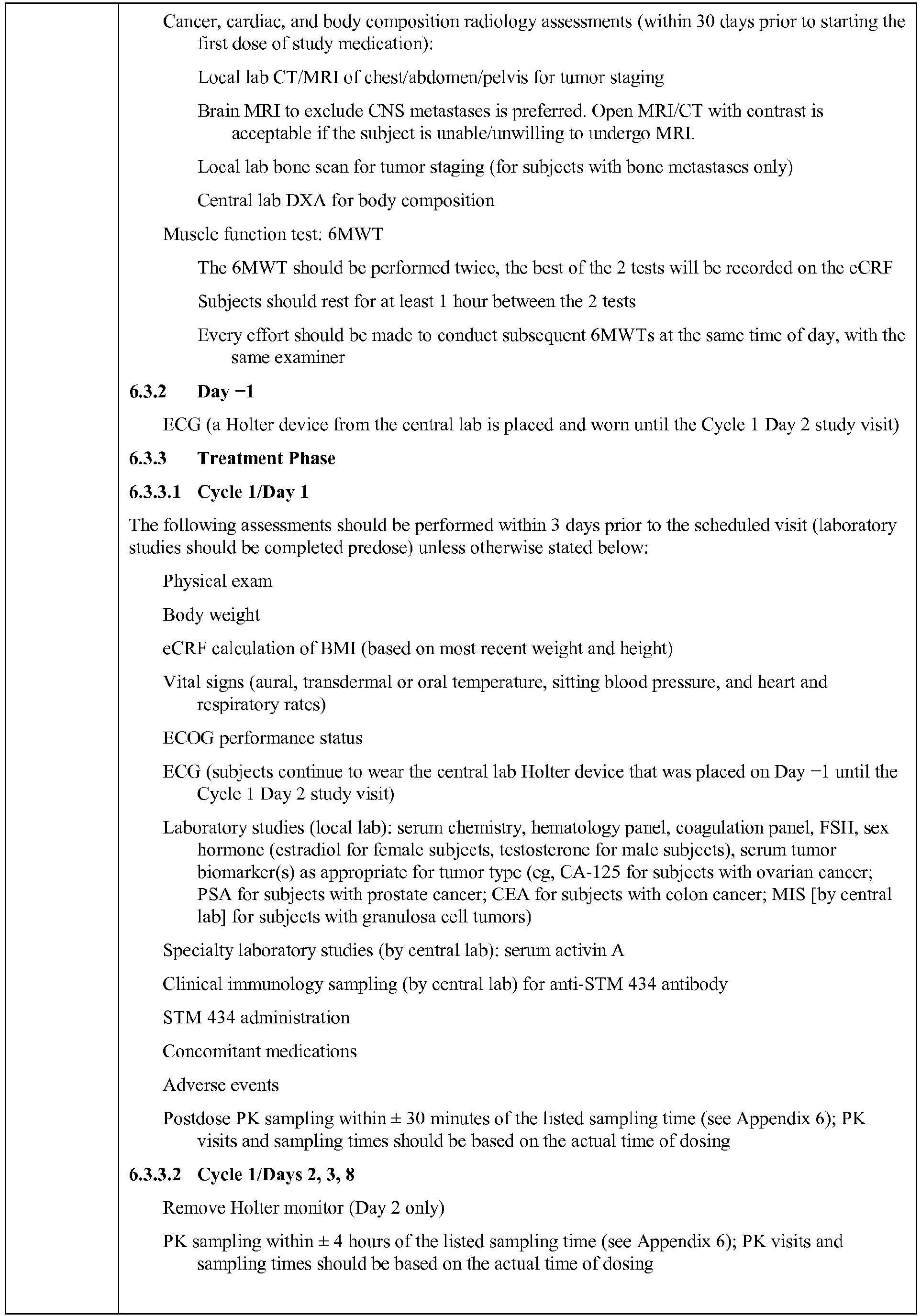

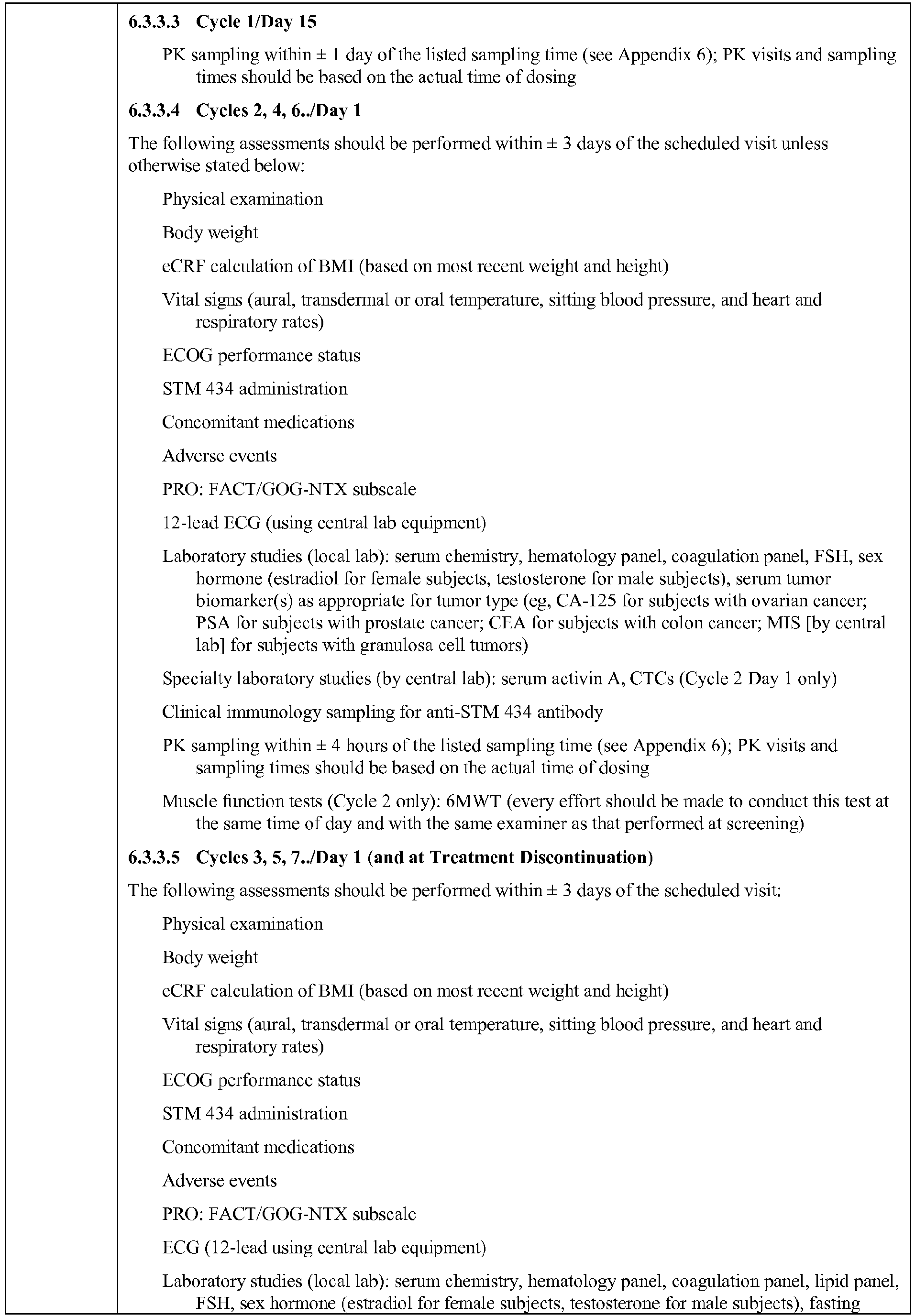

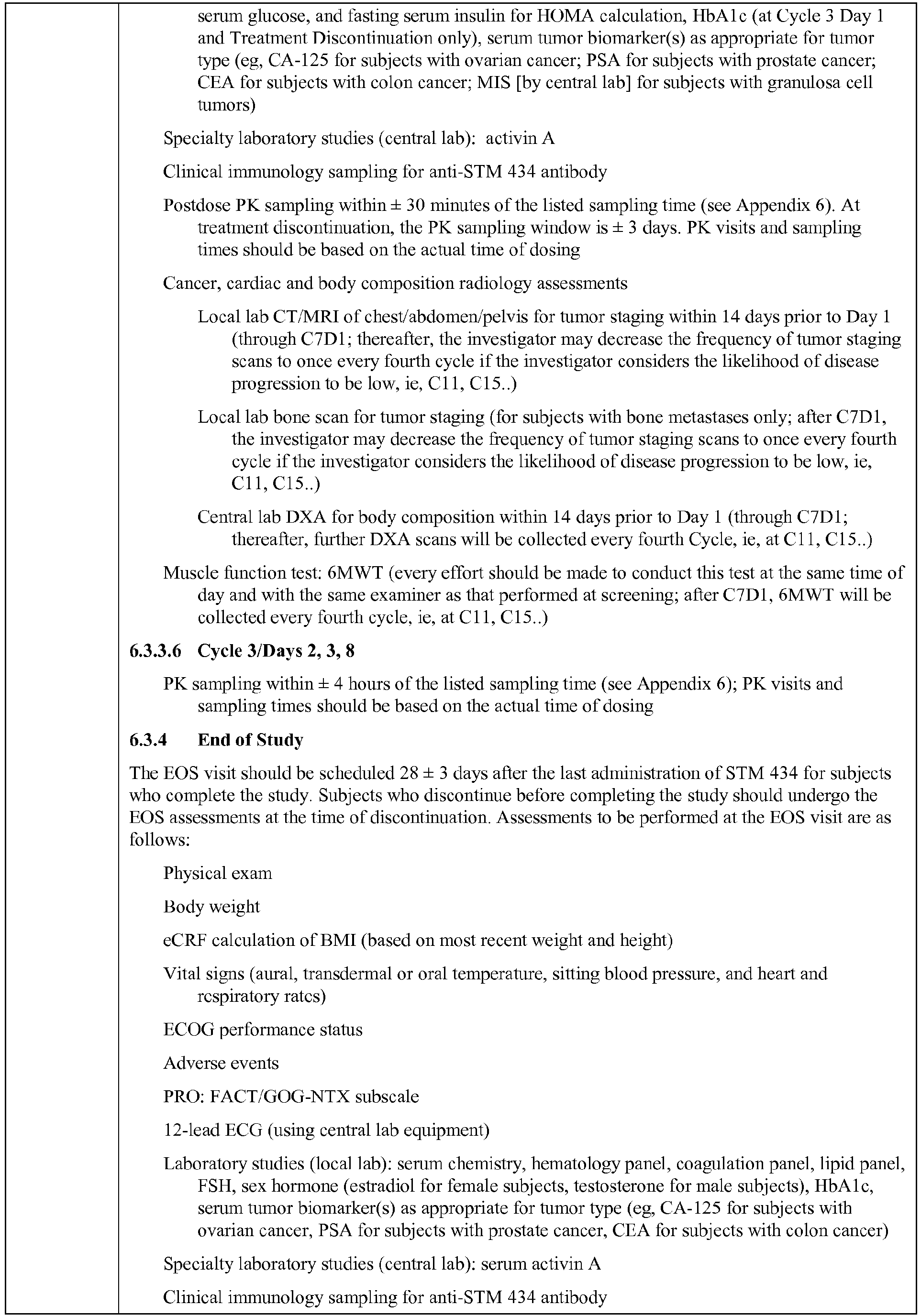

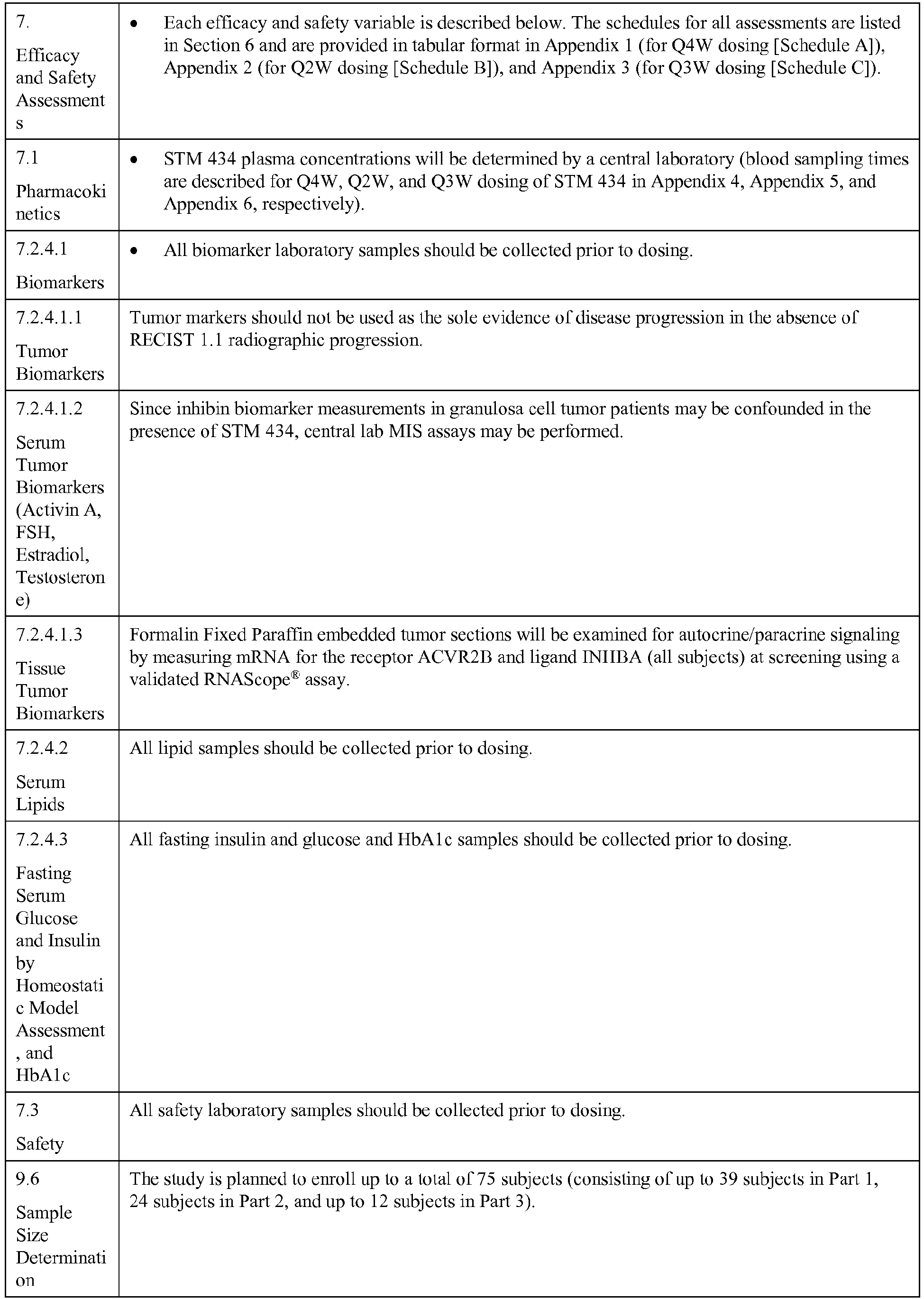

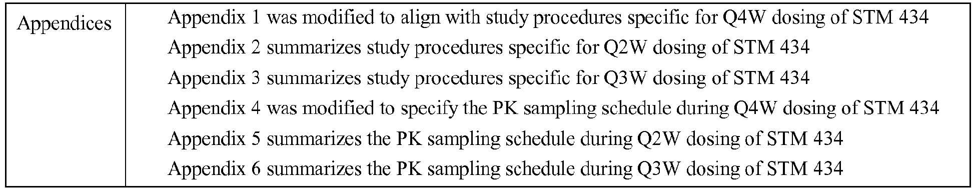

| **Appendix 1: Study Procedures for STM 434 Administered Q4W** | | | | | | | | | | | |
|---|---|---|---|---|---|---|---|---|---|---|---|
| **Evaluation** | **Screening** | | **Treatment Phase** | | | | | | | | **End of Stud y²** |
| | | | **Cycle 1** | | | **Cycles 2, 4, 6...** | **Cycles 3, 5, 7...** | | | | |
| | **Day s -14 to 1** | **Day -1** | **Day 1 (-3 days)** | **Day s 2, 3,8** | **Day 15 (± 1 day)** | **Day 1 (± 3 days)** | **Day 1 (± 3 days)** | **Day s2, 3,8** | **Day 15 (± 1 day)** | **Treat ment Discon 1** | |
| Consent form signed | X | | | | | | | | | | |
| Medical history, Demographics, Prior cancer therapies | X | | | | | | | | | | |
| Physical exam, Height and Weight³ | X | | X | | X | X | X | | | X | X |
| Stool guaiac exam | X⁴ | | | | | | | | | | |
| Vital signs⁵ | X | | X | | X | X | X | | | X | X |
| ECOG | X | | X | | X | X | X | | | X | X |
| ECG⁶ | X | X | | X⁶ | | X | X | | | X | X |
| FACT/GOG-NTX⁷ | X | | | | X | X | X | | | X | X |
| MUGA or ECHO | X⁸ | | | | | | X⁸ | | | X⁸ | |
| STM 434 administration | | | X | | | X | X | | | X | |
| Liposomal doxorubicin dosing administration⁹ | | | X | | | X | X | | | X | |
| Concomitant medications | X | | X | | X | X | X | | | X | X |
| Adverse events | X¹⁰ | | X | | X | X | X | | | X | X¹¹ |
| Hematology | X | | X | | X | X | X | | | X | X |
| Coagulation panel (PT, PTT, INR, TT¹², D-dimer¹²) | X | | X | | X | X | X | | | X | X |
| Serum chemistry | X | | X | | X | X | X | | | X | X |
| Fasting glucose and fasting insulin | X | | | | | | X | | | X | |
| HbA1c¹³ | X | | | | | | X¹³ | | | X¹³ | X |

| **Day s -14 to 1** | **Day -1** | **Day 1 (-3 days)** | **Day s 2, 3,8** | **Day 15 (± 1 day)** | **Day 1 (± 3 days)** | **Day 1 (± 3 days)** | **Day s 2, 3, 8** | **Day 15 (± 1 day)** | **Treat ment Discon 1** | | |
|---|---|---|---|---|---|---|---|---|---|---|---|
| Serum lipids | X | | | | | | X | | | X | X |
| Serum tumor biomarkers¹⁴ | X | | X | | | X | X | | | X | X |
| Tissue tumor biomarkers¹⁵ | X | | | | | | | | | | |
| Urinalysis (dipstick) | X | | | | | | | | | | |
| Serum pregnancy¹⁶ | X | | | | | | | | | | |
| Activin A (free and total) | X | | X | | X | X | X | | | X | X |
| CTCs | X¹⁷ | | | | | X¹⁷ | | | | | |
| FSH, estradiol (female subjects), testosterone (male subjects) | X | | X | | X | X | X | | | X | X |
| Central lab DXA | X¹⁸ | | | | | | X¹⁹ | | | X¹⁹ | |
| 6-minute walk test | X | | | | | X²⁰ | X | | | X | |
| Screening brain CT/MRI | X | | | | | | | | | | |
| Chest/abdomen/pe lvis CT/MRI | X¹⁸ | | | | | | X¹⁹ | | | X¹⁹ | |
| Bone scan²¹ | X¹⁸ | | | | | | X¹⁹ | | | X¹⁹ | |
| Disease progression assessment²² | | | | | | | X | | | X | |
| Anti-STM 434 antibody | X | | X | | | X | X | | | X | X |
| Predose PK | | | X²³ | | | X²³ | X²³ | | | X²³ | |
| Postdose PK | | | X²³ | X²³ | X²³ | X²³ | | X²³ | X²³ | | |

| | | | | | | | | | | | |
|---|---|---|---|---|---|---|---|---|---|---|---|
| **Footnotes to Study Procedures** ¹ The Treatment Discontinuation visit can occur at any scheduled or unscheduled visit when appropriate. At this visit, documentation to confirm progressive disease or unacceptable toxicity is required. ² The EOS visit should be scheduled 28 ± 3 days following the last administration of STM 434. ³ Subject weight is recorded at each visit; height is recorded only at the screening visit. ⁴ Stool guaiac is required at screening and may be repeated at the discretion of the investigator post-baseline. ⁵ Vital signs: aural, transdermal or oral temperature, sitting blood pressure, heart rate, and respiratory rate ⁶ After the screening visit, ECGs must be collected using equipment from the central laboratory. Starting on Day -1 subjects will wear a Holter monitor continuously until the Cycle 1/Day 2 visit, at which time the Holter monitor will be removed. On Day 1 of each of the subsequent cycles and at the EOS visit, 12-lead ECGs will be obtained with the central lab equipment. ⁷ The FACT/GOG-NTX may be collected at unscheduled visits if needed to assess an AE of neuropathy. ⁸ Only subjects in Part 3 require MUGA or ECHO to document the cardiac ejection fraction for liposomal doxorubicin administration. The choice of MUGA or ECHO is at the investigator's discretion, but once a modality is selected, the same method should be used for the subject throughout the study period. MUGA/ECHO is to be performed only at screening and Cycle 3 Day 1. In the event that a doxorubicin-treated subject in Part 3 discontinues prior to Cycle 3 Day 1, a MUGA/ECHO should be performed at the treatment discontinuation visit. ⁹ Liposomal doxorubicin administration is limited to subjects in Part 3 of the study. ¹⁰ Pretreatment SAEs should be reported from the time the informed consent document is signed until the administration of study medication on Cycle 1 Day 1. ¹¹ AE follow-up is required for 30 days following the last administration of STM 434 to determine if there are any new or ongoing AEs or SAEs regardless of attribution of causality. Follow-up may be conducted via telephone and must be documented in the source notes. ¹² Screening and Cycle 2 Day 1 only. ¹³ HbAlc will be collected and analyzed at screening, Cycle 3 Day 1, Treatment Discontinuation, and EOS only. ¹⁴ Serum tumor biomarkers can include CA-125, PSA, CA 19-9, CEA, or other biomarkers as appropriate based on the subject's tumor type. For patients with granulosa cell tumors, perform central lab MIS on Day 1 of each cycle. ¹⁵ Tissue tumor biomarkers include tumor sections/tumor block for ACVR2B and INHBA gene expression (all subjects) as well as gene mutation status (for subjects with clear cell/endometrial/granulosa cell tumors). ¹⁶ Females of childbearing potential only. ¹⁷ CTCs will be collected at screening and Cycle 2 Day 1 only. ¹⁸ Scans (DXA, CT, MRI, Bone) performed up to 30 days prior to Cycle 1 Day 1 can be used for the baseline assessments. ¹⁹ On-study DXA, CT/MRI and bone scans can be done within 14 days prior to the Day 1 visit. After scans are performed at the Cycle 7 Day 1 visit, subsequent scans may be performed every fourth cycle if the investigator considers the likelihood of disease progression low. ²⁰ 6-minute walk test will only be completed at the Cycle 2 Day 1 visit. ²¹ Bone scan assessments should be conducted at the investigator's discretion, for example, if the subject has tumors metastatic to bone. ²² The investigator should assess the radiographic scans according to RECIST 1.1 (Appendix 7) ²³ The detailed schedule for PK assessments is presented in Appendix 4 of this protocol. Note that the Day 2, 3, and 8 PK assessments are performed at Cycle 1 and Cycle 3 only. | | | | | | | | | | | |

| **Appendix 2. Study Procedures for STM 434 Administered Q2W (STM 434 Dosing on Days 1 and 15 of each 28-Day Cycle)** | | | | | | | | | | | | |
|---|---|---|---|---|---|---|---|---|---|---|---|---|
| **Evaluation** | **Screening** | | **Treatment Phase** | | | | | | | | | **End of Stud** y² |
| | | | **Cycle 1** | | | **Cycles 2, 4, 6...** | | **Cycles 3, 5, 7...** | | | | |
| | **Days -14 to 1** | **Day -1** | **Day 1 (-3 days)** | **Day s2, 3,8** | **Day 15 (± 1 day)** | **Day 1 (± 3 days)** | **Day 15 (± 3 days)** | **Day 1 (± 3 days)** | **Day 2, 3, 8** | **Day 15 (± 3 days)** | **Treat ment Disco n¹** | |
| Consent form signed | X | | | | | | | | | | | |
| Medical history, Demographics, Prior cancer therapies | X | | | | | | | | | | | |
| Physical exam, Height and Weight³ | X | | X | | X | X | X | X | | X | X | X |
| Stool guaiac exam | x⁴ | | | | | | | | | | | |
| Vital signs⁵ | X | | X | | X | X | X | X | | X | X | X |

| **Evaluation** | **Screening** | | **Treatment Phase** | | | | | | | | | **End of Stud y²** |
|---|---|---|---|---|---|---|---|---|---|---|---|---|
| | | | **Cycle 1** | | | **Cycles 2, 4, 6...** | | **Cycles 3, 5, 7...** | | | | |
| | **Days -14 to 1** | **Day -1** | **Day 1 (-3 days)** | **Day s2, 3,8** | **Day 15 (± 1 day)** | **Day 1 (±3 days)** | **Day 15 (±3 days)** | **Day 1 (±3 days)** | **Day 2, 3, 8** | **Day 15 (± 3 days)** | **Treat ment Disco n¹** | |
| ECOG | X | | X | | X | X | | X | | | X | X |
| ECG⁷ | X | X | | X⁶ | | X | | X | | | X | X |
| FACT/GOG-NTX⁷ | X | | | | X | X | | X | | | X | X |
| MUGA or ECHO | X⁸ | | | | | | | X⁸ | | | X⁸ | |
| STM 434 administration | | | X | | X | X | X | X | | X | X | |
| Liposomal doxorubicin dosing administration⁹ | | | X | | | X | | X | | | X | |
| Concomitant medications | X | | X | | X | X | X | X | | X | X | X |
| Adverse events | X¹⁰ | | X | | X | X | X | X | | X | X | X¹¹ |
| Hematology | X | | X | | X | X | | X | | | X | X |
| Coagulation panel (PT, PTT, INR, TT¹², D dimer¹²) | X | | X | | X | X | | X | | | X | X |
| Serum chemistry | X | | X | | X | X | | X | | | X | X |
| Fasting glucose and fasting insulin | X | | | | | | | X | | | X | |
| HbA1c¹³ | X | | | | | | | X¹³ | | | X¹³ | X |
| Serum lipids | X | | | | | | | X | | | X | X |
| Serum tumor biomarkers ¹⁴ | X | | X | | | X | | X | | | X | X |
| Tissue tumor biomarkers ¹⁵ | X | | | | | | | | | | | |
| Urinalysis (dipstick) | X | | | | | | | | | | | |
| Serum pregnancy¹⁶ | X | | | | | | | | | | | |
| Activin A (free and total) | X | | X | | X | X | | X | | | X | X |
| CTCs | X¹⁷ | | | | | X¹⁷ | | | | | | |

| **Days -14 to 1** | **Day -1** | **Day 1 (-3 days)** | **Day s2, 3,8** | **Day 15 (± 1 day)** | **Day 1 (± 3 days)** | **Day 15 (± 3 days)** | **Day 1 (± 3 days)** | **Day 2, 3, 8** | **Day 15 (± 3 days)** | **Treat ment Disco n¹** | | |
|---|---|---|---|---|---|---|---|---|---|---|---|---|
| FSH, estradiol (female subjects), testosterone (male subjects) | X | | X | | X | X | | X | | | X | X |
| Central lab DXA | X¹⁸ | | | | | | | X¹⁹ | | | X¹⁹ | |
| 6-minute walk test | X | | | | | X²⁰ | | X | | | X | |
| Screening brain CT/MRI | X | | | | | | | | | | | |
| Chest/abdomen/p elvis CT/MRI | X¹⁸ | | | | | | | X¹⁹ | | | X¹⁹ | |
| Bone scan²¹ | X¹⁸ | | | | | | | X¹⁹ | | | X¹⁹ | |
| Disease progression assessment²² | | | | | | | | X | | | X | |
| Anti-STM 434 antibody | X | | X | | | X | | X | | | X | X |
| Predose PK | | | X²³ | | | X²³ | X²³ | X²³ | | X²³ | X²³ | |
| Postdose PK | | | X²³ | X²³ | X²³ | X²³ | | | X²³ | | | |

| | | | | | | | | | | | | |
|---|---|---|---|---|---|---|---|---|---|---|---|---|
| **Footnotes to Study Procedures** ¹ The Treatment Discontinuation visit can occur at any scheduled or unscheduled visit when appropriate. At this visit, documentation to confirm progressive disease or unacceptable toxicity is required. ² The EOS visit should be scheduled 28 ± 3 days following the last administration of STM 434. ³ Subject weight is recorded at each visit; height is recorded only at the screening visit. ⁴ Stool guaiac is required at screening and may be repeated at the discretion of the investigator post-baseline. ⁵ Vital signs: aural, transdermal or oral temperature, sitting blood pressure, heart rate, and respiratory rate ⁶ After the screening visit, ECGs must be collected using equipment from the central laboratory. Starting on Day-1 subjects will wear a Holter monitor continuously until the Cycle 1/Day 2 visit, at which time the Holter monitor will be removed. On Day 1 of each of the subsequent cycles and at the EOS visit, 12-lead ECGs will be obtained with the central lab equipment. ⁷ The FACT/GOG-NTX may be collected at unscheduled visits if needed to assess an AE of neuropathy. ⁸ Only subjects in Part 3 require MUGA or ECHO to document the cardiac ejection fraction for liposomal doxorubicin administration. The choice of MUGA or ECHO is at the investigator's discretion, but once a modality is selected, the same method should be used for the subject throughout the study period. MUGA/ECHO is to be performed only at screening and Cycle 3 Day 1. In the event that a doxorubicin-treated subject in Part 3 discontinues prior to Cycle 3 Day 1, a MUGA/ECHO should be performed at the treatment discontinuation visit. ⁹ Liposomal doxorubicin administration is limited to subjects in Part 3 of the study. ¹⁰ Pretreatment SAEs should be reported from the time the informed consent document is signed until the administration of study medication on Cycle 1 Day 1. ¹¹ AE follow-up is required for 30 days following the last administration of STM 434 to determine if there are any new or ongoing AEs or SAEs regardless of attribution of causality. Follow-up may be conducted via telephone and must be documented in the source notes. ¹² Screening and Cycle 2 Day 1 only. ¹³ HbAlc will be collected and analyzed at screening, Cycle 3 Day 1, Treatment Discontinuation, and EOS only. ¹⁴ Serum tumor biomarkers can include CA-125, PSA, CA 19-9, CEA, or other biomarkers as appropriate based on the subject's tumor type. For patients with granulosa cell tumors, perform central lab MIS on Day 1 of each cycle. ¹⁵ Tissue tumor biomarkers include tumor sections/tumor block for ACVR2B and INHBA gene expression (all subjects) as well as gene mutation status (for subjects with clear cell/endometrial/granulosa cell tumors). ¹⁶ Females of childbearing potential only. ¹⁷ CTCs will be collected at screening and Cycle 2 Day 1 only. ¹⁸ Scans (DXA, CT, MRI, Bone) performed up to 30 days prior to Cycle 1 Day 1 can be used for the baseline assessments. ¹⁹ On-study DXA, CT/MRI and bone scans can be done within 14 days prior to the Day 1 visit. After scans are performed at the Cycle 7 Day 1 visit, subsequent scans may be performed every fourth cycle if the investigator considers the likelihood of disease progression low. ²⁰ 6-minute walk test will only be completed at the Cycle 2 Day 1 visit. ²¹ Bone scan assessments should be conducted at the investigator's discretion, for example, if the subject has tumors metastatic to bone. ²² The investigator should assess the radiographic scans according to RECIST 1.1 (Appendix 7) ²³ The detailed schedule for PK assessments is presented in Appendix 5 of this protocol. Note that the Day 2, 3, and 8 PK assessments are performed at Cycle 1 and Cycle 3 only. | | | | | | | | | | | | |

| **Appendix 3. Study Procedures for STM 434 Administered Q3W** | | | | | | | | | | |
|---|---|---|---|---|---|---|---|---|---|---|
| **Evaluation** | **Screening** | | **Treatment Phase** | | | | | | | **End of Stud y²** |
| | | | **Cycle 1** | | | **Cycles 2, 4, 6...** | **Cycles 3, 5, 7...** | | | |
| | **Days -14 to 1** | **Day -1** | **Day 1 (-3 days)** | **Day s 2, 3,8** | **Day 15 (± 1 day)** | **Day 1 (± 3 days)** | **Day 1 (± 3 days)** | **Day 2, 3,8** | **Treat ment Discon 1** | |
| Consent form signed | X | | | | | | | | | |
| Medical history, Demographics, Prior cancer therapies | X | | | | | | | | | |
| Physical exam, Height and Weight³ | X | | X | | | X | X | | X | X |
| Stool guaiac exam | x⁴ | | | | | | | | | |
| Vital signs⁵ | X | | X | | | X | X | | X | X |
| ECOG | X | | X | | | X | X | | X | X |
| ECG⁶ | X | X | | X⁶ | | X | X | | X | X |
| FACT/GOG-NTX⁷ | X | | | | | X | X | | X | X |
| STM 434 administration | | | X | | | X | X | | X | |
| Concomitant medications | X | | X | | | X | X | | X | X |
| Adverse events | X⁸ | | X | | | X | X | | X | X⁹ |
| Hematology | X | | X | | | X | X | | X | X |
| Coagulation panel (PT, PTT, | X | | X | | | X | X | | X | X |

| **Days -14 to 1** | **Day -1** | **Day 1 (-3 days)** | **Day s2, 3,8** | **Day 15 (± 1 day)** | **Day 1 (± 3 days)** | **Day 1 (± 3 days)** | **Day 2, 3,8** | **Treat ment Discon 1** | | |
|---|---|---|---|---|---|---|---|---|---|---|
| INR, TT¹⁰, D-dimer¹⁰) | | | | | | | | | | |
| Serum chemistry | X | | X | | | X | X | | X | X |
| Fasting glucose and fasting insulin | X | | | | | | X | | X | |
| HbA1c¹¹ | X | | | | | | X¹¹ | | X¹¹ | X |
| Serum lipids | X | | | | | | X | | X | X |
| Serum tumor biomarkers ¹² | X | | X | | | X | X | | X | X |
| Tissue tumor biomarkers ¹³ | X | | | | | | | | | |
| Urinalysis (dipstick) | X | | | | | | | | | |
| Serum pregnancy¹⁴ | X | | | | | | | | | |
| Activin A (free and total) | X | | X | | | X | X | | X | X |
| CTCs | X¹⁵ | | | | | X¹⁵ | | | | |
| FSH, estradiol (female subjects), testosterone (male subjects) | X | | X | | | X | X | | X | X |
| Central lab DXA | X¹⁶ | | | | | | X¹⁷ | | X¹⁷ | |
| 6-minute walk test | X | | | | | X¹⁸ | X | | X | |
| Screening brain CT/MRI | X | | | | | | | | | |
| Chest/abdomen/p elvis CT/MRI | X¹⁶ | | | | | | X¹⁷ | | X¹⁷ | |
| Bone scan¹⁹ | X¹⁶ | | | | | | X¹⁷ | | X¹⁷ | |
| Disease progression assessment²⁰ | | | | | | | X | | X | |
| Anti-STM 434 antibody | X | | X | | | X | X | | X | X |
| Predose PK | | | X²¹ | | | X²¹ | X²¹ | | X²¹ | |
| Postdose PK | | | X²¹ | X²¹ | X²¹ | X²¹ | | X²¹ | | |

| | | | | | | | | | | |
|---|---|---|---|---|---|---|---|---|---|---|
| **Footnotes to Study Procedures** ¹ The Treatment Discontinuation visit can occur at any scheduled or unscheduled visit when appropriate. At this visit, documentation to confirm progressive disease or unacceptable toxicity is required. ² The EOS visit should be scheduled 28 ± 3 days following the last administration of STM 434. ³ Subject weight is recorded at each visit; height is recorded only at the screening visit. ⁴ Stool guaiac is required at screening and may be repeated at the discretion of the investigator post-baseline. ⁵ Vital signs: aural, transdermal or oral temperature, sitting blood pressure, heart rate, and respiratory rate ⁶ After the screening visit, ECGs must be collected using equipment from the central laboratory. Starting on Day⁻¹ subjects will wear a Holter monitor continuously until the Cycle 1/Day 2 visit, at which time the Holter monitor will be removed. On Day 1 of each of the subsequent cycles and at the EOS visit, 12-lead ECGs will be obtained with the central lab equipment. ⁷ The FACT/GOG-NTX may be collected at unscheduled visits if needed to assess an AE of neuropathy. ⁸ Pretreatment SAEs should be reported from the time the informed consent document is signed until the administration of study medication on Cycle 1 Day 1. ⁹ AE follow-up is required for 30 days following the last administration of STM 434 to determine if there are any new or ongoing AEs or SAEs regardless of attribution of causality. Follow-up may be conducted via telephone and must be documented in the source notes. ¹⁰ Screening and Cycle 2 Day 1 only. ¹¹ HbAlc will be collected and analyzed at screening, Cycle 3 Day 1, Treatment Discontinuation, and EOS only. ¹² Serum tumor biomarkers can include CA-125, PSA, CA 19-9, CEA, or other biomarkers as appropriate based on the subject's tumor type. For patients with granulosa cell tumors, perform central lab MIS on Day 1 of each cycle. ¹³ Tissue tumor biomarkers include tumor sections/tumor block for ACVR2B and INHBA gene expression (all subjects) as well as gene mutation status (for subjects with clear cell/endometrial/granulosa cell tumors). ¹⁴ Females of childbearing potential only. ¹⁵ CTCs will be collected at screening and Cycle 2 Day 1 only. ¹⁶ Scans (DXA, CT, MRI, Bone) performed up to 30 days prior to Cycle 1 Day 1 can be used for the baseline assessments. ¹⁷ On-study DXA, CT/MRI and bone scans can be done within 14 days prior to the Day 1 visit. After scans are performed at the Cycle 7 Day 1 visit, subsequent scans may be performed every fourth cycle if the investigator considers the likelihood of disease progression low. ¹⁸ 6-minute walk test will only be completed at the Cycle 2 Day 1 visit. ¹⁹ Bone scan assessments should be conducted at the investigator's discretion, for example, if the subject has tumors metastatic to bone. ²⁰ The investigator should assess the radiographic scans according to RECIST 1.1 (Appendix 7) ²¹ The detailed schedule for PK assessments is presented in Appendix 6 of this protocol. Note that the Day 2, 3, and 8 PK assessments are performed at Cycle 1 and Cycle 3 only. | | | | | | | | | | |

| **Appendix 4. Pharmacokinetic Evaluation Schedule for Q4W Administration of STM 434** | | | | |
|---|---|---|---|---|
| **Cycle and Day** | **Part 1 (Dose Escalation)** | **Part 2 (Monotherapy Dose Expansion)** | **Part 3 (STM 434 + Liposomal Doxorubicin)** | |
| | **STM 434 Sampling^{a}** | **STM 434 Sampling^{a}** | **STM 434 Sampling^{a}** | **Doxorubicin/Doxorubicinol Sampling^{b}** |
| Cycle 1 Day 1 | Predose End of infusion 1, 2, 6 h after infusion | Predose End of infusion 1, 2, 6 h after infusion | Predose End of infusion 1, 2, 6 h after infusion | Predose End of infusion 1, 2, 3, 4, 6 h after the infusion |
| Cycle 1 Day 2 | 24 h after infusion | - | 24 h after infusion | 24 h after infusion |
| Cycle 1 Day 3 | 48 h after infusion | - | 48 h after infusion | 48 h after infusion |
| Cycle 1 Day 8 | 168 h after infusion | - | 168 h after infusion | 168 h after infusion |
| Cycle 1 Day 15 | 336 h after infusion | 336 h after infusion | 336 h after infusion | 336 h after infusion |
| Cycle ² Day 1 | 672 h after Cycle | 672 h after Cycle 1 | 672 h after Cycle 1 | End Cycle 1/Predose Cycle 2 |

| | 1 End of infusion Cycle 2 | End of infusion Cycle 2 | End of infusion Cycle 2 | End of infusion Cycle 2 |
|---|---|---|---|---|
| Cycle 3 Day 1 | 672 h after Cycle 2 End of infusion Cycle 3 1, 2, 6 h after infusion | 672 h after Cycle 2 End of infusion Cycle 3 1, 2, 6 h after infusion | 672 h after Cycle 2 End of infusion Cycle 3 1, 2, 6 h after infusion | End Cycle 2/Predose Cycle3 End of infusion Cycle 3 1, 2, 3, 4, 6 h after infusion |
| Cycle 3 Day 2 | 24 h after infusion | - | 24 h after infusion | 24 h after infusion |
| Cycle 3 Day 3 | 48 h after infusion | - | 48 h after infusion | 48 h after infusion |
| Cycle 3 Day 8 | 168 h after infusion | - | 168 h after infusion | 168 h after infusion |
| Cycle 3 Day 15 | 336 h after infusion | 336 h after infusion | 336 h after infusion | 336 h after infusion |
| Cycle 4 Day 1 | 672 h after Cycle 3 End of infusion Cycle 4 | 672 h after Cycle 3 End of infusion Cycle 4 | 672 h after Cycle 3 End of infusion Cycle 4 | End Cycle 3/Predose Cycle 4 End of infusion Cycle 4 |
| Cycle 5 Day 1 | 672 h after Cycle 4 End of infusion Cycle 5 | 672 h after Cycle 4 End of infusion Cycle 5 | 672 h after Cycle 4 End of infusion Cycle 5 | 672 h after Cycle 4 End of infusion Cycle 5 |
| Treatment Discontinuation | 672 h after the final infusion | - | 672 h after the final infusion | 672 h after the final infusion |

| | | | | |
|---|---|---|---|---|
| ^{a} All STM 434 sampling times are relative to STM 434 dosing. ^{b} All doxorubicin/doxorubicinol sampling times are relative to liposomal doxorubicin dosing. For Q4W dosing, postdose PK sampling windows are as follows: **Cycle 1** Day 1: ± 30 minutes Days 2, 3, 8: ± 4 hours Day 15: ± 1 day **Cycle 2** Day 1: ± 30 minutes **Cycle 3** Day 1: ± 30 minutes Days 2, 3, 8: ± 4 hours Day 15: ± 1 day **Cycle 4** Day 1: ± 30 minutes **Cycle 5** Day 1: ± 30 minutes | | | | |

| **Appendix 5. Pharmacokinetic Evaluation Schedule for Q2W Administration of STM 434** | | | | |
|---|---|---|---|---|
| **(STM 434 Dosing on Days 1 and 15 of each 28-Day Cycle)** | | | | |
| **Cycle and Day** | **Part 1 (Dose Escalation)** | **Part 2 (Monotherapy Dose Expansion)** | **Part 3 (STM 434 + Liposomal Doxorubicin)** | |
| | **STM 434 Sampling^{a}** | **STM 434 Sampling^{a}** | **STM 434 Sampling^{a}** | **Doxorubicin/Doxorubicinol Sampling^{a}** |
| Cycle 1 Day 1 | Predose End of infusion 1, 2, 6 h after infusion | Predose End of infusion 1, 2, 6 h after infusion | Predose End of infusion 1, 2, 6 h after infusion | Predose End of infusion 1, 2, 3, 4, 6 h after the infusion |
| Cycle 1 Day 2 | 24 h after infusion | - | 24 h after infusion | 24 h after infusion |
| Cycle 1 Day 3 | 48 h after infusion | - | 48 h after infusion | 48 h after infusion |
| Cycle 1 Day 8 | 168 h after infusion | - | 168 h after infusion | 168 h after infusion |
| Cycle 1 Day 15 | Predose | Predose | Predose | Predose |
| Cycle 2 Day 1 | Predose Cycle 2 | Predose Cycle 2 | Predose Cycle 2 | Predose Cycle 2 |
| Cycle 2 Day 15 | Predose | Predose | Predose | - |
| Cycle 3 Day 1 | Predose Cycle 3 End of infusion Cycle 3 1, 2, 6 h after infusion Cycle | Predose Cycle 3 End of infusion Cycle 3 1, 2, 6 h after infusion Cycle | Predose Cycle 3 End of infusion Cycle 3 1, 2, 6 h after infusion Cycle | Predose Cycle3 End of infusion Cycle 3 1, 2, 3, 4, 6 h after infusion |
| Cycle 3 Day 2 | 24 h after infusion | - | 24 h after infusion | 24 h after infusion |
| Cycle 3 Day 3 | 48 h after infusion | - | 48 h after infusion | 48 h after infusion |
| Cycle 3 Day 8 | 168 h after infusion | - | 168 h after infusion | 168 h after infusion |
| Cycle 3 Day 15 | Predose | Predose | Predose | Predose |
| Cycle 4 Day 1 | Predose Cycle 4 | Predose Cycle 4 | Predose Cycle 4 | Predose Cycle 4 |
| Cycle 4 Day 15 | Predose | Predose | Predose | Predose |
| Cycle 5 Day 1 | Predose Cycle 5 End of infusion Cycle 5 | Predose Cycle 5 End of infusion Cycle 5 | Predose Cycle 5 End of infusion Cycle 5 | Predose Cycle 5 End of infusion Cycle 5 |
| Treatment Discontinuation | 672 h after the final infusion | - | 672 h after the final infusion | 672 h after the final infusion |

| | | | | |
|---|---|---|---|---|
| ^{a} All STM 434 sampling times are relative to STM 434 dosing. ^{b} All doxorubicin/doxorubicinol sampling times are relative to liposomal doxorubicin dosing. For Q2W dosing, postdose PK sampling windows are as follows: **Cycle 1** Day 1: ± 30 minutes Days 2, 3, 8: ± 4 hours Day 15: within 1 day prior to dosing **Cycles 2, 4** Day 1: ± 4 hours Day 15: within 1 day prior to dosing **Cycle 3** Day 1: ± 30 minutes Days 2, 3, 8: ± 4 hours Day 15: within 1 day prior to dosing **Cycle 5** Day 1: ± 30 minutes | | | | |

| **Appendix 6. Pharmacokinetic Evaluation Schedule for Q3W Administration of STM 434** | | |
|---|---|---|
| **(STM 434 Dosing on Day 1 each 21-Day Cycle)** | | |
| **Cycle and Day** | **Part 1 (Dose Escalation)** | **Part 2 (Monotherapy Dose Expansion)** |
| | **STM 434 Sampling^{a}** | **STM 434 Sampling^{a}** |
| Cycle 1 Day 1 | Predose End of infusion 1, 2, 6 h after infusion | Predose End of infusion 1, 2, 6 h after infusion |
| Cycle 1 Day 2 | 24 h after infusion | - |
| Cycle 1 Day 3 | 48 h after infusion | - |
| Cycle 1 Day 8 | 168 h after infusion | - |
| Cycle 1 Day 15 | 336 h after infusion | 336 h after infusion |
| Cycle 2 Day 1 | Predose Cycle 2 | Predose Cycle 2 |
| Cycle 3 Day 1 | Predose Cycle 3 End of infusion Cycle 3 1, 2, 6 h after infusion Cycle 3 | Predose Cycle 3 End of infusion Cycle 3 1, 2, 6 h after infusion Cycle 3 |
| Cycle 3 Day 2 | 24 h after infusion | - |
| Cycle 3 Day 3 | 48 h after infusion | - |
| Cycle 3 Day 8 | 168 h after infusion | - |
| Cycle 4 Day 1 | Predose Cycle 4 | Predose Cycle 4 |
| Cycle 5 Day 1 | Predose Cycle 5 End of infusion Cycle 5 | Predose Cycle 5 End of infusion Cycle 5 |
| Treatment Discontinuation | 672 h after the final infusion | - |

| | | |
|---|---|---|
| ^{a} All STM 434 sampling times are relative to STM 434 dosing. For Q3W dosing, postdose PK sampling windows are as follows: **Cycle 1** Day 1: ± 30 minutes Days 2, 3, 8: ± 4 hours Day 15: ± 1 day **Cycle 2** Day 1: ± 4 hours **Cycle 3** Day 1: ± 30 minutes Days 2, 3, 8: ± 4 hours **Cycle 4** Day 1: ± 4 hours **Cycle 5** Day 1: ± 30 minutes | | |

### Example 7: STM 434 Stability.

The stability of formulated STM 434 stability was determined using various methods at various time points under various conditions. STM 434 was formulated as shown above (STM 434 Injection was formulated as 70 mg/mL STM 434, 10 mM potassium phosphate, 8.8% (w/v) sucrose, 0.006% (w/v) polysorbate 20, pH 6.7).

### Methods

The analytic methods used for assessing stability were as follows:

### Concentration by Ultraviolet (UV) Absorbance

The concentration of STM 434 in the drug product was measured by using a UV spectrophotometer. The absorbance of 280 nm light (A280) and the extinction coefficient at 280nm (ε280) of 1.7 mg-mL⁻¹·cm⁻¹ were used to determine the concentration based on Beer's law.

A protein at a given time point and temperature is considered to have equivalent stability to the protein at time zero (T0) or an identical protein, under otherwise identical conditions, at -70°C when the concentration is 70.0 ± 7.0 mg/mL. The percent stability of a given protein at a given time point can also be calculated relative to this number.

### Visual Appearance

This method provides for the visual assessment of STM 434 Injection. A vial was observed under ambient light, and the general appearance reported with respect to visible particles, degree of coloration, and clarity.

A protein at a given time point and temperature is considered to have equivalent stability to the protein at time zero (T0) or an identical protein, under otherwise identical conditions, at -70°C when the appearance is clear, colorless to slightly yellow liquid, and essentially free of particles.

### Osmolality

The osmolality of STM 434 Injection was measured using a freezing-point depression osmometer, which was calibrated against an osmolality standard of 290 mOsm/kg.

A protein at a given time point and temperature is considered to have equivalent stability to the protein at time zero (T0) or an identical protein, under otherwise identical conditions, at -70°C when the osmolality is 330.0 ± 50.0 mOsm/kg. The percentage stability of a given protein at a given time point can also be calculated relative to this number.

### pH

The pH of STM 434 Injection was measured using a pH meter calibrated with National Institute of Science and Technology (NIST)-traceable standards.

A protein at a given time point and temperature is considered to have equivalent stability to the protein at time zero (T0) or an identical protein, under otherwise identical conditions, at -70°C when the pH is 6.7 ± 0.3 at 25°C. The percent stability of a given protein at a given time point can also be calculated relative to this number.

### Volume in Container

The volume of STM 434 Injection solution in vials was measured.

A protein at a given time point and temperature is considered to have equivalent stability to the protein at time zero (T0) or an identical protein, under otherwise identical conditions, at -70°C when the volume in the container is not less than 1.0 mL. The percent stability of a given protein at a given time point can also be calculated relative to this number.

### Size Exclusion High Performance Liquid Chromatography (SE-HPLC)

SE-HPLC is a method of separating proteins by their hydrodynamic size, which is approximately correlated with molecular weight (MW). SE-HPLC allows for analysis of the molecular size distribution of STM 434 in the drug product, especially with respect to aggregation, fragmentation, and other impurities. Resolution of monomeric STM 434 from other species by SE-HPLC was performed on a TSKgel G3000SWx1 HPLC column, using an isocratic mobile phase of 100 mM sodium phosphate, 250 mM sodium chloride, pH 6.8, at a flow rate of 0.5 mL/min. Protein species were detected by A280 nm over a 35-minute assay run time. The elution time of proteins was inversely proportional to the log of their MW. Results from the assay are reported as percent A280 peak area relative to the total A280 peak area.

A protein at a given time point and temperature is considered to have equivalent stability to the protein at time zero (T0) or an identical protein, under otherwise identical conditions, at -70°C when the SE-HPLC main peak is ≥ 95.0%. The percent stability of a given protein at a given time point can also be calculated relative to this number.

### Imaged Capillary Isoelectric Focusing (icIEF)

cIEF is a high-resolution protein separation technique based on differences in isoelectric point (pI) among proteins. The Protein Simple (formerly Convergent Bioscience) iCE280 IEF analyzer system uses a whole capillary zone imaging detector that enables analysis without the mobilization step required for conventional cIEF instruments. With respect to STM 434 Injection, icIEF was used as a purity test.

In this icIEF method, STM 434 samples were treated with sialidase to reduce the substantial complexity imparted by the varying levels of sialic acid in the product. After desialylation, samples were mixed with carrier ampholytes to generate a pH gradient, methyl cellulose to reduce electroosmotic flow, and markers of known pI which absorb 280 nm light. The mixture was introduced into an internally coated fused silica capillary with a UV-transparent segment between inlet and outlet reservoirs assembled in a cartridge. When voltage was applied to the capillary segment, the solution forms a pH gradient, in which the ampholytes, pI markers, and protein species in the sample were focused at their respective pI. A whole capillary absorption image was taken with a charge-coupled device camera, allowing for monitoring of the focused zones in the capillary at 280 nm, corresponding to the various protein species and the pI markers. Inclusion of the pI markers allows for calibration of the pI gradient in the capillary, providing for reproducible determination of pI values of the various species in a sample. Comparable to HPLC, the icIEF results are provided as A280 peaks as a function of migration distance in the capillary. Results from the assay are reported as percent A280 peak area relative to the total A280 peak area for the protein species.

A protein at a given time point and temperature is considered to have equivalent stability to the protein at time zero (T0) or an identical protein, under otherwise identical conditions, at -70°C when the icIEF main peak is 65.0 ± 10.0%. The percent stability of a given protein at a given time point can also be calculated relative to this number.

### Sodium Dodecyl Sulfate Capillary Electrophoresis (CE-SDS), Non-reduced (nrCE-SDS)

CE-SDS, non-reduced was used as a purity test for STM 434 Injection. The methodology involves heat denaturation of proteins in the presence of the detergent SDS, which binds to polypeptides at a relatively constant ratio of SDS:polypeptide. The negatively charged SDS bound to proteins causes them to migrate in an electric field according to MW.

In this method, STM 434 samples were incubated at 70°C for 10 minutes with SDS, a free sulfhydryl blocking agent, and an internal MW standard under non-reducing conditions. After incubation, treated samples were loaded into an autosampler, which also carries solutions of rinses and a specialized SDS gel. The CE system was programmed for sequential rinses, gel loading, and electrokinetic sample injections into a bare fused silica capillary. For each sample, the injected proteins were subjected to an electric field for MW-based separation, including mobilization through a detector window, where the capillary coating had been removed and light passed through the capillary to a photodiode array detector, which in this method was set to collect 220 nm absorbance (A220). Results from the assay are reported as percent A220 peak area relative to the total A280 peak area for the protein species.

A protein at a given time point and temperature is considered to have equivalent stability to the protein at time zero (T0) or an identical protein, under otherwise identical conditions, at -70°C when the nrCE-SDS main peak is ≥ 88.0%. The percent stability of a given protein at a given time point can also be calculated relative to this number.

### CE-SDS, Reduced (rCE-SDS)

CE-SDS, reduced was used as a purity test for STM 434 Injection. The methodology involves heat denaturation of a specified concentration of protein in the presence of the detergent SDS, and a reducing agent which cleaves disulfide bonds into free sulfhydryls, which in the case of STM 434 causes the separation of the two polypeptide chains of the homodimer. The negatively charged SDS, which binds to polypeptide chains at a relatively constant ratio of SDS:polypeptide, causes the polypeptides to migrate in an electric field according to MW.

In this method, STM 434 samples were incubated at 70°C for 10 minutes with SDS, the reducing agent, beta-mercaptoethanol, and an internal MW standard under non-reducing conditions. After incubation, treated samples were loaded into an autosampler, which also carried solutions of rinses and a specialized SDS gel. The CE system was programmed for sequential rinses, gel loading, and electrokinetic sample injections into a bare fused silica capillary. For each sample, the injected proteins were subjected to an electric field for MW-based separation, including mobilization through a detector window, where the capillary coating had been removed and light passed through the capillary to a photodiode array detector, which in this method was set to collect 220 nm absorbance (A220). Results from the assay are reported as percent A220 peak area relative to the total A280 peak area for the protein species.

A protein at a given time point and temperature is considered to have equivalent stability to the protein at time zero (T0) or an identical protein, under otherwise identical conditions, at -70°C when the rCE-SDS main peak is ≥90.0%. The percent stability of a given protein at a given time point can also be calculated relative to this number.

### Product-specific ELISA

The methodology utilized in the STM 434 Injection identity test was a solid-phase sandwich ELISA. Microtiter strips were coated with an anti-STM 217 monoclonal antibody, which has high affinity for STM 434 as these proteins differ by a single amino acid (serine-20 in STM 217, threonine-20 in STM 434). In sequence, samples or controls were added to the wells, followed by a biotinylated monoclonal antibody generated against human ActR2B, followed by Neutravidin-horseradish peroxidase (HRP) conjugate. STM 434 present in samples binds to the immobilized capture antibody and the biotinylated secondary antibody, and Neutravidin-HRP binds this complex through the high affinity of avidin for biotin. The HRP moiety of the complex catalyzes the conversion of the chromogenic substrate tetramethyl benzidine (TMB) to a product which absorbs 450 nm light. Identity was confirmed in samples whose signal-to-noise ratio, equal to the sample's background-corrected A450 divided by the background-corrected A450 of blank wells, was no less than 10.0.

A protein at a given time point and temperature is considered to have equivalent stability to the protein at time zero (T0) or an identical protein, under otherwise identical conditions, at -70°C when the ELISA confirms the identity of the protein, e.g., relative to a positive control. The percent stability of a given protein at a given time point can also be calculated relative to the 450 nm number determined by ELISA.

### Cell-Based Bioassay (Bioassay)

The potency of STM 434 Injection was measured in a reporter gene expression assay using the C2C12 pMARE clone #44 skeletal muscle cell line. Murine C2C12 cells were stably transfected with a human myostatin/activin-responsive luciferase construct. When the engineered cell line was incubated with the ligand myostatin, signal transduction occured following myostatin binding to the activin receptors. This resulted in the activation of the luciferase reporter gene and the resulting production of luciferase. The reaction of luciferase with luciferin resulted in luminescence that was measured in a luminometer. STM 434 inhibits this signaling in a dose-dependent manner. The data was analyzed using a 4-parameter curve fit model. Once parallelism/similarity to the reference curve was established, biological concentrations were interpolated from the curve and a relative potency of the sample against the reference standard was calculated.

A protein at a given time point and temperature is considered to have equivalent stability or activity to the protein at time zero (T0) or an identical protein, under otherwise identical conditions, at -70°C when the bioassay shows a relative potency of 60 to 140%. The percent stability or activity of a given protein at a given time point can also be calculated relative to this number.

### Endotoxin

The method for quantifying bacterial endotoxin in STM 434 Injection utilized a kinetic chromogenic endotoxin detection system. Dilution series of endotoxin standard, STM 434 samples, and STM 434 samples spiked with endotoxin standard for system suitability were prepared, and loaded into a 96-well plate, along with water as a blank for system suitability. After incubating the plate at 37°C for 10 minutes, Limulus amebocyte lysate (LAL) reagent containing a peptide labeled with p-nitroaniline (pNA) was added to the wells. Endotoxin present in the samples and standards converted a proenzyme in LAL to an active enzyme which cleaved pNA from the colorless peptide to generate a signal at 405 nm absorbance (A405). The A405 from the endotoxin standards were plotted to generate a standard curve, and the endotoxin content of STM 434 samples was determined from their A405 readings against the standard curve. The results are reported in terms of endotoxin units per milligram (EU/mg) after determining the EU/mL of each sample and dividing by the sample's protein concentration in mg/mL.

A protein at a given time point and temperature is considered to have equivalent stability to the protein at time zero (T0) or an identical protein, under otherwise identical conditions, at -70°C when the endotoxin level is ≤ 0.5 endotoxin units (EU)/mg. The percent stability of a given protein at a given time point can also be calculated relative to this number.

### Sterility

The method for determining the sterility of STM 434 Injection utilized a membrane filtration technique to retain any microorganisms in the test articles.

A protein at a given time point and temperature is considered to have equivalent stability to the protein at time zero (T0) or an identical protein, under otherwise identical conditions, at -70°C when the sterility of the product meets the requirements of the United States Pharmacopeia. The percent stability of a given protein at a given time point can also be calculated relative to the numbers provided by the United States Pharmacopeia.

### Subvisible Particles

The amount of subvisible particles present in STM 434 Injection was determined using light obscuration. The results are reported as the number of particles with diameters 10 µm or larger, and with diameters 25 µm or larger.

A protein at a given time point and temperature is considered to have equivalent stability to the protein at time zero (T0) or an identical protein, under otherwise identical conditions, at -70°C when there are not more than 6,000 ≥10µm particles per container and there are not more than 600 ≥25µm particles per container. The percent stability of a given protein at a given time point can also be calculated relative to these numbers.

### Polysorbate 20 Concentration

The concentration of polysorbate 20 (PS20) in STM 434 Injection was determined by a method that utilizes a mixed-mode HPLC column run in reversed phase mode to separate PS20 from protein, with quantitative detection by charged aerosol detection (CAD). An injection sequence of PS20 standards provides a calibration curve based on the baseline-corrected CAD responses as a function of PS20 concentration, which fits a second-order polynomial. The reported PS20 concentration of an STM 434 sample was determined by entering its baseline-corrected CAD responses into the polynomial equation and solving for PS20 concentration.

A protein at a given time point and temperature is considered to have equivalent stability to the protein at time zero (T0) or an identical protein, under otherwise identical conditions, at -70°C when the polysorbate 20 concentration is 0.006 ± 0.003% (w/v). The percent stability of a given protein at a given time point can also be calculated relative to this number.

### Results

The tables below (Tables 18-39) show formulated STM 434 stability-related data generated using the above methods, at least in part, at the indicated time points and indicated conditions.

Table 18 shows that the tested characteristics of formulated 434 kept at 5°C for up to 54 months are similar to formulated 434 kept at -70°C for up to 54 months. Table 22 shows that the tested characteristics of formulated 434 kept at 5°C for up to 6 months are similar to those of formulated 434 kept at 5°C at 0 months. Tables 25 and 30 show that the tested characteristics of formulated 434 kept at 2-8°C for up to 12 months are similar to those of formulated 434 kept at 2-8°C at 0 months. Table 36 shows that the tested characteristics of formulated 434 kept in an upright position at 2-8°C for up to 6 months are similar to those of formulated 434 kept in an upright position at 2-8°C at 0 months. Table 37 shows that the tested characteristics of formulated 434 kept in an inverted position at 2-8°C for up to 6 months are similar to those of formulated 434 kept in an inverted position at 2-8°C at 0 months.

This data demonstrates that formulated STM 434 is highly stable for up to 54 months at refrigerated temperatures (e.g., 2-8°C (2, 3, 4, 5, 6, 7, 8°C); 5°C) and even at high concentrations such as 70mg/mL. This level of stability is much greater than the stability expected of proteins kept at 4°C, which is typically in the range of 1 month. *See* Pierce Technical Resource TR0043.1, *Protein Stability and Storage,* at Table 1 (http://sites.bio.indiana.edu/~chenlab/protocol_files/protein_storage.pdf); and Webster et al., Predicting Long-Term Storage Stability of Therapeutic Proteins, Pharmaceutical Technology, Volume 37, Issue 11 (Nov. 2, 2013). Thus formulated STM 434 is a highly stable composition that can be stored at refrigerated temperatures (e.g., 2-8°C) for an extended period of time greater than 1 month, e.g., up to 3, 6, 12, 18, 24, 36, 48, or 54 months or more.

**Table 18: 434 DP/DS Stability Sample Testing Results**

| | Sample | DP, 5°C | DP, -20°C | DP, -70°C | DS, 5°C | DS, -70°C |
|---|---|---|---|---|---|---|
| | Lot# | 001003696 5 | 001003696 5 | 001003696 5 | 00100351 06 | 001003510 6 |
| Method | Time point | 54 months | 54 months | 54 months | 54 months | 54 months |
| Visual Appearance | | Colorless, Clear, No visible particles | Colorless, Clear, No visible particles | Colorless, Clear, No visible particles | Colorless, Clear, No visible particles | Colorless, Clear, No visible particles |
| pH | | 6.7 | 6.7 | 6.7 | 6.8 | 6.7 |
| Protein Cone. (A280) | | 71.6 mg/mL | 71.3 mg/mL | 71.8 mg/mL | 76.0 mg/mL | 71.5 mg/mL |
| SE-HPLC | | 98.2% Main Pk 1.5% HMW 0.3% LMW | 98.6% Main Pk 1.4% HMW 0.0% LMW | 98.8% Main Pk 1.2% HMW 0.0% LMW | 98.2% Main Pk 1.5% HMW 0.3% LMW | 98.8% Main Pk 1.2% HMW 0.0% LMW |
| icIEF | | 60.8% Main Pk 22.9% APG 16.4% BPG | 69.9% Main Pk 14.8% APG 15.3% BPG | 72.4% Main Pk 15.5% APG 12.2% BPG | 65.6% Main Pk 24.7% APG 9.8% BPG | 65.8% Main Pk 20.6% APG 13.7% BPG |
| nrCE-SDS | | 94.1% Main Pk 5.9% Pre-peak | 94.2% Main Pk 5.8% Pre-peak | 94.7% Main Pk 5.3% Pre-peak | 94.5% Main Pk 5.5% Pre-peak | 94.4% Main Pk 5.6% Pre-peak |
| rCE-SDS | | 98.0% Main Pk | 98.0% Main Pk | 97.8% Main Pk | 97.7% Main Pk | 97.8% Main Pk |
| Cell Based Bioassay | | 87% Relative Potency | 99% Relative Potency | 111% Relative Potency | 101% Relative Potency | 99% Relative Potency |
| DP is drug product; DS is drug substance | | | | | | |

**Table 19: STM 434 Injection Lot 0010036965 Stability Data Summary: -70°C**

| **Condition: -70°C, Upright Start date: 9 Sep 2009** | | **Months** | | |
|---|---|---|---|---|
| **Method** | **Parameter** | **0** | **12** | **24** |
| Appearance | Color | CL | CL to SY | CL to SY |
| | Particles | None | None | None |
| pH | pH | 6.72 | 6.70 | 6.72 |
| CE-SDS, Reduced | % main peak | 98.972 | 98.905 | 98.404 |
| | % LMW | 1.028 | 1.095 | 1.096 |
| | % HMW | 0.0 | 0.0 | 0.498 |
| SE-HPLC | % main peak | 98.795 | 98.843 | 98.815 |
| | % HMW | 1.205 | 1.156 | 1.184 |
| | % MW | 0.0 | 0.0 | 0.0 |
| cIEF | % main peak | 68.445 | 72.046 | 70.181 |
| | % acidic | 16.282 | 15.191 | 16.026 |
| | % basic | 15.273 | 12.762 | 13.791 |
| Bioassay | % relative potency | 100 | 100 | 100 |
| Subvisible Particulates | ≥ 10 µm per container | < 10 | < 10 | < 10 |
| | ≥ 25 µm per container | < 10 | < 10 | < 10 |

| | | | | |
|---|---|---|---|---|
| CL = colorless; SY = slightly yellow; HMW = high molecular weight; LMW = low molecular weight | | | | |

**Table 20: STM 434 Injection Lot 0010036965 Stability Data Summary: -20°C**

| **Condition: -20°C, Upright Start date: 9 Sep 2009** | | **Months** | | | | | | | | |
|---|---|---|---|---|---|---|---|---|---|---|
| **Method** | **Parameter** | **0** | **1** | **3** | **6** | **9** | **12** | **18** | **24** | **30** |
| Appearance | Color | CL | CL to SY | CL to SY | CL to SY | CL to SY | CL to SY | CL to SY | CL to SY | CL to SY |
| | Particles | None | None | None | None | None | None | None | None | None |
| pH | pH | 6.72 | 6.69 | 6.70 | 6.67 | 6.64 | 6.73 | 6.72 | 6.70 | 6.68 |
| CE-SDS, Reduced | %main peak | 98.972 | 98.761 | 98.889 | 98.964 | 99.094 | 98.919 | 98.968 | 99.002 | 98.903 |
| | % MW | 1.028 | 1.239 | 1.111 | 1.036 | 0.906 | 1.081 | 1.031 | 0.997 | 1.096 |
| | % HMW | 0.0 | 0.0 | 0.0 | 0.0 | 0.0 | 0.0 | 0.0 | 0.0 | 0.0 |
| SE-HPLC | % main peak | 98.795 | 98.777 | 98.787 | 98.770 | 98.744 | 98.775 | 98.734 | 98.742 | 98.607 |
| | % HMW | 1.205 | 1.223 | 1.213 | 1.230 | 1.255 | 1.224 | 1.265 | 1.254 | 1.392 |
| | % MW | 0.0 | 0.0 | 0.0 | 0.0 | 0.0 | 0.0 | 0.0 | 0.0 | 0.0 |
| cIEF | % main peak | 68.445 | 68.390 | 68.516 | 67.255 | 71.142 | 69.844 | 68.774 | 70.214 | 69.094 |
| | % acidic | 16.282 | 17.454 | 16.285 | 17.599 | 16.522 | 14.354 | 17.089 | 15.963 | 16.242 |
| | % basic | 15.273 | 14.156 | 15.199 | 15.146 | 12.337 | 15.800 | 14.135 | 13.822 | 14.663 |
| Bioassay | % relative potency | 100 | 100 | 102 | 95 | 103 | 102 | 102 | 99 | 102 |
| Subvisible Particulates | ≥ 10 µm per container | < 10 | < 10 | < 10 | < 10 | < 10 | < 10 | < 10 | < 10 | < 10 |
| | ≥ 25 µm per container | < 10 | < 10 | < 10 | < 10 | < 10 | < 10 | < 10 | < 10 | < 10 |
| Sterility | Sterility | Pass | N.S. | N.S. | N.S. | N.S. | Pass | N.S. | Pass | N.S. |

| | | | | | | | | | | |
|---|---|---|---|---|---|---|---|---|---|---|
| CL = colorless; SY = slightly yellow; HMW = high molecular weight; LMW = low molecular weight; N.S. = not scheduled | | | | | | | | | | |

**Table 21: STM 434 Injection Lot 0010036965 Stability Data Summary: 5× Freeze/Thaw**

| **Condition: 5× F/T Start date: 9 Sep 2009** | | **Condition** | |
|---|---|---|---|
| **Method** | **Parameter** | **T = 0** | **5**×**F/T** |
| Appeara nce | Color | CL | CL to SY |
| | Particles | None | None |
| pH | pH | 6.72 | 6.68 |
| CE-SDS, Reduced | % main peak | 98.972 | 98.544 |
| | % MW | 1.028 | 1.455 |
| | % HMW | 0.0 | 0.0 |
| SE-HPLC | % main peak | 98.795 | 98.787 |
| | % HMW | 1.205 | 1.213 |
| | % MW | 0.0 | 0.0 |
| cIEF | % main peak | 68.445 | 68.255 |
| | % acidic | 16.282 | 18.293 |
| | % basic | 15.273 | 13.452 |
| Bioassay | % relative potency | 100 | 100 |
| Subvisib le Particula tes | ≥ 10 µm per container | < 10 | < 10 |
| | ≥ 25 µm per container | < 10 | < 10 |

| | | | |
|---|---|---|---|
| CL = colorless; SY = slightly yellow; HMW = high molecular weight; LMW = low molecular weight | | | |

**Table 22: STM 434 Injection Lot 0010036965 Stability Data Summary: 5°C**

| **Condition: 5°C, Upright Start date: 9 Sep 2009** | | **Months** | | | | |
|---|---|---|---|---|---|---|
| **Method** | **Parameter** | **0** | **0.5** | **1** | **3** | **6** |
| Appearance | Color | CL | CL to SY | CL to SY | CL to SY | CL to SY |
| | Particles | None | None | None | None | None |
| pH | pH | 6.72 | 6.70 | 6.69 | 6.71 | 6.67 |
| CE-SDS, Reduced | % main peak | 98.972 | 98.856 | 98.313 | 98.945 | 99.048 |
| | % MW | 1.028 | 1.144 | 1.687 | 1.055 | 0.952 |
| | % HMW | 0.0 | 0.0 | 0.0 | 0.0 | 0.0 |
| SE-HPLC | % main peak | 98.795 | 98.831 | 98.793 | 98.824 | 98.792 |
| | % HMW | 1.205 | 1.169 | 1.207 | 1.176 | 1.208 |
| | % MW | 0.0 | 0.0 | 0.0 | 0.0 | 0.0 |
| cIEF | % main peak | 68.445 | 67.703 | 67.012 | 67.239 | 69.441 |
| | % acidic | 16.282 | 16.196 | 18.094 | 17.907 | 15.290 |
| | % basic | 15.273 | 16.101 | 14.894 | 14.855 | 15.269 |
| Bioassay | % relative potency | 100 | 99 | 100 | 104 | 94 |
| Subvisible Particulates | ≥ 10 µm per container | < 10 | 11 | < 10 | < 10 | < 10 |
| | ≥ 25 µm per container | < 10 | < 10 | < 10 | < 10 | < 10 |

| | | | | | | |
|---|---|---|---|---|---|---|
| CL = colorless; SY = slightly yellow; HMW = high molecular weight; LMW = low molecular weight; N.S. = not scheduled | | | | | | |

**Table 23: STM 434 Injection Lot 0010036965 Stability Data Summary: 25°C**

| **Condition: 25°C, Upright Start date: 9 Sep 2009** | | **Months** | |
|---|---|---|---|
| **Method** | **Parameter** | **0** | **0.25** |
| Appearance | Color | CL | CL to SY |
| | Particles | None | None |
| pH | pH | 6.72 | 6.71 |
| CE-SDS, Reduced | % main peak | 98.972 | 98.851 |
| | % LMW | 1.028 | 1.150 |
| | % HMW | 0.0 | 0.0 |
| SE-HPLC | % main peak | 98.795 | 98.901 |
| | % HMW | 1.205 | 1.099 |
| | % LMW | 0.0 | 0.0 |
| cIEF | % main peak | 68.445 | 68.002 |
| | % acidic | 16.282 | 16.670 |
| | % basic | 15.273 | 15.328 |
| Bioassay | % relative potency | 100 | 97 |
| Subvisible Particulates | ≥ 10 µm per container | < 10 | < 10 |
| | ≥ 25 µm per container | < 10 | < 10 |

| | | | |
|---|---|---|---|
| CL = colorless; SY = slightly yellow; HMW = high molecular weight; LMW = low molecular weight; N.S. = not scheduled | | | |

**Table 24: STM 434 Injection Lot EG-13-0150 Stability Data Summary: -20°C (SPN-643)**

| **Condition: -20°C, Upright Start date: 17 Dec 2013** | | | **Months** | | | | | |
|---|---|---|---|---|---|---|---|---|
| **Method** | **Parameter** | **Acceptance Criteria** | **0** | **1** | **3** | **6** | **9** | **12** |
| Appearance | Clarity | CLR L | CLR L | CLR L | CLR L | CLR L | CLR L | CLR L |
| | Color | CL to LY | LY | CL | CL | CL | CL | CL |
| | Particles | EFOP | EFOP | NVP | NVP | NVP | NVP | NVP |
| pH | pH | 6.4-7.0 | 6.7 | 6.7 | 6.6 | 6.7 | 6.6 | 6.6 |
| Concentration by UV | mg/mL | 63-77 | 71.8 | 68.9 | 69.6 | 69.5 | 68.9 | 69.2 |
| CE-SDS, Reduced | % main peak | ≥ 90.0% | 98.7 | 98.8 | 99.2 | 99.4 99.3 99.2^{*} | 99.1 | 99.2 |
| CE-SDS, Non-Reduced | % main peak | ≥ 88.0% | 93.8 | 93.7 | 92.7 | 94.0 | 93.8 | 94.0 |
| | % pre-peak | -- | 6.2 | 6.3 | 6.7 | 6.0 | 6.2 | 6.0 |
| | % other | -- | N.D. | N.D. | 0.6 | N.D. | N.D. | 0.0 |
| SE-HPLC | % main peak | ≥ 95.0% | 98.7 | 98.7 | 98.7 | 98.7 | 98.6 | 98.6 |
| | % HMW | -- | 1.3 | 1.3 | 1.3 | 1.3 | 1.4 | 1.4 |
| | % MW | -- | **≤ 0.5** | **≤ 0.5** | **≤ 0.5** | **≤ 0.5** | ≤ 0.5 | 0.0 |
| icIEF | % main peak | 65.0±10.0 | 64.8 | 67.1 | 61.6 | 65.1 | 64.7 | 66.8 |
| | % APG | -- | 21.5 | 22.0 | 18.5 | 21.6 | 19.0 | 17.9 |
| | % BPG | -- | 13.9 | 10.9 | 20.0 | 13.3 | 16.3 | 15.4 |
| Bioassay | % relative potency | 60-140 | 99 | 93.2 | 94.4 | 110 | 99.3 | 105.0 |
| Subvisible Particulates | ≥ 10 µm | ≤ 6000 per container | 2 | N.S. | N.S. | N.S. | N.S. | 10 |
| | ≥ 25 µm | ≤ 600 per container | 0 | N.S. | N.S. | N.S. | N.S. | 0 |
| Container Closure Integrity | Dye ingress | No detectable dye ingress | N.S. | N.S. | N.S. | N.S. | N.S. | NDDI |

| | | | | | | | | |
|---|---|---|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled; NDDI = no detectable dye ingress *Three reported values due to deviation (analyst executed three injections; method calls for one) | | | | | | | | |

**Table 25: STM 434 Injection Lot EG-13-0150 Stability Data Summary: 2-8°C (SPN-644)**

| **Condition: 2-8°C, Upright Start date: 17 Dec 2013** | | | **Months** | | | | | |
|---|---|---|---|---|---|---|---|---|
| **Method** | **Parameter** | **Acceptance Criteria** | **0** | **1** | **3** | **6** | **9** | **12** |
| Appearance | Clarity | CLR L | CLR L | CLR L | CLR L | CLR L | CLR L | CLR L |
| | Color | CL to LY | LY | CL | CL | CL | CL | CL |
| | Particles | EFOP | EFOP | NVP | NVP | NVP | NVP | NVP |
| pH | pH | 6.4-7.0 | 6.7 | 6.7 | 6.6 | 6.7 | 6.6 | 6.6 |
| Concentration by UV | mg/mL | 63-77 | 71.8 | 68.9 | 68.7 | 69.3 | 68.5 | 69.9 |
| CE-SDS, Reduced | % main peak | ≥ 90.0% | 98.7 | 98.8 | 99.1 | 99.1 | 99.0 | 99.1 |
| | | | | | | 99.1 | | |
| | | | | | | 98.9^{*} | | |
| CE-SDS, Non-Reduced | % main peak | ≥ 88.0% | 93.8 | 93.7 | 93.0 | 94.0 | 94.3 | 93.6 |
| | % pre-peak | -- | 6.2 | 6.3 | 6.8 | 6.0 | 5.7 | 6.4 |
| | % other | -- | N.D. | N.D. | 0.2 | N.D. | N.D. | 0.0 |
| SE-HPLC | % main peak | ≥ 95.0% | 98.7 | 98.7 | 98.7 | 98.6 | 98.6 | 98.5 |
| | % HMW | -- | 1.3 | 1.3 | 1.3 | 1.4 | 1.4 | 1.4 |
| | % MW | -- | **≤ 0.5** | **≤ 0.5** | **≤ 0.5** | **≤ 0.5** | ≤ 0.5 | 0.1 |
| icIEF | % main peak | 65.0±10.0 | 64.8 | 67.1 | 57.7 | 64.4 | 65.2 | 66.9 |
| | % APG | -- | 21.5 | 22.0 | 18.5 | 23.0 | 19.6 | 19.3 |
| | % BPG | -- | 13.9 | 10.9 | 23.8 | 12.7 | 15.2 | 13.9 |
| Bioassay | % relative potency | 60-140 | 99 | 93.2 | 99.7 | 114 | 102.6 | 106.5 |
| Subvisible Particulates | ≥ 10 µm | ≤ 6000 per container | 2 | N.S. | N.S. | N.S. | N.S. | 236 |
| | ≥ 25 µm | ≤ 600 per container | 0 | N.S. | N.S. | N.S. | N.S. | 5 |
| Container Closure Integrity | Dye ingress | No detectable dye ingress | N.S. | N.S. | N.S. | N.S. | N.S. | NDDI |

| | | | | | | | | |
|---|---|---|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled; NDDI = no detectable dye ingress *Three reported values due to deviation (analyst executed three injections; method calls for one) | | | | | | | | |

**Table 26: STM 434 Injection Lot EG-13-0150 Stability Data Summary: 25°C**

| **Condition: 25°C, Upright Start date: 17 Dec 2013** | | | **Months** | |
|---|---|---|---|---|
| **Method** | **Parameter** | **Acceptance Criteria** | **0** | **1** |
| Appearance | Clarity | CLR L | CLR L | CLRL |
| | Color | CL to LY | LY | CL |
| | Particles | EFOP | EFOP | NVP |
| pH | pH | 6.4-7.0 | 6.7 | 6.6 |
| Concentration by UV | mg/mL | 63-77 | 71.8 | 69.4 |
| CE-SDS, Reduced | % main peak | ≥ 90.0% | 98.7 | 98.7 |
| CE-SDS, Non-Reduced | % main peak | ≥ 88.0% | 93.8 | 94.0 |
| | % pre-peak | -- | 6.2 | 6.0 |
| SE-HPLC | % main peak | ≥ 95.0% | 98.7 | 98.5 |
| | % HMW | -- | 1.3 | 1.3 |
| | % LMW | -- | **≤ 0.5** | ≤ 0.5 |
| icIEF | % main peak | 65.0±10.0 | 64.8 | 66.7 |
| | % APG | -- | 21.5 | 22.3 |
| | % BPG | -- | 13.9 | 11.1 |
| Bioassay | % relative potency | 60-140 | 99 | 86.9 |

| | | | | |
|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected | | | | |

**Table 27: STM 434 Injection Lot EG-13-0150 Stability Data Summary: 5× Freeze/Thaw**

| **Condition: 5× Freeze/Thaw** | | **Acceptance Criteria** | **T = 0** | **5×F/T** |
|---|---|---|---|---|
| **Start date: 13 Feb 2014** | | | | |
| **Method** | **Parameter** | | | |
| Appearance | Clarity | CLR L | CLR L | CLR L |
| | Color | CL to LY | LY | CL |
| | Particles | EFOP | EFOP | NVP |
| pH | pH | 6.4-7.0 | 6.7 | 6.7 |
| Concentration by UV | mg/mL | 63-77 | 71.8 | 70.5 |
| CE-SDS, Reduced | % main peak | ≥ 90.0% | 98.7 | 98.9 |
| CE-SDS, Non-Reduced | % main peak | ≥ 88.0% | 93.8 | 94.4 |
| | % pre-peak | -- | 6.2 | 5.6 |
| SE-HPLC | % main peak | ≥ 95.0% | 98.7 | 98.8 |
| | % HMW | -- | 1.3 | 1.2 |
| | % MW | -- | **≤ 0.5** | **≤ 0.5** |
| icIEF | % main peak | 65.0±10.0 | 64.8 | 69.4 |
| | % APG | -- | 21.5 | 17.0 |
| | % BPG | -- | 13.9 | 13.7 |
| Bioassay | % relative potency | 60-140 | 99 | 96 |

| | | | | |
|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected | | | | |

**Table 28: STM 434 Injection Lot FG-13-0230 Stability Data Summary: -70°C (SPN-657)**

| **Condition: -70°C, Upright** | | | **Months** | | |
|---|---|---|---|---|---|
| **Start date: 31 Jan 2014** | | | | | |
| **Method** | **Parameter** | **Acceptance Criteria** | **0** | **1** | **3** |
| Appearance | Clarity | CLR L | CLR L | CLR L | CLR L |
| | Color | CL to LY | LY | CL | CL |
| | Particles | EFOP | EFOP | NVP | NVP |
| pH | pH | 6.4-7.0 | 6.7 | 6.5 | 6.5 |
| Concentration by UV | mg/mL | 63-77 | 67.7 | 67.5 | 68.4 |
| CE-SDS, Reduced | %main peak | ≥ 90.0% | 98.7 | 99.0 | 98.9 |
| CE-SDS, Non-Reduced | % main peak | ≥ 88.0% | 94.4 | 94.6 | 94.4 |
| | % pre-peak | -- | 5.6 | 5.4 | 5.6 |
| SE-HPLC | % main peak | ≥ 95.0% | 98.9 | 98.9 | 98.8 |
| | % HMW | -- | 1.1 | 1.1 | 1.2 |
| | % LMW | -- | N.D. | **≤ 0.5** | **≤ 0.5** |
| icIEF | % main peak | 65.0±10.0 | 68.7 | 70.4 | 71.3 |
| | % APG | -- | 17.5 | 17.8 | 17.9 |
| | % BPG | -- | 13.9 | 11.9 | 11.0 |
| Bioassay | % relative potency | 60-140 | 99 | 115 | 107 |
| Subvisible Particulates | ≥ 10 µm | ≤ 6000 per container | 2 | N.S. | N.S. |
| | ≥ 25 µm | ≤ 600 per container | 0 | N.S. | N.S. |
| Container Closure Integrity | Dye ingress | No detectable dye ingress | N.S. | N.S. | N.S. |

| | | | | | |
|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | | |

**Table 29: STM 434 Injection Lot FG-13-0230 Stability Data Summary: -20°C (SPN-648)**

| **Condition: -20°C, Upright** | | | **Months** | | | | | |
|---|---|---|---|---|---|---|---|---|
| **Start date: 31 Jan 2014** | | | | | | | | |
| **Method** | **Parameter** | **Acceptance Criteria** | **0** | **1** | **3** | **6** | **9** | **12** |
| Appearance | Clarity | CLR L | CLR L | CLR L | CLR L | CLR L | CLR L | CLR L |
| | Color | CL to LY | LY | CL | CL | CL | CL | CL |
| | Particles | EFOP | EFOP | NVP | NVP | NVP | NVP | NVP |
| pH | pH | 6.4-7.0 | 6.7 | 6.5 | 6.5 | 6.5 | 6.5 | 6.4 |
| Concentration by UV | mg/mL | 63-77 | 67.7 | 66.8 | 68.4 | 67.5 | 69.2 | 70.9 |
| CE-SDS, Reduced | %main peak | ≥ 90.0% | 98.7 | 99.1 | 99.1 | 99.2 | 99.2 | 99.5 |
| CE-SDS, Non-Reduced | %main peak | ≥ 88.0% | 94.4 | 93.9 | 94.0 | 93.2 | 93.8 | 94.1 |
| | % pre-peak | -- | 5.6 | 6.1 | 6.0 | 6.8 | 6.2 | 5.9 |
| SE-HPLC | % main peak | ≥ 95.0% | 98.9 | 98.9 | 98.8 | 98.6 | 98.8 | 98.8 |
| | % HMW | -- | 1.1 | 1.1 | 1.2 | 1.2 | 1.2 | 1.2 |
| | % MW | -- | N.D. | **≤ 0.5** | **≤ 0.5** | ≤ 0.5 | ≤ 0.5 | **0.0** |
| icIEF | % main peak | 65.0±10.0 | 68.7 | 67.7 | 69.6 | 66.0 | 64.7 | 70.2 |
| | % APG | -- | 17.5 | 19.0 | 19.2 | 19.3 | 16.6 | 17.7 |
| | % BPG | -- | 13.9 | 13.4 | 11.3 | 14.8 | 18.8 | 12.1 |
| Bioassay | % relative potency | 60-140 | 99 | 100 | 115 | 96 | 94 | 106 |
| Subvisible Particulates | ≥ 10 µm | ≤ 6000 per container | 2 | N.S. | N.S. | N.S. | N.S. | N.S. |
| | ≥ 25 µm | ≤ 600 per container | 0 | N.S. | N.S. | N.S. | N.S. | N.S. |
| Container Closure Integrity | Dye ingress | No detectable dye ingress | N.S. | N.S. | N.S. | N.S. | N.S. | N.S. |

| | | | | | | | | |
|---|---|---|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | | | | | |

**Table 30: STM 434 Injection Lot FG-13-0230 Stability Data Summary: 2-8°C (SPN-649)**

| **Condition: 2-8°C, Upright** | | | **Months** | | | | | |
|---|---|---|---|---|---|---|---|---|
| **Start date: 31 Jan 2014** | | | | | | | | |
| **Method** | **Parameter** | **Acceptance Criteria** | **0** | **1** | **3** | **6** | **9** | **12** |
| Appearance | Clarity | CLR L | CLR L | CLR L | CLR L | CLR L | CLR L | CLR L |
| | Color | CL to LY | LY | CL | CL | CL | CL | CL |
| | Particles | EFOP | EFOP | NVP | NVP | NVP | NVP | NVP |
| pH | pH | 6.4-7.0 | 6.7 | 6.5 | 6.5 | 6.5 | 6.4 | 6.5 |
| Concentration by UV | mg/mL | 63-77 | 67.7 | 68.1 | 68.3 | 67.5 | 68.1 | 72.5 |
| CE-SDS, Reduced | %main peak | ≥ 90.0% | 98.7 | 99.1 | 99.1 | 99.2 | 99.2 | 99.5 |
| CE-SDS, Non-Reduced | %main peak | ≥ 88.0% | 94.4 | 94.4 | 94.3 | 93.8 | 95.1 | 93.6 |
| | % pre-peak | -- | 5.6 | 5.6 | 5.7 | 6.2 | 4.9 | 6.4 |
| SE-HPLC | % main peak | ≥ 95.0% | 98.9 | 98.9 | 98.8 | 98.8 | 98.8 | 98.8 |
| | % HMW | -- | 1.1 | 1.1 | 1.1 | 1.2 | 1.2 | 1.2 |
| | % MW | -- | N.D. | **≤ 0.5** | **≤ 0.5** | ≤ 0.5 | ≤ 0.5 | **0.0** |
| icIEF | % main peak | 65.0±10.0 | 68.7 | 68.5 | 68.8 | 65.3 | 64.2 | 67.5 |
| | % APG | -- | 17.5 | 18.9 | 19.5 | 21.2 | 17.8 | 18.0 |
| | % BPG | -- | 13.9 | 12.6 | 11.7 | 13.6 | 18.2 | 14.5 |
| Bioassay | % relative potency | 60-140 | 99 | 116 | 108 | 93 | 116 | 121 |
| Subvisible Particulates | ≥ 10 µm | ≤ 6000 per container | 2 | N.S. | N.S. | N.S. | N.S. | N.S. |
| | ≥ 25 µm | ≤ 600 per container | 0 | N.S. | N.S. | N.S. | N.S. | N.S. |
| Container Closure Integrity | Dye ingress | No detectable dye ingress | N.S. | N.S. | N.S. | N.S. | N.S. | N.S. |

| | | | | | | | | |
|---|---|---|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | | | | | |

**Table 31: STM 434 Injection Lot FG-13-0230 Stability Data Summary: 25°C (SPN-650)**

| **Condition: 25°C, Upright** | | | **Months** | |
|---|---|---|---|---|
| **Start date: 31 Jan 2014** | | | | |
| **Method** | **Parameter** | **Acceptance Criteria** | **0** | **1** |
| Appearance | Clarity | CLR L | CLR L | CLR L |
| | Color | CL to LY | LY | CL |
| | Particles | EFOP | EFOP | NVP |
| pH | pH | 6.4-7.0 | 6.7 | 6.5 |
| Concentration by UV | mg/mL | 63-77 | 67.7 | 67.3 |
| CE-SDS, Reduced | % main peak | ≥ 90.0% | 98.7 | 99.0 |
| CE-SDS, Non-Reduced | % main peak | ≥ 88.0% | 94.4 | 94.4 |
| | % pre-peak | -- | 5.6 | 5.6 |
| SE-HPLC | % main peak | ≥ 95.0% | 98.9 | 98.9 |
| | % HMW | -- | 1.1 | 1.1 |
| | % MW | -- | N.D. | **≤ 0.5** |
| icIEF | % main peak | 65.0±10.0 | 68.7 | 68.6 |
| | % APG | -- | 17.5 | 19.8 |
| | % BPG | -- | 13.9 | 11.7 |
| Bioassay | % relative potency | 60-140 | 99 | 116 |
| Subvisible Particulates | ≥ 10 µm | ≤ 6000 per container | 2 | 10 |
| | ≥ 25 µm | ≤ 600 per container | 0 | 3 |
| Container Closure Integrity | Dye ingress | No detectable dye ingress | N.S. | No detectable dye ingress |

| | | | | |
|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | |

**Table 32: STM 434 Injection Lot FG-14-0056 Stability Data Summary: -20°C (SPN-654)**

| **Condition: 2-8°C, Upright** | | | **Months** | |
|---|---|---|---|---|
| **Start date: 20 Feb 2014** | | | | |
| **Method** | **Parameter** | **Acceptance Criteria** | **0** | **1** |
| Appearance | Clarity | CLR L | CLR L | CLR L |
| | Color | CL to LY | LY | CL |
| | Particles | EFOP | EFOP | NVP |
| pH | pH | 6.4-7.0 | 6.7 | 6.5 |
| Concentration by UV | mg/mL | 63-77 | 67.7 | 68.2 |
| CE-SDS, Reduced | % main peak | ≥ 90.0% | 98.7 | 98.9 |
| CE-SDS, Non-Reduced | %main peak | ≥ 88.0% | 94.4 | 93.8 |
| | % pre-peak | -- | 5.6 | 5.2 |
| SE-HPLC | % main peak | ≥ 95.0% | 98.9 | 98.7 |
| | % HMW | - | 1.1 | 1.3 |
| | % LMW | - | N.D. | ≤ 0.5 |
| icIEF | % main peak | 65.0±10.0 | 68.7 | 70.9 |
| | % APG | - | 17.5 | 17.6 |
| | % BPG | -- | 13.9 | 11.6 |
| Bioassay | % relative potency | 60-140 | 99 | 117 |
| Subvisible Particulates | ≥ 10 µm | ≤ 6000 per container | 2 | N.S. |
| | ≥ 25 µm | ≤ 600 per container | 0 | N.S. |
| Container Closure Integrity | Dye ingress | No detectable dye ingress | N.S. | N.S. |

| | | | | |
|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | |

**Table 33: STM 434 Injection Lot FG-14-0056 Stability Data Summary: 2-8°C (SPN-655)**

| **Condition: 2-8°C,** Upright | | | **Months** | |
|---|---|---|---|---|
| **Start** date: **20** Feb 2014 | | | | |
| Method | Parameter | Acceptance **Criteria** | **0** | **1** |
| Appearance | Clarity | CLR L | CLR L | CLR L |
| | Color | CL to LY | LY | CL |
| | Particles | EFOP | EFOP | NVP |
| pH | pH | 6.4-7.0 | 6.7 | 6.5 |
| Concentration by UV | mg/mL | 63-77 | 67.7 | 68.4 |
| CE-SDS, Reduced | % main peak | ≥ 90.0% | 98.7 | 98.8 |
| CE-SDS, Non-Reduced | %main peak | ≥ 88.0% | 94.4 | 93.4 |
| | % pre-peak | -- | 5.6 | 6.6 |
| SE-HPLC | % main peak | ≥ 95.0% | 98.9 | 98.8 |
| | % HMW | -- | 1.1 | 1.2 |
| | % LMW | -- | N.D. | ≤ 0.5 |
| icIEF | % main peak | 65.0±10.0 | 68.7 | 67.6 |
| | % APG | -- | 17.5 | 19.2 |
| | % BPG | -- | 13.9 | 13.3 |
| Bioassay | % relative potency | 60-140 | 99 | 102 |
| Subvisible Particulates | ≥ 10 µm | ≤ 6000 per container | 2 | N.S. |
| | ≥ 25 µm | ≤ 600 per container | 0 | N.S. |
| Container Closure Integrity | Dye ingress | No detectable dye ingress | N.S. | N.S. |

| | | | | |
|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | |

**Table 34: STM 434 Injection Lot FG-14-0056 Stability Data Summary: 25°C (SPN-656)**

| **Condition: 25°C, Upright** | | | **Months** | |
|---|---|---|---|---|
| **Start date: 20 Feb 2014** | | | | |
| **Method** | **Parameter** | **Acceptance Criteria** | **0** | **1** |
| Appearance | Clarity | CLR L | CLR L | CLR L |
| | Color | CL to LY | LY | CL |
| | Particles | EFOP | EFOP | NVP |
| pH | pH | 6.4-7.0 | 6.7 | 6.5 |
| Concentration by UV | mg/mL | 63-77 | 67.7 | 68.5 |
| CE-SDS, Reduced | % main peak | ≥ 90.0% | 98.7 | 98.7 |
| CE-SDS, Non-Reduced | %main peak | ≥ 88.0% | 94.4 | 93.7 |
| | % pre-peak | -- | 5.6 | 6.3 |
| SE-HPLC | % main peak | ≥ 95.0% | 98.9 | 98.5 |
| | % HMW | -- | 1.1 | 1.3 |
| | % LMW | -- | N.D. | 0.3 |
| icIEF | % main peak | 65.0±10.0 | 68.7 | 70.8 |
| | % APG | -- | 17.5 | 17.1 |
| | % BPG | -- | 13.9 | 12.2 |
| Bioassay | % relative potency | 60-140 | 99 | 122 |
| Subvisible Particulates | ≥ 10 µm | ≤ 6000 per container | 2 | N.S. |
| | ≥ 25 µm | ≤ 600 per container | 0 | N.S. |
| Container Closure Integrity | Dye ingress | No detectable dye ingress | N.S. | N.S. |

| | | | | |
|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | |

**Table 35: STM 434 Injection Lot FG-14-0109 Stability Data Summary: -20°C Upright (SPN-676)**

| **Condition: -20°C, Upright** | | | **Months** | | | |
|---|---|---|---|---|---|---|
| **Start date: 25 Jun 2014** | | | | | | |
| **Method** | **Parameter** | **Acceptance Criteria** | **0** | **3** | **6** | |
| Appearance | Clarity | CLR L | CLR L | CLR L | CLR L | |
| | Color | CL to LY | CL | CL | CL | |
| | Particles | EFOP | FOP | NVP | NVP | |
| pH | pH | 6.4-7.0 | 6.6 | 6.5 | 6.5 | |
| Concentration by UV | mg/mL | 63-77 | 67.8 | 67.5 | 67.2 | |
| CE-SDS, Reduced | %main peak | ≥ 90.0% | 98.5 | 98.6 | 99.2 | |
| CE-SDS, Non-Reduced | %main peak | ≥ 88.0% | 94.4 | 94.2 | 93.8 | |
| | % pre-peak | -- | 5.6 | 5.8 | 6.4 | |
| SE-HPLC | % main peak | ≥ 95.0% | 98.8 | 98.9 | 98.8 | |
| | % HMW | -- | 1.2 | 1.1 | 1.2 | |
| | % LMW | -- | N.D. | ≤ 0.5 | 0.0 | |
| icIEF | % main peak | 65.0±10.0 | 65.4 | 65.7 | 69.1 | |
| | % APG | -- | 23.0 | 18.7 | 17.9 | |
| | % BPG | -- | 11.6 | 15.6 | 13.1 | |
| Bioassay | % relative potency | 60-140 | 98 | 96 | 101 | |
| Subvisible Particulates | ≥ 10 µm | ≤ 6000 per container | 2 | N.S. | N.S. | |
| | ≥ 25 µm | ≤ 600 per container | 0 | N.S. | N.S. | |
| Container Closure Integrity | Dye ingress | No detectable dye ingress | N.S. | N.S. | N.S. | |

| | | | | | | |
|---|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | | | |

**Table 36: STM 434 Injection Lot FG--14-0109 Stability Data Summary: 2-8°C Upright (SPN-677)**

| **Condition: 2-8°C, Upright** | | | **Months** | | | |
|---|---|---|---|---|---|---|
| **Start date: 25 Jun 2014** | | | | | | |
| **Method** | Parameter | **Acceptance Criteria** | **0** | **3** | **6** | |
| Appearance | Clarity | CLR L | CLR L | CLR L | CLR L | |
| | Color | CL to LY | CL | CL | CL | |
| | Particles | EFOP | FOP | NVP | NVP | |
| pH | pH | 6.4-7.0 | 6.6 | 6.5 | 6.5 | |
| Concentration by UV | mg/mL | 63-77 | 67.8 | 67.3 | 67.5 | |
| CE-SDS, Reduced | %main peak | ≥ 90.0% | 98.5 | 98.7 | 99.0 | |
| CE-SDS, Non-Reduced | %main peak | ≥ 88.0% | 94.4 | 94.1 | 94.0 | |
| | % pre-peak | -- | 5.6 | 5.9 | 6.0 | |
| SE-HPLC | % main peak | ≥ 95.0% | 98.8 | 98.9 | 98.8 | |
| | % HMW | -- | 1.2 | 1.1 | 1.2 | |
| | % LMW | -- | N.D. | ≤ 0.5 | 0.1 | |
| icIEF | % main peak | 65.0±10.0 | 65.4 | 65.7 | 67.3 | |
| | % APG | -- | 23.0 | 18.9 | 19.2 | |
| | % BPG | -- | 11.6 | 15.5 | 13.8 | |
| Bioassay | % relative potency | 60-140 | 98 | 92 | 107 | |
| Subvisible Particulates | ≥ 10 µm | ≤ 6000 per container | 2 | N.S. | N.S. | |
| | ≥ 25 µm | ≤ 600 per container | 0 | N.S. | N.S. | |
| Container Closure Integrity | Dye ingress | No detectable dye ingress | N.S. | N.S. | N.S. | |

| | | | | | | |
|---|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | | | |

**Table 37: STM 434 Injection Lot FG--14-0109 Stability Data Summary: 2-8°C Inverted (SPN-678)**

| **Condition: 2-8°C, Inverted** | | | **Months** | | | |
|---|---|---|---|---|---|---|
| **Start date: 25 Jun 2014** | | | | | | |
| **Method** | **Parameter** | **Acceptance Criteria** | **0** | **3** | **6** | |
| Appearance | Clarity | CLR L | CLR L | CLR L | CLR L | |
| | Color | CL to LY | CL | CL | CL | |
| | Particles | EFOP | FOP | NVP | NVP | |
| pH | pH | 6.4-7.0 | 6.6 | 6.5 | 6.5 | |
| Concentration by UV | mg/mL | 63-77 | 67.8 | 67.7 | 67.7 | |
| CE-SDS, Reduced | %main peak | ≥ 90.0% | 98.5 | 98.8 | 99.1 | |
| CE-SDS, Non-Reduced | % main peak | ≥ 88.0% | 94.4 | 94.3 | 94.0 | |
| | % pre-peak | -- | 5.6 | 5.7 | 6.0 | |
| SE-HPLC | % main peak | ≥ 95.0% | 98.8 | 98.9 | 98.8 | |
| | % HMW | -- | 1.2 | 1.1 | 1.2 | |
| | % LMW | -- | N.D. | ≤ 0.5 | 0.1 | |
| icIEF | % main peak | 65.0±10.0 | 65.4 | 65.5 | 68.5 | |
| | % APG | -- | 23.0 | 19.5 | 18.9 | |
| | % BPG | -- | 11.6 | 15.1 | 12.8 | |
| Bioassay | % relative potency | 60-140 | 98 | 96 | 112 | |
| Subvisible Particulates | ≥ 10 µm | ≤ 6000 per container | 2 | N.S. | N.S. | |
| | ≥ 25 µm | ≤ 600 per container | 0 | N.S. | N.S. | |
| Container Closure Integrity | Dye ingress | No detectable dye ingress | N.S. | N.S. | N.S. | |

| | | | | | | |
|---|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled | | | | | | |

**Table 38: STM 434 Injection Lot FG-14-0109 Stability Data Summary: 25°C Upright (SPN-679)**

| **Condition: 25°C, Upright** | | | **Months** | | |
|---|---|---|---|---|---|
| **Start date: 25 Jun 2014** | | | | | |
| **Method** | **Parameter** | **Acceptance Criteria** | **0** | **3** | **6** |
| Appearance | Clarity | CLR L | CLR L | CLR L | CLR L |
| | Color | CL to LY | CL | CL | CL |
| | Particles | EFOP | FOP | NVP | NVP |
| pH | pH | 6.4-7.0 | 6.6 | 6.5 | 6.5 |
| Concentration by UV | mg/mL | 63-77 | 67.8 | 68.3 | 67.7 |
| CE-SDS, Reduced | %main peak | ≥ 90.0% | 98.5 | 98.7 | 98.8 |
| CE-SDS, Non-Reduced | %main peak | ≥ 88.0% | 94.4 | 94.3 | 93.6 |
| | % pre-peak | -- | 5.6 | 5.7 | 6.4 |
| SE-HPLC | % main peak | ≥ 95.0% | 98.8 | 98.5 | 98.3 |
| | % HMW | -- | 1.2 | 1.2 | 1.4 |
| | % LMW | -- | N.D. | ≤ 0.5 | 0.3 |
| icIEF | % main peak | 65.0±10.0 | 65.4 | 61.5 | 62.5 |
| | % APG | -- | 23.0 | 22.6 | 25.1 |
| | % BPG | -- | 11.6 | 16.0 | 12.5 |
| Bioassay | % relative potency | 60-140 | 98 | 91 | 105 |
| Subvisible Particulates | ≥ 10 µm | ≤ 6000 per container | 2 | N.S. | 36 |
| | ≥ 25 µm | ≤ 600 per container | 0 | N.S. | 2 |
| Container Closure Integrity | Dye ingress | No detectable dye ingress | N.S. | N.S. | NDDI |

| | | | | | |
|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled; NDDI = no detectable dye ingress | | | | | |

**Table 39: STM 434 Injection Lot FG-14-0109 Stability Data Summary: 25°C Inverted (SPN-680)**

| **Condition: 25°C, Inverted** | | | **Months** | | |
|---|---|---|---|---|---|
| **Start date: 25 Jun 2014** | | | | | |
| **Method** | **Parameter** | **Acceptance Criteria** | **0** | **3** | **6** |
| Appearance | Clarity | CLR L | CLR L | CLR L | CLR L |
| | Color | CL to LY | CL | CL | CL |
| | Particles | EFOP | FOP | NVP | NVP |
| pH | pH | 6.4-7.0 | 6.6 | 6.5 | 6.5 |
| Concentration by UV | mg/mL | 63-77 | 67.8 | 67.7 | 67.2 |
| CE-SDS, Reduced | % main peak | ≥ 90.0% | 98.5 | 98.7 | 98.6 |
| CE-SDS, Non-Reduced | %main peak | ≥ 88.0% | 94.4 | 94.2 | 93.9 |
| | % pre-peak | -- | 5.6 | 5.8 | 6.1 |
| SE-HPLC | % main peak | ≥ 95.0% | 98.8 | 98.5 | 98.3 |
| | % HMW | -- | 1.2 | 1.2 | 1.4 |
| | % LMW | -- | N.D. | ≤ 0.5 | 0.3 |
| icIEF | % main peak | 65.0±10.0 | 65.4 | 62.3 | 61.5 |
| | % APG | -- | 23.0 | 22.5 | 26.8 |
| | % BPG | -- | 11.6 | 15.4 | 11.8 |
| Bioassay | % relative potency | 60-140 | 98 | 94 | 98 |
| Subvisible Particulates | ≥ 10 µm | ≤ 6000 per container | 2 | N.S. | 7 |
| | ≥ 25 µm | ≤ 600 per container | 0 | N.S. | 2 |
| Container Closure Integrity | Dye ingress | No detectable dye ingress | N.S. | N.S. | NDDI |

| | | | | | |
|---|---|---|---|---|---|
| CLR L = clear liquid; CL = colorless; LY = light yellow; EFOP = essentially free of particles; NVP = no visible particles; HMW = high molecular weight; LMW = low molecular weight; APG = acidic peak group; BPG = basic peak group; N.D. = not detected; N.S. = not scheduled; NDDI = no detectable dye ingress | | | | | |

### Example 8: STM 434 administration alters human body composition.

A human subject was selected for treatment with STM 434 using the above protocol in Example 4. The subject was a 62 year old African-American male with recurrent papillary renal cell carcinoma metastatic to the contralateral kidney and pelvic lymph nodes. He had received 8 prior lines of antitumor therapy with Torisel, Pazopanib, and investigational agents including B7-H3 mAb, cMet inhibitors, a CHK1 inhibitor, an Aurora kinase inhibitor, and recombinant human Interleukin 10.

### STM 434 Formulation, Packing, and Storage

STM 434 was formulated as a sterile aqueous solution intended for IV administration, containing 70 mg/mL STM 434, 10 mM potassium phosphate buffer, 8.8% (w/v) sucrose, and 0.006% (w/v) polysorbate 20 at pH 6.7. Formulated STM 434 solution was packaged into 5-mL glass vials, with 13 mm fluoropolymer stoppers and 13 mm seals. Vials of STM 434 were stored in a non-frost-free freezer at a temperature of -20°C (± 5°C). Prior to use, STM 434 was thawed overnight in a refrigerator at 2°C to 8°C.

### STM 434 Administration and Monitoring

STM 434 was administered to the subject at 0.25 mg/kg IV approximately every 4 weeks (Cohort 2).

First DXA scan was 30 Dec 2014; Dosing was 6 Jan 2015, 3 Feb 2015, and 3 Mar 2015; Follow up DXA scan was on 31 March 2015.

### Changes from baseline in lean body mass, appendicular lean mass, and fat mass (visceral and subcutaneous)

Various body composition measurements were taken approximately three months apart to assess the impact of STM 434 on body composition, e.g., muscle and fat. Lean body mass, appendicular lean mass, and fat mass, and fat distribution (visceral and subcutaneous) were determined by DXA scans. DXA scans were analyzed by the site radiologist and confirmed by the central radiology laboratory using IBIS (Imaging Biomarker Information System) software to ensure accurate subject positioning and machine calibration.

Table 40 shows the results of the measurements for the subject. The results demonstrate substantial increases in lean body mass and substantial decreases in fat mass after only approximately 3 months of treatment at a relatively low dose of STM 434.

| **Table 40** | | | | | | | | | | |
|---|---|---|---|---|---|---|---|---|---|---|
| DATE | TLBM | ALM | TFM | VFM | SFM | TFDIS | VFDIS | SFDIS | TBMD | RDCOM |
| 30-Dec-2014 | 57398 | 22492 | 15793 | 657 | 353 | 20.7 | 65 | 35 | 1.383 | N/A |
| 31-Mar-2015 | 64460 | 24389 | 8204 | 4 | 321 | 10.8 | 1.4 | 98.6 | 1.365 | N/A |

| | | | | | | | | | | |
|---|---|---|---|---|---|---|---|---|---|---|
| % Δ | 12.30% | 8.40% | -48% | -99% | -9.1% | -47.8% | -97.8% | 282% | -1.3% | N/A |
| TLBM= total lean body mass; | | | | | | | | | | |
| ALM= Appendicular lean mass; | | | | | | | | | | |
| TFM= total fat mass; | | | | | | | | | | |
| VFM= visceral fat mass; | | | | | | | | | | |
| SFM= subcutaneous fat mass; | | | | | | | | | | |
| TBMD= total bone mineral density; | | | | | | | | | | |
| TFDIS= total fat distribution; | | | | | | | | | | |
| VFDIS= visceral fat distribution; | | | | | | | | | | |
| SFDIS= subcutaneous fat distribution; | | | | | | | | | | |

While the invention has been particularly shown and described with reference to a preferred embodiment and various alternate embodiments, it will be understood by persons skilled in the relevant art that various changes in form and details can be made therein without departing from the scope of the invention.

**SEQUENCES**

| **SEQ ID NO** | **Description** |
|---|---|
| 1 | ActRIIB extracellular domain, polynucleotide |
| 2 | ActRIIB extracellular domain, polypeptide |
| 3 | svActRIIB (E28W, S44T) polynucleotide with signal sequence |
| 4 | svActRIIB (E28W, S44T) polypeptide with signal sequence |
| 5 | svActRIIB (E28W, S44T) polynucleotide without signal sequence |
| 6 | svActRIIB (E28W, S44T) polypeptide without signal sequence |
| 7 | svActRIIB-Fc (E28W, S44T) polynucleotide with signal sequence |
| 8 | svActRIIB-Fc (E28W, S44T) polypeptide with signal sequence |
| 9 | svActRIIB-Fc (E28W, S44T) polynucleotide without signal sequence |
| 10 | svActRIIB-Fc (E28W, S44T) polypeptide without signal sequence (STM 434) |
| 11 | svActRIIB (E28Y, S44T) polynucleotide with signal sequence |
| 12 | svActRIIB (E28Y, S44T) polypeptide with signal sequence |
| 13 | svActRIIB (E28Y, S44T) polynucleotide without signal sequence |
| 14 | svActRIIB (E28Y, S44T) polypeptide without signal sequence |
| 15 | svActRIIB-Fc (E28Y, S44T) polynucleotide with signal sequence |
| 16 | svActRIIB-Fc (E28Y, S44T) polypeptide with signal sequence |
| 17 | svActRIIB-Fc (E28Y, S44T) polynucleotide without signal sequence |
| 18 | svActRIIB-Fc (E28Y, S44T) polypeptide without signal sequence |
| 19 | ActRIIB (E28W) polypeptide, without signal sequence |
| 20 | ActRIIB-Fc (E28W) polynucleotide, without signal sequence |
| 21 | ActRIIB-Fc (E28W) polypeptide, without signal sequence |
| 22 | IgG2Fc polypeptide sequence |
| 23 | IgG1Fc polypeptide sequence |
| 24 | IgG4 Fc polypeptide sequence |
| 25 | Linker amino acid sequence |
| 26 | Hinge linker #1 polynucleotide sequence |
| 27 | Hinge linker #1 peptide sequence |

| **SEQ ID NO** | **SEQUENCE** |
|---|---|
| 1 | |
| | |
| 2 | |
| 3 | |
| 4 | |
| 53 | |
| 6 | |
| 7 | |
| 8 | |
| 9 | |
| | |
| 10 | |
| 11 | |
| 12 | |
| 13 | |
| 14 | |
| 15 | |
| | |
| 16 | |
| 17 | |
| 18 | |
| 19 | |
| 20 | |
| 21 | |
| | |
| 22 | |
| 23 | |
| 24 | |
| 25 | GGGGS |
| 26 | GGAGGGGGAG GATCTGTCGA GTGCCCACCG TGCCCA |
| 27 | GGGGSVECPP CP |

## Claims

1. A composition comprising a solution of a protein, a sucrose excipient, a potassium phosphate buffer, and a polysorbate 20 surfactant, wherein the composition comprises a pH of 4-12, wherein the protein comprises a polypeptide capable of binding myostatin, activin A, or GDF-11, wherein the polypeptide is selected from the group consisting of:
(a) a polypeptide consisting of the amino acid sequence set forth in the group consisting of SEQ ID NO: 4, 6, 12, and 14;
(b) a polypeptide having at least 90% sequence identity to (a), and the polypeptide has a W or a Y at the position corresponding to position 28 of the sequence set forth in SEQ ID NO:2 and a T at the position corresponding to position 44 of the sequence set forth in SEQ ID NO:2, and
(c) a polypeptide having at least 95% sequence identity to (a), wherein the polypeptide has a W or a Y at the position corresponding to position 28 of the sequence set forth in SEQ ID NO:2 and a T at the position corresponding to position 44 of the sequence set forth in SEQ ID NO:2; and
wherein the protein in the composition retains at least 80% stability for a time period of greater than 1 month in solution when kept at 2-8°C or 5°C relative to the protein in the composition at the beginning of the time period (0 months) or relative to an identical protein kept under otherwise identical conditions for greater than 1 month at -20°C or -70°C;
and, optionally wherein the composition further comprises a chemotherapeutic agent or a second therapeutic agent.

2. The composition of claim 1, wherein the protein consists of the sequence set forth in SEQ ID NO:10 at a concentration of 70 mg/mL, the excipient is 8.8% weight/volume (w/v) sucrose, the buffer is 10 mM potassium phosphate buffer, the surfactant is 0.006% (w/v) polysorbate 20, and comprising a pH of 6.7; and wherein the protein in the composition retains at least 90% stability for a time period of greater than 6 months in solution when kept at 2-8°C or 5°C relative to the protein in the composition at the beginning of the time period (0 months) or relative to an identical protein kept under otherwise identical conditions for greater than 6 months at -20°C or -70°C.

3. The composition of claim 1, comprising a pH of least 4, 5, 6, 7, 8, or 9, optionally wherein the pH is 4-10, 4-8, or 5-7, optionally wherein the pH is at least 6-7, optionally wherein the pH is at least 6.0, 6.1, 6.2, 6.3, 6.4, 6.5, 6.6, 6.7, 6.8, 6.9, or 7.0.

4. The composition of any preceding claim except claim 2, wherein:
(i) the excipient concentration in the composition is at least 7-11, 8-10, 8-9, 8.8, 7, 8, 9, 10, 11, 8.0, 8.1, 8.2, 8.3, 8.4, 8.5, 8.6, 8.7, 8.9, or 9.0% weight/volume (w/v);
(ii) the buffer concentration in the composition is at least 7-13, 8-12, 9-11, 8, 9, 10, 11, or 12 mM;
(iii) the surfactant concentration is 0.001-0.10, 0.05-0.10, 0.001, 0.002, 0.003, 0.004, 0.005, 0.006, 0.007, 0.008, 0.009, 0.010, 0.02, 0.03, 0.04, 0.05, 0.06, 0.07, 0.08, 0.09, or 0.10% (w/v);
(iv) the protein in the composition retains at least 99.5-80%, 90-85%, 99.5%, 99%, 98%, 97%, 96%, 95%, 94%, 93%, 92%, 91%, 90%, 89%, 88%, 87%, 86%, 85%, 84%, 83%, 82%, 81%, or 80% stability for a time period of greater than 1-54, 6-48, 12-36, 24-36, 1, 2, 3, 6, 12, 18, 24, 36, 48, or 54 month(s) in solution when kept at 2-8°C or 5°C relative to the protein in the composition at the beginning of the time period (0 months); or
(v) the protein in the composition retains at least 99.5-80%, 90-85%, 99.5%, 99%, 98%, 97%, 96%, 95%, 94%, 93%, 92%, 91%, 90%, 89%, 88%, 87%, 86%, 85%, 84%, 83%, 82%, 81% or 80% stability for a time period of greater than 1-54, 6-48, 12-36, 24-36, 1, 2, 3, 6, 12, 18, 24, 36, 48, or 54 month(s) in solution when kept at 2-8°C or 5°C relative to an identical protein kept under otherwise identical conditions for an equivalent time period at - 20°C or -70°C.

5. The composition of any preceding claim, wherein the protein in the composition retains at least 99.5-80%, 90-85%, 99.5%, 99%, 98%, 97%, 96%, 95%, 94%, 93%, 92%, 91%, 90%, 89%, 88%, 87%, 86%, 85%, 84%, 83%, 82%, 81%, or 80% activity for a time period of greater than 1-54, 6-48, 12-36, 24-36, 1, 2, 3, 6, 12, 18, 24, 36, 48, or 54 month(s) in solution when kept at 2-8°C or 5°C relative to the protein in the composition at the beginning of the time period (0 months), optionally wherein activity is determined using a cell-based bioassay.

6. The composition of any preceding claim, wherein the protein in the composition retains at least 99.5-80%, 90-85%, 99.5%, 99%, 98%, 97%, 96%, 95%, 94%, 93%, 92%, 91%, 90%, 89%, 88%, 87%, 86%, 85%, 84%, 83%, 82%, 81% or 80% activity for a time period of greater than 1-54, 6-48, 12-36, 24-36, 1, 2, 3, 6, 12, 18, 24, 36, 48, or 54 month(s) in solution when kept at 2-8°C or 5°C relative to an identical protein kept under otherwise identical conditions for an equivalent time period at -20°C or -70°C, optionally wherein activity is determined using a cell-based bioassay.

7. The composition of any preceding claim, wherein percent stability of the protein in the composition is determined by one or more of the following: visual appearance, osmolality, pH, volume, size exclusion high performance liquid chromatography (SE-HPLC), imaged capillary isoelectric focusing (icIEF), sodium dodecul sulfate capillary electrophoresis (CE-SDS), non reduced (nrCE-SDS), reduced CE-SDS, enzyme-linked immuno-specific asssay (ELISA), cell-based bioassay, endotoxin level, sterility, subvisible particles, and polysorbate 20 concentration.

8. The composition of any preceding claim except claim 2, wherein the peptide consists of the amino acid sequence set forth in SEQ ID NO:6 or wherein the protein consists of the amino acid sequence set forth in SEQ ID NO:10.

9. The composition of any preceding claim except claim 2, wherein the concentration of the protein is at least 50-90, 60-80, 65-70, 70-75, 60, 61, 62, 63, 64, 65, 66, 67, 68, 69, 70, 71, 72, 73, 74, 75, 76, 77, 78, 79, or 80 mg/mL.

10. The composition of any preceding claim except claim 2, wherein the polypeptide comprises the sequence set forth in SEQ ID NO:6, the excipient is 8.8% (w/v) sucrose, the buffer is 10 mM potassium phosphate buffer, the surfactant is 0.006% (w/v) polysorbate 20, and the pH is 6.7.

11. The composition of any preceding claim except claim 2, wherein the protein consists of the amino acid sequence set forth in SEQ ID NO:10 at a concentration of 70 mg/mL, the excipient is 8.8% (w/v) sucrose, the buffer is 10 mM potassium phosphate buffer, the surfactant is 0.006% (w/v) polysorbate 20, and the pH is 6.7.

12. A composition as defined in any one of the preceding claims for use in a method of inhibiting a solid tumor growth in a subject, or treating a solid tumor in a subject, or inhibiting, reducing, or treating cachexia in a subject, comprising administering a dose of the composition of any of the above composition claims to the subject.

13. A composition as defined in any one of claims 1 to 11, for use in a method of inhibiting, reducing, or treating cachexia in a subject, comprising administering a fixed dose ranging from at least 0.1 mg/kg to 20 mg/kg of the composition of any preceding composition claim to the subject.

14. A method of producing the composition of any one of claims 1 to 11, wherein the protein comprises a polypeptide capable of binding myostatin, activin A, or GDF-11, wherein the polypeptide is selected from the group consisting of: (a) a polypeptide consisting of the amino acid sequence set forth in the group consisting of SEQ ID NO: 4, 6, 12 and 14; (b) a polypeptide having at least 90% sequence identity to (a), and the polypeptide has a W or a Y at the position corresponding to position 28 of the sequence set forth in SEQ ID NO:2 and a T at the position corresponding to position 44 of the sequence set forth in SEQ ID NO:2, and (c) a polypeptide having at least 95% sequence identity to (a), and the polypeptide has a W or a Y at the position corresponding to position 28 of the sequence set forth in SEQ ID NO:2 and a T at the position corresponding to position 44 of the sequence set forth in SEQ ID NO:2, the method comprising:
culturing a CS9 Chinese hamster ovary cell line engineered to express the protein in a cell culture;
harvesting the protein from the culture; and
purifying the protein.

## Patentansprüche

1. Zusammensetzung, umfassend eine Lösung aus einem Protein, einem Saccharose-Hilfsstoff, einem Kaliumphosphatpuffer und einem Polysorbat-20-Tensid, wobei die Zusammensetzung einen pH-Wert von 4-12 umfasst, wobei das Protein ein Polypeptid umfasst, das Myostatin, Aktivin-A oder GDF-11 binden kann, wobei das Polypeptid ausgewählt ist aus der Gruppe bestehend aus:
(a) einem Polypeptid, das auf der Aminosäuresequenz besteht, die in der Gruppe dargelegt ist, bestehend aus SEQ ID NO: 4, 6, 12 und 14;
(b) einem Polypeptid mit mindestens 90 % Sequenzidentität mit (a) und wobei das Polypeptid ein W oder ein Y an der Position hat, die Position 28 der Sequenz entspricht, die in SEQ ID NO: 2 dargelegt ist, und ein T an der Position hat, die Position 44 der Sequenz entspricht, die in SEQ ID NO: 2 dargelegt ist, und
(c) einem Polypeptid mit mindestens 95 % Sequenzidentität mit (a), wobei das Polypeptid ein W oder ein Y an der Position hat, die Position 28 der Sequenz entspricht, die in SEQ ID NO: 2 dargelegt ist, und ein T an der Position hat, die Position 44 der Sequenz entspricht, die in SEQ ID NO: 2 dargelegt ist; und
wobei das Protein in der Zusammensetzung mindestens 80 % Stabilität für einen Zeitraum von länger als 1 Monat in Lösung behält, wenn es bei 2-8 °C oder 5 °C gehalten wird, relativ zu dem Protein in der Zusammensetzung am Anfang des Zeitraums (0 Monate) oder relativ zu einem identischen Protein, das unter ansonsten identischen Bedingungen für länger als 1 Monat bei -20 °C oder -70 °C gehalten wird;
und optional wobei die Zusammensetzung ferner ein Chemotherapeutikum oder ein zweites Therapeutikum umfasst.

2. Zusammensetzung nach Anspruch 1, wobei das Protein aus der Sequenz besteht, die in SEQ ID NO: 10 dargelegt ist, in einer Konzentration von 70 mg/ml, der Hilfsstoff 8,8 % Gewicht/Volumen (w/v) Saccharose ist, der Puffer 10 mM Kaliumphosphatpuffer ist, das Tensid 0,006 % (w/v) Polysorbat 20 ist, und umfassend einen pH-Wert von 6,7; und wobei das Protein in der Zusammensetzung mindestens 90 % Stabilität für einen Zeitraum von länger als 6 Monate in Lösung behält, wenn es bei 2-8 °C oder 5 °C gehalten wird, relativ zu dem Protein in der Zusammensetzung am Anfang des Zeitraums (0 Monate) oder relativ zu einem identischen Protein, das unter ansonsten identischen Bedingungen für länger als 6 Monate bei -20 °C oder -70 °C gehalten wird.

3. Zusammensetzung nach Anspruch 1, umfassend einen pH-Wert von mindestens 4, 5, 6, 7, 8 oder 9, optional wobei der pH-Wert 4-10, 4-8 oder 5-7 ist, optional wobei der pH-Wert mindestens 6-7 ist, optional wobei der pH-Wert mindestens 6,0, 6,1, 6,2, 6,3, 6,4, 6,5, 6,6, 6,7, 6,8, 6,9 oder 7,0 ist.

4. Zusammensetzung nach einem vorhergehenden Anspruch außer Anspruch 2, wobei:
(i) die Hilfsstoffkonzentration in der Zusammensetzung mindestens 7-11, 8-10, 8-9, 8,8, 7, 8, 9, 10, 11, 8,0, 8,1, 8,2, 8,3, 8,4, 8,5, 8,6, 8,7, 8,9 oder 9,0 % Gewicht/Volumen (w/v) ist;
(ii) die Pufferkonzentration in der Zusammensetzung mindestens 7-13, 8-12, 9-11, 8, 9, 10, 11 oder 12 mM ist;
(iii) die Tensidkonzentration 0,001-0,10, 0,05-0,10, 0,001, 0,002, 0,003, 0,004, 0,005, 0,006, 0,007, 0,008, 0,009, 0,010, 0,02, 0,03, 0,04, 0,05, 0,06, 0,07, 0,08, 0,09 oder 0,10 % (w/v) ist;
(iv) das Protein in der Zusammensetzung mindestens 99,5-80 %, 90-85 %, 99,5 %, 99 %, 98 %, 97 %, 96 %, 95 %, 94 %, 93 %, 92 %, 91 %, 90 %, 89 %, 88 %, 87 %, 86 %, 85 %, 84 %, 83 %, 82 %, 81 % oder 80 % Stabilität für einen Zeitraum von länger als 1-54, 6-48, 12-36, 24-36, 1, 2, 3, 6, 12, 18, 24, 36, 48 oder 54 Monat(e) in Lösung behält, wenn es bei 2-8 °C oder 5 °C gehalten wird, relativ zu dem Protein in der Zusammensetzung am Anfang des Zeitraums (0 Monate); oder
(v) das Protein in der Zusammensetzung mindestens 99,5-80 %, 90-85 %, 99,5 %, 99 %, 98 %, 97 %, 96 %, 95 %, 94 %, 93 %, 92 %, 91 %, 90 %, 89 %, 88 %, 87 %, 86 %, 85 %, 84 %, 83 %, 82 %, 81 % oder 80 % Stabilität für einen Zeitraum von länger als 1-54, 6-48, 12-36, 24-36, 1, 2, 3, 6, 12, 18, 24, 36, 48 oder 54 Monat(e) in Lösung behält, wenn es bei 2-8 °C oder 5 °C gehalten wird, relativ zu einem identischen Protein, das unter ansonsten identischen Bedingungen für einen gleichwertigen Zeitraum bei -20 °C oder -70 °C gehalten wird.

5. Zusammensetzung nach einem vorhergehenden Anspruch, wobei das Protein in der Zusammensetzung mindestens 99,5-80 %, 90-85 %, 99,5 %, 99 %, 98 %, 97 %, 96 %, 95 %, 94 %, 93 %, 92 %, 91 %, 90 %, 89 %, 88 %, 87 %, 86 %, 85 %, 84 %, 83 %, 82 %, 81 % oder 80 % Aktivität für einen Zeitraum von länger als 1-54, 6-48, 12-36, 24-36, 1, 2, 3, 6, 12, 18, 24, 36, 48 oder 54 Monat(e) in Lösung behält, wenn es bei 2-8 °C oder 5 °C gehalten wird, relativ zu dem Protein in der Zusammensetzung am Anfang des Zeitraums (0 Monate), optional wobei Aktivität unter Verwendung eines zellbasierten Bioassays bestimmt wird.

6. Zusammensetzung nach einem vorhergehenden Anspruch, wobei das Protein in der Zusammensetzung mindestens 99,5-80 %, 90-85 %, 99,5 %, 99 %, 98 %, 97 %, 96 %, 95 %, 94 %, 93 %, 92 %, 91 %, 90 %, 89 %, 88 %, 87 %, 86 %, 85 %, 84 %, 83 %, 82 %, 81 % oder 80 % Aktivität für einen Zeitraum von länger als 1-54, 6-48, 12-36, 24-36, 1, 2, 3, 6, 12, 18, 24, 36, 48 oder 54 Monat(e) in Lösung behält, wenn es bei 2-8 °C oder 5 °C gehalten wird, relativ zu einem identischen Protein, das unter ansonsten identischen Bedingungen für einen gleichwertigen Zeitraum bei -20 °C oder -70 °C gehalten wird, optional wobei Aktivität unter Verwendung eines zellbasierten Bioassays bestimmt wird.

7. Zusammensetzung nach einem vorhergehenden Anspruch, wobei Prozentstabilität des Proteins in der Zusammensetzung durch ein oder mehrere aus dem Folgenden bestimmt ist: visuelles Erscheinungsbild, Osmolalität, pH-Wert, Volumen, Größenausschluss-Hochleistungsflüssigkeitschromatographie (SE-HPLC), abgebildete kapillare isoelektrische Fokussierung (icIEF), Natriumdodeculsulfat-Kapillarelektrophorese (CE-SDS), nicht reduzierte (nrCE-SDS), reduzierte CE-SDS, enzymgebundener immunspezifischer Test (ELISA), zellbasiertes Bioassay, Endotoxin-Spiegel, Sterilität, subvisible Partikel und Polysorbat-20-Konzentration.

8. Zusammensetzung nach einem vorhergehenden Anspruch außer Anspruch 2, wobei das Peptid aus der Aminosäuresequenz besteht, die in SEQ ID NO: 6 dargelegt ist, oder wobei das Protein aus der Aminosäuresequenz besteht, die in SEQ ID NO: 10 dargelegt ist.

9. Zusammensetzung nach einem vorhergehenden Anspruch außer Anspruch 2, wobei die Konzentration des Proteins mindestens 50-90, 60-80, 65-70, 70-75, 60, 61, 62, 63, 64, 65, 66, 67, 68, 69, 70, 71, 72, 73, 74, 75, 76, 77, 78, 79 oder 80 mg/ml ist.

10. Zusammensetzung nach einem vorhergehenden Anspruch außer Anspruch 2, wobei das Polypeptid die Sequenz umfasst, die in SEQ ID NO: 6 dargelegt ist, der Hilfsstoff 8,8 % (w/v) Saccharose ist, der Puffer 10 mM Kaliumphosphatpuffer ist, das Tensid 0,006 % (w/v) Polysorbat 20 ist und der pH-Wert 6,7 ist.

11. Zusammensetzung nach einem vorhergehenden Anspruch außer Anspruch 2, wobei das Protein aus der Aminosäuresequenz besteht, die in SEQ ID NO: 10 dargelegt ist, in einer Konzentration von 70 mg/ml, der Hilfsstoff 8,8 % (w/v) Saccharose ist, der Puffer 10 mM Kaliumphosphatpuffer ist, das Tensid 0,006 % (w/v) Polysorbat 20 ist und der pH-Wert 6,7 ist.

12. Zusammensetzung nach einem der vorhergehenden Ansprüche zur Verwendung in einem Verfahren des Hemmens des Wachstums eines festen Tumors in einem Subjekt oder des Behandelns eines festen Tumors in einem Subjekt oder des Hemmens, Verringerns oder Behandelns von Kachexie in einem Subjekt, umfassend das Verabreichen einer Dosis der Zusammensetzung nach einem der oben stehenden Zusammensetzungsansprüche an das Subj ekt.

13. Zusammensetzung nach einem der Ansprüche 1 bis 11 zur Verwendung in einem Verfahren des Hemmens, Verringerns oder Behandelns von Kachexie in einem Subjekt, umfassend das Verabreichen einer festen Dosis im Bereich von mindestens 0,1 mg/kg bis 20 mg/kg der Zusammensetzung nach einem vorhergehenden Zusammensetzungsanspruch an das Subjekt.

14. Verfahren des Erzeugens der Zusammensetzung nach einem der Ansprüche 1 bis 11, wobei das Protein ein Polypeptid umfasst, das Myostatin, Aktivin-A oder GDF-11 binden kann, wobei das Polypeptid ausgewählt ist aus der Gruppe bestehend aus: (a) einem Polypeptid, das auf der Aminosäuresequenz besteht, die in der Gruppe dargelegt ist, bestehend aus SEQ ID NO: 4, 6, 12 und 14; (b) einem Polypeptid mit mindestens 90 % Sequenzidentität mit (a) und wobei das Polypeptid ein W oder ein Y an der Position hat, die Position 28 der Sequenz entspricht, die in SEQ ID NO: 2 dargelegt ist, und ein T an der Position hat, die Position 44 der Sequenz entspricht, die in SEQ ID NO: 2 dargelegt ist, und (c) einem Polypeptid mit mindestens 95 % Sequenzidentität mit (a), wobei das Polypeptid ein W oder ein Y an der Position hat, die Position 28 der Sequenz entspricht, die in SEQ ID NO: 2 dargelegt ist, und ein T an der Position hat, die Position 44 der Sequenz entspricht, die in SEQ ID NO: 2 dargelegt ist, wobei das Verfahren Folgendes umfasst:
Kultivieren einer CS9 Zelllinie aus Ovarien des Chinesischen Zwerghamsters, die gebaut ist, um das Protein in einer Zellkultur zu exprimieren;
Ernten des Proteins aus der Kultur; und
Reinigen des Proteins.

## Revendications

1. Composition comprenant une solution d'une protéine, d'un excipient de saccharose, d'un tampon phosphate de potassium et d'un tensioactif polysorbate 20, dans laquelle la composition comprend un pH de 4 à 12, dans laquelle la protéine comprend un polypeptide capable de se lier à la myostatine, à l'activine A ou au GDF-11, dans laquelle le polypeptide est choisi dans le groupe constitué par :
(a) un polypeptide constitué par la séquence d'acides aminés présentée dans le groupe constitué par SEQ ID NO: 4, 6, 12 et 14 ;
(b) un polypeptide ayant au moins 90% d'identité de séquence avec (a), et le polypeptide a un W ou un Y à la position correspondant à la position 28 de la séquence présentée dans SEQ ID NO: 2 et un T à la position correspondant à la position 44 de la séquence présentée dans SEQ ID NO: 2, et
(c) un polypeptide ayant au moins 95% d'identité de séquence avec (a), dans laquelle le polypeptide a un W ou un Y à la position correspondant à la position 28 de la séquence présentée dans SEQ ID NO: 2 et un T à la position correspondant à la position 44 de la séquence présentée dans SEQ ID NO: 2 ; et
dans laquelle la protéine dans la composition conserve au moins 80% de stabilité pendant une période de temps supérieure à 1 mois en solution lorsqu'elle est maintenue à 2 à 8 °C ou 5 °C par rapport à la protéine dans la composition au début de la période de temps (0 mois) ou par rapport à une protéine identique maintenue dans des conditions par ailleurs identiques pendant plus d'un mois à -20 °C ou -70 °C ;
et, éventuellement dans laquelle la composition comprend en outre un agent chimiothérapeutique ou un deuxième agent thérapeutique.

2. Composition selon la revendication 1, dans laquelle la protéine est constituée par la séquence indiquée dans SEQ ID NO: 10 à une concentration de 70 mg/mL, l'excipient contient 8,8% poids/volume (p/v) de saccharose, le tampon est un tampon contenant 10 mM de phosphate de potassium, le tensioactif contient 0,006% (p/v) de polysorbate 20, et comprenant un pH de 6,7 ; et dans laquelle la protéine dans la composition conserve au moins 90% de stabilité pendant une période de temps supérieure à 6 mois en solution lorsqu'elle est maintenue à 2 à 8 °C ou 5 °C par rapport à la protéine dans la composition au début de la période de temps (0 mois) ou par rapport à une protéine identique maintenue dans des conditions par ailleurs identiques pendant plus de 6 mois à -20 °C ou -70 °C.

3. Composition selon la revendication 1, comprenant un pH d'au moins 4, 5, 6, 7, 8 ou 9, éventuellement dans laquelle le pH est de 4 à 10, 4 à 8 ou 5 à 7, éventuellement dans laquelle le pH est d'au moins 6 à 7, éventuellement dans laquelle le pH est d'au moins 6,0, 6,1, 6,2, 6,3, 6,4, 6,5, 6,6, 6,7, 6,8, 6,9 ou 7,0.

4. Composition selon l'une quelconque des revendications précédentes à l'exception de la revendication 2, dans laquelle :
(i) la concentration d'excipient dans la composition est d'au moins 7 à 11, 8 à 10, 8 à 9, 8.8, 7, 8, 9, 10, 11, 8,0, 8,1, 8,2, 8,3, 8,4, 8,5, 8,6, 8,7, 8,9 ou 9,0% en poids/volume (p/v) ;
(ii) la concentration de tampon dans la composition est d'au moins 7 à 13, 8 à 12, 9 à 11, 8, 9, 10, 11 ou 12 mM;
(iii) la concentration de tensioactif est de 0,001 à 0,10, 0,05 à 0,10, 0,001, 0,002, 0,003, 0,004, 0,005, 0,006, 0,007, 0,008, 0,009, 0,010, 0,02, 0,03, 0,04, 0,05, 0,06, 0,07, 0,08, 0,09 ou 0,10% (p/v) ;
(iv) la protéine dans la composition conserve au moins 99,5 à 80%, 90 à 85%, 99,5%, 99%, 98%, 97%, 96%, 95%, 94%, 93%, 92%, 91%, 90%, 89%, 88%, 87%, 86%, 85%, 84%, 83%, 82%, 81% ou 80% de stabilité pendant une période supérieure à 1 à 54, 6 à 48, 12 à 36, 24 à 36, 1, 2, 3, 6, 12, 18, 24, 36, 48 ou 54 mois en solution lorsqu'elle est maintenue à 2 à 8 °C ou 5 °C par rapport à la protéine dans la composition au début de la période (0 mois) ; ou
(v) la protéine dans la composition conserve au moins 99,5 à 80%, 90 à 85%, 99,5%, 99%, 98%, 97%, 96%, 95%, 94%, 93%, 92%, 91%, 90%, 89%, 88%, 87%, 86%, 85%, 84%, 83%, 82%, 81% ou 80% de stabilité pendant une période supérieure à 1 à 54, 6 à 48, 12 à 36, 24 à 36, 1, 2, 3, 6, 12, 18, 24, 36, 48 ou 54 mois en solution lorsqu'elle est maintenue à 2 à 8 °C ou 5 °C par rapport à une protéine identique maintenue dans des conditions par ailleurs identiques pendant une période de temps équivalente à -20 °C ou -70 °C.

5. Composition selon l'une quelconque des revendications précédentes, dans laquelle la protéine dans la composition conserve au moins 99,5 à 80%, 90 à 85%, 99,5%, 99%, 98%, 97%, 96%, 95%, 94%, 93%, 92%, 91%, 90%, 89%, 88%, 87%, 86%, 85%, 84%, 83%, 82%, 81% ou 80% d'activité pendant une période supérieure à 1 à 54, 6 à 48, 12 à 36, 24 à 36, 1, 2, 3, 6, 12, 18, 24, 36, 48 ou 54 mois en solution lorsqu'elle est maintenue à 2 à 8 °C ou 5 °C par rapport à la protéine dans la composition au début de la période de temps (0 mois), éventuellement dans laquelle l'activité est déterminée en utilisant un essai biologique à base de cellules.

6. Composition selon l'une quelconque des revendications précédentes, dans laquelle la protéine dans la composition conserve au moins 99,5 à 80%, 90 à 85%, 99,5%, 99%, 98%, 97%, 96%, 95%, 94%, 93%, 92%, 91%, 90%, 89%, 88%, 87%, 86%, 85%, 84%, 83%, 82%, 81% ou 80% d'activité pendant une période supérieure à 1 à 54, 6 à 48, 12 à 36, 24 à 36, 1, 2, 3, 6, 12, 18, 24, 36, 48 ou 54 mois en solution lorsqu'elle est maintenue à 2 à 8 °C ou 5 °C par rapport à une protéine identique maintenue dans des conditions par ailleurs identiques pendant une période de temps équivalente à -20 °C ou -70 °C, éventuellement dans laquelle l'activité est déterminée en utilisant un essai biologique à base de cellules.

7. Composition selon l'une quelconque des revendications précédentes, dans laquelle le pourcentage de stabilité de la protéine dans la composition est déterminé par un ou plusieurs des éléments suivants l'aspect visuel, l'osmolalité, le pH, le volume, la chromatographie d'exclusion liquide à haute performance (SE-HPLC), la focalisation isoélectrique capillaire imagée (icIEF), l'électrophorèse capillaire en présence de dodécyl sulfate de sodium (CE-SDS), la CE-SDS non réduite (nrCE-SDS), la CE-SDS réduite, l'essai immuno-spécifique lié à une enzyme (ELISA), l'essai biologique à base de cellules, le niveau d'endotoxine, la stérilité, les particules subvisibles et la concentration de polysorbate 20.

8. Composition selon l'une quelconque des revendications précédentes, à l'exception de la revendication 2, dans laquelle le peptide est constitué par la séquence d'acides aminés présentée dans SEQ ID NO: 6 ou dans laquelle la protéine est constituée par la séquence d'acides aminés présentée dans SEQ ID NO: 10.

9. Composition selon l'une quelconque des revendications précédentes à l'exception de la revendication 2, dans laquelle la concentration de la protéine est d'au moins 50 à 90, 60 à 80, 65 à 70, 70 à 75, 60, 61, 62, 63, 64, 65, 66, 67, 68, 69, 70, 71, 72, 73, 74, 75, 76, 77, 78, 79 ou 80 mg/ml.

10. Composition selon l'une quelconque des revendications précédentes à l'exception de la revendication 2, dans laquelle le polypeptide comprend la séquence indiquée dans SEQ ID NO: 6, l'excipient contient 8,8% (p/v) de saccharose, le tampon est un tampon contenant 10 mM de phosphate de potassium, le tensioactif contient 0,006% (p/v) de polysorbate 20 et le pH est de 6,7.

11. Composition selon l'une quelconque des revendications précédentes à l'exception de la revendication 2, dans laquelle la protéine est constituée par la séquence d'acides aminés indiquée dans SEQ ID NO: 10 à une concentration de 70 mg/mL, l'excipient contient 8,8% (p/v) de saccharose, le tampon est un tampon contenant 10 mM de phosphate de potassium, le tensioactif contient 0,006% (p/v) de polysorbate 20 et le pH est de 6,7.

12. Composition telle que définie dans l'une quelconque des revendications précédentes à utiliser dans un procédé d'inhibition de la croissance d'une tumeur solide chez un sujet, ou de traitement d'une tumeur solide chez un sujet, ou d'inhibition, de réduction ou de traitement de la cachexie chez un sujet, comprenant l'administration d'une dose de la composition de l'une quelconque des revendications ci-dessus au sujet.

13. Composition telle que définie dans l'une quelconque des revendications 1 à 11, à utiliser dans un procédé d'inhibition, de réduction ou de traitement de la cachexie chez un sujet, comprenant l'administration d'une dose fixe allant d'au moins 0,1 mg/kg à 20 mg/kg de la composition de l'une quelconque des revendications précédentes au sujet.

14. Procédé de production de la composition selon l'une quelconque des revendications 1 à 11, dans lequel la protéine comprend un polypeptide capable de se lier à la myostatine, à l'activine A ou au GDF-11, dans lequel le polypeptide est choisi dans le groupe constitué par : (a) un polypeptide constitué par la séquence d'acides aminés présentée dans le groupe constitué par SEQ ID NO: 4, 6, 12 et 14 ; (b) un polypeptide ayant au moins 90% d'identité de séquence avec (a), et le polypeptide a un W ou un Y à la position correspondant à la position 28 de la séquence présentée dans SEQ ID NO: 2 et un T à la position correspondant à la position 44 de la séquence présentée dans SEQ ID NO: 2, et (c) un polypeptide ayant au moins 95% d'identité de séquence avec (a), et le polypeptide a un W ou un Y à la position correspondant à la position 28 de la séquence présentée dans SEQ ID NO: 2 et un T à la position correspondant à la position 44 de la séquence présentée dans SEQ ID NO: 2, le procédé comprenant :
la culture d'une lignée de cellules ovariennes de hamster chinois CS9 conçue pour exprimer la protéine en culture cellulaire ;
la récolte de la protéine depuis la culture ; et
la purification de la protéine.
